# EUROPEAN PATENT APPLICATION

(11) **EP 3 061 754 A1**
(43) Date of publication of application: **31.08.2016**
(21) Application number: 14856224.2
(22) Date of filing: 22.10.2014
(51) Int. Cl.: C07D 239/28, A61K 31/426, A61K 31/4965, A61K 31/497, A61K 31/505, A61K 31/506, A61K 31/5377, A61K 31/5386, A61K 31/55, A61P 25/18, A61P 25/28, A61P 43/00, C07D 239/36, C07D 239/42, C07D 239/47, C07D 239/48, C07D 239/54, C07D 239/56, C07D 241/24, C07D 241/26, C07D 277/20

(54) **HETEROCYCLIC COMPOUND**

(30) Priority: 23.10.2013 JP 2013220194
(71) Applicant: Takeda Pharmaceutical Company Limited, Osaka-shi, Osaka 541-0045 (JP)
(72) Inventor: SETO, Masaki, Fujisawa-shi Kanagawa 251-0012 (JP); BANNO, Yoshihiro, Fujisawa-shi Kanagawa 251-0012 (JP); IMAEDA, Toshihiro, New York, New York 10021 (US); KAJITA, Yuichi, Fujisawa-shi Kanagawa 251-0012 (JP); ASHIZAWA, Tomoko, Fujisawa-shi Kanagawa 251-0012 (JP); KAWASAKI, Masanori, Kanagawa 251-0012 (JP); NAKAMURA, Shinji, Fujisawa-shi Kanagawa 251-0012 (JP); MIKAMI, Satoshi, Fujisawa-shi Kanagawa 251-0012 (JP); NOMURA, Izumi, Fujisawa-shi Kanagawa 251-0012 (JP); TANIGUCHI, Takahiko, Fujisawa-shi Kanagawa 251-0012 (JP); MARUI, Shogo, Fujisawa-shi Kanagawa 251-0012 (JP)
(74) Representative: Huenges, Martin
(86) International application number: PCT/JP2014/078151
(87) International publication number: WO 2015/060368

(57) **Abstract**

The problem of the present invention is to provide a compound having a PDE2A inhibitory action, and useful as a prophylactic or therapeutic drug for schizophrenia, Alzheimer's disease and the like. The present invention relates to a compound represented by the formula (I): 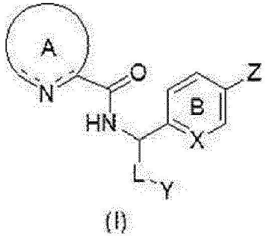 wherein each symbol is as described in the DESCRIPTION, or a salt thereof.

## Description

### Technical Field

The present invention relates to a nitrogen-containing heterocyclic compound having a PDE2A selective inhibitory action, and useful as a prophylactic or therapeutic drug for schizophrenia, Alzheimer's disease and the like.

### (Background of the Invention)

Cyclic nucleotide phosphodiesterases (PDEs) are enzymes that regulate the cellular levels of the second messengers, cyclic adenosine monophosphate (cAMP) and cyclic guanosine monophosphate (cGMP), by controlling their rates of degradation. PDEs are a superfamily of enzymes encoded by 21 genes and subdivided into 11 distinct families according to structural and functional properties. The PDE enzymes selectively catalyze the hydrolysis of the 3'-ester bond of cAMP and/or cGMP, forming the inactive 5'-monophosphate. On the basis of substrate specificity, the PDE families can be further classified into three groups: i) the cAMP-PDEs (PDE4, PDE7 and PDE8), ii) the cGMP-PDEs (PDE5, PDE6 and PDE9), and iii) the dual-substrate PDEs (PDE1, PDE2, PDE3, PDE10 and PDE11).

cAMP and cGMP are involved in the regulation of virtually every physiological process such as pro-inflammatory mediator production and action, ion channel function, muscle relaxation, learning and memory formation, differentiation, apoptosis, lipogenesis, glycogenolysis and gluconeogenesis. Especially, in neurons, these second messengers have an important role in the regulation of synaptic transmission as well as in neuronal differentiation and survival (Non-Patent Document 1). Regulation of these processes by cAMP and cGMP are accompanied by activation of protein kinase A (PKA) and protein kinase G (PKG), which in turn phosphorylate a variety of substrates including transcription factors, ion channels and receptors that regulate a variety of physiological processes. Intracellular cAMP and cGMP concentrations seem to be temporally, spatially, and functionally compartmentalized by regulation of adenylate cyclase and guanylate cyclase in response to extracellular signaling and their degradation by PDEs (Non-Patent Document 2). PDEs provide the only means of degrading the cyclic nucleotides cAMP and cGMP in cells, thus PDEs play an essential role in cyclic nucleotide signaling. Thereby, PDEs may be promising targets for various therapeutic drugs.

Phosphodiesterase 2A (PDE2A) is a dual substrate enzyme that hydrolyzes both cAMP and cGMP. It is organized into four domains, N-terminus, GAF-A, GAF-B, and catalytic domains, and functions as a homodimer. PDE2A catalytic activity is allosterically stimulated by cGMP binding. GAF-B domain binds with a high affinity and a high selectivity to cGMP. A conformational change is caused by the cGMP binding in the PDE2A homodimer which causes an increase in the catalytic activity of the enzyme to several-folds or more (Non-Patent Document 3-6). In contrast, there are as yet no known in vivo examples that cAMP stimulates PDE2A catalytic activity. Furthermore, binding affinity of cAMP to the GAF-B is 30-100-fold lower than cGMP (Non-Patent Document 6 and 7). PDE2A activity may become functionally significant under conditions in which cellular cGMP concentrations are elevated, which shows a physiological role for GAF domain-regulation of the enzyme.

PDE2A is expressed in a wide variety of tissues and highly in the brain. The protein was originally purified from heart, liver, adrenal gland, platelets, endothelial cells, and macrophages (Non-Patent Document 8-13). In the brain, the PDE2A mRNA levels are the highest in the caudate lobe, nucleus accumbens, cortex (frontal, parietal and temporal) and the hippocampus, and are at least 10-fold lower expression in other brain regions (Non-Patent Document 14). This suggests that PDE2A may control intraneuronal cAMP and cGMP levels in areas that are important for learning and memory formation.

Inhibition of PDE2A results in increased cAMP and cGMP levels that could improve cognitive function. In both cortical neurons and hippocampal slices, a PDE2A inhibitor potently increased cGMP concentrations in the presence of guanylate cyclase activators and also increased cAMP concentrations in the presence of forskolin (Non-Patent Document 15). The PDE2A inhibitor was also found to potently increase the induction of long-term potentiation (LTP) in hippocampal slices in response to a weak tetanizing stimulus. This effect on LTP in the slices suggests that PDE2A inhibition has positive effects on learning and memory in vivo (Non-Patent Document 15). In fact, the same PDE2A inhibitor increased retention on both novel object recognition and social recognition tests in rats, and improved object memory and object recognition in 3-, 12-, and 24-month old rats. It also attenuated the extradimensional (ED) shift deficit on extradimensional-intradimensional (ED/ID) cognitive test in subchronic PCP-treated rats (Non-Patent Document 15-17). These results suggest that PDE2A inhibition could facilitate learning and memory processes through cAMP and cGMP-regulated signaling cascades.

Increased cGMP levels by PDE2A inhibition could also influence anxiety and stress-related events. PDE2A inhibitors decreased oxidative stress and induced the expression of NADPH oxidase subunits in oxidative stress inducer-treated mice. It improved anxiety-related behavior in elevated plus maze, open-field, and hole-board tests through the NADPH oxidase pathway (Non-Patent Document 18). In addition, PDE2A inhibitors also produced anxiolytic effects on behavior in non-stressed mice in the elevated plus-maze and hole-board tests (Non-Patent Document 19). PDE2A may be a novel pharmacological target for treatment of not only cognitive deficit, but also anxiety in neuropsychiatric and neurodegenerative disorders.

These unique distribution and functions in the brain indicate that PDE2A represents an important novel target for the treatment of neuropsychiatric and neurodegenerative disorders, in particular schizophrenia and Alzheimer's disease.

As heterocyclic compounds, the following compounds are known. Patent document 1 describes that a compound represented by the formula: 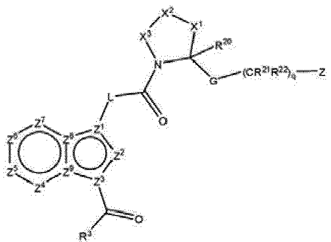 wherein each symbol is as defined in patent document 1, is a Factor D inhibitor, and is useful for the treatment of age-related muscular weakness, diabetic retinopathy, neurological disease, Parkinson's disease, obesity and the like.

Non-patent document 20 describes a compound represented by the following formula:

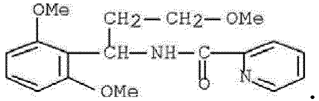

Patent document 2 describes that a compound represented by the following formula: 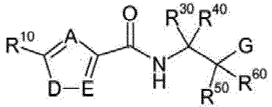 wherein each symbol is as defined in patent document 2, is a cathepsin A inhibitor, and is useful for the treatment of cardiac-related diseases (myocardial infarction, arteriosclerosis), chronic bronchitis and the like.

Patent document 3 describes that a compound represented by the following formula (I): 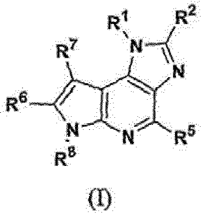 wherein each symbol is as defined in patent document 3, is a NF-κB inhibitor, and is useful for the treatment of inflammatory diseases, autoimmune diseases and the like.

Patent document 4 describes that a compound represented by the following formula: 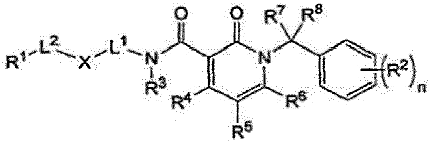 wherein each symbol is as defined in patent document 4, is a PDK1 inhibitor, and is useful for the treatment of cancer and the like.

Patent document 5 describes that a compound represented by the following formula: 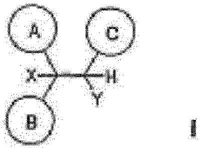 wherein each symbol is as defined in patent document 5, is a potassium channel antagonist, and is useful for the treatment of arrhythmia, supraventricular tachycardia, thromboembolic event and the like.

Patent document 6 describes that a compound represented by the following formula I: 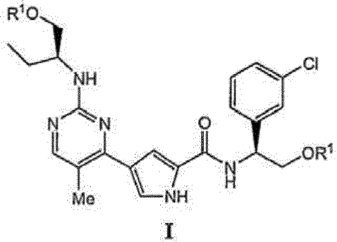 wherein each symbol is as defined in patent document 6, is a ERK protein kinase inhibitor, and is useful for the treatment of cancer, diabetes, cardiovascular disease, Alzheimer's disease, autoimmune disease and the like.

Patent document 7 describes that a compound represented by the following formula I: 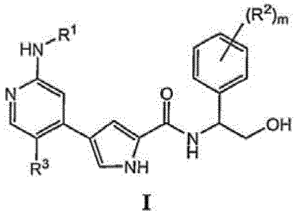 wherein each symbol is as defined in patent document 7, and a compound represented by the following formula II: 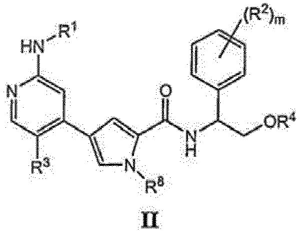 wherein each symbol is as defined in patent document 7, are ERK protein kinase inhibitors, and are useful as chemotherapeutic drugs, anti-proliferative disorder drugs, antiinflammatory drugs, immunosuppressive drugs, and therapeutic drugs for neurological disease.

Patent document 8 describes that a compound represented by the following formula I: 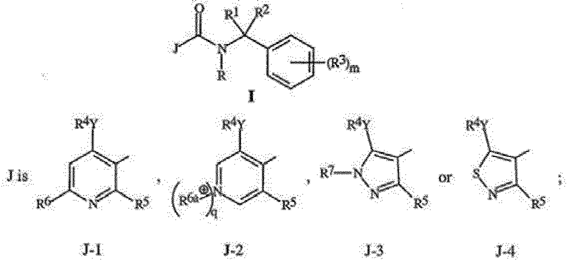 wherein each symbol is as defined in patent document 8, is useful as a herbicide.

Non-patent document 21 describes that a compound represented by the following formula: 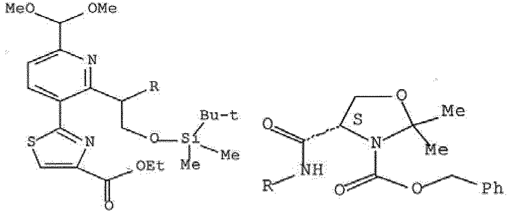 is useful as an antibiotic agent.

Patent document 9 describes that a compound represented by the following formula II: 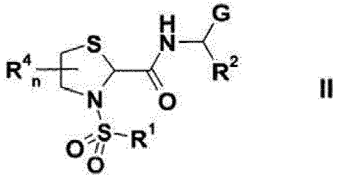 wherein each symbol is as defined in patent document 9, is a prostaglandin receptor inhibitor, and is useful for the treatment or prophylaxis of dysmenorrhea, premature labor pain and the like.

Patent document 10 describes that a compound represented by the following formula (I): 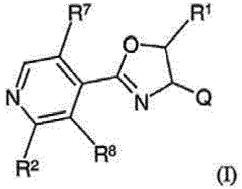 wherein each symbol is as defined in patent document 10, is useful for insect proofing and the like.

Patent document 11 describes that a compound represented by the formula (1):

**Ar¹** (**Alk^{a}** )**ᵣL¹Ar²CH**(**R¹**)**C**(**R^{a}**)(**R^{a}**')**R** **(1)**

wherein each symbol is as defined in patent document 11, is an α4 integrin inhibitor, and is useful for the prophylaxis of immunoinflammation and inflammatory diseases.

Also, a compound represented by the following formula:

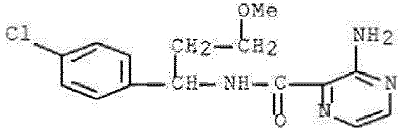

(CAS Registry Number: 1436363-71-1),
a compound represented by the following formula:

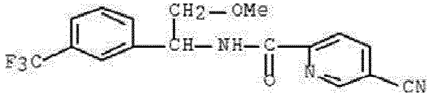

(CAS Registry Number: 1436087-15-8),
a compound represented by the following formula:

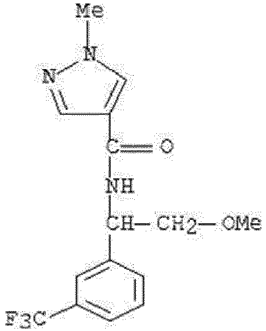

(CAS Registry Number: 1385385-84-1),
a compound represented by the following formula:

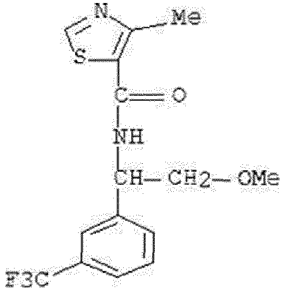

(CAS Registry Number: 1385296-42-3),
a compound represented by the following formula:

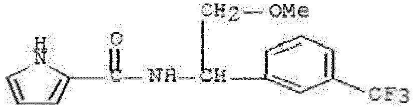

(CAS Registry Number: 1376017-63-8),
a compound represented by the following formula:

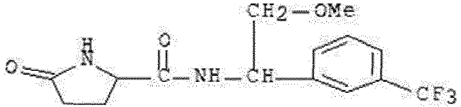

(CAS Registry Number: 1375841-11-4),
a compound represented by the following formula:

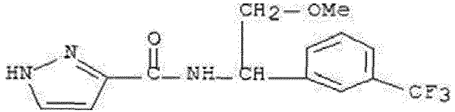

(CAS Registry Number: 1355852-02-6),
a compound represented by the following formula:

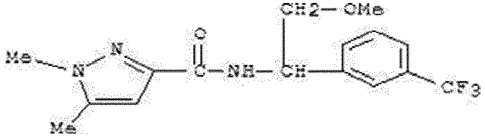

(CAS Registry Number: 1334014-40-2),
a compound represented by the following formula:

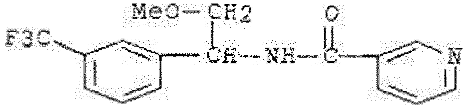

(CAS Registry Number: 1333974-47-2),
a compound represented by the following formula:

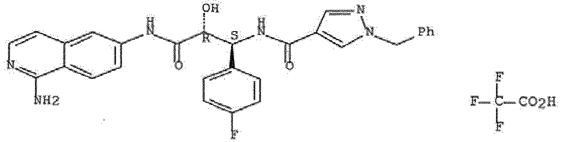

(CAS Registry Number: 1279038-43-5),
a compound represented by the following formula:

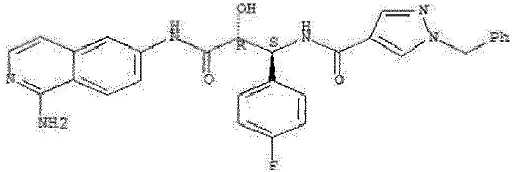

(CAS Registry Number: 1279038-42-4),
a compound represented by the following formula:

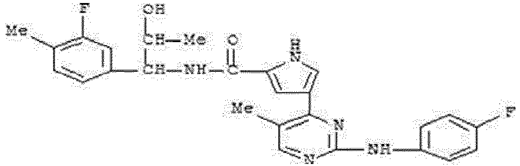

(CAS Registry Number: 1027515-44-1),
a compound represented by the following formula:

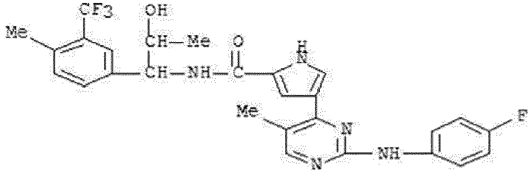

(CAS Registry Number: 1027368-00-8),
a compound represented by the following formula:

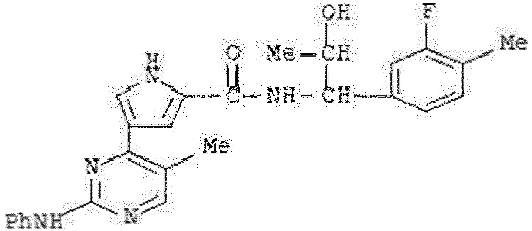

(CAS Registry Number: 1027221-79-9),
a compound represented by the following formula:

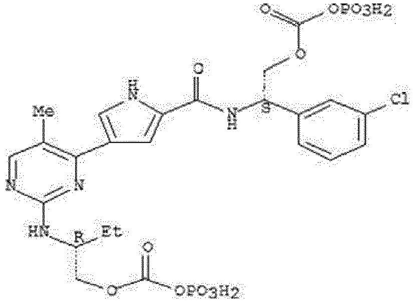

(CAS Registry Number: 1026985-09-0), and
a compound represented by the following formula:

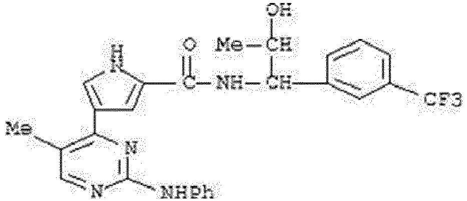

(CAS Registry Number: 1026285-23-3) are known.

### [Document List]

### [Patent Documents]

patent document 1: WO 2012/093101
patent document 2: WO 2011/092187
patent document 3: WO 2006/122137
patent document 4: WO 2008/005457
patent document 5: WO 2006/015159
patent document 6: WO 2005/113546
patent document 7: WO 2005/113541
patent document 8: WO 2005/070889
patent document 9: WO 03/082278
patent document 10: WO 01/98296
patent document 11: WO 00/32575

### [non-patent documents]

non-patent document 1: Nat. Rev. Drug Discov. 2006, vol. 5: 660-670
non-patent document 2: Circ. Res. 2007, vol. 100: 950-966
non-patent document 3: J. Biol. Chem. 1971, vol. 246: 3841-3846
non-patent document 4: J. Biol. Chem. 1973, vol. 248: 1334-1340
non-patent document 5: PNAS 2005, vol. 99: 13260-13265
non-patent document 6: British J. Pharmacol. 2010, vol. 161: 1645-1660
non-patent document 7: J. Biol. Chem. 2004, vol. 279: 37928-37938
non-patent document 8: J. Biol. Chem. 1982, vol. 257: 1973-1979
non-patent document 9: J. Biol. Chem. 1983, vol. 258: 12526-12533
non-patent document 10: Phosphodiesterase Inhibitors, Academic Press: 21-40
non-patent document 11: Rev. Physiol. Biochem. Pharmacol. 1999, vol. 135: 67-104
non-patent document 12: Cell Signal 2004, vol. 16: 365-374
non-patent document 13: J. Histochem. Cytochem. 2009, vol. 57: 933-949
non-patent document 14: Neuropharmacology 2010, vol. 59: 367-374
non-patent document 15: Neuropharmacology 2004, vol. 47: 1081-1092
non-patent document 16: Mol. Neurobiol. 2010, vol. 41: 129-137
non-patent document 17: Neuropharmacology 2012, vol. 62: 1182-1190
non-patent document 18: J. Pharmacol. Exp. Ther. 2008, vol. 326: 369-379
non-patent document 19: J. Pharmacol. Exp. Ther. 2009, vol. 331: 690-699
non-patent document 20: J. Am. Chem. Soc. 2012, vol. 134: 7313-7316
non-patent document 21: Heterocycles 2003, vol. 61: 45-50

### SUMMARY OF THE INVENTION

### Problems to be Solved by the Invention

The present invention aims to provide a compound having a PDE2A selective inhibitory action, and useful as a prophylactic or therapeutic drug for schizophrenia, Alzheimer's disease and the like.

### Means of Solving the Problems

The present inventors have conducted intensive studies and found that a compound represented by the formula (I) to be described later unexpectedly has a superior PDE2A selective inhibitory action, and therefore, is useful as a prophylactic or therapeutic drug for schizophrenia, Alzheimer's disease and the like, and completed the present invention based on these findings.

Accordingly, the present invention is as follows.
[1] A compound represented by the formula (I): 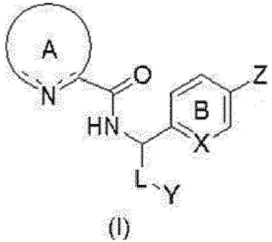 wherein
   ring A is an optionally further substituted 5- or 6-membered nitrogen-containing heterocycle, 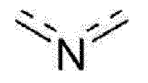 as a ring A-constituting atom is =N- or -N=,
   ring B is a benzene ring or a pyridine ring, each of which is optionally further substituted,
   X is a carbon atom or a nitrogen atom,
   L is a bond or an optionally substituted C₁₋₂ alkylene group,
   Y is
      (1) the formula -CH₂-O-R¹ wherein R¹ is a substituent, or
      (2) the formula: 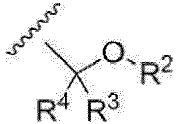 wherein R² and R³ are each independently a hydrogen atom or a substituent, R⁴ is a substituent, or R³ and R⁴ are joined to optionally form, together with the adjacent carbon atom, an optionally further substituted ring, provided when L is a bond, then R³ and R⁴ are not 3-pyridyl groups at the same time, and Z is a substituent, (provided that 3-amino-N-[1-(4-chlorophenyl)-3-methoxypropyl]pyrazine-2-carboxamide is excluded), or a salt thereof (hereinafter to be also referred to as compound (I) or compound (1)).
[2] The compound of the above-mentioned [1], wherein Y is
   (1) the formula -CH₂-O-R¹ wherein R¹ is an optionally substituted C₁₋₆ alkyl group; or
   (2) the formula: 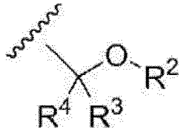 wherein R² and R³ are each independently a hydrogen atom or an optionally substituted C₁₋₆ alkyl group, and R⁴ is an optionally substituted C₁₋₆ alkyl group,
      or a salt thereof.
[3] The compound of the above-mentioned [1], wherein ring A is an optionally further substituted 5- or 6-membered nitrogen-containing heterocycle; 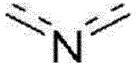 as a ring A-constituting atom is =N- or -N=;
   ring B is a benzene ring or a pyridine ring, each of which is optionally further substituted;
   X is a carbon atom or a nitrogen atom;
   L is a bond or an optionally substituted C₁₋₂ alkylene group;
   Y is
   (1) the formula -CH₂-O-R¹ wherein R¹ is an optionally substituted C₁₋₆ alkyl group; or
   (2) the formula: 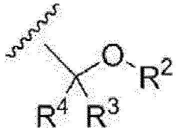 wherein R² and R³ are each independently a hydrogen atom or an optionally substituted C₁₋₆ alkyl group, and R⁴ is an optionally substituted C₁₋₆ alkyl group; and
      Z is
      (1) an optionally substituted C₁₋₆ alkoxy group,
      (2) an optionally substituted C₁₋₆ alkyl group,
      (3) an optionally substituted C₃₋₁₀ cycloalkyl group, or
      (4) an optionally substituted heterocyclic group,
         the above-mentioned [1] compound described in or a salt thereof.
[4] The compound of the above-mentioned [1] to [3], wherein ring A is a 5- or 6-membered nitrogen-containing heterocycle optionally substituted by 1 to 3 substituents selected from
   (1) a C₁₋₆ alkyl group optionally substituted by 1 to 3 substituents selected from
      (i) a C₆₋₁₄ aryl group,
      (ii) a C₁₋₆ alkoxy group, and
      (iii) a heterocyclic group,
   (2) a halogen atom,
   (3) a cyano group,
   (4) a C₂₋₆ alkenyl group,
   (5) a C₂₋₆ alkynyl group optionally substituted by 1 to 3 C₃₋₁₀ cycloalkyl groups,
   (6) a C₃₋₁₀ cycloalkyl group optionally substituted by 1 to 3 substituents selected from
      (i) an optionally halogenated C₁₋₆ alkyl group,
      (ii) a C₆₋₁₄ aryl group, and
      (iii) a C₁₋₆ alkoxy group,
   (7) a C₃₋₁₀ cycloalkenyl group optionally substituted by 1 to 3 C₁₋₆ alkoxy groups,
   (8) a C₆₋₁₄ aryl group optionally substituted by 1 to 3 substituents selected from
      (i) a C₁₋₆ alkyl group,
      (ii) a halogen atom, and
      (iii) a C₁₋₆ alkoxy group,
   (9) a C₁₋₆ alkoxy group,
   (10) a C₁₋₆ alkylthio group,
   (11) an amino group optionally mono- or di-substituted by a substituent selected from
      (i) a C₁₋₆ alkyl group optionally substituted by 1 to 3 substituents selected from a C₁₋₆ alkoxy group, a C₃₋₁₀ cycloalkyl group and a C₆₋₁₄ aryl group,
      (ii) a C₃₋₁₀ cycloalkyl group,
      (iii) a C₆₋₁₄ aryl group optionally substituted by 1 to 3 C₁₋₆ alkoxy groups,
      (iv) a heterocyclic group optionally substituted by 1 to 3 C₁₋₆ alkyl groups, and
      (v) a C₃₋₁₀ cycloalkyl-carbonyl group,
   (12) a non-aromatic heterocyclic group optionally substituted by 1 to 3 substituents selected from
      (i) a C₁₋₆ alkoxy group,
      (ii) a halogen atom,
      (iii) a C₁₋₆ alkyl group optionally substituted by 1 to 3 substituents selected from a halogen atom and a C₁₋₆ alkoxy group, and
      (iv) an oxo group,
   (13) an aromatic heterocyclic group optionally substituted by 1 to 3 substituents selected from
      (i) an optionally halogenated C₁₋₆ alkoxy group,
      (ii) a halogen atom,
      (iii) an optionally halogenated C₁₋₆ alkyl group, and
      (iv) a C₃₋₁₀ cycloalkyl group,
   (14) a heterocyclyloxy group, and
   (15) an oxo group; 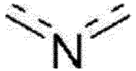 as a ring A-constituting atom is =N- or -N=;
      ring B is a benzene ring optionally substituted by a halogen atom;
      X is a carbon atom;
      L is a bond or methylene;
      Y is
      (1) the formula -CH₂-O-R¹ wherein R¹ is a C₁₋₆ alkyl group; or
      (2) the formula: 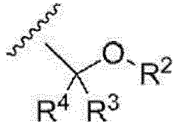
         wherein R² is a hydrogen atom;
         R³ is a C₁₋₆ alkyl group; and
         R⁴ is a C₁₋₆ alkyl group; and
         Z is
            (1) an optionally halogenated C₁₋₆ alkoxy group,
            (2) an optionally halogenated C₁₋₆ alkyl group,
            (3) a C₃₋₁₀ cycloalkyl group, or
            (4) a heterocyclic group,
               or a salt thereof.
[5] The compound of the above-mentioned [1] to [4], wherein ring A is a 5- or 6-membered nitrogen-containing heterocycle optionally substituted by 1 to 3 substituents selected from
   (1) a C₁₋₆ alkyl group optionally substituted by 1 to 3 substituents selected from
      (i) a C₆₋₁₄ aryl group,
      (ii) a C₁₋₆ alkoxy group, and
      (iii) a heterocyclic group,
   (2) a cyano group,
   (3) a C₃₋₁₀ cycloalkyl group optionally substituted by 1 to 3 substituents selected from
      (i) an optionally halogenated C₁₋₆ alkyl group,
      (ii) a C₆₋₁₄ aryl group, and
      (iii) a C₁₋₆ alkoxy group,
   (4) a C₃₋₁₀ cycloalkenyl group optionally substituted by 1 to 3 C₁₋₆ alkoxy groups,
   (5) a C₆₋₁₄ aryl group optionally substituted by 1 to 3 substituents selected from
      (i) a C₁₋₆ alkyl group,
      (ii) a halogen atom, and
      (iii) a C₁₋₆ alkoxy group,
   (6) an amino group optionally mono- or di-substituted by a substituent selected from
      (i) a C₁₋₆ alkyl group optionally substituted by 1 to 3 substituents selected from a C₁₋₆ alkoxy group, a C₃₋₁₀ cycloalkyl group and a C₆₋₁₄ aryl group,
      (ii) a C₃₋₁₀ cycloalkyl group,
      (iii) a C₆₋₁₄ aryl group optionally substituted by 1 to 3 C₁₋₆ alkoxy groups,
      (iv) a heterocyclic group optionally substituted by 1 to 3 C₁₋₆ alkyl groups, and
      (v) a C₃₋₁₀ cycloalkyl-carbonyl group,
   (7) a non-aromatic heterocyclic group optionally substituted by 1 to 3 substituents selected from
      (i) a C₁₋₆ alkoxy group,
      (ii) a halogen atom,
      (iii) a C₁₋₆ alkyl group optionally substituted by 1 to 3 substituents selected from a halogen atom and a C₁₋₆ alkoxy group, and
      (iv) an oxo group,
   (8) an aromatic heterocyclic group optionally substituted by 1 to 3 substituents selected from
      (i) an optionally halogenated C₁₋₆ alkoxy group,
      (ii) a halogen atom,
      (iii) an optionally halogenated C₁₋₆ alkyl group, and
      (iv) a C₃₋₁₀ cycloalkyl group, and
   (9) an oxo group; 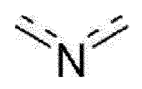 as a ring A-constituting atom is =N- or -N=;
      ring B is a benzene ring optionally substituted by a halogen atom;
      X is a carbon atom;
      L is a bond;
      Y is
      (1) the formula -CH₂-O-R¹ wherein R¹ is a C₁₋₆ alkyl group; or
      (2) the formula: 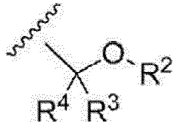
         wherein R² is a hydrogen atom;
         R³ is a C₁₋₆ alkyl group; and
         R⁴ is a C₁₋₆ alkyl group; and
         Z is
            (1) an optionally halogenated C₁₋₆ alkoxy group, or
            (2) a heterocyclic group,
               or a salt thereof.
[6] The compound of the above-mentioned [1] to [5], wherein ring A is a 5- or 6-membered nitrogen-containing heterocycle optionally substituted by 1 to 3 substituents selected from
   (1) a C₁₋₆ alkyl group,
   (2) a cyano group,
   (3) a C₃₋₁₀ cycloalkyl group optionally substituted by 1 to 3, optionally halogenated C₁₋₆ alkyl group,
   (4) a C₃₋₁₀ cycloalkenyl group optionally substituted by 1 to 3 C₁₋₆ alkoxy groups,
   (5) a C₆₋₁₄ aryl group,
   (6) an amino group optionally mono- or di-substituted by a C₁₋₆ alkyl group,
   (7) a non-aromatic heterocyclic group optionally substituted by 1 to 3 C₁₋₆ alkoxy groups,
   (8) an aromatic heterocyclic group optionally substituted by 1 to 3 substituents selected from
      (i) an optionally halogenated C₁₋₆ alkoxy group,
      (ii) a halogen atom, and
      (iii) an optionally halogenated C₁₋₆ alkyl group, and
   (9) an oxo group; 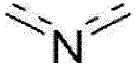 as a ring A-constituting atom is =N- or -N=;
      ring B is a benzene ring optionally substituted by a halogen atom;
      X is a carbon atom;
      L is a bond;
      Y is
      (1) the formula -CH₂-O-R¹ wherein R¹ is a C₁₋₆ alkyl group; or
      (2) the formula: 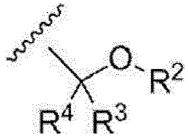
         wherein R² is a hydrogen atom;
         R³ is a C₁₋₆ alkyl group; and
         R⁴ is a C₁₋₆ alkyl group; and
         Z is
            (1) an optionally halogenated C₁₋₆ alkoxy group, or
            (2) a heterocyclic group,
               or a salt thereof.
[7] The compound of the above-mentioned [1] to [6], wherein ring A is a 5- or 6-membered nitrogen-containing heterocycle optionally substituted by 1 to 3 substituents selected from
   (1) a C₃₋₁₀ cycloalkyl group optionally substituted by 1 to 3 C₁₋₆ alkyl groups,
   (2) a non-aromatic heterocyclic group,
   (3) an aromatic heterocyclic group optionally substituted by 1 to 3 substituents selected from
      (i) a C₁₋₆ alkoxy group, and
      (ii) a halogen atom, and
   (4) an oxo group; 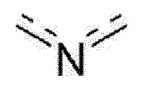 as a ring A-constituting atom is =N- or -N=;
      ring B is a benzene ring optionally substituted by a halogen atom;
      X is a carbon atom;
      L is a bond;
      Y is a group represented by the formula -CH₂-O-R¹ wherein R¹ is a C₁₋₆ alkyl group; and
      Z is a halogenated C₁₋₆ alkoxy group,
      or a salt thereof.
[8] The compound of the above-mentioned [1] to [7], wherein ring A is a dihydropyrimidine ring optionally substituted by 1 to 3 substituents selected from
   (1) a pyrrolidinyl group,
   (2) a pyridyl group optionally substituted by 1 to 3 substituents selected from
      (i) a C₁₋₆ alkoxy group, and
      (ii) a halogen atom, and
   (3) an oxo group; 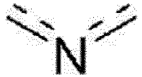 as a ring A-constituting atom is =N- or -N=;
      ring B is a benzene ring optionally substituted by a halogen atom;
      X is a carbon atom;
      L is a bond;
      Y is the formula -CH₂-O-R¹ wherein R¹ is a C₁₋₆ alkyl group; and Z is a halogenated C₁₋₆ alkoxy group,
      or a salt thereof.
[9] N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide, or a salt thereof.
[10] 2-(3,5-Dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide, or a salt thereof.
[11] 2-(3-Fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide, or a salt thereof.
[12] A medicament comprising the compound of any of the above-mentioned [1] to [11], or a salt thereof.
[13] The medicament of the above-mentioned [12], which is a phosphodiesterase 2A inhibitor.
[14] The medicament of the above-mentioned [12], which is a prophylactic or therapeutic drug for schizophrenia.
[15] The compound of any of the above-mentioned [1] to [11] for use in the prophylaxis or treatment of schizophrenia, or a salt thereof.
[16] A method of inhibiting phosphodiesterase 2A in a mammal, comprising administering an effective amount of the compound of any of the above-mentioned [1] to [11] or a salt thereof to the mammal.
[17] A method for the prophylaxis or treatment of schizophrenia in a mammal, comprising administering an effective amount of the compound of any of the above-mentioned [1] to [11] or a salt thereof to the mammal.
[18] Use of the compound of any of the above-mentioned [1] to [11] or a salt thereof in the production of a prophylactic or therapeutic drug for schizophrenia.

### Effect of the Invention

According to the present invention, a compound having a PDE2A selective inhibitory action, and useful as a prophylactic or therapeutic drug for schizophrenia, Alzheimer's disease and the like can be provided.

### (Detailed Description of the Invention)

The present invention is explained in detail in the following.

The definition of each substituent used in the present specification is described in detail in the following. Unless otherwise specified, each substituent has the following definition.

In the present specification, examples of the "halogen atom" include fluorine, chlorine, bromine and iodine.

In the present specification, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

In the present specification, examples of the "optionally halogenated C₁₋₆ alkyl group" include a C₁₋₆ alkyl group optionally having 1 to 7, preferably 1 to 5, halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.

In the present specification, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl and 5-hexenyl.

In the present specification, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and 4-methyl-2-pentynyl.

In the present specification, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.

In the present specification, examples of the "optionally halogenated C₃₋₁₀ cycloalkyl group" include a C₃₋₁₀ cycloalkyl group optionally having 1 to 7, preferably 1 to 5, halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl and cyclooctyl.

In the present specification, examples of the "C₃₋₁₀ cycloalkenyl group" include cyclopropenyl, cyclobutenyl, cyclopentenyl, cyclohexenyl, cycloheptenyl and cyclooctenyl.

In the present specification, examples of the "C₆₋₁₄ aryl group" include phenyl, 1-naphthyl, 2-naphthyl, 1-anthryl, 2-anthryl and 9-anthryl.

In the present specification, examples of the "C₇₋₁₆ aralkyl group" include benzyl, phenethyl, naphthylmethyl and phenylpropyl.

In the present specification, examples of the "C₁₋₆ alkoxy group" include methoxy, ethoxy, propoxy, isopropoxy, butoxy, isobutoxy, sec-butoxy, tert-butoxy, pentyloxy and hexyloxy.

In the present specification, examples of the "optionally halogenated C₁₋₆ alkoxy group" include a C₁₋₆ alkoxy group optionally having 1 to 7, preferably 1 to 5, halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy and hexyloxy.

In the present specification, examples of the "C₃₋₁₀ cycloalkyloxy group" include cyclopropyloxy, cyclobutyloxy, cyclopentyloxy, cyclohexyloxy, cycloheptyloxy and cyclooctyloxy.

In the present specification, examples of the "C₁₋₆ alkylthio group" include methylthio, ethylthio, propylthio, isopropylthio, butylthio, sec-butylthio, tert-butylthio, pentylthio and hexylthio.

In the present specification, examples of the "optionally halogenated C₁₋₆ alkylthio group" include a C₁₋₆ alkylthio group optionally having 1 to 7, preferably 1 to 5, halogen atoms. Specific examples thereof include methylthio, difluoromethylthio, trifluoromethylthio, ethylthio, propylthio, isopropylthio, butylthio, 4,4,4-trifluorobutylthio, pentylthio and hexylthio.

In the present specification, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.

In the present specification, examples of the "optionally halogenated C₁₋₆ alkyl-carbonyl group" include a C₁₋₆ alkyl-carbonyl group optionally having 1 to 7, preferably 1 to 5, halogen atoms. Specific examples thereof include acetyl, chloroacetyl, trifluoroacetyl, trichloroacetyl, propanoyl, butanoyl, pentanoyl and hexanoyl.

In the present specification, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexyloxycarbonyl.

In the present specification, examples of the "C₆₋₁₄ aryl-carbonyl group" include benzoyl, 1-naphthoyl and 2-naphthoyl.

In the present specification, examples of the "C₇₋₁₆ aralkyl-carbonyl group" include phenylacetyl and phenylpropionyl.

In the present specification, examples of the "5- to 14-membered aromatic heterocyclylcarbonyl group" include nicotinoyl, isonicotinoyl, thenoyl and furoyl.

In the present specification, examples of the "3- to 14-membered non-aromatic heterocyclylcarbonyl group" include morpholinylcarbonyl, piperidinylcarbonyl and pyrrolidinylcarbonyl.

In the present specification, examples of the "mono- or di-C₁₋₆ alkyl-carbamoyl group" include methylcarbamoyl, ethylcarbamoyl, dimethylcarbamoyl, diethylcarbamoyl and N-ethyl-N-methylcarbamoyl.

In the present specification, examples of the "mono- or di-C₇₋₁₆ aralkyl-carbamoyl group" include benzylcarbamoyl and phenethylcarbamoyl.

In the present specification, examples of the "C₁₋₆ alkylsulfonyl group" include methylsulfonyl, ethylsulfonyl, propylsulfonyl, isopropylsulfonyl, butylsulfonyl, sec-butylsulfonyl and tert-butylsulfonyl.

In the present specification, examples of the "optionally halogenated C₁₋₆ alkylsulfonyl group" include a C₁₋₆ alkylsulfonyl group optionally having 1 to 7, preferably 1 to 5, halogen atoms. Specific examples thereof include methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl and hexylsulfonyl.

In the present specification, examples of the "C₆₋₁₄ arylsulfonyl group" include phenylsulfonyl, 1-naphthylsulfonyl and 2-naphthylsulfonyl.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following substituent group A.

### [substituent group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g., methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g., benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino, toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group and
(62) a C₆₋₁₄ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5-to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and 8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3- to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and 9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include the "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

Examples of the "acyl group" also include a hydrocarbon-sulfonyl group, a heterocyclyl-sulfonyl group, a hydrocarbon-sulfinyl group and a heterocyclyl-sulfinyl group.

Here, the hydrocarbon-sulfonyl group means a hydrocarbon group-bonded sulfonyl group, the heterocyclyl-sulfonyl group means a heterocyclic group-bonded sulfonyl group, the hydrocarbon-sulfinyl group means a hydrocarbon group-bonded sulfinyl group and the heterocyclyl-sulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkyl-carbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxycarbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted amino group include an amino group, a mono- or di-(optionally halogenated C₁₋₆ alkyl) amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxy-carbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl)amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl)(C₁₋₆ alkyl-carbonyl)amino group (e.g., N-acetyl-N-methylamino) and a (C₁₋₆ alkyl) (C₆₋₁₄ aryl-carbonyl) amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g., benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxy group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from substituent group A".

Preferable examples of the optionally substituted silyl group include a tri-C₁₋₆ alkylsilyl group (e.g., trimethylsilyl, tert-butyl(dimethyl)silyl).

In the present specification, examples of the "C₁₋₆ alkylene group" include -CH₂-, -(CH₂)₂-, -(CH₂)₃-, -(CH₂)₄-,-(CH₂)₅-, -(CH₂)₆-, -CH(CH₃)-, -C(CH₃)₂-, -CH(C₂H₅)-, -CH(C₃H₇)-,-CH(CH(CH₃)₂)-, -(CH(CH₃))₂-, -CH₂-CH(CH₃)-, -CH(CH₃) -CH₂-, -CH₂-CH₂-C(CH₃)₂-, -C(CH₃)₂-CH₂-CH₂-, -CH₂-CH₂-CH₂-C (CH₃)₂- and-C(CH₃)₂-CH₂-CH₂-CH₂-.

In the present specification, examples of the "C₂₋₆ alkenylene group" include -CH=CH-, -CH₂-CH=CH-, -CH=CH-CH₂-,-C (CH₃) ₂-CH=CH-, -CH=CH-C (CH₃)₂-, -CH₂-CH=CH-CH₂-, -CH₂-CH₂-CH=CH-, -CH=CH-CH₂-CH₂-, -CH=CH-CH=CH-, -CH=CH-CH₂-CH₂-CH₂- and -CH₂-CH₂-CH₂-CH=CH-.

In the present specification, examples of the "C₂₋₆ alkynylene group" include -C≡C-, -CH₂-C≡C-, -C≡C-CH2-,-C(CH₃)₂-C≡C-, -C≡C-C(CH₃)₂-, -CH₂-C≡C-CH₂-, -CH₂-CH₂-C≡C-, -C≡ C-CH₂-CH₂-, -C≡C-C≡C-, -C≡C-CH₂-CH₂-CH₂- and -CH₂-CH₂-CH₂-C≡C-.

The definition of each symbol in the formula (I) is described in detail in the following.

Ring A is an optionally further substituted 5- or 6-membered nitrogen-containing heterocycle.

As the "5- or 6-membered nitrogen-containing heterocycle" of the "optionally further substituted 5- or 6-membered nitrogen-containing heterocycle" for ring A, pyrrole, pyrazole, triazole, oxazole, thiazole, oxadiazole, thiadiazole, pyridine, pyrazine, pyridazine, pyrimidine, triazine and dihydropyrimidine can be mentioned.

Preferred are thiazole, pyrazine, pyrimidine and dihydropyrimidine, and more preferred is dihydropyrimidine.

The "5- or 6-membered nitrogen-containing heterocycle" for ring A may be further substituted by, for example, the aforementioned "substituent", and the number of the substituents is, for example, 1 to 3. When the number of the substituents is two or more, each substituent may be the same or different.

Ring A is preferably a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl) optionally substituted by 1 to 3 substituents selected from
   (i) a C₆₋₁₄ aryl group (e.g., phenyl),
   (ii) a C₁₋₆ alkoxy group (e.g., methoxy), and
   (iii) a heterocyclic group (preferably, aromatic heterocyclic group, more preferably, 5- or 6-membered monocyclic aromatic heterocyclic group (e.g., pyridyl)),
(2) a halogen atom (e.g., fluorine atom, chlorine atom),
(3) a cyano group,
(4) a C₂₋₆ alkenyl group (e.g., 1-propenyl),
(5) a C₂₋₆ alkynyl group (e.g., ethynyl, 1-propynyl) optionally substituted by 1 to 3 C₃₋₁₀ cycloalkyl groups (e.g., cyclopropyl),
(6) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a C₆₋₁₄ aryl group (e.g., phenyl), and
   (iii) a C₁₋₆ alkoxy group (e.g., methoxy),
(7) a C₃₋₁₀ cycloalkenyl group (e.g., cyclopentenyl, cyclohexenyl) optionally substituted by 1 to 3 C₁₋₆ alkoxy groups (e.g., methoxy),
(8) a C₆₋₁₄ aryl group (e.g., phenyl) optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(9) a C₁₋₆ alkoxy group (e.g., methoxy),
(10) a C₁₋₆ alkylthio group (e.g., methylthio),
(11) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a C₆₋₁₄ aryl group (e.g., phenyl),
   (ii) a C₃₋₁₀ cycloalkyl group (e.g., cyclopropyl),
   (iii) a C₆₋₁₄ aryl group (e.g., phenyl) optionally substituted by 1 to 3 C₁₋₆ alkoxy groups (e.g., methoxy),
   (iv) a heterocyclic group (preferably, aromatic heterocyclic group, more preferably, 5- or 6-membered monocyclic aromatic heterocyclic group (e.g., pyrazolyl)) optionally substituted by 1 to 3 C₁₋₆ alkyl groups (e.g., methyl), and
   (v) a C₃₋₁₀ cycloalkyl-carbonyl group (e.g., cyclopropylcarbonyl),
(12) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy), and
   (iv) an oxo group,
(13) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(14) a heterocyclyloxy group (preferably, aromatic heterocyclyloxy group, more preferably, 5- or 6-membered monocyclic aromatic heterocyclyloxy group (e.g., pyridyloxy)), and
(15) an oxo group.

In another embodiment, ring A is preferably a thiazole ring, a pyrazine ring, a pyrimidine ring or a dihydropyrimidine ring, each optionally substituted by 1 to 3 substituents selected from
(1) a C₁₋₆ alkyl group (e.g., methyl, isopropyl, tert-butyl) optionally substituted by 1 to 3 substituents selected from
   (i) a phenyl group,
   (ii) a C₁₋₆ alkoxy group (e.g., methoxy), and
   (iii) a pyridyl group,
(2) a halogen atom (e.g., fluorine atom, chlorine atom),
(3) a cyano group,
(4) a C₂₋₆ alkenyl group (e.g., 1-propenyl),
(5) a C₂₋₆ alkynyl group (e.g., ethynyl, 1-propynyl) optionally substituted by 1 to 3 C₃₋₁₀ cycloalkyl groups (e.g., cyclopropyl),
(6) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a phenyl group, and
   (iii) a C₁₋₆ alkoxy group (e.g., methoxy),
(7) a C₃₋₁₀ cycloalkenyl group (e.g., cyclopentenyl, cyclohexenyl) optionally substituted by 1 to 3 C₁₋₆ alkoxy groups (e.g., methoxy),
(8) a phenyl group optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(9) a C₁₋₆ alkoxy group (e.g., methoxy),
(10) a C₁₋₆ alkylthio group (e.g., methylthio),
(11) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a phenyl group,
   (ii) a C₃₋₁₀ cycloalkyl group (e.g., cyclopropyl),
   (iii) a phenyl group optionally substituted by 1 to 3 C₁₋₆ alkoxy groups (e.g., methoxy),
   (iv) a pyrazolyl group optionally substituted by 1 to 3 C₁₋₆ alkyl groups (e.g., methyl), and
   (v) a C₃₋₁₀ cycloalkyl-carbonyl group (e.g., cyclopropylcarbonyl),
(12) an azetidinyl group, a pyrrolidinyl group, a tetrahydrofuryl group, an isoxazolidinyl group, a piperidyl group, a tetrahydropyranyl group, a morpholinyl group, an azepanyl group, a 3-oxa-8-azabicyclo[3.2.1]octyl group, a 3-azabicyclo[3.1.0]hexyl group or a 6-oxa-3-azabicyclo[3.1.1]heptyl group, each optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy), and
   (iv) an oxo group,
(13) a thienyl group, a furyl group, an imidazolyl group, a pyrazolyl group, a thiazolyl group, a pyridyl group, a pyrazinyl group, or a benzimidazolyl group, each optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(14) a pyridyloxy group, and
(15) an oxo group;

Ring A is more preferably a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl) optionally substituted by 1 to 3 substituents selected from
   (i) a C₆₋₁₄ aryl group (e.g., phenyl),
   (ii) a C₁₋₆ alkoxy group (e.g., methoxy), and
   (iii) a heterocyclic group (preferably, aromatic heterocyclic group, more preferably, 5- or 6-membered monocyclic aromatic heterocyclic group (e.g., pyridyl)),
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a C₆₋₁₄ aryl group (e.g., phenyl), and
   (iii) a C₁₋₆ alkoxy group (e.g., methoxy),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a C₆₋₁₄ aryl group (e.g., phenyl) optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(6) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a C₆₋₁₄ aryl group (e.g., phenyl),
   (ii) a C₃₋₁₀ cycloalkyl group (e.g., cyclopropyl),
   (iii) a C₆₋₁₄ aryl group (e.g., phenyl) optionally substituted by 1 to 3 C₁₋₆ alkoxy groups (e.g., methoxy),
   (iv) a heterocyclic group (preferably, aromatic heterocyclic group, more preferably, 5- or 6-membered monocyclic aromatic heterocyclic group (e.g., pyrazolyl)) optionally substituted by 1 to 3 C₁₋₆ alkyl groups (e.g., methyl), and
   (v) a C₃₋₁₀ cycloalkyl-carbonyl group (e.g., cyclopropylcarbonyl),
(7) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy), and
   (iv) an oxo group,
(8) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(9) an oxo group.

Ring A is further preferably a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl)
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 optionally halogenated C₁₋₆ alkyl groups (e.g., methyl, trifluoromethyl),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a C₆₋₁₄ aryl group (e.g., phenyl),
(6) an amino group optionally mono- or di-substituted by a C₁₋₆ alkyl group (e.g., methyl, ethyl, isopropyl, isobutyl),
(7) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 C₁₋₆ alkoxy groups (e.g., methoxy, isopropoxy),
(8) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
      and
   (iii) an optionally halogenated C₁₋₆ alkyl group (e.g., methyl, trifluoromethyl), and
(9) an oxo group.

In another embodiment, ring A is further preferably a dihydropyrimidine ring optionally substituted by 1 to 3 substituents selected from
(1) a C₁₋₆ alkyl group (e.g., methyl, isopropyl, tert-butyl)
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 C₁₋₆ alkyl groups (e.g., methyl),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a phenyl group,
(6) an amino group optionally di-substituted by a C₁₋₆ alkyl group (e.g., methyl, ethyl, isopropyl, isobutyl),
(7) an azetidinyl group, a pyrrolidinyl group or a 6-oxa-3-azabicyclo[3.1.1]heptyl group, each optionally substituted by 1 to 3 C₁₋₆ alkoxy groups (e.g., methoxy, isopropoxy),
(8) a thienyl group, a furyl group, a pyrazolyl group or a pyridyl group, each optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
      and
   (iii) an optionally halogenated C₁₋₆ alkyl group (e.g., methyl, trifluoromethyl), and
(9) an oxo group.

Ring A is further more preferably a 5- or 6-membered nitrogen-containing heterocycle (e.g., dihydropyrimidine) optionally substituted by 1 to 3 substituents selected from
(1) a C₃₋₁₀ cycloalkyl group (e.g., cyclopropyl) optionally substituted by 1 to 3 C₁₋₆ alkyl groups (e.g., methyl),
(2) a non-aromatic heterocyclic group (preferably, 4- to 10-membered non-aromatic heterocyclic group (e.g., pyrrolidinyl)),
(3) an aromatic heterocyclic group (preferably, 5- to 10-membered aromatic heterocyclic group (e.g., pyridyl)) optionally substituted by 1 to 3 substituents selected from
   (i) a C₁₋₆ alkoxy group (e.g., methoxy), and
   (ii) a halogen atom (e.g., fluorine atom), and
(4) an oxo group.

In another embodiment, ring A is further more preferably a dihydropyrimidine ring optionally substituted by 1 to 3 substituents selected from
(1) a C₃₋₁₀ cycloalkyl group (e.g., cyclopropyl) optionally substituted by 1 to 3 C₁₋₆ alkyl groups (e.g., methyl),
(2) a pyrrolidinyl group,
(3) a pyridyl group optionally substituted by 1 to 3 substituents selected from
   (i) a C₁₋₆ alkoxy group (e.g., methoxy), and
   (ii) a halogen atom (e.g., fluorine atom), and
(4) an oxo group.

Ring A is particularly preferably a dihydropyrimidine ring optionally substituted by 1 to 3 substituents selected from
(1) a pyrrolidinyl group,
(2) a pyridyl group optionally substituted by 1 to 3 substituents selected from
   (i) a C₁₋₆ alkoxy group (e.g., methoxy), and
   (ii) a halogen atom (e.g., fluorine atom), and
(4) an oxo group.

When tautomers of the following formula (A) and the following formula (B) are present in the "5- or 6-membered nitrogen-containing heterocycle" of the "optionally further substituted 5- or 6-membered nitrogen-containing heterocycle" of ring A, either tautomer can be used.

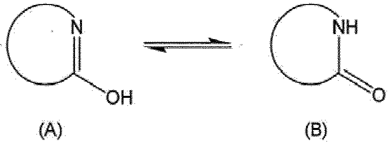

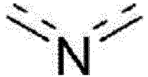

as a ring A-constituting atom is =N- or -N=.

Carbonyl of amide (-CO-NH-), a linker, binds to a ring-constituting atom adjacent to the nitrogen atom in

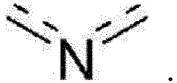

Ring B is a benzene ring or a pyridine ring, each of which is optionally further substituted.

The "benzene ring or a pyridine ring" of the "benzene ring or a pyridine ring, each of which is optionally further substituted" for ring B may be further substituted by, for example, a substituent selected from the aforementioned substituent group A, and the number of the substituents is, for example, 1 to 3. When the number of the substituents is two or more, each substituent may be the same or different.

Ring B is preferably a benzene ring optionally substituted by a halogen atom (e.g., fluorine atom).

X is a carbon atom or a nitrogen atom.

X is preferably a carbon atom.

L is a bond or an optionally substituted C₁₋₂ alkylene group.

The "C₁₋₂ alkylene group" of the "optionally substituted C₁₋₂ alkylene group" for L may be substituted by, for example, a substituent selected from the aforementioned substituent group A, and the number of the substituents is, for example, 1 to 3. When the number of the substituents is two or more, each substituent may be the same or different.

L is preferably a bond or methylene.

L is more preferably a bond.

Y is
(1) the formula -CH₂-O-R¹ wherein R¹ is a substituent; or
(2) the formula: 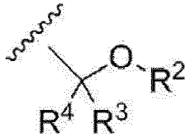
wherein R² and R³ are each independently a hydrogen atom or a substituent, R⁴ is a substituent, or R³ and R⁴ are joined to optionally form, together with the adjacent carbon atom, an optionally further substituted ring, provided when L is a bond, then R³ and R⁴ are not 3-pyridyl groups at the same time.

Examples of the "ring" of the "optionally further substituted ring" jointly formed by R³ and R⁴ together with the adjacent carbon atom include a ring corresponding to a C₃₋₁₀ cycloalkyl group, a ring corresponding to a C₃₋₁₀ cycloalkenyl group, and a ring corresponding to a non-aromatic heterocyclic group.

The "ring" of the "optionally further substituted ring" jointly formed by R³ and R⁴ together with the adjacent carbon atom may be further substituted by, for example, a substituent selected from the aforementioned substituent group A, and the number of the substituents is, for example, 1 to 3. When the number of the substituents is two or more, each substituent may be the same or different.

Y is preferably
(1) the formula -CH₂-O-R¹ wherein R¹ is an optionally substituted C₁₋₆ alkyl group; or
(2) the formula: 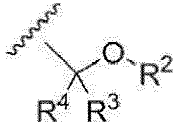
wherein R² and R³ are each independently a hydrogen atom or an optionally substituted C₁₋₆ alkyl group, and R⁴ is an optionally substituted C₁₋₆ alkyl group.

Y is more preferably
(1) the formula -CH₂-O-R¹ wherein R¹ is a C₁₋₆ alkyl group (e.g., methyl); or
(2) the formula:

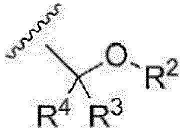

wherein R² is a hydrogen atom;
R³ is a, C₁₋₆ alkyl group (e.g., methyl); and
R⁴ is a C₁₋₆ alkyl group (e.g., methyl).

Y is further preferably,
(1) the formula -CH₂-O-R¹ wherein R¹ is a C₁₋₆ alkyl group (e.g., methyl).

In another embodiment, preferably, the above-mentioned R⁴ is not an optionally substituted carboxyl group, an optionally substituted carbamoyl group, a cyano group or a tetrazolyl group.

In another embodiment, preferably, the above-mentioned R³ and R⁴ are not optionally substituted cyclic groups (optionally substituted C₃₋₁₀ cycloalkyl group, optionally substituted C₃₋₁₀ cycloalkenyl group, optionally substituted C₆₋₁₄ aryl group, optionally substituted heterocyclic group).

Z is a substituent.

Z is preferably
(1) an optionally substituted C₁₋₆ alkoxy group,
(2) an optionally substituted C₁₋₆ alkyl group,
(3) an optionally substituted C₃₋₁₀ cycloalkyl group, or
(4) an optionally substituted heterocyclic group.

Z is more preferably
(1) an optionally halogenated C₁₋₆ alkoxy group (e.g., trifluoromethoxy),
(2) an optionally halogenated C₁₋₆ alkyl group (e.g., trifluoromethyl),
(3) a C₃₋₁₀ cycloalkyl group (e.g., cyclopropyl), or
(4) a heterocyclic group (preferably, aromatic heterocyclic group, more preferably, 5- or 6-membered monocyclic aromatic heterocyclic group (e.g., pyrrolyl, pyrazolyl)).

In another embodiment, Z is more preferably
(1) an optionally halogenated C₁₋₆ alkoxy group (e.g., trifluoromethoxy),
(2) an optionally halogenated C₁₋₆ alkyl group (e.g., trifluoromethyl),
(3) a C₃₋₁₀ cycloalkyl group (e.g., cyclopropyl), or
(4) a pyrrolyl group or a pyrazolyl group.

Z is further preferably
(1) an optionally halogenated C₁₋₆ alkoxy group (e.g., trifluoromethoxy),
(2) an optionally halogenated C₁₋₆ alkyl group (e.g., trifluoromethyl), or
(3) a C₃₋₁₀ cycloalkyl group (e.g., cyclopropyl).

In another embodiment, Z is further preferably
(1) an optionally halogenated C₁₋₆ alkoxy group (e.g., trifluoromethoxy), or
(2) a heterocyclic group (preferably, aromatic heterocyclic group, more preferably, 5- or 6-membered monocyclic aromatic heterocyclic group (e.g., pyrrolyl, pyrazolyl)).

In still another embodiment, Z is further preferably
(1) a halogenated C₁₋₆ alkoxy group (e.g., trifluoromethoxy), or
(2) a pyrazolyl group.

Z is particularly preferably
(1) an optionally halogenated C₁₋₆ alkoxy group (e.g., trifluoromethoxy).

In another embodiment, Z is particularly preferably
(1) a halogenated C₁₋₆ alkoxy group (e.g., trifluoromethoxy).

In another embodiment, preferably, the above-mentioned Z is not 1-methyl-4-(methylamino)-1,6-dihydroimidazo[4,5-d]pyrrolo[2,3-b]pyridin-7-yl.

In another embodiment, preferably, the above-mentioned Z is not an optionally substituted carbamoyl group, an optionally substituted sulfamoyl group, an optionally substituted arylcarbonyloxy group, an optionally substituted aromatic heterocyclylcarbonyloxy group, an optionally substituted amino group, an optionally substituted aryloxy group, an optionally substituted aromatic heterocyclyloxy group, an optionally substituted aryl group or an optionally substituted aromatic heterocyclic group.

In still another embodiment, preferably, the above-mentioned Z is not a halogen atom.

Preferable examples of compound (I) include the following compounds.

### [Compound A]

Compound (I), wherein ring A is an optionally further substituted 5- or 6-membered nitrogen-containing heterocycle; 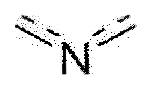 as a ring A-constituting atom is =N- or -N=;
ring B is a benzene ring or a pyridine ring, each of which is optionally substituted;
X is a carbon atom or a nitrogen atom;
L is a bond or an optionally substituted C₁₋₂ alkylene group; Y is
(1) the formula -CH₂-O-R¹ wherein R¹ is an optionally substituted C₁₋₆ alkyl group; or
(2) the formula: 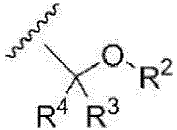
   wherein R² and R³ are each independently a hydrogen atom or an optionally substituted C₁₋₆ alkyl group, and R⁴ is an optionally substituted C₁₋₆ alkyl group; and
   Z is
      (1) an optionally substituted C₁₋₆ alkoxy group,
      (2) an optionally substituted C₁₋₆ alkyl group,
      (3) an optionally substituted C₃₋₁₀ cycloalkyl group, or
      (4) an optionally substituted heterocyclic group.

### [Compound B]

Compound (I), wherein ring A is a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl) optionally substituted by 1 to 3 substituents selected from
   (i) a C₆₋₁₄ aryl group (e.g., phenyl),
   (ii) a C₁₋₆ alkoxy group (e.g., methoxy), and
   (iii) a heterocyclic group (preferably, aromatic heterocyclic group, more preferably, 5- or 6-membered monocyclic aromatic heterocyclic group (e.g., pyridyl)),
(2) a halogen atom (e.g., fluorine atom, chlorine atom),
(3) a cyano group,
(4) a C₂₋₆ alkenyl group (e.g., 1-propenyl),
(5) a C₂₋₆ alkynyl group (e.g., ethynyl, 1-propynyl) optionally substituted by 1 to 3 C₃₋₁₀ cycloalkyl groups (e.g., cyclopropyl),
(6) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a C₆₋₁₄ aryl group (e.g., phenyl), and
   (iii) a C₁₋₆ alkoxy group (e.g., methoxy),
(7) a C₃₋₁₀ cycloalkenyl group (e.g., cyclopentenyl, cyclohexenyl) optionally substituted by 1 to 3 C₁₋₆ alkoxy groups (e.g., methoxy),
(8) a C₆₋₁₄ aryl group (e.g., phenyl) optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(9) a C₁₋₆ alkoxy group (e.g., methoxy),
(10) a C₁₋₆ alkylthio group (e.g., methylthio),
(11) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a C₆₋₁₄ aryl group (e.g., phenyl),
   (ii) a C₃₋₁₀ cycloalkyl group (e.g., cyclopropyl),
   (iii) a C₆₋₁₄ aryl group (e.g., phenyl) optionally substituted by 1 to 3 C₁₋₆ alkoxy groups (e.g., methoxy),
   (iv) a heterocyclic group (preferably, aromatic heterocyclic group, more preferably, 5- or 6-membered monocyclic aromatic heterocyclic group (e.g., pyrazolyl)) optionally substituted by 1 to 3 C₁₋₆ alkyl groups (e.g., methyl), and
   (v) a C₃₋₁₀ cycloalkyl-carbonyl group (e.g., cyclopropylcarbonyl),
(12) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy), and
   (iv) an oxo group,
(13) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(14) a heterocyclyloxy group (preferably, aromatic heterocyclyloxy group, more preferably, 5- or 6-membered monocyclic aromatic heterocyclyloxy group (e.g., pyridyloxy)), and
(15) an oxo group; 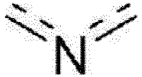 as a ring A-constituting atom is =N- or -N=;
   ring B is a benzene ring optionally substituted by a halogen atom (e.g., fluorine atom);
   X is a carbon atom;
   L is a bond or methylene;
   Y is
   (1) the formula -CH₂-O-R¹ wherein R¹ is a C₁₋₆ alkyl group (e.g., methyl); or
   (2) the formula: 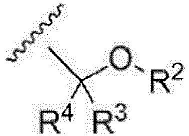
      wherein R² is a hydrogen atom;
      R³ is a C₁₋₆ alkyl group (e.g., methyl); and
      R⁴ is a C₁₋₆ alkyl group (e.g., methyl); and
      Z is
         (1) an optionally halogenated C₁₋₆ alkoxy group (e.g., trifluoromethoxy),
         (2) an optionally halogenated C₁₋₆ alkyl group (e.g., trifluoromethyl),
         (3) a C₃₋₁₀ cycloalkyl group (e.g., cyclopropyl), or
         (4) a heterocyclic group (preferably, aromatic heterocyclic group, more preferably, 5- or 6-membered monocyclic aromatic heterocyclic group (e.g., pyrrolyl, pyrazolyl).

### [Compound B-1]

Compound (I), wherein ring A is a thiazole ring, a pyrazine ring, a pyrimidine ring or a dihydropyrimidine ring, each optionally substituted by 1 to 3 substituents selected from
(1) a C₁₋₆ alkyl group (e.g., methyl, isopropyl, tert-butyl) optionally substituted by 1 to 3 substituents selected from
   (i) a phenyl group,
   (ii) a C₁₋₆ alkoxy group (e.g., methoxy), and
   (iii) a pyridyl group,
(2) a halogen atom (e.g., fluorine atom, chlorine atom),
(3) a cyano group,
(4) a C₂₋₆ alkenyl group (e.g., 1-propenyl),
(5) a C₂₋₆ alkynyl group (e.g., ethynyl, 1-propynyl) optionally substituted by 1 to 3 C₃₋₁₀ cycloalkyl groups (e.g., cyclopropyl),
(6) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a phenyl group, and
   (iii) a C₁₋₆ alkoxy group (e.g., methoxy),
(7) a C₃₋₁₀ cycloalkenyl group (e.g., cyclopentenyl, cyclohexenyl) optionally substituted by 1 to 3 C₁₋₆ alkoxy groups (e.g., methoxy),
(8) a phenyl group optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(9) a C₁₋₆ alkoxy group (e.g., methoxy),
(10) a C₁₋₆ alkylthio group (e.g., methylthio),
(11) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a phenyl group,
   (ii) a C₃₋₁₀ cycloalkyl group (e.g., cyclopropyl),
   (iii) a phenyl group optionally substituted by 1 to 3 C₁₋₆ alkoxy groups (e.g., methoxy),
   (iv) a pyrazolyl group optionally substituted by 1 to 3 C₁₋₆ alkyl groups (e.g., methyl), and
   (v) a C₃₋₁₀ cycloalkyl-carbonyl group (e.g., cyclopropylcarbonyl),
(12) an azetidinyl group, a pyrrolidinyl group, a tetrahydrofuryl group, an isoxazolidinyl group, a piperidyl group, a tetrahydropyranyl group, a morpholinyl group, an azepanyl group, a 3-oxa-8-azabicyclo[3.2.1]octyl group, a 3-azabicyclo[3.1.0]hexyl group or a 6-oxa-3-azabicyclo[3.1.1]heptyl group, each optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy),
      and
   (iv) an oxo group,
(13) a thienyl group, a furyl group, an imidazolyl group, a pyrazolyl group, a thiazolyl group, a pyridyl group, a pyrazinyl group, or a benzimidazolyl group, each optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(14) a pyridyloxy group, and
(15) an oxo group; 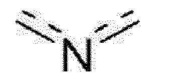 as a ring A-constituting atom is =N- or -N=;
   ring B is a benzene ring optionally substituted by a halogen atom (e.g., fluorine atom);
   X is a carbon atom;
   L is a bond or methylene;
   Y is
   (1) the formula -CH₂-O-R¹ wherein R¹ is a C₁₋₆ alkyl group (e.g., methyl); or
   (2) the formula: 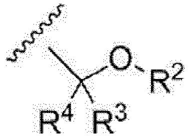
      wherein R² is a hydrogen atom;
      R³ is a C₁₋₆ alkyl group (e.g., methyl); and
      R⁴ is a C₁₋₆ alkyl group (e.g., methyl); and
      Z is
         (1) an optionally halogenated C₁₋₆ alkoxy group (e.g., trifluoromethoxy),
         (2) an optionally halogenated C₁₋₆ alkyl group (e.g., trifluoromethyl),
         (3) a C₃₋₁₀ cycloalkyl group (e.g., cyclopropyl), or
         (4) a pyrrolyl group or a pyrazolyl group.

### [Compound B-2]

Compound (I), wherein ring A is a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl) optionally substituted by 1 to 3 substituents selected from
   (i) a C₆₋₁₄ aryl group (e.g., phenyl),
   (ii) a 6C₁₋₆ alkoxy group (e.g., methoxy), and
   (iii) a heterocyclic group (preferably, aromatic heterocyclic group, more preferably, 5- or 6-membered monocyclic aromatic heterocyclic group (e.g., pyridyl)),
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 substituents selected from
   (i) an optionally halogenated C₁₋₆ alkyl group (e.g., methyl, trifluoromethyl),
   (ii) a C₆₋₁₄ aryl group (e.g., phenyl), and
   (iii) a C₁₋₆ alkoxy group (e.g., methoxy),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a C₆₋₁₄ aryl group (e.g., phenyl) optionally substituted by 1 to 3 substituents selected from
   (i) a C₁₋₆ alkyl group (e.g., methyl),
   (ii) a halogen atom (e.g., fluorine atom), and
   (iii) a C₁₋₆ alkoxy group (e.g., methoxy),
(6) an amino group optionally mono- or di-substituted by a substituent selected from
   (i) a C₁₋₆ alkyl group (e.g., methyl, ethyl, isopropyl, isobutyl) optionally substituted by 1 to 3 substituents selected from a C₁₋₆ alkoxy group (e.g., methoxy), a C₃₋₁₀ cycloalkyl group (e.g., cyclopropyl) and a C₆₋₁₄ aryl group (e.g., phenyl),
   (ii) a C₃₋₁₀ cycloalkyl group (e.g., cyclopropyl),
   (iii) a C₆₋₁₄ aryl group (e.g., phenyl) optionally substituted by 1 to 3 C₁₋₆ alkoxy groups (e.g., methoxy),
   (iv) a heterocyclic group (preferably, aromatic heterocyclic group, more preferably, 5- or 6-membered monocyclic aromatic heterocyclic group (e.g., pyrazolyl)) optionally substituted by 1 to 3 C₁₋₆ alkyl groups (e.g., methyl), and
   (v) a C₃₋₁₀ cycloalkyl-carbonyl group (e.g., cyclopropylcarbonyl),
(7) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 substituents selected from
   (i) a C₁₋₆ alkoxy group (e.g., methoxy, isopropoxy),
   (ii) a halogen atom (e.g., fluorine atom),
   (iii) a C₁₋₆ alkyl group (e.g., methyl) optionally substituted by 1 to 3 substituents selected from a halogen atom (e.g., fluorine atom) and a C₁₋₆ alkoxy group (e.g., methoxy), and
   (iv) an oxo group,
(8) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
   (iii) an optionally halogenated C₁₋₆ alkyl group (e.g., methyl, trifluoromethyl), and
   (iv) a C₃₋₁₀ cycloalkyl group (e.g., cyclopropyl),
(9) an oxo group; 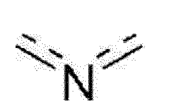 as a ring A-constituting atom is =N- or -N=;
   ring B is a benzene ring optionally substituted by a halogen atom (e.g., fluorine atom);
   X is a carbon atom;
   L is a bond;
   Y is
   (1) the formula -CH₂-O-R¹ wherein R¹ is a C₁₋₆ alkyl group (e.g., methyl); or
   (2) the formula: 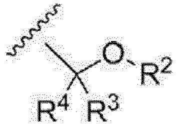
      wherein R² is a hydrogen atom;
      R³ is a C₁₋₆ alkyl group (e.g., methyl); and
      R⁴ is a C₁₋₆ alkyl group (e.g., methyl); and
      Z is
         (1) an optionally halogenated C₁₋₆ alkoxy group (e.g., trifluoromethoxy), or
         (2) a heterocyclic group (preferably, aromatic heterocyclic group, more preferably, 5- or 6-membered monocyclic aromatic heterocyclic group (e.g., pyrrolyl, pyrazolyl)).

### [Compound B-3]

Compound (I), wherein ring A is a 5- or 6-membered nitrogen-containing heterocycle (e.g., thiazole, pyrazine, pyrimidine or dihydropyrimidine) optionally substituted by 1 to 3 substituents selected from
(1) a C₁₋₆ alkyl group (e.g., methyl, isopropyl, tert-butyl)
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 optionally halogenated C₁₋₆ alkyl groups (e.g., methyl, trifluoromethyl),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a C₆₋₁₄ aryl group (e.g., phenyl),
(6) an amino group optionally mono- or di-substituted by a C₁₋₆ alkyl group (e.g., methyl, ethyl, isopropyl, isobutyl),
(7) a non-aromatic heterocyclic group (preferably, 4- to 10-membered non-aromatic heterocyclic group (e.g., azetidinyl, pyrrolidinyl, tetrahydrofuryl, isoxazolidinyl, piperidyl, tetrahydropyranyl, morpholinyl, azepanyl, 3-oxa-8-azabicyclo[3.2.1]octyl, 3-azabicyclo[3.1.0]hexyl, 6-oxa-3-azabicyclo[3.1.1]heptyl)) optionally substituted by 1 to 3 C₁₋₆ alkoxy groups (e.g., methoxy, isopropoxy),
(8) an aromatic heterocyclic group (preferably, 5- to 10-membered aromatic heterocyclic group (e.g., thienyl, furyl, imidazolyl, pyrazolyl, thiazolyl, pyridyl, pyrazinyl, benzimidazolyl)) optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
      and
   (iii) an optionally halogenated C₁₋₆ alkyl group (e.g., methyl, trifluoromethyl), and
(9) an oxo group; 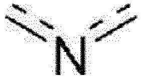 as a ring A-constituting atom is =N- or -N=;
   ring B is a benzene ring optionally substituted by a halogen atom (e.g., fluorine atom);
   X is a carbon atom;
   L is a bond;
   Y is
   (1) the formula -CH₂-O-R¹ wherein R¹ is a C₁₋₆ alkyl group (e.g., methyl); or
   (2) the formula: 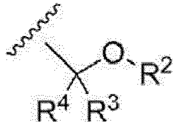
      wherein R² is a hydrogen atom;
      R³ is a C₁₋₆ alkyl group (e.g., methyl); and
      R⁴ is a C₁₋₆ alkyl group (e.g., methyl); and
      Z is
         (1) an optionally halogenated C₁₋₆ alkoxy group (e.g., trifluoromethoxy), or
         (2) a heterocyclic group (preferably, aromatic heterocyclic group, more preferably, 5- or 6-membered monocyclic aromatic heterocyclic group (e.g., pyrrolyl, pyrazolyl)).

### [Compound B-4]

Compound (I), wherein ring A is a dihydropyrimidine ring optionally substituted by 1 to 3 substituents selected from
(1) a C₁₋₆ alkyl group (e.g., methyl, isopropyl, tert-butyl)
(2) a cyano group,
(3) a C₃₋₁₀ cycloalkyl group (e.g., cyclopropyl, cyclobutyl, cyclopentyl, cyclohexyl, bicyclo[3.1.0]hexyl) optionally substituted by 1 to 3 C₁₋₆ alkyl groups (e.g., methyl),
(4) a C₃₋₁₀ cycloalkenyl group (e.g., cyclopentenyl, cyclohexenyl) optionally substituted by 1 to 3 C₁₋₆ alkoxy groups (e.g., methoxy),
(5) a phenyl group,
(6) an amino group optionally di-substituted by a C₁₋₆ alkyl group (e.g., methyl, ethyl, isopropyl, isobutyl),
(7) an azetidinyl group, a pyrrolidinyl group or a 6-oxa-3-azabicyclo[3.1.1]heptyl group, each optionally substituted by 1 to 3 C₁₋₆ alkoxy groups (e.g., methoxy, isopropoxy),
(8) a thienyl group, a furyl group, a pyrazolyl group or a pyridyl group, each optionally substituted by 1 to 3 substituents selected from
   (i) an optionally halogenated C₁₋₆ alkoxy group (e.g., methoxy, ethoxy, isopropoxy, difluoromethoxy),
   (ii) a halogen atom (e.g., fluorine atom, chlorine atom),
      and
   (iii) an optionally halogenated C₁₋₆ alkyl group (e.g., methyl, trifluoromethyl), and
(9) an oxo group; 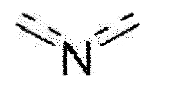 as a ring A-constituting atom is =N- or -N=;
   ring B is a benzene ring optionally substituted by a halogen atom (e.g., fluorine atom);
   X is a carbon atom;
   L is a bond;
   Y is
   (1) the formula -CH₂-O-R¹ wherein R¹ is a C₁₋₆ alkyl group (e.g., methyl); or
   (2) the formula: 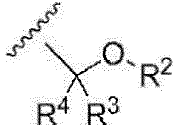
      wherein R² is a hydrogen atom;
      R³ is a C₁₋₆ alkyl group (e.g., methyl); and
      R⁴ is a C₁₋₆ alkyl group (e.g., methyl); and
      Z is
         (1) an optionally halogenated C₁₋₆ alkoxy group (e.g., trifluoromethoxy), or
         (2) a pyrazolyl group.

### [Compound C]

Compound (I), wherein ring A is a 5- or 6-membered nitrogen-containing heterocycle (e.g., dihydropyrimidine) optionally substituted by 1 to 3 substituents selected from
(1) a C₃₋₁₀ cycloalkyl group (e.g., cyclopropyl) optionally substituted by 1 to 3 C₁₋₆ alkyl groups (e.g., methyl),
(2) a non-aromatic heterocyclic group (preferably, 4- to 10-membered non-aromatic heterocyclic group (e.g., pyrrolidinyl)),
(3) an aromatic heterocyclic group (preferably, 5- to 10-membered aromatic heterocyclic group (e.g., pyridyl)) optionally substituted by 1 to 3 substituents selected from
   (i) a C₁₋₆ alkoxy group (e.g., methoxy), and
   (ii) a halogen atom (e.g., fluorine atom), and
(4) an oxo group; 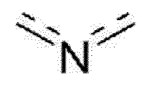 as a ring A-constituting atom is =N- or -N=;
   ring B is a benzene ring optionally substituted by a halogen atom (e.g., fluorine atom);
   X is a carbon atom;
   L is a bond;
   Y is the formula -CH₂-O-R¹ wherein R¹ is a C₁₋₆ alkyl group (e.g., methyl); and
   Z is an optionally halogenated C₁₋₆ alkoxy group (e.g., trifluoromethoxy).

### [Compound C-1]

Compound (I), wherein ring A is a 5- or 6-membered nitrogen-containing heterocycle (e.g., dihydropyrimidine) optionally substituted by 1 to 3 substituents selected from
(1) a C₃₋₁₀ cycloalkyl group (e.g., cyclopropyl) optionally substituted by 1 to 3 C₁₋₆ alkyl groups (e.g., methyl),
(2) a non-aromatic heterocyclic group (preferably, 4- to 10-membered non-aromatic heterocyclic group (e.g., pyrrolidinyl)),
(3) an aromatic heterocyclic group (preferably, 5- to 10-membered aromatic heterocyclic group (e.g., pyridyl)) optionally substituted by 1 to 3 substituents selected from
   (i) a C₁₋₆ alkoxy group (e.g., methoxy), and
   (ii) a halogen atom (e.g., fluorine atom), and
(4) an oxo group; 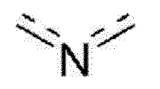 as a ring A-constituting atom is =N- or -N=;
   ring B is a benzene ring optionally substituted by a halogen atom (e.g., fluorine atom);
   X is a carbon atom;
   L is a bond;
   Y is the formula -CH₂-O-R¹ wherein R¹ is a C₁₋₆ alkyl group (e.g., methyl); and
   Z is a halogenated C₁₋₆ alkoxy group (e.g., trifluoromethoxy).

### [Compound C-2]

Compound (I), wherein ring A is a dihydropyrimidine ring optionally substituted by 1 to 3 substituents selected from
(1) a C₃₋₁₀ cycloalkyl group (e.g., cyclopropyl) optionally substituted by 1 to 3 C₁₋₆ alkyl groups (e.g., methyl),
(2) a pyrrolidinyl group,
(3) a pyridyl group optionally substituted by 1 to 3 substituents selected from
   (i) a C₁₋₆ alkoxy group (e.g., methoxy), and
   (ii) a halogen atom (e.g., fluorine atom), and
(4) an oxo group; 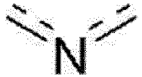 as a ring A-constituting atom is =N- or -N=;
   ring B is a benzene ring optionally substituted by a halogen atom (e.g., fluorine atom);
   X is a carbon atom;
   L is a bond;
   Y is the formula -CH₂-O-R¹ wherein R¹ is a C₁₋₆ alkyl group (e.g., methyl); and
   Z is a halogenated C₁₋₆ alkoxy group (e.g., trifluoromethoxy).

### [Compound C-3]

Compound (I), wherein ring A is a dihydropyrimidine ring optionally substituted by 1 to 3 substituents selected from
(1) a pyrrolidinyl group,
(2) a pyridyl group optionally substituted by 1 to 3 substituents selected from
   (i) a C₁₋₆ alkoxy group (e.g., methoxy), and
   (ii) a halogen atom (e.g., fluorine atom), and
(3) an oxo group; 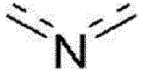 as a ring A-constituting atom is =N- or -N=;
   ring B is a benzene ring optionally substituted by a halogen atom (e.g., fluorine atom);
   X is a carbon atom;
   L is a bond;
   Y is the formula -CH₂-O-R¹ wherein R¹ is a C₁₋₆ alkyl group (e.g., methyl); and
   Z is a halogenated C₁₋₆ alkoxy group (e.g., trifluoromethoxy).

Specific examples of compound (I) include the compounds of Examples 1 - 189, from which N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide, 2-(3,5-dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide, and 2-(3-fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide, or a salt thereof are preferable.

Examples of the salt of the compound represented by the formula (I) include a metal salt, an ammonium salt, a salt with organic base, a salt with inorganic acid, a salt with organic acid, a salt with basic or acidic amino acid, and the like.

Preferable examples of the metal salt include alkali metal salts such as sodium salt, potassium salt and the like; alkaline earth metal salts such as calcium salt, magnesium salt, barium salt and the like; an aluminum salt, and the like.

Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like.

Preferable examples of the salt with inorganic acid include salts with hydrochloric acid, hydrobromic acid, nitric acid, sulfuric acid, phosphoric acid and the like.

Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysine, ornithine and the like, and preferable examples of the salt with acidic amino acid include salts with aspartic acid, glutamic acid and the like.

Of the above-mentioned salts, a pharmaceutically acceptable salt is preferable.

A prodrug of compound (I) means a compound which is converted to the compound (I) by a reaction due to an enzyme, an gastric acid, etc. under the physiological condition in the living body, that is, a compound which is enzymatically converted to the compound (I) by oxidation, reduction, hydrolysis, etc.; a compound which is converted to the compound (I) by hydrolysis etc. due to gastric acid, etc.

A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation or dimethylaminomethylcarbonylation); a compound obtained by subjecting a carboxyl group in compound (I) to an esterification or amidation (e.g., a compound obtained by subjecting a carboxyl group in compound (I) to C₁₋₆ alkyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation) and the like. Of these, compound (I) wherein a carboxyl group is esterified with C₁₋₆ alkyl such as methyl, ethyl, tert-butyl and the like is preferably used. These compounds can be produced from compound (I) by a method known *per se.*

The prodrug of compound (I) may be a compound that converts to compound (I) under physiological conditions as described in "Development of Pharmaceutical Products", vol. 7, Molecule Design, pages 163-198, Hirokawa Shoten (1990).

Unless otherwise specified, each symbol in the compounds in the following reaction schemes is as defined above. Each compound described in the reaction schemes may form a salt as long as it does not inhibit the reaction. Examples of such salt include those similar to the salts of compound (I).

While the compound obtained in each step can be directly used as a crude product in the form of a reaction mixture for the next reaction, it can also be isolated from the reaction mixture according to a conventional method, and further purified with ease by a separation means such as recrystallization, distillation, chromatography and the like.

The production method of the compound of the present invention is explained in the following.

The starting materials and reagents used in each step in the following production method, and the obtained compounds each may form a salt. Examples of the salt include those similar to the aforementioned salts of the compound of the present invention and the like.

When the compound obtained in each step is a free compound, it can be converted to a desired salt by a method known per se. Conversely, when the compound obtained in each step is a salt, it can be converted to a free form or a desired other kind of salt by a method known per se.

The compound obtained in each step can also be used for the next reaction as a reaction mixture thereof or after obtaining a crude product thereof. Alternatively, the compound obtained in each step can be isolated and/or purified from the reaction mixture by a separation means such as concentration, crystallization, recrystallization, distillation, solvent extraction, fractionation, chromatography and the like according to a conventional method.

When the starting materials and reagent compounds of each step are commercially available, the commercially available products can be used as they are.

In the reaction of each step, while the reaction time varies depending on the reagents and solvents to be used, unless otherwise specified, it is generally 1 min - 48 hr, preferably 10 min - 8 hr.

In the reaction of each step, while the reaction temperature varies depending on the reagents and solvents to be used, unless otherwise specified, it is generally -78°C to 300°C, preferably -78°C to 150°C.

In the reaction of each step, while the pressure varies depending on the reagents and solvents to be used, unless otherwise specified, it is generally 1 atm - 20 atm, preferably 1 atm - 3 atm.

In the reaction of each step, for example, microwave synthesizers such as Initiator manufactured by Biotage and the like are sometimes used. While the reaction temperature varies depending on the reagents and solvents to be used, unless otherwise specified, it is generally room temperature - 300°C, preferably 50°C - 250°C. While the reaction time varies depending on the reagents and solvents to be used, unless otherwise specified, it is generally 1 min - 48 hr, preferably 1 min - 8 hr.

In the reaction of each step, unless otherwise specified, a reagent is used in 0.5 equivalent - 20 equivalents, preferably 0.8 equivalent - 5 equivalents, relative to the substrate. When a reagent is used as a catalyst, the reagent is used in 0.001 equivalent - 1 equivalent, preferably 0.01 equivalent - 0.2 equivalent, relative to the substrate. When the reagent is also a reaction solvent, the reagent is used in a solvent amount.

In the reaction of each step, unless otherwise specified, it is performed without solvent or by dissolving or suspending in a suitable solvent. Specific examples of the solvent include those described in Examples and the following. alcohols: methanol, ethanol, tert-butyl alcohol, 2-methoxyethanol and the like; ethers: diethyl ether, diphenyl ether, tetrahydrofuran, 1,2-dimethoxyethane and the like; aromatic hydrocarbons: chlorobenzene, toluene, xylene and the like; saturated hydrocarbons: cyclohexane, hexane and the like; amides: N,N-dimethylformamide, N-methylpyrrolidone and the like; halogenated hydrocarbons: dichloromethane, carbon tetrachloride and the like; nitriles: acetonitrile and the like; sulfoxides: dimethyl sulfoxide and the like; aromatic organic bases: pyridine and the like; acid anhydrides: acetic anhydride and the like; organic acids: formic acid, acetic acid, trifluoroacetic acid and the like; inorganic acids: hydrochloric acid, sulfuric acid and the like; esters: ethyl acetate and the like; ketones: acetone, methyl ethyl ketone and the like; and water.

Two or more kinds of the above-mentioned solvents may be used by mixing at an appropriate ratio.

When a base is used in the reaction of each step, for example, bases shown below or those described in Examples are used. inorganic bases: sodium hydroxide, magnesium hydroxide and the like; basic salts: sodium carbonate, calcium carbonate, sodium hydrogen carbonate and the like; organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine and the like; metal alkoxides: sodium ethoxide, potassium tert-butoxide and the like; alkali metal hydrides: sodium hydride and the like; metal amides: sodium amide, lithium diisopropyl amide, lithium hexamethyl disilazide and the like; and organolithium compounds: n-butyllithium and the like.

When an acid or acidic catalyst is used in the reaction of each step, for example, acids and acidic catalysts shown below or those described in Examples are used. inorganic acids: hydrochloric acid, sulfuric acid, nitric acid, hydrobromic acid, phosphoric acid and the like; organic acids: acetic acid, trifluoroacetic acid, citric acid, p-toluenesulfonic acid, 10-camphorsulfonic acid and the like; and
Lewis acids: boron trifluoride diethyl ether complex, zinc iodide, anhydrous aluminum chloride, anhydrous zinc chloride, anhydrous iron chloride and the like.

Unless otherwise specified, the reaction of each step is performed according to a method known per se, for example, the methods described in Jikken Kagaku Kouza 5th edition, vol. 13 - vol. 19 (The Chemical Society of Japan ed.); Shinjikken Kagaku Kouza, vol. 14 - vol. 15 (The Chemical Society of Japan ed.); Fine Organic Chemistry rev. 2nd edition (L. F. Tietze, Th. Eicher, NANKODO); rev. Organic Name Reactions, Their Mechanism and Essence (Hideo Togo, Kodansha); ORGANIC SYNTHESES Collective Volume I - VII (John Wiley & Sons Inc); Modern Organic Synthesis in the Laboratory, A Collection of Standard Experimental Procedures (Jie Jack Li, OXFORD UNIVERSITY); Comprehensive Heterocyclic Chemistry III, Vol. 1 - Vol. 14 (Elsevier Japan KK); Strategic Applications of Named Reactions in Organic Synthesis (translation supervisor Kiyoshi Tomioka, KAGAKUDOJIN); Comprehensive Organic Transformations (VCH Publishers Inc.), 1989 and the like, or the methods described in the Examples.

In each step, protection or deprotection of a functional group is performed by the method known per se, for example, the methods described in "Protective Groups in Organic Synthesis, 4th Ed." (Theodora W. Greene, Peter G. M. Wuts) Wiley-Interscience, 2007; "Protecting Groups 3rd Ed." (P. J. Kocienski) Thieme, 2004 and the like, or the methods described in the Examples.

Examples of the protecting group of the hydroxy group of alcohol and the like and a phenolic hydroxy group include ether protecting groups such as methoxymethyl ether, benzyl ether, t-butyldimethylsilyl ether, tetrahydropyranyl ether and the like; carboxylate protecting groups such as acetate and the like; sulfonate ester protecting groups such as methanesulfonate ester and the like; carbonate ester protecting groups such as t-butylcarbonate and the like, and the like.

Examples of the protecting group of the carbonyl group of aldehyde include acetal protecting groups such as dimethyl acetal and the like; cyclic acetal protecting groups such as cyclic 1,3-dioxane and the like, and the like.

Examples of the protecting group of the carbonyl group of ketone include ketal protecting groups such as dimethyl ketal and the like; cyclic ketal protecting groups such as cyclic 1,3-dioxane and the like; oxime protecting groups such as O-methyloxime and the like; hydrazone protecting groups such as N,N-dimethylhydrazone and the like, and the like.

Examples of the carboxyl protecting group include ester protecting groups such as methyl ester and the like; amide protecting groups such as N,N-dimethylamide and the like, and the like.

Examples of the thiol protecting group include ether protecting groups such as benzyl thioether and the like; ester protecting groups such as thioacetate ester, thiocarbonate, thiocarbamate and the like, and the like.

Examples of the protecting group of an amino group and an aromatic heterocycle such as imidazole, pyrrole, indole and the like include carbamate protecting groups such as benzyl carbamate and the like; amide protecting groups such as acetamide and the like; alkylamine protecting groups such as N-triphenylmethylamine and the like, sulfonamide protecting groups such as methanesulfonamide and the like, and the like.

The protecting group can be removed by a method known per se, for example, a method using acid, base, ultraviolet light, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide), a reduction method and the like.

When a reduction reaction is performed in each step, examples of the reducing agent to be used include metal hydrides such as lithium aluminum hydride, sodium triacetoxyborohydride, sodium cyanoborohydride, diisobutylaluminum hydride (DIBAL-H), sodium borohydride, tetramethylammonium triacetoxyborohydride and the like; boranes such as borane tetrahydrofuran complex and the like; Raney nickel; Raney cobalt; hydrogen; formic acid and the like. When a carbon-carbon double bond or triple bond is reduced, a method using a catalyst such as palladium-carbon, Lindlar catalyst and the like.

When an oxidation reaction is performed in each step, examples of an oxidant to be used include peracids such as m-chloroperbenzoic acid (mCPBA), hydrogen peroxide, t-butyl hydroperoxide and the like; perchlorates such as tetrabutylammonium perchlorate and the like; chlorates such as sodium chlorate and the like; chlorites such as sodium chlorite and the like; periodic acids such as sodium periodate and the like; hypervalent iodine reagents such as iodosylbenzene and the like; reagents containing manganese such as manganese dioxide, potassium permanganate and the like; leads such as lead tetraacetate and the like; reagents containing chromium such as pyridinium chlorochromate (PCC), pyridinium dichromate (PDC), Jones reagent and the like; halogen compounds such as N-bromosuccinimide (NBS) and the like; oxygen; ozone; sulfur trioxide pyridine complex; osmium tetraoxide; selenium dioxide; 2,3-dichloro-5,6-dicyano-1,4-benzoquinone (DDQ) and the like.

When a radical cyclization reaction is performed in each step, examples of the radical initiator to be used include azo compounds such as azobisisobutyronitrile (AIBN) and the like; water-soluble radical initiators such as 4,4'-azobis-4-cyanopentanoic acid (ACPA) and the like; triethylborane in the presence of air or oxygen; benzoyl peroxide and the like. In addition, examples of the radical reaction reagent to be used include tributylstannane, tristrimethylsilylsilane, 1,1,2,2-tetraphenyldisilane, diphenylsilane, samarium iodide and the like.

When the Wittig reaction is performed in each step, examples of the Wittig reagent to be used include alkylidenephosphoranes and the like. Alkylidenephosphoranes can be prepared by a method known per se, for example, by reacting a phosphonium salt with a strong base.

When the Horner-Emmons reaction is performed in each step, examples of the reagent to be used include phosphonoacetic acid esters such as methyl dimethylphosphonoacetate, ethyl diethylphosphonoacetate and the like; and bases such as alkali metal hydrides, organolithium compounds and the like.

When the Friedel-Crafts reaction is performed in each step, examples of the reagent to be used include Lewis acid and acid chloride or alkylating agents (e.g., alkyl halides, alcohol, olefins and the like). Alternatively, an organic acid and an inorganic acid can also be used instead of the Lewis acid, and acid anhydride such as acetic anhydride and the like can also be used instead of acid chloride.

When an aromatic nucleophilic substitution reaction is performed in each step, a nucleophilic agent (e.g., amines, imidazole and the like) and a base (e.g., basic salts, organic bases and the like) are used as the reagent.

When a nucleophilic addition reaction with carbanion, a nucleophilic 1,4-addition reaction with carbanion (Michael addition reaction) or a nucleophilic substitution reaction with carbanion is performed in each step, examples of the base to be used for generating carbanion include organolithium compounds, metal alkoxides, inorganic bases, organic bases and the like.

When the Grignard reaction is performed in each step, examples of the Grignard reagent include aryl magnesium halides such as phenyl magnesium bromide and the like; and alkyl magnesium halides such as methyl magnesium bromide and the like. The Grignard reagent can be prepared by a method known per se, for example, by reacting alkyl halide or aryl halide with magnesium metal in ether or tetrahydrofuran as a solvent.

When the Knoevenagel condensation reaction is performed in each step, an active methylene compound held between two electron-withdrawing groups (e.g., malonic acid, diethyl malonate, malononitrile and the like) and a base (e.g., organic bases, metal alkoxides, inorganic bases) are used as the reagents.

When the Vilsmeier-Haack reaction is performed in each step, phosphoryl chloride and an amide derivative (e.g., N,N-dimethylformamide and the like) are used as the reagents.

When an azidation reaction of alcohols, alkylhalides or sulfonate esters is performed in each step, examples of the azidation reagent to be used include diphenylphosphoryl azide (DPPA), trimethylsilylazide, sodium azide and the like. For example, when alcohols are azidated, a method using diphenylphosphoryl azide and 1,8-diazabicyclo[5,4,0]undec-7-ene (DBU), a method using trimethylsilylazide and the Lewis acid and the like can be employed.

When a reductive amination reaction is performed in each step, examples of the reducing agent to be used include sodium triacetoxyborohydride, sodium cyanoborohydride, hydrogen, formic acid and the like. When the substrate is an amine compound, examples of the carbonyl compound to be used besides para-formaldehyde include aldehydes such as acetaldehyde and the like, ketones such as cyclohexanone and the like. When the substrate is a carbonyl compound, examples of the amines to be used include primary amines such as ammonia, methylamine and the like; secondary amines such as dimethylamine and the like, and the like.

When the Mitsunobu reaction is performed in each step, azodicarboxylate esters (e.g., diethyl azodicarboxylate (DEAD), diisopropyl azodicarboxylate (DIAD) and the like) and triphenylphosphine are used as the reagents.

When an esterification reaction, amidation reaction or ureation reaction is performed in each step, examples of the reagent to be used include acyl halide forms such as acid chloride, acid bromide and the like; and activated carboxylic acids such as acid anhydride, active ester form, sulfate ester form and the like. Examples of the activator of the carboxylic acid include carbodiimide condensing agents such as 1-ethyl-3-(3-dimethylaminopropyl)carbodiimide hydrochloride (WSCD) and the like; triazine condensing agents such as 4-(4,6-dimethoxy-1,3,5-triazin-2-yl)-4-methylmorpholinium chloride-n-hydrate (DMT-MM) and the like; carbonate ester condensing agents such as 1,1-carbonyldiimidazole (CDI) and the like; diphenylphosphoryl azide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformates such as ethyl chloroformate and the like; 0-(7-azabenzotriazol-1-yl)-N,N,N',N'-tetramethyluronium hexafluorophosphate (HATU); sulfuric acid; a combination thereof and the like. When a carbodiimide condensing agent is used, additives such as 1-hydroxybenzotriazole (HOBt), N-hydroxysuccinimide (HOSu), dimethylaminopyridine (DMAP) and the like can be further added to the reaction.

When a coupling reaction is performed in each step, examples of the metal catalyst to be used include palladium compounds such as palladium(II) acetate, tetrakis(triphenylphosphine)palladium(0), dichloro bis(triphenylphosphine)palladium(II), dichloro bis(triethylphosphine)palladium(II), tris(dibenzylideneacetone)dipalladium(0), 1,1'-bis(diphenylphosphino)ferrocene palladium(II) chloride, palladium(II) acetate and the like; nickel compounds such as tetrakis(triphenylphosphine)nickel(0) and the like; rhodium compounds such as tris(triphenylphosphine)rhodium(III) chloride and the like; a cobalt compound; copper compounds such as copper oxide, copper(I) iodide and the like; a platinum compound and the like. A base may be further added to the reaction and examples of such base include inorganic bases, basic salts and the like.

When a thiocarbonylation reaction is performed in each step, diphosphorus pentasulfide is representatively used as a thiocarbonylating agent. Besides diphosphorus pentasulfide, a reagent having a 1,3,2,4-dithiadiphosphetane-2,4-disulfide structure such as 2,4-bis(4-methoxyphenyl-1,3,2,4-dithiadiphosphetane-2,4-disulfide (Lawesson reagent) and the like may also be used.

When the Wohl-Ziegler reaction is performed in each step, examples of the halogenating agent to be used include N-iodosuccinimide, N-bromosuccinimide (NBS), N-chlorosuccinimide (NCS), bromine, sulfuryl chloride and the like. Furthermore, the reaction can be accelerated by adding heat, light, radical initiators such as benzoyl peroxide, azobisisobutyronitrile and the like to the reaction.

When a halogenating reaction of a hydroxy group is performed in each step, examples of the halogenating agent to be used include acid halide of hydrohalic acid and inorganic acid; specifically, hydrochloric acid, thionyl chloride, phosphorus oxychloride and the like for chlorination, and 48% hydrobromic acid, phosphorus tribromide, and the like for bromination. In addition, a method of obtaining an alkyl halide form from alcohol by reacting with triphenylphosphine and carbon tetrachloride or carbon tetrabromide, and the like may be used. Alternatively, a method of synthesizing an alkyl halide form via a two-step reaction including conversion of alcohol to sulfonate ester, and reacting same with lithium bromide, lithium chloride or sodium iodide may also be used.

When the Arbuzov reaction is performed in each step, examples of the reagent to be used include alkyl halides such as ethyl bromoacetate and the like; and phosphites such as triethyl phosphite, tri(isopropyl)phosphite and the like.

When a sulfonate esterification reaction is performed in each step, examples of the sulfonating agent to be used include methanesulfonyl chloride, p-toluenesulfonyl chloride, methanesulfonic anhydride, p-toluenesulfonic anhydride and the like.

When hydrolysis is performed in each step, an acid or a base is used as the reagent. In addition, when acid hydrolysis of t-butyl ester is performed, formic acid, triethylsilane and the like are sometimes added to reductively trap the by-produced t-butyl cation.

When a dehydrating reaction is performed in each step, examples of the dehydrating agent to be used include sulfuric acid, phosphorus pentoxide, phosphorus oxychloride, N,N'-dicyclohexylcarbodiimide, alumina, polyphosphoric acid and the like.

Compound (I) can be produced by a method known per se, for example, methods shown in Reaction schemes 1 to 9, or a method analogous thereto. 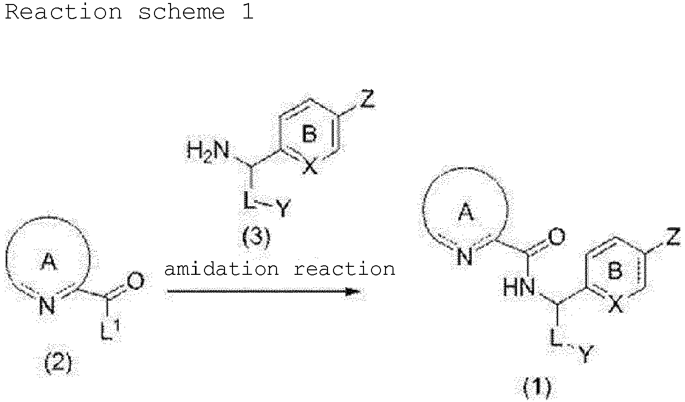 wherein L¹ is a leaving group, and other symbols are as defined above.

Compound (2) can be obtained as a commercially available product, or can be produced by a method known per se or a method analogous thereto.

Compound (9) which is compound (3) in reaction scheme 1 wherein -L-Y is -CH₂-OR¹ can be produced, for example, by a method shown in reaction scheme 2 or a method analogous thereto. 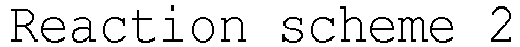 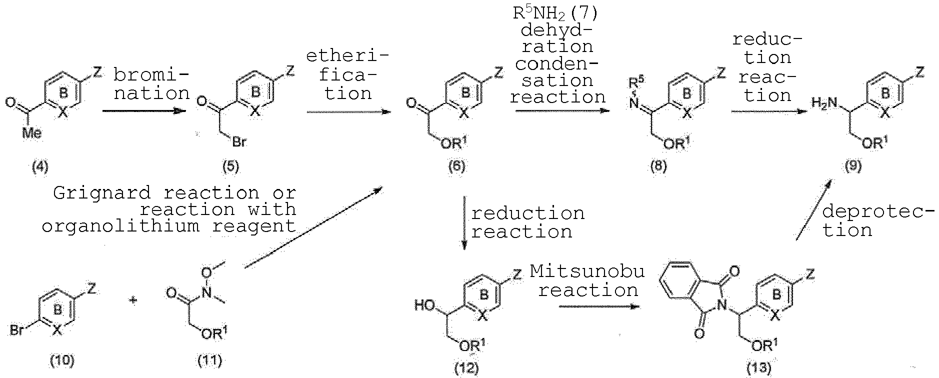 wherein R⁵ is a hydroxy group, an optionally substituted C₁₋₃ alkoxy group or an optionally substituted benzyl group, and other symbols are as defined above.

Compound (4), compound (7), compound (10) and compound (11) can be obtained as commercially available products, or can be produced by a method known per se or a method analogous thereto.

Compound (5) can be produced by bromination of compound (4). Examples of the brominating agent include bromine, phenyltrimethylammonium tribromide, N-bromosuccinimide and the like.

Compound (6) can be produced by etherification of compound (5). This reaction is performed by, for example, reacting compound (5) with desired alcohol (R¹OH) in the presence of silver(I) carbonate or silver(I) oxide and boron trifluoride diethyl ether complex. This reaction can also be performed by reacting compound (5) and a desired alcohol (R¹OH) in the presence of a base. Examples of such base include tertiary amines such as triethylamine, tripropylamine, tributylamine, diisopropylethylamine and the like, basic salts, metal hydride complex compounds, metal alkoxides, metal amides, organolithium compounds and the like.

Compound (6) can also be produced by a Grignard reaction using compound (10) and compound (11) or a reaction with an organolithium reagent. In the reaction with an organolithium reagent, an organolithium reagent can be prepared by a method known per se, for example, by reacting compound (10) with, for example, an organolithium compound such as n-butyllithium and the like in ether or tetrahydrofuran as a solvent.

Compound (8) can be produced by a dehydration condensation reaction of compound (6) and compound (7). When compound (7) forms a salt, this reaction is generally performed by adding a base. Examples of such base include tertiary amines such as triethylamine, tripropylamine, tributylamine, diisopropylethylamine and the like, aromatic amines such as pyridine, 2,6-lutidine and the like, basic salts, inorganic bases, alkali metal hydrides, metal alkoxides and the like. This reaction can also be promoted by adding a dehydrating agent such as molecular sieve and the like, or p-toluenesulfonic acid, zinc chloride, phosphoryl chloride, boron trifluoride, titanium tetrachloride, acetic acid, trifluoroacetic acid and the like to the system, or removing water generated in the system by using Dean-Stark apparatus and the like, or combining these.

Compound (21) which is compound (3) in reaction scheme 1 wherein -L-Y is -C(R³)(R⁴)-OH can be produced, for example, by a method shown in reaction scheme 3 or a method analogous thereto. 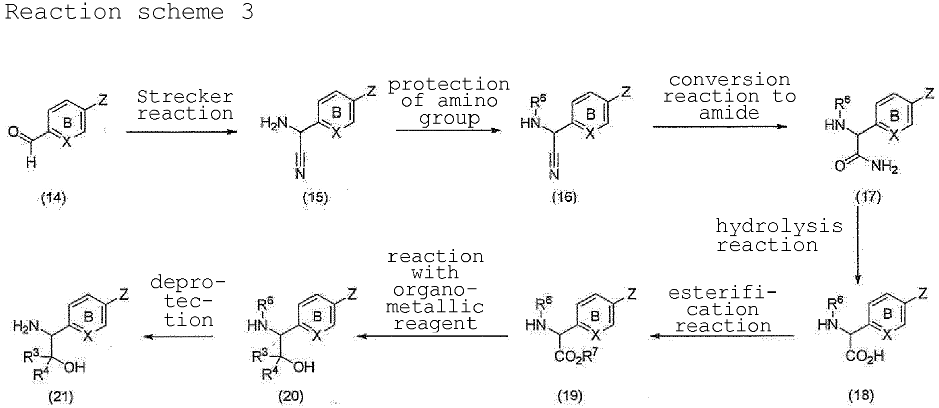 wherein R⁶ is an amino-protecting group, R⁷ is an optionally substituted C₁₋₆ alkyl group, and each of other symbols is as defined above.

Compound (14) can be obtained as a commercially available product, or can be produced by a method known per se or a method analogous thereto.

Compound (15) can be produced by subjecting compound (14) to a Strecker reaction.

In this reaction, generally, compound (14) and ammonia or an equivalent thereof, and hydrogen cyanide or an equivalent thereof are condensed to give a corresponding α-aminonitrile (15).

Examples of the ammonia equivalent include ammonium chloride, ammonium carbonate, benzylamine and the like. Examples of the hydrogen cyanide equivalent include sodium cyanide, potassium cyanide, trimethylsilyl cyanide and the like. This reaction may be performed by adding, where necessary, a Lewis acid such as titanium (IV) tetraisopropoxide and the like.

Compound (17) can be produced from compound (16).

This reaction can be performed by a method known per se, for example, according to the method described in Synthesis, vol. 12, page 949-950, 1989, or a method analogous thereto. For example, a reaction using potassium carbonate and an aqueous hydrogen peroxide solution and the like can be mentioned.

Compound (20) wherein both R³ and R⁴ are substituents can be produced by reacting compound (19) with an organometallic reagent for introducing a substituent defined by R³ and R⁴. Examples of such organometallic reagent include organomagnesium compounds, organolithium compounds and the like.

Compound (21) which is compound (3) in reaction scheme 1 wherein -L-Y is -C(R³)(R⁴)-OH can be produced, for example, by a method shown in reaction scheme 4 or a method analogous thereto. 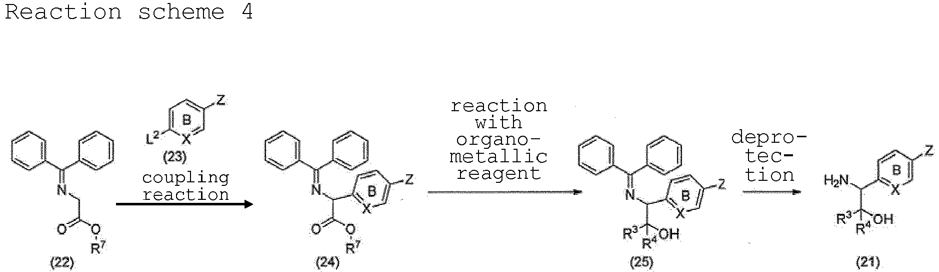 wherein L² is a leaving group, and each of other symbols is as defined above.

Compound (22) and compound (23) can be obtained as a commercially available product, or can be produced by a method known per se or a method analogous thereto.

Compound (24) can be produced by subjecting compound (22) and compound (23) to a coupling reaction. Examples of the transition metal catalyst to be used for this reaction include, besides the aforementioned palladium catalysts, palladium compounds such as bis(tri-tert-butylphosphine)palladium(0), tris(dibenzylideneacetone)dipalladium(0) and the like. Examples of the base include organic bases, inorganic bases, basic salts and the like. This reaction is also performed in the co-presence of a phosphine ligand. Examples of the phosphine ligand include tri-tert-butylphosphine and the like.

Compound (25) wherein both R³ and R⁴ are substituents can be produced by reacting compound (24) with an organometallic reagent for introducing a substituent defined by R³ and R⁴. Examples of such organometallic reagent include organomagnesium compounds, organolithium compounds and the like.

Compound (29) which is compound (3) in reaction scheme 1 wherein -L-Y is -CH₂CH₂-OR¹ can be produced, for example, by a method shown in reaction scheme 5 or a method analogous thereto. Reaction scheme 5 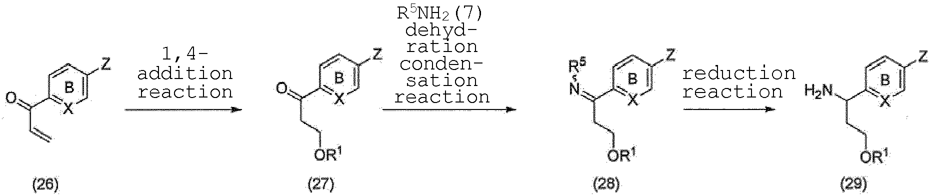 wherein each symbol is as defined above.

Compound (26) can be obtained as a commercially available product, or can be produced by a method known per se or a method analogous thereto.

Compound (27) can be produced by reacting compound (26) and a desired alcohol (R¹OH) in the presence of a palladium(II) catalyst.

Examples of such palladium(II) catalyst include bis(acetonitrile)palladium chloride, palladium chloride, palladium acetate and the like.

Compound (28) can be produced by a dehydration condensation reaction of compound (27) and compound (7). When compound (7) forms a salt, this reaction is generally performed by adding a base. Examples of such base include tertiary amines such as triethylamine, tripropylamine, tributylamine, diisopropylethylamine and the like, aromatic amines such as pyridine, 2,6-lutidine and the like, basic salts, inorganic bases, alkali metal hydrides, metal alkoxides and the like. This reaction can also be promoted by adding a dehydrating agent such as molecular sieve and the like, or p-toluenesulfonic acid, zinc chloride, phosphoryl chloride, boron trifluoride, titanium tetrachloride, acetic acid, trifluoroacetic acid and the like to the system, or removing water generated in the system by using Dean-Stark apparatus and the like, or combining these.

Of compounds (2) in reaction scheme 1, compound (32) can be produced by, for example, the method shown in reaction scheme 6 or a method analogous thereto. 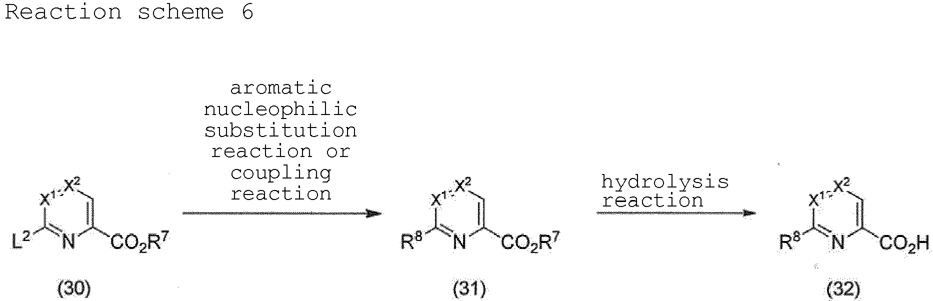 wherein X¹ is a group represented by CR⁹ (R⁹ is hydrogen atom, optionally substituted hydrocarbon group, optionally substituted C₁₋₆ alkoxy group, optionally substituted amino group, cyano group or optionally substituted heterocyclic group), or a nitrogen atom, X² is a group represented by CR¹⁰ (R¹⁰ is hydrogen atom, optionally substituted hydrocarbon group, optionally substituted C₁₋₆ alkoxy group, optionally substituted amino group, cyano group, or optionally substituted heterocyclic group), or a nitrogen atom, R⁸ is an optionally substituted hydrocarbon group, an optionally substituted C₁₋₆ alkoxy group, an optionally substituted C₁₋₆ alkylthio group, an optionally substituted C₆₋₁₄ aryloxy group, an optionally substituted 5- to 14-membered aromatic heterocyclyloxy group, an optionally substituted amino group, an optionally substituted C₆₋₁₄ aryl group, or an optionally substituted heterocyclic group, and each of other symbols is as defined above.

Compound (30) can be obtained as a commercially available product, or can be produced by a method known per se or a method analogous thereto.

Compound (31) can be produced by a coupling reaction or aromatic nucleophilic substitution reaction of compound (30). The coupling reaction is also performed in the co-presence of a phosphine ligand. Examples of the phosphine ligand include dicyclohexyl(2',4',6'-triisopropylbiphenyl-2-yl)phosphine, dicyclohexyl(2',6'-dimethoxybiphenyl-2-yl)phosphine, 4,5-bis(diphenylphosphino)-9,9-dimethylxanthene and the like.

Of compounds (1) in reaction scheme 1, compound (43) can be produced by, for example, the method shown in reaction scheme 7 or a method analogous thereto. 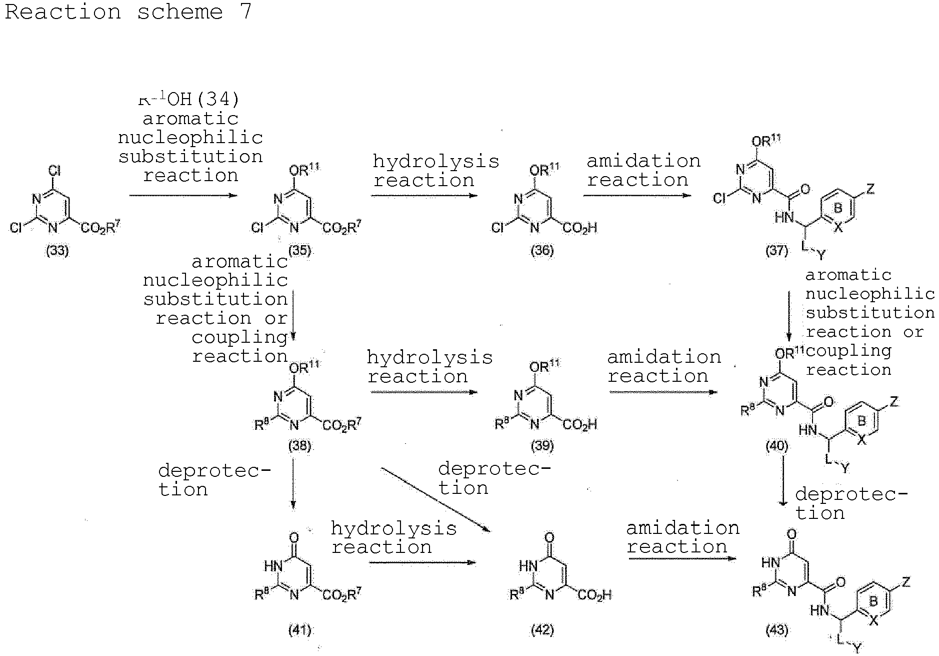 wherein R¹¹ is an optionally substituted C₁₋₆ alkyl group, and each of other symbols is as defined above.

Compound (33) and compound (34) can be obtained as a commercially available product, or can be produced by a method known per se or a method analogous thereto.

Compound (38) can be produced by a coupling reaction or aromatic nucleophilic substitution reaction of compound (35). The coupling reaction is also performed in the co-presence of a phosphine ligand. Examples of the phosphine ligand include dicyclohexyl(2',4',6'-triisopropylbiphenyl-2-yl)phosphine, dicyclohexyl(2',6'-dimethoxybiphenyl-2-yl)phosphine, 4,5-bis(diphenylphosphino)-9,9-dimethylxanthene and the like.

Compound (40) can be produced by a coupling reaction or aromatic nucleophilic substitution reaction of compound (37). The coupling reaction is also performed in the co-presence of a phosphine ligand. Examples of the phosphine ligand include dicyclohexyl(2',4',6'-triisopropylbiphenyl-2-yl)phosphine, dicyclohexyl(2',6'-dimethoxybiphenyl-2-yl)phosphine, 4,5-bis(diphenylphosphino)-9,9-dimethylxanthene and the like.

Of compounds (1) in reaction scheme 1, compound (47) can be produced by, for example, the method shown in reaction scheme 8 or a method analogous thereto. 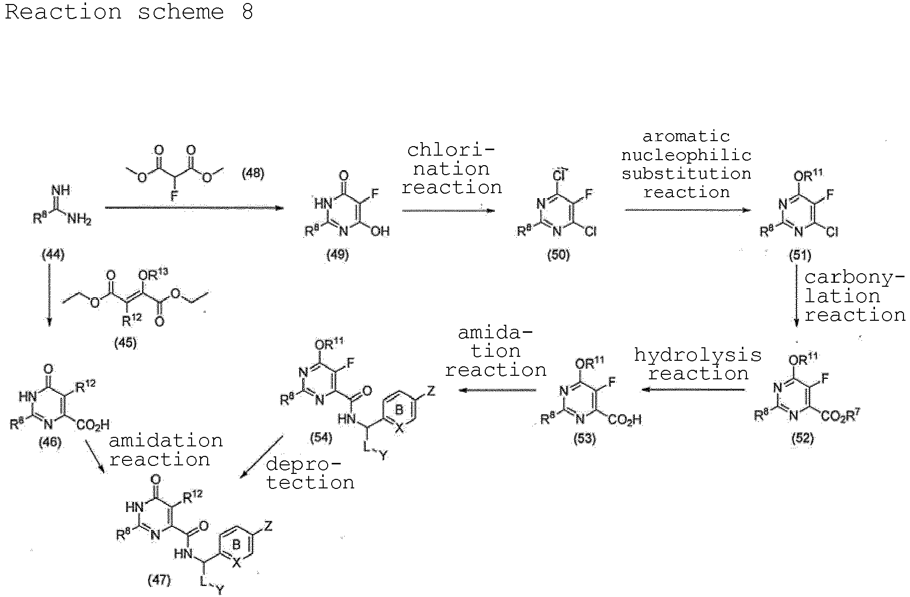 wherein R¹² is hydrogen or an optionally substituted C₁₋₆ alkyl group or a halogen atom, R¹³ is hydrogen or an alkali metal, and each of other symbols is as defined above.

Compound (44) and compound (45) can be obtained as a commercially available product, or can be produced by a method known per se or a method analogous thereto, and compound (48) can be obtained as a commercially available product.

Compound (46) can be produced by a pyrimidine ring cyclization reaction of compound (44) and compound (45). This reaction is performed using inorganic bases such as sodium hydroxide and the like. This reaction can also be performed using an acid catalyst, for example, Eaton reagent and the like.

Compound (49) can be produced by reacting compound (44) and compound (48) in the presence of metal alkoxides.

Compound (50) can be produced by reacting compound (49) with a chlorinating agent.

Examples of the chlorinating agent include phosphorus oxychloride, thionyl chloride and the like. This reaction can also be promoted by adding a base. As such base, tertiary amines such as triethylamine, diisopropylethylamine, N,N-dimethylaniline and the like, and the like can be mentioned.

This reaction can also be promoted by adding amides. As such amides, N,N-dimethylformamide and the like can be mentioned.

Compound (52) can be produced by reacting compound (51) and carbon monoxide and R⁷OH in the presence of a transition metal catalyst.

Examples of the transition metal catalyst include palladium(II) acetate, tris(dibenzylideneacetone)dipalladium(0), bis(tri-tert-butylphosphine)palladium(0), bis(triphenylphosphine)palladium(II) chloride, [1,1'-bis(diphenylphosphino)ferrocene]palladium(II) chloride and the like. This reaction is generally performed using a base. Examples of such base include basic salts such as sodium acetate and the like, tertiary amines such as triethylamine, diisopropylethylamine, N,N-dimethylaniline and the like, and the like.

This reaction is carried out under a carbon monoxide atmosphere. The carbon monoxide pressure is generally about 1 - 100 pressure, preferably about 1 - 20 pressure.

This reaction is also performed in the co-presence of a phosphine ligand. Examples of the phosphine ligand include tri-tert-butylphosphine, triphenylphosphine, 1,1'-bis(diphenylphosphino)ferrocene and the like.

This reaction is advantageously performed using a solvent inert to the reaction. While such solvent is not particularly limited as long as the reaction proceeds, for example, a solvent such as amides, hydrocarbons, alcohols corresponding to R⁷OH, ethers and the like, or a mixed solvent thereof and the like are preferable.

Of compounds (1) in reaction scheme 1, compound (60) can be produced by, for example, the method shown in reaction scheme 9 or a method analogous thereto. 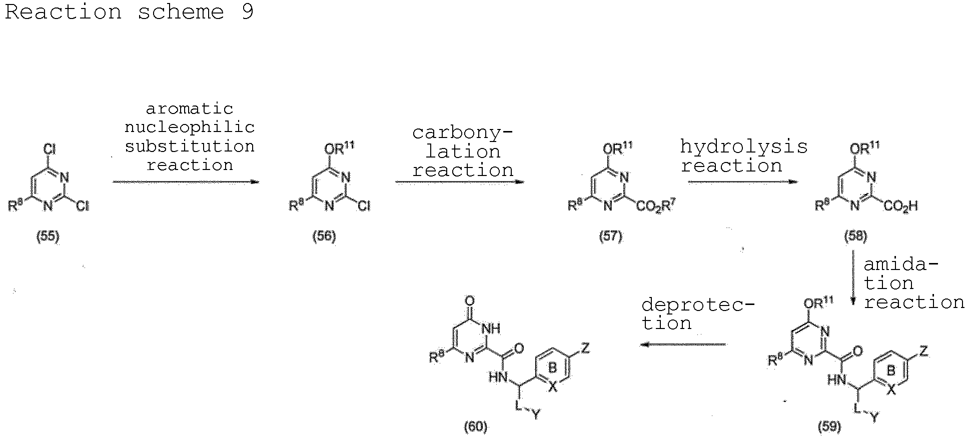 wherein each symbol is as defined above.

Compound (55) can be obtained as a commercially available product, or can be produced by a method known per se or a method analogous thereto.

Compound (57) can be produced by reacting compound (56) and carbon monoxide and R⁷OH in the presence of a transition metal catalyst.

Examples of the transition metal catalyst include palladium(II) acetate, tris(dibenzylideneacetone)dipalladium(0), bis(tri-tert-butylphosphine)palladium(0), bis(triphenylphosphine)palladium(II) chloride, [1,1'-bis(diphenylphosphino)ferrocene]palladium(II) chloride and the like. This reaction is generally performed using a base. Examples of such base include basic salts such as sodium acetate and the like, tertiary amines such as triethylamine, diisopropylethylamine, N,N-dimethylaniline and the like, and the like.

This reaction is performed under a carbon monoxide atmosphere. The carbon monoxide pressure is generally about 1 - 100 pressure, preferably about 1 - 20 pressure.

This reaction is also performed in the co-presence of a phosphine ligand. Examples of the phosphine ligand include tri-tert-butylphosphine, triphenylphosphine, 1,1'-bis(diphenylphosphino)ferrocene and the like.

This reaction is advantageously performed using a solvent inert to the reaction. While such solvent is not particularly limited as long as the reaction proceeds, for example, a solvent such as amides, hydrocarbons, alcohols corresponding to R⁷OH, ethers and the like, or a mixed solvent thereof and the like are preferable.

Compounds (I) obtained by each of the above-mentioned production methods can be isolated and purified by a known means such as concentration, concentrated under reduced pressure, solvent extraction, crystallization, recrystallization, phase transfer, chromatography and the like. Respective starting compounds used in each of the above-mentioned production methods can be isolated and purified by a known means similar to the aforementioned means. Alternatively, these starting compounds in the form of a reaction mixture may be directly used without isolation as a starting material for the next step.

When compound (I) contains an isomer such as an optical isomer, a stereoisomer, a regioisomer, a rotamer and the like, any isomer and a mixture thereof are also encompassed in compound (I). For example, when compound (I) has an optical isomer, an optical isomer resolved from racemate is also encompassed in compound (I). These isomers can be obtained as a single product by a synthetic means or a separation means known per se (e.g., concentration, solvent extraction, column chromatography, recrystallization etc.), optical resolution method (e.g., fractional recrystallization method, chiral column method, diastereomer method etc.) and the like.

As the optical resolution method, for example, a method known per se, for example, fractional recrystallization, chiral column method, diastereomer method and the like can be used.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallization method, and if desired, a neutralization step to give a free optical isomer.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENALTIO-OVM (manufactured by Tosoh Corporation), CHIRAL series (manufactured by Daicel Chemical Industries, Ltd.) and the like, and developed with water, various buffers (e.g., phosphate buffer) and organic solvents (e.g., ethanol, methanol, isopropanol, acetonitrile, trifluoroacetic acid, diethylamine) as an eluent, solely or in admixture of a mixture of the optical isomers to separate the optical isomers. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method etc.) and the like, and is subjected to a chemical treatment such as hydrolysis and the like to separate an optically active reagent moiety, whereby an optical isomer is obtained. For example, when compound (1) contains hydroxy group, or primary or secondary amino group within a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or the amide compound, respectively. When compound (1) has a carboxyl group, this compound and an optically active amine or an optically active alcohol reagent are subjected to condensation reaction to give diastereomers of the amide compound or the ester compound, respectively. The separated diastereomer is converted to an optical isomer of the original compound by acid hydrolysis or base hydrolysis.

Compound (I) may be a crystal. Even if compound (I) is in a single crystal form or mixed crystal form, it can be encompassed in compound (I) of the present invention. Crystal can be produced by crystallization by applying a crystallization method known per se.

Compound (I) may be a solvate (e.g., hydrate etc.) or a non-solvate (e.g., non-hydrate etc.), both of which are encompassed in compound (I).

Compound (I) may be a pharmaceutically acceptable co-crystal or a co-crystal salt. As used herein, co-crystal or co-crystal salt means a crystalline substance constituted of two or more distinct solids at room temperature, each of which has different physical properties (e.g., structure, melting point, melting heat, hygroscopicity, solubility and stability etc.). The co-crystal or co-crystal salt can be produced by a co-crystallization method known per se.

Compound (I) also encompasses a compound labeled or substituted with an isotope (e.g., ²H, ³H, ¹¹C, ¹⁴C, ¹⁸F, ³⁵S, ¹²⁵I etc.) and the like.

Compound (I) also encompasses a deuterium conversion form wherein ¹H is converted to ²H(D).

Compound (I) labeled or substituted with an isotope can be used, for example, as a tracer (PET tracer) used for positron emission tomography (PET), and is useful in the field such as medical diagnosis and the like.

Since the compound of the present invention has a superior PDE2A inhibitory action, shows low toxicity (e.g., phototoxicity, acute toxicity, chronic toxicity, genetic toxicity, reproductive toxicity, cardiac toxicity, drug interaction, carcinogenicity etc., particularly phototoxicity), is superior in stability (particularly, metabolic stability) and in vivo kinetics (absorbability, distribution, metabolism, excretion etc.), and further shows high solubility, it is useful as a pharmaceutical product. The compound of the present invention has a PDE2A inhibitory action on mammals (e.g., mouse, rat, hamster, rabbit, cat, dog, bovine, horse, sheep, monkey, human etc.), and can be used as a prophylactic or therapeutic drug for the following diseases and symptoms:
(1) psychotic disorder (e.g., brief psychotic disorder, shared psychotic disorder),
(2) psychosis induced by alcohol, amphetamine, cannabis, cocaine, hallucinogens, obesity, inhalants, opioids or phencyclidine,
(3) delusional disorder,
(4) anxiety disorder,
(5) movement disorder,
(6) mood disorder,
(7) major depressive disorder,
(8) major depressive disorder superimposed on a psychotic disorder (including delusional disorder and schizophrenia),
(9) major depressive episode of the mild, moderate or severe type,
(10) manic or mixed mood episode,
(11) hypomanic mood episode,
(12) depressive episode with atypical features,
(13) depressive episode with melancholic features,
(14) depressive episode with catatonic features,
(15) mood episode with postpartum onset;
(16) post-stroke depression,
(17) dysthymic disorder,
(18) minor depressive disorder,
(19) autism;
(20) drug addiction,
(21) neurodegenerative disorder,
(22) neurodegeneration associated with cerebral trauma,
(23) neurodegeneration associated with stroke,
(24) neurodegeneration associated with cerebral infarct,
(25) neurodegeneration associated with hypoglycemia,
(26) neurodegeneration associated with epileptic seizure,
(27) neurodegeneration associated with neurotoxin poisoning,
(28) multi-system atrophy,
(29) Alzheimer's disease,
(30) dementia,
(31) multi-infarct dementia,
(32) alcoholic dementia or other drug-related dementia,
(33) dementia associated with intracranial tumors or cerebral trauma,
(34) dementia associated with Huntington's disease or Parkinson's disease,
(35) AIDS-related dementia,
(36) frontotemperal lobe dementia,
(37) delirium,
(38) amnestic disorder,
(39) post-traumatic stress disorder,
(40) mental retardation,
(41) learning disorder (e.g., reading disorder, mathematics disorder, or a disorder of written expression),
(42) attention-deficit/hyperactivity disorder;
(43) age-related cognitive decline,
(44) premenstrual dysphoric disorder,
(45) post-psychotic depressive disorder of schizophrenia,
(46) bipolar disorder (including bipolar I disorder and bipolar II disorder),
(47) cyclothymic disorder,
(48) Parkinson's disease,
(49) Huntington's disease,
(50) paranoia,
(51) schizophrenia (positive symptom, a negative symptom, cognitive functional disorder as main symptoms) (e.g., paranoid schizophrenia, disorganized schizophrenia, catatonic schizophrenia, undifferentiated schizophrenia, residual schizophrenia),
(52) schizophreniform disorder,
(53) schizoaffective disorder of the delusional type or the depressive type,
(54) personality disorder of the paranoid type,
(55) personality disorder of the schizoid type,
(56) obesity,
(57) metabolic syndrome,
(58) non-insulin dependent diabetes (NIDDM),
(59) glucose intolerance,
(60) pneumonia,
(61) osteoarthritis,
(62) acute lung disorder,
(63) migraine headache,
(64) fragile X syndrome.

Particularly, the compound of the present invention is useful for the prophylaxis or treatment of schizophrenia and Alzheimer's disease.

Since the compound of the present invention is superior in metabolic stability, it is expected to show a superior treatment effect on the above-mentioned diseases even at a low dose. Moreover, the compound of the present invention is superior in penetration into the brain.

Since the compound of the present invention shows low toxicity, a pharmaceutical composition containing the compound of the present invention (hereinafter to be referred to as "the medicament of the present invention") can be safely administered solely or by mixing with a pharmacologically acceptable carrier according to a method known per se (e.g., the method described in the Japanese Pharmacopoeia etc.) as the production method of a pharmaceutical preparation, and in the form of, for example, tablet (including sugar-coated tablet, film-coated tablet, sublingual tablet, orally disintegrating tablet, buccal and the like), pill, powder, granule, capsule (including soft capsule, microcapsule), troche, syrup, liquid, emulsion, suspension, release control preparation (e.g., immediate-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal and intratumor administrations, administration to the vicinity of tumor, and direct administration to the lesion).

The above-mentioned "pharmacologically acceptable carrier" may be exemplified by various organic or inorganic carrier materials that are conventionally used as preparation materials, for example, excipient, lubricant, binding agent and disintegrant for solid preparations; or solvent, solubilizing agent, suspending agent, isotonic agent, buffering agent, soothing agent and the like for liquid preparations. Furthermore, when necessary, ordinary additives such as preservative, antioxidant, colorant, sweetening agent, adsorbing agent, wetting agent and the like can also be used as appropriate in an appropriate amount.

Examples of the excipient include lactose, sucrose, D-mannitol, D-sorbitol, starch, α-starch, cornstarch, dextrin, crystalline cellulose, low-substituted hydroxypropylcellulose, sodium carboxymethylcellulose, gum arabic, pullulan, light anhydrous silicic acid, synthetic aluminum silicate, magnesium aluminometasilicate and the like.

Examples of the lubricant include magnesium stearate, calcium stearate, talc, colloidal silica and the like.

Examples of the binder include α-starch, crystalline cellulose, sucrose, gum arabic, D-mannitol, trehalose, dextrin, pullulan, hydroxypropylcellulose, hydroxypropylmethylcellulose, polyvinylpyrrolidone, starch, sucrose, gelatin, methylcellulose, carboxymethylcellulose, carboxymethylcellulose sodium and the like.

Examples of the disintegrant include lactose, sucrose, starch, carboxymethylcellulose, carboxymethylcellulose calcium, croscarmellose sodium, carboxymethylstarch sodium, light anhydrous silicic acid, low-substituted hydroxypropylcellulose and the like.

Examples of the solvent include water for injection, physiological saline, Ringer's injection, alcohol, propylene glycol, polyethylene glycol, macrogol, sesame oil, corn oil, olive oil, cottonseed oil and the like.

Examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose, polysorbate, polyoxyethylene hydrogenated castor oil and the like; and the like.

Examples of the isotonic agent include glucose, D-sorbitol, sodium chloride, glycerin, D-mannitol and the like.

Examples of the buffering agent include buffer solutions such as phosphate salts, acetate salts, carbonate salts, citrate salts and the like.

Examples of the soothing agent include benzyl alcohol and the like.

Examples of the preservative include parahydroxybenzoates, chlorobutanol, benzyl alcohol, phenethyl alcohol, dehydroacetic acid, sorbic acid and the like.

Examples of the antioxidant include sulfite salts, ascorbic acid, α-tocopherol and the like.

Examples of the colorant include water-soluble food tar color (e.g., Food Color Red No. 2 and No. 3, Food Color Yellow No. 4 and No. 5, Food Color Blue No. 1 and No. 2 etc.), water-insoluble lake dye (e.g., aluminum salt of the aforementioned water-soluble food tar color), natural dye (e.g., β-carotene, chlorophyll, ferric oxide red) and the like.

Examples of the sweetening agent include saccharin sodium, dipotassium glycyrrhizinate, aspartame, stevia and the like.

While the content of the compound of the present invention in the medicament of the present invention varies depending on the dosage form, dose of the compound of the present invention and the like, it is, for example, about 0.01 - 100 wt%, preferably about 0.1 - 95 wt%, of the whole medicament.

While the dose of the compound of the present invention varies depending on the subject of administration, administration route, target disease, symptom and the like, it is generally about 0.1 - about 20 mg/kg body weight, preferably about 0.2 - about 10 mg/kg body weight, more preferably about 0.5 - about 10 mg/kg body weight, for oral administration to schizophrenia patients (adult, about 60 kg body weight), and the dose is desirably administered in about one to several (e.g., 1 - 3) portions per day according to the symptoms.

The compound of the present invention can be administered as a single active substance, or can be administered in combination with other medicaments such as other drugs used in the treatment of psychotic disorder (particularly schizophrenia and bipolar disorder), obsessive-compulsive disorder, major depression, Parkinson's disease, Alzheimer's disease, cognitive disorder, memory loss and the like (hereinafter to be abbreviated as concomitant drug).

Examples of the concomitant drug include nicotinic α7 agonists, nicotinic α7 partial agonists, nicotinic α7 positive allosteric modulators, PDE2 inhibitors, PDE4 inhibitors, PDE5 inhibitors, PDE10 inhibitors, other PDE inhibitors, calcium channel blockers, muscarinic m1 and m2 modulators, adenosine receptor modulators, ampakines, Glycine transporter 1 inhibitors, NMDA-R modulators, mGluR modulators, dopamine modulators, serotonin modulators, selective serotonin reuptake inhibitors, serotonin and norepinephrine reuptake inhibitors, norepinephrine and dopamine reuptake inhibitors, triple reuptake inhibitors, cannabinoid modulators, cholinesterase inhibitors (e.g., donepezil, rivastigmine, galanthamine) and the like.

In addition, examples of the concomitant drug include, but are not limited to, other suitable schizophrenia drugs (e.g., haloperidol, clozapine, olanzapine, risperidone, aripiprazole, ziprasidone, paliperidone, quetiapine fumarate etc.), bipolar disorder drug (e.g., lithium, olanzapine, aripiprazole, valproic acid etc.), Parkinson's disease drugs (e.g., levodopa, bromocriptine, pergolide, pramipexole, tolcapone, procyclidine, trihexyphenidyl, benztropine etc.), agents used in the treatment of major depression (e.g., amitriptyline, imipramine, desipramine, nortriptyline, paroxetine, fluoxetine, sertraline, bupropion, escitalopram, mirtazapine, venlafaxine, duloxetine etc.), agents used in the treatment of Alzheimer's disease (e.g., galanthamine, tacrine, donepezil, rivastigmine, memantine, neotropin, selegiline, estrogen, clioquinol etc.), agents used in the treatment of dementia (e.g., thioridazine, haloperidol, risperidone, tacrine, donepezil, rivastigmine etc.), agents used in the treatment of epilepsy (e.g., phenytoin, phenobarbital, carbamazepine, valproic acid, ethosuximide, gabapentin, solfeton, felbatol etc.), agents used in the treatment of multiple sclerosis (e.g., tolterodine, oxybutynin, oxycodone, interferon beta-1b, interferon beta-1a, azathioprine, methotrexate, glatiramer etc.), agents used in the treatment of Huntington's disease (e.g., amitriptyline, imipramine, desipramine, nortriptyline, paroxetine, fluoxetine, sertraline, tetrabenazine, haloperidol, chlorpromazine, thioridazine, sulpiride, quetiapine, clozapine, risperidone etc.), agents used in the treatment of diabetes [e.g., PPAR ligands (e.g. agonists or antagonists such as rosiglitazone, troglitazone, pioglitazone etc.), insulin secretagogues (e.g., sulfonylurea drugs such as glyburide, glimepiride, chlopropamide, tolbutamide, glipizide etc., and non-sulfonyl secretagogues etc.), α-glucosidase inhibitors (e.g., acarbose, miglitol, voglibose etc), insulin sensitizers (e.g., PPAR-γ agonists such as glitazones etc.; biguanides, PTP-1b inhibitors, DPP-iv inhibitors, 11 beta-HSD inhibitors etc.), hepatic glucose output lowering compounds (e.g., glucagon antagonists and metformin such as glucophage, glucophage XR etc.), insulin and insulin derivatives (including both long and short acting forms of insulin and insulin formulations)], antiobesity drugs [e.g., β-3 agonists, CB-1 agonists, neuropeptide Y5 inhibitors, ciliary neurotrophic factor and derivatives (e.g., axokine), appetite suppressants (e.g., sibutramine), lipase inhibitors (e.g., orlistat) etc.].

The administration form of the combination drug of the present invention is not particularly limited, and the compound of the present invention and a concomitant drug only need to be combined on administration. Examples of such administration mode include the following:
(1) administration of a single preparation obtained by simultaneously processing the compound of the present invention and the concomitant drug,
(2) simultaneous administration of two kinds of preparations of the compound of the present invention and the concomitant drug, which have been separately produced, by the same administration route,
(3) administration of two kinds of preparations of the compound of the present invention and the concomitant drug, which have been separately produced, by the same administration route in a staggered manner,
(4) simultaneous administration of two kinds of preparations of the compound of the present invention and the concomitant drug, which have been separately produced, by different administration routes,
(5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

These administration forms are summarized below, and abbreviated as the concomitant drug of the present invention.

When the combination drug of the present invention is administered, the concomitant drug and the compound of the present invention can be administered simultaneously. In addition, the compound of the present invention can be administered after administration of the concomitant drug administration, and the concomitant drug can be administered after administration of the compound of the present invention. When administered in a staggered manner, the administration time varies depending on the active ingredient to be administered, dosage form and administration method.

For example, when the concomitant drug or a pharmaceutical composition thereof is to be administered first, the compound of the present invention or a pharmaceutical composition thereof can be administered within 1 min to 3 days, preferably within 10 min to 1 day, more preferably within 15 min to 1 hour after administration of the concomitant drug or a pharmaceutical composition thereof. In addition, when the compound of the present invention or a pharmaceutical composition thereof is to be administered first, the concomitant drug or a pharmaceutical composition thereof can be administered within 1 min to 1 day, preferably within 10 min to 6 hours, more preferably within 15 min to 1 hour after administration of the compound of the present invention or a pharmaceutical composition thereof.

When the concomitant drug does not pose problems of side effects, it can be administered at any dose. While the dose of the concomitant drug varies depending on the dosage form, subject of administration, administration route, target disease, symptom and the like, it is, for example, generally about 0.1 - about 20 mg/kg body weight, preferably about 0.2 - about 10 mg/kg body weight, more preferably about 0.5 - about 10 mg/kg body weight for oral administration to schizophrenia patients (adult, about 60 kg body weight), and the dose is desirably administered in about one to several (e.g., 1 - 3) portions per day according to the symptoms.

When the compound of the present invention is used in combination with a concomitant drug, the dose thereof can be reduced within the safe range in consideration of the opposite effects of the both drugs.

The combination drug of the present invention shows low toxicity and, for example, the compound of the present invention or(and) the above-mentioned concomitant drug may be mixed with a pharmacologically acceptable carrier according to a method known per se to give a pharmaceutical composition, for example, tablet (including sugar-coated tablet, film-coated tablet and the like), powder, granule, capsule (including soft capsule), liquid, emulsion, suspension, injection, suppository, sustained-release preparation (e.g., sublingual tablet, microcapsule etc.), plaster, orally disintegrating tablet, orally disintegrable film and the like, which can be safely administered orally or parenterally (e.g., subcutaneous, topical, rectal, intravenous administration etc.).

The pharmaceutically acceptable carriers that can be used for manufacturing the combination drug of the present invention can be the same as those used in the medicament of the present invention as mentioned above.

A mixing ratio between the compound of the present invention and the concomitant drug in the combination drug of the present invention can be selected appropriately based on the administration subjects, administration routes, target diseases and the like.

The concomitant drug in the combination drug of the present invention can be combined at an appropriate proportion if two or more drugs are combined.

A dosage of the concomitant drug can be selected appropriately based on the dosages used clinically. In addition, a mixing ratio between the compound of the present invention and the concomitant drug can be selected appropriately based on the administration subjects, administration routes, target diseases, symptoms, combinations, etc. For example, if the administration subject is humans, a concomitant drug may be used in an amount ranging from about 0.01 to 100 parts by weight relative to 1 part by weight of the compound of the present invention.

For example, while the content of the compound of the present invention in the combination drug of the present invention varies depending on the preparation form, it is generally about 0.01 - 99.9 wt%, preferably about 0.1 - 50 wt%, more preferably about 0.5 - 20 wt%, of the whole preparation.

The content of the concomitant drug in the combination drug of the present invention varies depending on the preparation form, and is generally about 0.01 to 99.9% by weight, preferably about 0.1 to 50% by weight, further preferably about 0.5 to 20% by weight, of the whole preparation.

While the content of the additive such as a carrier and the like in the combination drug of the present invention varies depending on the form of a preparation, it is generally about 1 to 99.99% by weight, preferably about 10 to 90% by weight, based on the whole preparation.

When the compound of the present invention and the concomitant drug are separately prepared, the same content may be adopted.

Since the dosage may vary under various conditions as mentioned above, a dosage less than the dosages may be sufficient or it may be necessary to administer at a dosage exceeding the above-mentioned ranges.

### Examples

The present invention is explained in detail in the following by referring to Examples, Experimental Examples and Formulation Examples, which do not limit the present invention and the invention may be changed within the scope of the present invention.

In the following Examples, "room temperature" indicates generally approximately 10°C to 35°C. The ratios indicated for mixed solvents are volume mixing ratios, unless otherwise specified. % means wt%, unless otherwise specified.

In silica gel column chromatography, NH means use of aminopropylsilane-bonded silica gel, and Diol means use of 3-(2,3-dihydroxypropoxy)propylsilane-bonded silica gel. In HPLC (high performance liquid chromatography), C18 means use of octadecyl-bonded silica gel. The ratios of elution solvents are volume mixing ratios, unless otherwise specified.

In the following Examples, the following abbreviations are used.
MS: mass spectrum
[M+H]⁺, [M-H]⁻: molecular ion peak
M: mol concentration
N: N
CDCl₃: deuterated chloroform
DMSO-d₆: deuterated dimethyl sulfoxide
CD₃OD: deuterated methanol
¹H NMR: proton nuclear magnetic resonance
LC/MS: liquid chromatography mass spectrometer
ESI: ElectroSpray Ionization
APCI: Atomospheric Pressure Chemical Ionization

¹H NMR was measured by Fourier-transform type NMR. For the analysis, ACD/SpecManager (trade name) and the like were used. Peaks with very mild protons such as hydroxyl group, amino group and the like are not described.

MS was measured by LC/MS. As the ionization method, ESI method or API method was used. The data indicate measured values (found). Generally, a molecular ion peak is observed. In the case of a compound having a tert-butoxycarbonyl group, a peak after elimination of tert-butoxycarbonyl group or tert-butyl group may be observed as a fragment ion. In the case of a compound having a hydroxy group, a peak after elimination of water (H₂O) may be observed as a fragment ion. In the case of a salt, a molecular ion peak or fragment ion peak of free form is generally observed. In the case of a compound having an amino group (NH₂), a peak after elimination of amino group is sometimes observed as a fragment ion.

### Example 1

### N-(2-methoxy-1-(4-(trifluoromethoxy)phenyl)ethyl)-2-phenyl-1,3-thiazole-4-carboxamide

### A) 2-methoxy-1-(4-(trifluoromethoxy)phenyl)ethanone

To a solution of 2-bromo-1-(4-(trifluoromethoxy)phenyl)ethanone (6.12 g) in methanol (150 mL) were added silver carbonate (I) (7.75 g) and boron trifluoride diethyl ether complex (3.29 mL) at room temperature. The reaction mixture was stirred at 50°C for 20 hr. Insoluble material was filtered off, and the filtrate was concentrated under reduced pressure. Water was added to the residue, and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (4.78 g).
MS: [M+H]⁺ 235.1.

### B) 2-methoxy-1-(4-(trifluoromethoxy)phenyl)ethanamine hydrochloride

To a solution of 2-methoxy-1-(4-(trifluoromethoxy)phenyl)ethanone (4.78 g) in ethanol (120 mL) were added hydroxylamine hydrochloride (2.84 g) and triethylamine (5.69 mL) at room temperature. The reaction mixture was stirred at room temperature for 5.5 hr and the solvent was evaporated under reduced pressure. Water was added to the residue and the mixture was extracted with ethyl acetate. The extract was washed with water and saturated brine, and dried over anhydrous magnesium sulfate. The solvent was evaporated under reduced pressure to give N-hydroxy-2-methoxy-1-(4-(trifluoromethoxy)phenyl)ethanimine as a crude product. To a solution of the obtained crude product in ethanol (160 mL) was added 10% palladium-carbon (containing water (50%), 350 mg). The reaction mixture was stirred under a hydrogen atmosphere, at room temperature for 16 hr. The catalyst was filtered off, and the filtrate was distilled under reduced pressure. To a solution of the residue in ethyl acetate (10 mL) was added 4 M hydrogen chloride/ethyl acetate solution (100 mL), and the mixture was stirred at room temperature for 2 hr. The solvent was evaporated under reduced pressure. The residue was washed with diisopropyl ether to give the title compound (4.2 g).
¹H NMR (300 MHz, DMSO-d₆) δ 3.34 (3H, s), 3.58-3.74 (2H, m), 4.58 (1H, dd, J = 6.6, 5.5 Hz), 7.46 (2H, d, J = 8.7 Hz), 7.65 (2H, d, J = 8.3 Hz), 8.57 (3H, brs).

### C) N-(2-methoxy-1-(4-(trifluoromethoxy)phenyl)ethyl)-2-phenyl-1,3-thiazole-4-carboxamide

To a solution of 2-phenyl-1,3-thiazole-4-carboxylic acid (92 mg) and 2-methoxy-1-(4-(trifluoromethoxy)phenyl)ethanamine hydrochloride (122 mg) in N,N-dimethylformamide (5.0 mL) were added 1-(3-dimethylaminopropyl)-3-ethylcarbodiimide hydrochloride (104 mg), 1-hydroxybenzotriazole (73 mg) and triethylamine (0.12 mL) at room temperature. The reaction mixture was stirred at room temperature overnight, poured into water, and the mixture was extracted with ethyl acetate. The extract was washed with water and saturated brine, and dried over anhydrous magnesium sulfate. The solvent was evaporated under reduced pressure. The residue was purified by silica gel column chromatography (NH, ethyl acetate/hexane) to give the title compound (132 mg).
¹H NMR (300 MHz, DMSO-d₆) δ 3.32 (3H, s), 3.66 (1H, dd, J = 10.0, 5.5 Hz), 3.83 (1H, dd, J = 9.8, 7.9 Hz), 5.22-5.45 (1H, m), 7.35 (2H, d, J = 7.9 Hz), 7.52-7.63 (5H, m), 8.09 (2H, dd, J = 6.4, 3.0 Hz), 8.32 (1H, s), 8.84 (1H, d, J = 8.7 Hz).

### Example 2

### 2-anilino-N-(2-methoxy-1-(4-(trifluoromethoxy)phenyl)ethyl)pyrimidine-4-carboxamide

### A) 2-anilinopyrimidine-4-carboxylic acid

A mixture of 2-chloropyrimidine-4-carboxylic acid (200 mg), aniline (0.115 mL), 6 M hydrochloric acid (0.053 mL) and dimethyl sulfoxide (2.0 mL) was stirred at 80°C overnight. The reaction mixture was cooled to room temperature, water was added, and the precipitated solid was collected by filtration. The solid was washed with water to give the title compound (112.1 mg).
¹H NMR (300 MHz, DMSO-d₆) δ 6.93-7.00 (1H, m), 7.25-7.33 (3H, m), 7.80-7.86 (2H, m), 8.70 (1H, d, J = 5.1 Hz), 9.96 (1H, s), 13.54-13.82 (1H, m).

### B) 2-anilino-N-(2-methoxy-1-(4-(trifluoromethoxy)phenyl)ethyl)pyrimidine-4-carboxamide

By a method similar to that of Example 1, step C, the title compound was obtained.

### Example 3

### N-(2-methoxy-1-(4-(trifluoromethoxy)phenyl)ethyl)-2-((1-methyl-1H-pyrazol-4-yl)amino)pyrimidine-4-carboxamide

### A) 2-((1-methyl-1H-pyrazol-4-yl)amino)pyrimidine-4-carboxylic acid

A mixture of 2-chloropyrimidine-4-carboxylic acid (200 mg), 1-methyl-1H-pyrazole-4-amine hydrochloride (147 mg), 6 M hydrochloric acid (0.105 mL) and 1,2-dimethoxyethane (3.0 mL) was stirred at 80°C overnight. To the reaction mixture was added water at room temperature, and the precipitated solid was collected by filtration to give the title compound (59.3 mg). ¹H NMR (300 MHz, DMSO-d₆) δ 3.80 (3H, s), 7.18 (1H, d, J = 4.8 Hz), 7.53 (1H, brs), 7.93 (1H, s), 8.63 (1H, d, J = 4.8 Hz), 9.84 (1H, brs), 13.58 (1H, brs).

### B) N-(2-methoxy-1-(4-(trifluoromethoxy)phenyl)ethyl)-2-((1-methyl-1H-pyrazol-4-yl)amino)pyrimidine-4-carboxamide

By a method similar to that of Example 1, step C, the title compound was obtained.

### Example 4

### 2-anilino-6-methoxy-N-(2-methoxy-1-(4-(trifluoromethoxy)phenyl)ethyl)pyrimidine-4-carboxamide

### A) methyl 2-chloro-6-methoxypyrimidine-4-carboxylate

To a solution of methyl 2,4-dichloropyrimidine-6-carboxylate (1.24 g) in acetonitrile (20.0 mL) and methanol (5.0 mL) was added potassium carbonate (2.50 g). The reaction mixture was stirred at 70°C overnight, and the precipitate was filtered off. The filtrate was distilled under reduced pressure to give the title compound (1.00 g).
¹H NMR (300 MHz, DMSO-d₆) δ 4.00 (3H, s), 4.08 (3H, s), 7.37 (1H, s).

### B) methyl 2-anilino-6-methoxypyrimidine-4-carboxylate

By a method similar to that of Example 2, step A, the title compound was obtained.
MS: [M+H]⁺ 260.1.

### C) 2-anilino-6-methoxypyrimidine-4-carboxylic acid

To a mixture of 2-anilino-6-methoxypyrimidine-4-carboxylic acid methyl (49.3 mg), methanol (5.0 mL) and tetrahydrofuran (5.0 mL) was added 2 M aqueous sodium hydroxide solution (0.19 mL) at room temperature. The mixture was stirred at room temperature overnight, neutralized with 1 M hydrochloric acid at 0°C, and extracted with ethyl acetate. The extract was washed with water and saturated brine, and dried over anhydrous magnesium sulfate. The solvent was evaporated under reduced pressure to give the title compound (40 mg).
MS: [M+H]⁺ 246.1.

### D) 2-anilino-6-methoxy-N-(2-methoxy-1-(4-(trifluoromethoxy)phenyl)ethyl)pyrimidine-4-carboxamide

By a method similar to that of Example 1, step C, the title compound was obtained.

### Example 5

### N-(2-methoxy-1-(4-(trifluoromethoxy)phenyl)ethyl)pyrimidine-4-carboxamide

By a method similar to that of Example 1, step C, the title compound was obtained.

### Example 6

### 2-anilino-N-(2-methoxy-1-(4-(trifluoromethoxy)phenyl)ethyl)-6-oxo-1,6-dihydropyrimidine-4-carboxamide

### A) butyl 2-anilino-6-oxo-1,6-dihydropyrimidine-4-carboxylate

To a solution of methyl 2-chloro-6-methoxypyrimidine-4-carboxylate (1.01 g) in n-butanol (20.0 mL) were added aniline (0.46 mL) and 6 M hydrochloric acid (0.42 mL), and the mixture was stirred at 120°C overnight. To the reaction mixture was added water, and the mixture was extracted with ethyl acetate. The extract was washed with water and saturated brine, and dried over anhydrous magnesium sulfate. The solvent was evaporated under reduced pressure. The residue was crystallized from ethyl acetate/isopropyl ether to give the title compound (540 mg).
MS: [M+H]⁺ 288.1.

### B) 2-anilino-6-oxo-1,6-dihydropyrimidine-4-carboxylic acid

By a method similar to that of Example 4, step C, the title compound was obtained.
¹H NMR (300 MHz, DMSO-d₆) δ 6.36 (1H, brs), 6.98-7.11 (1H, m), 7.32 (2H, t, J = 7.9 Hz), 7.71 (2H, d, J = 7.9 Hz), 9.08 (1H, brs), 11.06 (1H, brs), 13.31 (1H, brs).

### C) 2-anilino-N-(2-methoxy-1-(4-(trifluoromethoxy)phenyl)ethyl)-6-oxo-1,6-dihydropyrimidine-4-carboxamide

By a method similar to that of Example 1, step C, the title compound was obtained.

### Example 7

### 2-anilino-N-(2-methoxy-1-(4-(trifluoromethoxy)phenyl)ethyl)-6-methylpyrimidine-4-carboxamide

By a method similar to that of Example 6, step A, Example 4, step C and Example 1, step C, the title compound was obtained.

### Example 8

### 5-amino-N-(2-methoxy-1-(4-(trifluoromethoxy)phenyl)ethyl)pyrimidine-4-carboxamide

By a method similar to that of Example 1, step C, the title compound was obtained.

### Example 9

### 2-(benzylamino)-N-(2-methoxy-1-(4-(trifluoromethoxy)phenyl)ethyl)pyrimidine-4-carboxamide

### A) 2-chloro-N-(2-methoxy-1-(4-(trifluoromethoxy)phenyl)ethyl)pyrimidine-4-carboxamide

To a solution of 2-chloropyrimidine-4-carboxylic acid (100 mg) in tetrahydrofuran (5.0 mL) were added a catalytic amount of N,N-dimethylformamide and oxalyl chloride (0.108 mL) at room temperature. After stirring at room temperature for 1 hr, the solvent was evaporated under reduced pressure. To a mixture of the residue and tetrahydrofuran (5.0 mL) were added 2-methoxy-1-(4-(trifluoromethoxy)phenyl)ethanamine hydrochloride (206 mg) and diisopropylethylamine (0.33 mL) at room temperature. After stirring at room temperature for 3 hr, water was added to the reaction mixture, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (227 mg).
MS: [M-H]- 374.1.

### B) 2-(benzylamino)-N-(2-methoxy-1-(4-(trifluoromethoxy)phenyl)ethyl)pyrimidine-4-carboxamide

A mixture of 2-chloro-N-(2-methoxy-1-(4-(trifluoromethoxy)phenyl)ethyl)pyrimidine-4-carboxamide (50 mg), benzylamine (0.022 mL), triethylamine (0.037 mL) and acetonitrile (5.0 mL) was stirred under a nitrogen atmosphere at 90°C for 2 days. To the reaction mixture was added an aqueous sodium hydrogen carbonate solution, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (50.2 mg).
¹H NMR (300 MHz, CDCl₃) δ 3.39 (3H, s), 3.71 (2H, d, J = 4.8 Hz), 4.66 (2H, d, J = 6.0 Hz), 5.22-5.30 (1H, m), 5.59-5.77 (1H, m), 7.16 (2H, d, J = 7.8 Hz), 7.28-7.41 (8H, m), 8.43-8.53 (2H, m).

### Example 10

### N-(2-methoxy-1-(4-(trifluoromethoxy)phenyl)ethyl)-5-(methylamino)pyrimidine-4-carboxamide

To a mixture of 5-amino-N-(2-methoxy-1-(4-(trifluoromethoxy)phenyl)ethyl)pyrimidine-4-carboxamide (120 mg) and ethanol (10 mL) was added a formalin solution (61 mg) at room temperature. The mixture was stirred at 50°C for 3 hr, cooled to room temperature, sodium borohydride (250 mg) was added and the mixture was stirred again at 50°C for 2 hr. The mixture was cooled to room temperature, water was added, a volatile compound was removed under reduced pressure, and the organic product was extracted with ethyl acetate. The ethyl acetate layer was dried over anhydrous sodium sulfate, and concentrated under reduced pressure. The residue was fractionated by HPLC (C18, mobile phase: water/acetonitrile (0.05% TF-Acontaining system). To the obtained fraction was added a saturated aqueous sodium hydrogen carbonate solution, and the mixture was extracted with ethyl acetate, dried over anhydrous magnesium sulfate, and concentrated under reduced pressure to give the title compound (50 mg).

### Example 11

### 2-anilino-6-chloro-N-(2-methoxy-1-(4-(trifluoromethoxy)phenyl)ethyl)pyrimidine-4-carboxamide

### A) butyl 2-anilino-6-chloropyrimidine-4-carboxylate

To butyl 2-anilino-6-oxo-1,6-dihydropyrimidine-4-carboxylate (804 mg) were added thionyl chloride (5 mL) and N,N-dimethylformamide (102 mg) under ice-cooling. The reaction mixture was stirred at room temperature for 2 hr, and the solvent was evaporated under reduced pressure. The residue was added to a saturated aqueous sodium hydrogen carbonate solution, and the mixture was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (440 mg).
¹H NMR (300 MHz, CDCl₃) δ 0.93-1.06 (3H, m), 1.39-1.54 (2H, m), 1.72-1.87 (2H, m), 4.41 (2H, t, J = 6.8 Hz), 7.05-7.15 (1H, m), 7.31-7.46 (4H, m), 7.65 (2H, d, J = 7.5 Hz).

### B) 2-anilino-6-chloro-N-(2-methoxy-1-(4-(trifluoromethoxy)phenyl)ethyl)pyrimidine-4-carboxamide

By a method similar to that of Example 4, step C and Example 1, step C, the title compound was obtained.

### Example 12

### 2-anilino-6-cyano-N-(2-methoxy-1-(4-(trifluoromethoxy)phenyl)ethyl)pyrimidine-4-carboxamide

To a solution of 2-anilino-6-chloro-N-(2-methoxy-1-(4-(trifluoromethoxy)phenyl)ethyl)pyrimidine-4-carboxamide (140 mg) in N,N-dimethylacetamide (10.0 mL) were added tetrakis(triphenylphosphine)palladium(0) (34.7 mg) and zinc cyanide (52.8 mg) at room temperature, and the reaction mixture was stirred at 100°C overnight. The reaction mixture was poured into water, and the mixture was extracted with ethyl acetate. The extract was washed with water and saturated aqueous sodium hydrogen carbonate solution, and dried over anhydrous magnesium sulfate. The solvent was evaporated under reduced pressure. The residue was purified by silica gel column chromatography (NH, ethyl acetate/hexane) to give the title compound (46 mg).
¹H NMR (300 MHz, DMSO-d₆) δ 3.30-3.32 (5H, m), 3.61-3.81 (2H, m), 5.17-5.34 (1H, m), 7.05-7.14 (1H, m), 7.30-7.42 (4H, m), 7.56 (2H, d, J = 8.7 Hz), 7.65-7.76 (3H, m), 8.87 (1H, d, J = 7.9 Hz), 10.43 (1H, s).

### Example 13

### N-(2-methoxy-1-(4-(trifluoromethoxy)phenyl)ethyl)-2-(1H-pyrazol-5-yl)pyrimidine-4-carboxamide

### A) N-(2-methoxy-1-(4-(trifluoromethoxy)phenyl)ethyl)-2-(1-(tetrahydro-2H-pyran-2-yl)-1H-pyrazol-5-yl)pyrimidine-4-carboxamide

A mixture of 2-chloro-N-(2-methoxy-1-(4-(trifluoromethoxy)phenyl)ethyl)pyrimidine-4-carboxamide (178 mg), 1-(tetrahydro-2H-pyran-2-yl)-5-(4,4,5,5-tetramethyl-1,3,2-dioxaborolan-2-yl)-1H-pyrazole (171 mg), (1,1'-bis(diphenylphosphino)ferrocene)dichloropalladium(II) (34.7 mg), 2 M aqueous sodium carbonate solution (0.947 mL) and 1,2-dimethoxyethane (10 mL) was stirred under an argon atmosphere, at 90°C for 24 hr. After cooling to room temperature, water was added to the reaction mixture, and the mixture was extracted with ethyl acetate. The extract was washed with saturated brine, and dried over anhydrous sodium sulfate. The solvent was evaporated under reduced pressure. The residue was purified by silica gel column chromatography (ethyl acetate/hexane) to give the title compound (170 mg).
MS: [M-H]- 490.2.

### B) N-(2-methoxy-1-(4-(trifluoromethoxy)phenyl)ethyl)-2-(1H-pyrazol-5-yl)pyrimidine-4-carboxamide

To a mixture of N-(2-methoxy-1-(4-(trifluoromethoxy)phenyl)ethyl)-2-(1-(tetrahydro-2H-pyran-2-yl)-1H-pyrazol-5-yl)pyrimidine-4-carboxamide (169 mg) and methanol (3.0 mL) was added 2 M hydrogen chloride/methanol solution (5.0 mL), and the mixture was stirred at room temperature for 2 hr was stirred. The solvent was evaporated under reduced pressure, an aqueous sodium hydrogen carbonate solution was added to the residue, and the mixture was extracted with ethyl acetate. The extract was washed with water and saturated brine, and dried over anhydrous sodium sulfate. The solvent was evaporated under reduced pressure. The residue was purified by silica gel column chromatography (NH, methanol/ethyl acetate) to give the title compound (100 mg).
¹H NMR (300 MHz, CDCl₃) δ 3.46 (3H, s), 3.81 (2H, d, J = 4.8 Hz), 5.30-5.39 (1H, m), 7.13 (1H, d, J = 2.1 Hz), 7.17-7.23 (2H, m), 7.46 (2H, d, J = 8.7 Hz), 7.74 (1H, d, J = 2.1 Hz), 7.98 (1H, d, J = 4.8 Hz), 8.69 (1H, d, J = 8.1 Hz), 8.99 (1H, d, J = 4.8 Hz), 10.87-11.17 (1H, m).

### Example 14

### 2-anilino-5-fluoro-N-(2-methoxy-1-(4-(trifluoromethoxy)phenyl)ethyl)pyrimidine-4-carboxamide

### A) ethyl 2-anilino-5-fluoropyrimidine-4-carboxylate

By a method similar to that of Example 6, step A, the title compound was obtained.
MS: [M+H]⁺ 262.1.

### B) 2-anilino-5-fluoro-N-(2-methoxy-1-(4-(trifluoromethoxy)phenyl)ethyl)pyrimidine-4-carboxamide

To a solution of ethyl 2-anilino-5-fluoropyrimidine-4-carboxylate (35 mg) in ethanol (5.0 mL) was added 1 M aqueous sodium hydroxide solution (1.0 mL) at room temperature, and the mixture was stirred at room temperature for 1 hr. To the reaction mixture was added 1 M hydrochloric acid (1.0 mL) at room temperature and the solvent was evaporated under reduced pressure. A mixture of the residue, 2-methoxy-1-(4-(trifluoromethoxy)phenyl)ethanamine hydrochloride (19.1 mg), 1-(3-dimethylaminopropyl)-3-ethylcarbodiimide hydrochloride (13.5 mg), 1-hydroxybenzotriazole (9.5 mg), triethylamine (0.02 mL) and N,N-dimethylformamide (5.0 mL) was stirred at room temperature for 3 hr. Water was added to the reaction mixture, and the mixture was extracted with ethyl acetate. The extract was washed with water and saturated aqueous sodium hydrogen carbonate solution, dried over anhydrous magnesium sulfate. The solvent was evaporated under reduced pressure. The residue was purified by silica gel column chromatography (ethyl acetate/hexane) to give the title compound (6.1 mg).
¹H NMR (300 MHz, CD₃OD) δ 3.39 (3H, s), 3.74 (2H, d, J = 5.5 Hz), 5.28 (1H, t, J = 5.5 Hz), 6.99-7.07 (1H, m), 7.23-7.34 (4H, m), 7.52 (2H, d, J = 8.4 Hz), 7.61-7.67 (2H, m), 8.54 (1H, d, J = 2.7 Hz).

### Example 15

### 2-(benzyl(methyl)amino)-N-(2-methoxy-1-(4-(trifluoromethoxy)phenyl)ethyl)-6-oxo-1,6-dihydropyrimidine-4-carboxamide

### A) 2-(benzyl(methyl)amino)-6-methoxy-N-(2-methoxy-1-(4-(trifluoromethoxy)phenyl)ethyl)pyrimidine-4-carboxamide

By a method similar to that of Example 9, step B, Example 4, step C and Example 1, step C, the title compound was obtained.
MS: [M+H]⁺ 491.2.

### B) 2-(benzyl(methyl)amino)-N-(2-methoxy-1-(4-(trifluoromethoxy)phenyl)ethyl)-6-oxo-1,6-dihydropyrimidine-4-carboxamide

To a solution of 2-(benzyl(methyl)amino)-6-methoxy-N-(2-methoxy-1-(4-(trifluoromethoxy)phenyl)ethyl)pyrimidine-4-carboxamide (221 mg) in N,N-dimethylformamide (10.0 mL) was added pyridine hydrochloride (520 mg) at room temperature. The reaction mixture was stirred at 130°C overnight, cooled to room temperature, water (30 mL) was added and the mixture was stirred at room temperature for 30 min. The precipitated crystals were collected by filtration, and the filtrated crystal was recrystallized from ethyl acetate/hexane to give the title compound (190 mg).
¹H NMR (300 MHz, DMSO-d₆) δ 3.14 (3H, s), 3.21-3.28 (3H, m), 3.55-3.78 (2H, m), 4.89 (2H, s), 5.17 (1H, d, J = 7.2 Hz), 7.23-7.39 (8H, m), 7.45 (2H, d, J = 8.3 Hz), 8.66 (1H, brs), 11.37 (1H, brs).

### Example 16

### 2-(cyclopropylamino)-N-(2-methoxy-1-(4-(trifluoromethoxy)phenyl)ethyl)-6-oxo-1,6-dihydropyrimidine-4-carboxamide

### A) methyl 2-(cyclopropylamino)-6-methoxypyrimidine-4-carboxylate

By a method similar to that of Example 9, step B, the title compound was obtained.
MS: [M+H]⁺ 224.1.

### B) 2-(cyclopropylamino)-6-oxo-1,6-dihydropyrimidine-4-carboxylic acid

To a solution of methyl 2-(cyclopropylamino)-6-methoxypyrimidine-4-carboxylate (201 mg) in acetic acid (5.0 mL) were added 6 M hydrochloric acid (1.5 mL) at room temperature. The reaction mixture was stirred at 120°C overnight. The solvent was evaporated under reduced pressure to give the title compound (170 mg).
¹H NMR (300 MHz, DMSO-d₆) δ 0.47-0.56 (2H, m), 0.70-0.81 (2H, m), 2.63-2.78 (1H, m), 6.12 (1H, s), 7.71 (1H, brs).

### C) 2-(cyclopropylamino)-N-(2-methoxy-1-(4-(trifluoromethoxy)phenyl)ethyl)-6-oxo-1,6-dihydropyrimidine-4-carboxamide

By a method similar to that of Example 1, step C, the title compound was obtained.

### Example 17

### 2-anilino-N-(2-methoxy-1-(4-(trifluoromethoxy)phenyl)ethyl)-5-(methylamino)pyrimidine-4-carboxamide

### A) ethyl 2-anilino-5-(methylamino)pyrimidine-4-carboxylate

A mixture of ethyl 2-chloro-5-(methylamino)pyrimidine-4-carboxylate (38 mg), aniline (0.032 mL), tris(dibenzylideneacetone)dipalladium(0) (16.1 mg), dicyclohexyl(2',4',6'-triisopropylbiphenyl-2-yl)phosphine (33.6 mg), cesium carbonate (172 mg) and 1,2-dimethoxyethane (10 mL) was stirred at 85°C for 40 hr. To the reaction mixture was added water at room temperature, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (25 mg).
MS: [M+H]⁺ 273.1.

### B) 2-anilino-N-(2-methoxy-1-(4-(trifluoromethoxy)phenyl)ethyl)-5-(methylamino)pyrimidine-4-carboxamide

By a method similar to that of Example 14, step B, the title compound was obtained.

### Example 18

### N-(2-methoxy-1-(4-(trifluoromethoxy)phenyl)ethyl)-2-(methylamino)-6-oxo-1,6-dihydropyrimidine-4-carboxamide

By a method similar to that of Example 9, step B, Example 4, step C, Example 1, step C and Example 15, step B, the title compound was obtained.

### Example 19

### 2-(dimethylamino)-N-(2-methoxy-1-(4-(trifluoromethoxy)phenyl)ethyl)-6-oxo-1,6-dihydropyrimidine-4-carboxamide

### A) methyl 2-(dimethylamino)-6-methoxypyrimidine-4-carboxylate

By a method similar to that of Example 9, step B, the title compound was obtained.
MS: [M+H]⁺ 212.1.

### B) 2-(dimethylamino)-6-methoxypyrimidine-4-carboxylic acid

By a method similar to that of Example 4, step C, the title compound was obtained.
¹H NMR (300 MHz, DMSO-d₆) δ 3.15 (6H, s), 3.89 (3H, s), 6.46 (1H, s).

### C) 2-(dimethylamino)-6-methoxy-N-(2-methoxy-1-(4-(trifluoromethoxy)phenyl)ethyl)pyrimidine-4-carboxamide

By a method similar to that of Example 1, step C, the title compound was obtained.
MS: [M+H]⁺ 415.1.

### D) 2-(dimethylamino)-N-(2-methoxy-1-(4-(trifluoromethoxy)phenyl)ethyl)-6-oxo-1,6-dihydropyrimidine-4-carboxamide

By a method similar to that of Example 15, step B, the title compound was obtained.

### Example 20

### 2-(dimethylamino)-N-((1S)-2-methoxy-1-(4-(trifluoromethoxy)phenyl)ethyl)-6-oxo-1,6-dihydropyrimidine-4-carboxamide

A racemate (300 mg) of 2-(dimethylamino)-N-(2-methoxy-1-(4-(trifluoromethoxy)phenyl)ethyl)-6-oxo-1,6-dihydropyrimidine-4-carboxamide was fractionated by HPLC (column: CHIRALPAK AS, 50 mmID×500 mmL, manufactured by Daicel Corporation, mobile phase: hexane/ethanol = 600/400) and recrystallized from ethyl acetate/hexane to give the title compound (118 mg) having a shorter retention time.

### Example 21

### 2-(dimethylamino)-N-((1R)-2-methoxy-1-(4-(trifluoromethoxy)phenyl)ethyl)-6-oxo-1,6-dihydropyrimidine-4-carboxamide

A racemate (300 mg) of 2-(dimethylamino)-N-(2-methoxy-1-(4-(trifluoromethoxy)phenyl)ethyl)-6-oxo-1,6-dihydropyrimidine-4-carboxamide was fractionated by HPLC (column: CHIRALPAK AS, 50 mmID×500 mmL, manufactured by Daicel Corporation, mobile phase: hexane/ethanol = 600/400), and recrystallized from ethyl acetate/hexane to give the title compound (106 mg) having a longer retention time.

### Example 22

### 2-((3,4-dimethoxyphenyl)amino)-N-(2-methoxy-1-(4-(trifluoromethoxy)phenyl)ethyl)-6-oxo-1,6-dihydropyrimidine-4-carboxamide

By a method similar to that of Example 6, step A, Example 4, step C and Example 1, step C, the title compound was obtained.

### Example 23

### 2-((cyclopropylcarbonyl)amino)-N-(2-methoxy-1-(4-(trifluoromethoxy)phenyl)ethyl)pyrimidine-4-carboxamide

A mixture of 2-chloro-N-(2-methoxy-1-(4-(trifluoromethoxy)phenyl)ethyl)pyrimidine-4-carboxamide (100 mg), cyclopropanecarboxamide (27.2 mg), tris(dibenzylideneacetone)dipalladium(0) (24.4 mg), 4,5-bis(diphenylphosphino)-9,9-dimethylxanthene (46.2 mg), cesium carbonate (121 mg) and toluene (3.0 mL) was stirred overnight under an argon atmosphere at 110°C. To the reaction mixture was added water at room temperature, and the mixture was extracted with ethyl acetate. The extract was washed with water and saturated brine, and dried over anhydrous sodium sulfate. The solvent was evaporated under reduced pressure. The residue was fractionated by silica gel column chromatography (Diol, ethyl acetate/hexane) and HPLC (C18, mobile phase: water/acetonitrile (0.1% TFA-containing system)), saturated aqueous sodium hydrogen carbonate solution were added to the obtained fraction, and the mixture was extracted with ethyl acetate, dried over anhydrous magnesium sulfate, and concentrated under reduced pressure to give the title compound (39.3 mg).
¹H NMR (300 MHz, CDCl₃) δ 0.95-1.03 (2H, m), 1.19-1.26 (2H, m), 2.26-2.38 (1H, m), 3.42 (3H, s), 3.74-3.78 (2H, m), 5.25-5.33 (1H, m), 7.16-7.22 (2H, m), 7.43 (2H, d, J = 8.7 Hz), 7.75 (1H, d, J = 4.9 Hz), 8.19 (1H, s), 8.52-8.58 (1H, m), 8.80 (1H, d, J = 4.9 Hz).

### Example 24

### N-(2-methoxy-1-(4-(trifluoromethoxy)phenyl)ethyl)-6-oxo-1,6-dihydropyrimidine-4-carboxamide

### A) 6-chloro-N-(2-methoxy-1-(4 (trifluoromethoxy)phenyl)ethyl)pyrimidine-4-carboxamide

By a method similar to that of Example 9, step A, the title compound was obtained.
MS: [M+H]⁺ 376.1.

### B) 6-((2,4-dimethoxybenzyl)oxy)-N-(2-methoxy-1-(4 (trifluoromethoxy)phenyl)ethyl)pyrimidine-4-carboxamide

To a suspension of sodium hydride (60% in oil, 73.8 mg) in N,N-dimethylformamide (10 mL) was added 2,4-dimethoxybenzylalcohol (331 mg) at 0°C. Under a nitrogen atmosphere, the mixture was stirred at room temperature for 1 hr. To the reaction mixture was added dropwise a solution of 6-chloro-N-(2-methoxy-1-(4-(trifluoromethoxy)phenyl)ethyl)pyrimidine-4-carboxamide (254 mg) in N,N-dimethylformamide (5.0 mL) at room temperature. Under a nitrogen atmosphere, the reaction mixture stirred at 60°C for 2 hr, water was added at room temperature, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (199 mg).
MS: [M+H]⁺ 508.2.

### C) N-(2-methoxy-1-(4-(trifluoromethoxy)phenyl)ethyl)-6-oxo-1,6-dihydropyrimidine-4-carboxamide

To a solution of 6-((2,4-dimethoxybenzyl)oxy)-N-(2-methoxy-1-(4-(trifluoromethoxy)phenyl)ethyl)pyrimidine-4-carboxamide (199 mg) in toluene (5.0 mL) was added trifluoroacetic acid (2.0 mL), and the mixture was stirred at room temperature for 5 days. The reaction mixture was neutralized with aqueous sodium hydrogen carbonate solution at 0°C, and extracted with ethyl acetate. The extract was washed with water and saturated brine, and dried over anhydrous sodium sulfate. The solvent was evaporated under reduced pressure. The residue was purified by silica gel column chromatography (methanol/ethyl acetate) to give the title compound (104 mg). ¹H NMR (300 MHz, CDCl₃) δ 3.41 (3H, s), 3.74 (2H, d, J = 5.1 Hz), 5.23-5.31 (1H, m), 7.16-7.22 (2H, m), 7.29 (1H, d, J = 1.1 Hz), 7.41 (2H, d, J = 8.3 Hz), 8.16 (1H, d, J = 1.1 Hz), 8.48 (1H, d, J = 7.8 Hz), 12.11-12.29 (1H, m).

### Example 25

### N-(2-methoxy-1-(4-(trifluoromethoxy)phenyl)ethyl)-2-methyl-6-oxo-1,6-dihydropyrimidine-4-carboxamide

By a method similar to that of Example 1, step C, the title compound was obtained.

### Example 26

### 2-benzyl-N-(2-methoxy-1-(4-(trifluoromethoxy)phenyl)ethyl)-6-oxo-1,6-dihydropyrimidine-4-carboxamide

### A) 2-chloro-6-methoxypyrimidine-4-carboxylic acid

By a method similar to that of Example 4, step C, the title compound was obtained.
¹H NMR (300 MHz, CDCl₃) δ 4.11 (3H, s), 7.46 (1H, s).

### B) 2-chloro-6-methoxy-N-(2-methoxy-1-(4-(trifluoromethoxy)phenyl)ethyl)pyrimidine-4-carboxamide

By a method similar to that of Example 9, step A, the title compound was obtained.
MS: [M+H]⁺ 406.1.

### C) 2-benzyl-6-methoxy-N-(2-methoxy-1-(4-(trifluoromethoxy)phenyl)ethyl)pyrimidine-4-carboxamide

A mixture of 2-chloro-6-methoxy-N-(2-methoxy-1-(4-(trifluoromethoxy)phenyl)ethyl)pyrimidine-4-carboxamide (50 mg), 2-benzyl-4,4,5,5-tetramethyl-1,3,2-dioxaborolane (53.8 mg), (1,1'-bis(diphenylphosphino)ferrocene)dichloropalladium(II) dichloromethane adduct (10 mg), tripotassium phosphate (78 mg), 1,2-dimethoxyethane (3.0 mL) and water (1.0 mL) was heated under microwave irradiation at 120°C for 3 hr. After cooling to room temperature, water was added to the reaction mixture, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (33 mg).
MS: [M+H]⁺ 462.2.

### D) 2-benzyl-N-(2-methoxy-1-(4-(trifluoromethoxy)phenyl)ethyl)-6-oxo-1,6-dihydropyrimidine-4-carboxamide

By a method similar to that of Example 15, step B, the title compound was obtained.

### Example 27

### N-(2-methoxy-1-(4-(trifluoromethoxy)phenyl)ethyl)-6-oxo-2-(pyrrolidin-1-yl)-1,6-dihydropyrimidine-4-carboxamide

By a method similar to that of Example 9, step B and Example 15, step B, the title compound was obtained.

### Example 28

### 2-(3-fluoroazetidin-1-yl)-N-((1S)-2-methoxy-1-(4-(trifluoromethoxy)phenyl)ethyl)-6-oxo-1,6-dihydropyrimidine-4-carboxamide

### A) tert-butyl (2-methoxy-1-(4-(trifluoromethoxy)phenyl)ethyl)carbamate

To a mixture of 2-methoxy-1-(4-(trifluoromethoxy)phenyl)ethanamine hydrochloride (100 mg), triethylamine (0.109 mL) and tetrahydrofuran (6 mL) was added di-tert-butyl dicarbonate (0.097 mL), and the mixture was stirred at 50°C for 3 hr. To the reaction mixture was added 1 M hydrochloric acid, and the mixture was extracted with ethyl acetate. The extract was washed with saturated brine, and dried over anhydrous sodium sulfate. The solvent was evaporated under reduced pressure. The residue was purified by silica gel column chromatography (hexane/ethyl acetate) to give the title compound (131 mg).
¹H NMR (300 MHz, CDCl₃) δ 1.41 (9H, brs), 3.34 (3H, s), 3.46-3.71 (2H, m), 4.80 (1H, brs), 5.29 (1H, brs), 7.17 (2H, d, J = 7.9 Hz), 7.30-7.39 (2H, m).

### B) tert-butyl ((1S)-2-methoxy-1-(4-(trifluoromethoxy)phenyl)ethyl)carbamate

A racemate (12.7 g) of tert-butyl (2-methoxy-1-(4-(trifluoromethoxy)phenyl)ethyl)carbamate was fractionated by HPLC (column: CHIRALPAK IC, 50 mmID×500 mmL, manufactured by Daicel Corporation, mobile phase: hexane/ethanol = 930/70) to give the title compound (6.0 g) having a longer retention time.
¹H NMR (300 MHz, CDCl₃) δ 1.41 (9H, brs), 3.35 (3H, s), 3.49-3.66 (2H, m), 4.81 (1H, brs), 5.29 (1H, brs), 7.13-7.21 (2H, m), 7.30-7.39 (2H, m).

### C) (1S)-2-methoxy-1-(4-(trifluoromethoxy)phenyl)ethanamine hydrochloride

A mixture of tert-butyl ((1S)-2-methoxy-1-(4-(trifluoromethoxy)phenyl)ethyl)carbamate (5.95 g) and 4 M hydrogen chloride/ethyl acetate solution (30 mL) was stirred at room temperature for 1 hr. The solvent was evaporated under reduced pressure, and the residue was washed with diisopropyl ether to give the title compound (4.3 g).
¹H NMR (300 MHz, DMSO-d₆) δ 3.33 (3H, s), 3.60-3.77 (2H, m), 4.52-4.61 (1H, m), 7.46 (2H, d, J = 8.3 Hz), 7.68 (2H, d, J = 8.7 Hz), 8.67 (3H, brs).

### D) 2-(3-fluoroazetidin-1-yl)-6-((4-methoxybenzyl)oxy)-N-((1S)-2-methoxy-1-(4-(trifluoromethoxy)phenyl)ethyl)pyrimidine-4-carboxamide

A mixture of methyl 2,6-dichloropyrimidine-4-carboxylate (500 mg), 4-methoxybenzylalcohol (1.51 mL), potassium carbonate (334 mg) and acetonitrile (5.0 mL) was stirred at 70°C for 48 hr. The solid was removed by filtration and the solvent was evaporated under reduced pressure. The residue was purified by silica gel column chromatography (ethyl acetate/hexane) to give a yellow solid (544 mg). A mixture of the obtained solid (120 mg), 3-fluoroazetidine hydrochloride (49.7 mg), triethylamine (0.124 mL) and acetonitrile (5.0 mL) was stirred at 60°C for 2 hr. Water was added to the reaction mixture, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give a white solid (104 mg). Using the obtained solid (104 mg) and by an operation similar to that in Example 14, step B, the title compound (121 mg) was obtained. MS: [M+H]⁺ 551.2.

### E) 2-(3-fluoroazetidin-1-yl)-N-((1S)-2-methoxy-1-(4-(trifluoromethoxy)phenyl)ethyl)-6-oxo-1,6-dihydropyrimidine-4-carboxamide

By a method similar to that of Example 24, step C, the title compound was obtained.

### Example 29

### N-(2-methoxy-1-(4-(trifluoromethoxy)phenyl)ethyl)-2-(1-methyl-1H-pyrazol-3-yl)-6-oxo-1,6-dihydropyrimidine-4-carboxamide

By a method similar to that of Example 13, step A and Example 15, step B, the title compound was obtained.

### Example 30

### 2-((2-methoxyethyl)(methyl)amino)-N-(2-methoxy-1-(4-(trifluoromethoxy)phenyl)ethyl)-6-oxo-1,6-dihydropyrimidine-4-carboxamide

By a method similar to that of Example 9, step B and Example 15, step B, the title compound was obtained.

### Example 31

### 2-(dimethylamino)-N-(1-(3-fluoro-4-(trifluoromethoxy)phenyl)-2-methoxyethyl)-6-oxo-1,6-dihydropyrimidine-4-carboxamide

### A) 3-fluoro-N-methoxy-N-methyl-4-(trifluoromethoxy)benzamide

To a solution of 3-fluoro-4-(trifluoromethoxy)benzoic acid (20.0 g), N,O-dimethylhydroxylamine hydrochloride (10.5 g), and triethylamine (24.9 mL) in N,N-dimethylformamide (300 mL) were added 1-hydroxybenzotriazole monohydrate (16.4 g) and 1-(3-dimethylaminopropyl)-3-ethylcarbodiimide hydrochloride (20.5 g) at room temperature. The reaction mixture was stirred at room temperature overnight, saturated aqueous sodium hydrogen carbonate solution was added, and the mixture was extracted with ethyl acetate. The extract was washed with water and saturated brine, and dried over anhydrous magnesium sulfate. The solvent was evaporated under reduced pressure to give the title compound (22.9 g).
¹H NMR (300 MHz, CDCl₃) δ 3.38 (3H, s), 3.56 (3H, s), 7.30-7.42 (1H, m), 7.51-7.65 (2H, m).

### B) 1-(3-fluoro-4-(trifluoromethoxy)phenyl)ethanone

To a solution of 3-fluoro-N-methoxy-N-methyl-4-(trifluoromethoxy)benzamide (3.52 g) in tetrahydrofuran (60 mL) was slowly added 1 M methylmagnesium bromide/tetrahydrofuran solution (39.5 mL) at 0°C. The reaction mixture was stirred at room temperature for 4 hr, poured into a saturated aqueous ammonium chloride solution, and the mixture was extracted with ethyl acetate. The extract was dried over anhydrous sodium sulfate, and the solvent was evaporated under reduced pressure. The residue was purified by silica gel column chromatography (hexane/ethyl acetate) to give the title compound (2.33 g).
¹H NMR (300 MHz, DMSO-d₆) δ 2.62 (3H, s), 7.68-7.80 (1H, m), 7.86-7.96 (1H, m), 8.04 (1H, dd, J = 11.0, 2.1 Hz).

### C) 2-bromo-1-(3-fluoro-4-(trifluoromethoxy)phenyl)ethanone

To a solution of 1-(3-fluoro-4-(trifluoromethoxy)phenyl)ethanone (4.51 g) in acetic acid (50 mL) was slowly added a solution of bromine (1.12 mL) in acetic acid (5 mL) at room temperature. The reaction mixture was stirred at 50°C for 1.5 hr, and the solvent was evaporated under reduced pressure. An aqueous sodium hydrogen carbonate solution was added, and the mixture was extracted with ethyl acetate. The extract was washed with saturated aqueous sodium hydrogen carbonate solution, and dried over anhydrous sodium sulfate. The solvent was evaporated under reduced pressure. The residue was purified by silica gel column chromatography (hexane/ethyl acetate) to give the title compound (6.01 g).
¹H NMR (300 MHz, DMSO-d₆) δ 4.99 (2H, s), 7.73-7.84 (1H, m), 7.92-8.00 (1H, m), 8.12 (1H, dd, J = 11.0, 2.0 Hz).

### D) 1-(3-fluoro-4-(trifluoromethoxy)phenyl)-2-methoxyethanone

To a solution of 2-bromo-1-(3-fluoro-4-(trifluoromethoxy)phenyl)ethanone (6.01 g) in methanol (60 mL) were added silver carbonate (I) (7.53 g) and boron trifluoride diethyl ether complex (3.10 mL). The reaction mixture was stirred under a nitrogen atmosphere at 60°C for 4 hr. Insoluble material was filtrated off, and insoluble material was washed with ethyl acetate. The filtrate was concentrated under reduced pressure, and ethyl acetate and brine were added. The precipitated solid was filtered off, the filtrate was separated into an organic layer and an aqueous layer, and the aqueous layer was extracted with ethyl acetate. The extract was dried over anhydrous sodium sulfate, and the solvent was evaporated under reduced pressure. The residue was purified by silica gel column chromatography (hexane/ethyl acetate) to give the title compound (3.57 g).
¹H NMR (300 MHz, DMSO-d₆) δ 3.36 (3H, s), 4.81 (2H, s), 7.70-7.81 (1H, m), 7.83-7.92 (1H, m), 8.01 (1H, dd, J = 10.9, 2.3 Hz).

### E) 1-(3-fluoro-4-(trifluoromethoxy)phenyl)-2-methoxyethanamine hydrochloride

To a mixture of 1-(3-fluoro-4-(trifluoromethoxy)phenyl)-2-methoxyethanone (3.57 g), hydroxylamine hydrochloride (2.01 g) and ethanol (50 mL) was added triethylamine (3.99 mL). The reaction mixture was stirred at room temperature for 16 hr and the solvent was evaporated under reduced pressure. Water was added to the residue and the mixture was extracted with ethyl acetate. The extract was washed with saturated brine, and dried over anhydrous sodium sulfate. The solvent was evaporated under reduced pressure to give 1-(3-fluoro-4-(trifluoromethoxy)phenyl)-N-hydroxy-2-methoxyethanimine as a crude product (3.89 g). To a solution of the obtained crude product (3.89 g) in ethanol (60 mL) was added 20% palladium hydroxide-carbon (1.00 g). The reaction mixture was stirred under a hydrogen atmosphere, at room temperature for 5 hr. Insoluble material was filtered off, and the filtrate was concentrated under reduced pressure. The residue was dissolved in ethyl acetate (10 mL), and 4 M hydrogen chloride/ethyl acetate solution (10 mL) was added. The solvent was evaporated under reduced pressure and the precipitated solid was washed with diisopropyl ether to give the title compound (2.86 g).
¹H NMR (300 MHz, DMSO-d₆) δ 3.32 (3H, s), 3.60-3.78 (2H, m), 4.53-4.66 (1H, m), 7.46-7.55 (1H, m), 7.62-7.84 (2H, m), 8.70 (3H, brs).

### F) 2-((2-methoxyethyl)(methyl)amino)-N-(2-methoxy-1-(4 (trifluoromethoxy)phenyl)ethyl)-6-oxo-1,6-dihydropyrimidine-4-carboxamide

By a method similar to that of Example 1, step C and Example 15, step B, the title compound was obtained.

### Example 32

### 2-(dimethylamino)-N-((1R)-1-(3-fluoro-4-(trifluoromethoxy)phenyl)-2-methoxyethyl)-6-oxo-1,6-dihydropyrimidine-4-carboxamide

A racemate (130 mg) of 2-(dimethylamino)-N-(1-(3-fluoro-4-(trifluoromethoxy)phenyl)-2-methoxyethyl)-6-oxo-1,6-dihydropyrimidine-4-carboxamide was fractionated by HPLC (column: CHIRALPAK IC, 50 mmID×500 mmL, manufactured by Daicel Corporation, mobile phase: hexane/ethanol = 400/600) and recrystallized from ethyl acetate/hexane to give the title compound (54 mg) having a shorter retention time.

### Example 33

### 2-(dimethylamino)-N-((1S)-1-(3-fluoro-4-(trifluoromethoxy)phenyl)-2-methoxyethyl)-6-oxo-1,6-dihydropyrimidine-4-carboxamide

A racemate (130 mg) of 2-(dimethylamino)-N-(1-(3-fluoro-4-(trifluoromethoxy)phenyl)-2-methoxyethyl)-6-oxo-1,6-dihydropyrimidine-4-carboxamide was fractionated by HPLC (column: CHIRALPAK IC, 50 mmID×500 mmL, manufactured by Daicel Corporation, mobile phase: hexane/ethanol = 400/600) and recrystallized from ethyl acetate/hexane to give the title compound (47 mg) having a longer retention time.

### Example 34

### 2-methoxy-N-(2-methoxy-1-(4-(trifluoromethoxy)phenyl)ethyl)-6-oxo-1,6-dihydropyrimidine-4-carboxamide

### A) 2-methoxy-6-oxo-1,6-dihydropyrimidine-4-carboxylic acid

To diethyl oxaloacetate (2.79 mL) was added 2 M aqueous sodium hydroxide solution (25 mL) at room temperature, and the mixture was stirred at room temperature for 30 min. O-methylisourea sulfate (2.0 g) was added, and the mixture was stirred at room temperature overnight. The reaction mixture was acidified with 2 M hydrochloric acid and the precipitate was collected by filtration to give the title compound (1.27 g). ¹H NMR (300 MHz, DMSO-d₆) δ 3.92 (3H, s), 6.52 (1H, s), 12.73 (1H, brs), 13.39 (1H, brs).

### B) 2-methoxy-N-(2-methoxy-1-(4-(trifluoromethoxy)phenyl)ethyl)-6-oxo-1,6-dihydropyrimidine-4-carboxamide

By a method similar to that of Example 1, step C, the title compound was obtained.

### Example 35

### N-(2-methoxy-1-(4-(trifluoromethoxy)phenyl)ethyl)-2-(methylsulfanyl)-6-oxo-1,6-dihydropyrimidine-4-carboxamide

By a method similar to that of Example 34, step A, Example 1, step C, the title compound was obtained.

### Example 36

### 2-(cyclopropyl(methyl)amino)-N-((1S)-2-methoxy-1-(4-(trifluoromethoxy)phenyl)ethyl)-6-oxo-1,6-dihydropyrimidine-4-carboxamide

### A) 2-chloro-6-methoxy-N-((1S)-2-methoxy-1-(4-(trifluoromethoxy)phenyl)ethyl)pyrimidine-4-carboxamide

By a method similar to that of Example 9, step A, the title compound was obtained.

### MS: [M+H]⁺ 406.1.

### B) 2-(cyclopropyl(methyl)amino)-N-((1S)-2-methoxy-1-(4-(trifluoromethoxy)phenyl)ethyl)-6-oxo-1,6-dihydropyrimidine-4-carboxamide

By a method similar to that of Example 9, step B and Example 15, step B, the title compound was obtained.

### Example 37

### 2-(diethylamino)-N-((1S)-2-methoxy-1-(4-(trifluoromethoxy)phenyl)ethyl)-6-oxo-1,6-dihydropyrimidine-4-carboxamide

By a method similar to that of Example 9, step B and Example 15, step B, the title compound was obtained.

### Example 38

### 2-(ethyl(methyl)amino)-N-((1S)-2-methoxy-1-(4-(trifluoromethoxy)phenyl)ethyl)-6-oxo-1,6-dihydropyrimidine-4-carboxamide

By a method similar to that of Example 9, step B and Example 15, step B, the title compound was obtained.

### Example 39

### N-((1S)-2-methoxy-1-(4-(trifluoromethoxy)phenyl)ethyl)-2-(3-methylpyrrolidin-1-yl)-6-oxo-1,6-dihydropyrimidine-4-carboxamide

A mixture of 2-chloro-6-methoxy-N-((1S)-2-methoxy-1-(4-(trifluoromethoxy)phenyl)ethyl)pyrimidine-4-carboxamide (90 mg), 3-methylpyrrolidine (28.3 mg), triethylamine (0.046 mL) and acetonitrile (3.0 mL) was stirred at 70°C for 2 hr. The solvent was evaporated under reduced pressure. To the residue were added pyridine hydrochloride (256 mg) and N,N-dimethylacetamide (3.0 mL), and the mixture was stirred at 130°C overnight. The solvent was evaporated under reduced pressure. An aqueous sodium hydrogen carbonate solution was added to the residue, and the precipitated solid was collected by filtration. The solid was purified by silica gel column chromatography (ethyl acetate/hexane) to give the title compound (56 mg).
¹H NMR (300 MHz, CDCl₃) δ 1.16 (3H, d, J = 6.3 Hz), 1.62-1.75 (1H, m), 2.13-2.28 (1H, m), 2.37-2.52 (1H, m), 3.05-3.16 (1H, m), 3.40 (3H, s), 3.49-3.62 (1H, m), 3.67-3.83 (4H, m), 5.22-5.29 (1H, m), 6.54 (1H, s), 7.15-7.21 (2H, m), 7.40 (2H, d, J = 8.7 Hz), 8.51 (1H, d, J = 8.4 Hz), 10.63-10.80 (1H, m).

### Example 40

### 2-((3R)-3-methoxypyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 41

### 6-chloro-N-(2-methoxy-1-(4 (trifluoromethoxy)phenyl)ethyl)pyrazine-2-carboxamide

A mixture of 6-chloropyrazine-2-carboxylic acid (150 mg), thionyl chloride (0.345 mL), N,N-dimethylformamide (0.05 mL) and toluene (5 mL) was stirred at 100°C for 1 hr. The mixture was concentrated under reduced pressure, to the residue were added tetrahydrofuran (5 mL), triethylamine (0.659 mL) and 2-methoxy-1-(4-(trifluoromethoxy)phenyl)ethanamine hydrochloride (257 mg), and the mixture was stirred at room temperature for 5 hr. The mixture was poured into a saturated aqueous sodium hydrogen carbonate solution, and the mixture was extracted with ethyl acetate. The extract was washed with saturated aqueous sodium hydrogen carbonate solution and saturated brine, dried over magnesium sulfate and concentrated under reduced pressure. The residue was purified by silica gel column chromatography (ethyl acetate/hexane) to give the title compound (225 mg).
¹H NMR (300 MHz, CDCl₃) δ 3.43 (3H, s), 3.77 (2H, d, J = 4.9 Hz), 5.23-5.46 (1H, m), 7.13-7.25 (2H, m), 7.39-7.53 (2H, m), 8.27 (1H, d, J = 7.5 Hz), 8.77 (1H, s), 9.27 (1H, s).

### Example 42

### N-(2-methoxy-1-(4-(trifluoromethoxy)phenyl)ethyl)-6-(pyrrolidin-1-yl)pyrazine-2-carboxamide

A mixture of 6-chloro-N-(2-methoxy-1-(4-(trifluoromethoxy)phenyl)ethyl)pyrazine-2-carboxamide (30 mg), pyrrolidine (11.4 mg), potassium carbonate (22.1 mg) and dimethyl sulfoxide (3 mL) was stirred at 80°C for 2 hr. The mixture was poured into saturated brine, and the mixture was extracted with ethyl acetate. The extract was washed with saturated brine, dried over magnesium sulfate and concentrated under reduced pressure. The residue was purified by silica gel column chromatography (hexane/ethyl acetate) to give the title compound (26.6 mg).
¹H NMR (300 MHz, CDCl₃) δ 1.99-2.19 (4H, m), 3.41 (3H, s), 3.55 (4H, t, J = 6.6 Hz), 3.68-3.93 (2H, m), 5.24-5.41 (1H, m), 7.11-7.23 (2H, m), 7.35-7.48 (2H, m), 8.05 (1H, s), 8.50 (1H, d, J = 7.9 Hz), 8.57 (1H, s).

### Example 43

### N-((1S)-2-methoxy-1-(4-(trifluoromethoxy)phenyl)ethyl)-2-(2-methylpyrrolidin-1-yl)-6-oxo-1,6-dihydropyrimidine-4-carboxamide

By a method similar to that of Example 39, the title compound was obtained.

### Example 44

### 2-(3-azabicyclo[3.1.0]hexa-3-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 45

### 2-((3S)-3-methoxypyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 46

### 2-(3,3-difluoropyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 47

### N-(2-methoxy-1-(4-(trifluoromethoxy)phenyl)ethyl)-6-oxo-2-(piperidin-1-yl)-1,6-dihydropyrimidine-4-carboxamide

By a method similar to that of Example 9, step B and Example 15, step B, the title compound was obtained.

### Example 48

### N-(2-methoxy-1-(4-(trifluoromethoxy)phenyl)ethyl)-2-(morpholin-4-yl)-6-oxo-1,6-dihydropyrimidine-4-carboxamide

By a method similar to that of Example 9, step B and Example 15, step B, the title compound was obtained.

### Example 49

### 2-(azepane-1-yl)-N-(2-methoxy-1-(4-(trifluoromethoxy)phenyl)ethyl)-6-oxo-1,6-dihydropyrimidine-4-carboxamide

By a method similar to that of Example 39, the title compound was obtained.

### Example 50

### 2-((2R)-2-(methoxymethyl)pyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 51

### N-(2-methoxy-1-(4-(trifluoromethoxy)phenyl)ethyl)-6-oxo-2-(pyridin-2-yl)-1,6-dihydropyrimidine-4-carboxamide

### A) 6-oxo-2-(pyridin-2-yl)-1,6-dihydropyrimidine-4-carboxylic acid

By a method similar to that of Example 34, step A, the title compound was obtained.
MS: [M+H]⁺ 218.1.

### B) N-(2-methoxy-1-(4-(trifluoromethoxy)phenyl)ethyl)-6-oxo-2-(pyridin-2-yl)-1,6-dihydropyrimidine-4-carboxamide

By a method similar to that of Example 1, step C, the title compound was obtained.

### Example 52

### N-((1S)-2-methoxy-1-(4-(trifluoromethoxy)phenyl)ethyl)-6-oxo-2-(pyrrolidin-1-yl)-1,6-dihydropyrimidine-4-carboxamide

### A) methyl 6-methoxy-2-(pyrrolidin-1-yl)pyrimidine-4-carboxylate

To a solution of methyl 2-chloro-6-methoxypyrimidine-4-carboxylate (5.08 g) in acetonitrile (84 mL) were added pyrrolidine (2.51 mL) and triethylamine (5.24 mL) at 0°C. The mixture was stirred at room temperature for 2 hr, poured into water at room temperature, and the mixture was extracted with ethyl acetate. The extract was washed with saturated brine, dried over anhydrous magnesium sulfate, and the solvent was evaporated under reduced pressure. The residue was purified by silica gel column chromatography (ethyl acetate/hexane) to give the title compound (3.56 g).
MS: [M+H]⁺ 238.1.

### B) 6-methoxy-2-(pyrrolidin-1-yl)pyrimidine-4-carboxylic acid

To a solution of methyl 6-methoxy-2-(pyrrolidin-1-yl)pyrimidine-4-carboxylate (3.56 g) in methanol (37.5 mL) and tetrahydrofuran (37.5 mL) was added 2 M aqueous sodium hydroxide solution (15.0 mL) at room temperature, and the mixture was stirred at room temperature for 30 min. The reaction mixture was acidified with 1 M hydrochloric acid at 0°C, and extracted with ethyl acetate. The extract was dried over anhydrous magnesium sulfate, and the solvent was evaporated under reduced pressure to give the title compound (3.04 g).
MS: [M+H]⁺ 224.1.

### C) N-((1S)-2-methoxy-1-(4-(trifluoromethoxy)phenyl)ethyl)-6-oxo-2-(pyrrolidin-1-yl)-1,6-dihydropyrimidine-4-carboxamide

By a method similar to that of Example 1, step C and Example 15, step B, the title compound was obtained.

### Example 53

### N-(2-methoxy-1-(4-(trifluoromethoxy)phenyl)ethyl)-6-oxo-2-(1H-pyrazol-1-yl)-1,6-dihydropyrimidine-4-carboxamide

By a method similar to that of Example 42 and Example 15, step B, the title compound was obtained.

### Example 54

### 2-(azetidin-1-yl)-N-(2-methoxy-1-(4-(trifluoromethoxy)phenyl)ethyl)-6-oxo-1,6-dihydropyrimidine-4-carboxamide

### A) 2-(azetidin-1-yl)-6-((4-methoxybenzyl)oxy)-N-((1S)-2-methoxy-1-(4-(trifluoromethoxy)phenyl)ethyl)pyrimidine-4-carboxamide

By a method similar to that of Example 28, step D, the title compound was obtained.
MS: [M+H]⁺ 553.2.

### B) 2-(azetidin-1-yl)-N-(2-methoxy-1-(4-(trifluoromethoxy)phenyl)ethyl)-6-oxo-1,6-dihydropyrimidine-4-carboxamide

By a method similar to that of Example 24, step C, the title compound was obtained.

### Example 55

### 2-(3-methoxyazetidin-1-yl)-N-((1S)-2-methoxy-1-(4-(trifluoromethoxy)phenyl)ethyl)-6-oxo-1,6-dihydropyrimidine-4-carboxamide

### A) 2-(3-methoxyazetidin-1-yl)-6-((4-methoxybenzyl)oxy)-N-((1S)-2-methoxy-1-(4-(trifluoromethoxy)phenyl)ethyl)pyrimidine-4-carboxamide

By a method similar to that of Example 28, step D, the title compound was obtained.
MS: [M+H]⁺ 563.2.

### B) 2-(3-methoxyazetidin-1-yl)-N-((1S)-2-methoxy-1-(4-(trifluoromethoxy)phenyl)ethyl)-6-oxo-1,6-dihydropyrimidine-4-carboxamide

By a method similar to that of Example 24, step C, the title compound was obtained.

### Example 56

### 2-((2S)-2-(methoxymethyl)pyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 57

### N-(2-methoxy-1-(4-(trifluoromethoxy)phenyl)ethyl)-2-(1-methyl-1H-pyrazol-4-yl)-6-oxo-1,6-dihydropyrimidine-4-carboxamide

By a method similar to that of Example 13, step A and Example 15, step B, the title compound was obtained.

### Example 58

### 2-cyclopentyl-N-((1S)-2-methoxy-1-(4-(trifluoromethoxy)phenyl)ethyl)-6-oxo-1,6-dihydropyrimidine-4-carboxamide

### A) 2-(cyclopent-1-en-1-yl)-6-methoxy-N-((1S)-2-methoxy-1-(4-(trifluoromethoxy)phenyl)ethyl)pyrimidine-4-carboxamide

A mixture of 2-chloro-6-methoxy-N-((1S)-2-methoxy-1-(4-(trifluoromethoxy)phenyl)ethyl)pyrimidine-4-carboxamide (165.9 mg), 2-(cyclopent-1-en-1-yl)-4,4,5,5-tetramethyl-1,3,2-dioxaborolane (238 mg), bis(di-tert-butyl(4-dimethylaminophenyl)phosphine)dichloropalladium(II) (29.0 mg), 2 M aqueous cesium carbonate solution (0.409 mL) and 2-methyl-2-butanol (10 mL) was heated under microwave irradiation at 130°C for 1 hr. After cooling to room temperature, the reaction mixture was poured into water, and the mixture was extracted with ethyl acetate. The extract was washed with water and saturated brine, dried over anhydrous sodium sulfate, and the solvent was evaporated under reduced pressure. The residue was purified by silica gel column chromatography (ethyl acetate/hexane) to give the title compound (135 mg).
MS: [M+H]⁺ 438.1.

### B) 2-cyclopentyl-6-methoxy-N-((1S)-2-methoxy-1-(4-(trifluoromethoxy)phenyl)ethyl)pyrimidine-4-carboxamide

To a mixture of 2-(cyclopent-1-en-1-yl)-6-methoxy-N-((1S)-2-methoxy-1-(4-(trifluoromethoxy)phenyl)ethyl)pyrimidine-4-carboxamide (135.1 mg) and tetrahydrofuran (6.2 mL) was added 5% palladium-carbon (containing water (50%), 100 mg), and the mixture was stirred at under a hydrogen atmosphere, at room temperature for 6 hr. Insoluble material was removed by filtration, and the filtrate was concentrated under reduced pressure. The residue was purified by silica gel column chromatography (ethyl acetate/hexane) to give the title compound (119 mg).
MS: [M+H]⁺ 440.0.

### C) 2-cyclopentyl-N-((1S)-2-methoxy-1-(4-(trifluoromethoxy)phenyl)ethyl)-6-oxo-1,6-dihydropyrimidine-4-carboxamide

By a method similar to that of Example 15, step B, the title compound was obtained.

### Example 59

### N-(1-(3-fluoro-4-(trifluoromethoxy)phenyl)-2-methoxyethyl)-6-oxo-2-(pyrrolidin-1-yl)-1,6-dihydropyrimidine-4-carboxamide

### A) N-(1-(3-fluoro-4-(trifluoromethoxy)phenyl)-2-methoxyethyl)-6-methoxy-2-(pyrrolidin-1-yl)pyrimidine-4-carboxamide

To a solution of 6-methoxy-2-(pyrrolidin-1-yl)pyrimidine-4-carboxylic acid (112 mg) in N,N-dimethylformamide (3.33 mL) were added 1-(3-fluoro-4-(trifluoromethoxy)phenyl)-2-methoxyethanamine hydrochloride (174 mg), 1-(3-dimethylaminopropyl)-3-ethylcarbodiimide hydrochloride (115 mg), 1-hydroxybenzotriazole (81 mg) and triethylamine (0.084 mL) at room temperature. The mixture was stirred at room temperature for 2 hr, poured into water, and the mixture was extracted with ethyl acetate. The extract was washed with water and saturated brine, dried over anhydrous magnesium sulfate, and the solvent was evaporated under reduced pressure. The residue was separated and purified by silica gel column chromatography (ethyl acetate/hexane) to give the title compound (231 mg).
MS: [M+H]⁺ 459.1.

### B) N-(1-(3-fluoro-4-(trifluoromethoxy)phenyl)-2-methoxyethyl)-6-oxo-2-(pyrrolidin-1-yl)-1,6-dihydropyrimidine-4-carboxamide

A mixture of N-(1-(3-fluoro-4-(trifluoromethoxy)phenyl)-2-methoxyethyl)-6-methoxy-2-(pyrrolidin-1-yl)pyrimidine-4-carboxamide (231 mg), pyridine hydrochloride (582 mg) and N,N-dimethylacetamide (3.36 mL) was stirred at 130°C overnight. Water was added to the reaction mixture at room temperature, and the precipitated solid was collected by filtration. The obtained solid was fractionated by HPLC (C18, mobile phase: water/acetonitrile (0.1% TFA-containing system)), to the obtained fraction was added a saturated aqueous sodium hydrogen carbonate solution, and the mixture was extracted with ethyl acetate. The extract was dried over anhydrous magnesium sulfate, and concentrated under reduced pressure to give the title compound (149 mg).

### Example 60

### N-((1R)-1-(3-fluoro-4-(trifluoromethoxy)phenyl)-2-methoxyethyl)-6-oxo-2-(pyrrolidin-1-yl)-1,6-dihydropyrimidine-4-carboxamide

A racemate (104 mg) of N-(1-(3-fluoro-4-(trifluoromethoxy)phenyl)-2-methoxyethyl)-6-oxo-2-(pyrrolidin-1-yl)-1,6-dihydropyrimidine-4-carboxamide was fractionated by HPLC (column: CHIRALPAK IC, 50 mmID×500 mmL, manufactured by Daicel Corporation, mobile phase: hexane/ethanol = 300/700) and crystallized from diisopropyl ether/hexane to give the title compound (39 mg) having a shorter retention time.

### Example 61

### N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide

A racemate (104 mg) of N-(1-(3-fluoro-4-(trifluoromethoxy)phenyl)-2-methoxyethyl)-6-oxo-2-(pyrrolidin-1-yl)-1,6-dihydropyrimidine-4-carboxamide was fractionated by HPLC (column: CHIRALPAK IC, 50 mmID×500 mmL, manufactured by Daicel Corporation, mobile phase: hexane/ethanol = 300/700) and crystallized from diisopropyl ether /hexane to give the title compound (46 mg) having a longer retention time.
¹H NMR (300 MHz, DMSO-d₆) δ 1. 92 (4H, brs), 3.30 (3H, s), 3.53 (4H, brs), 3.61-3.70 (1H, m), 3.70-3.81 (1H, m), 5.12-5.28 (1H, m), 6.05 (1H, s), 7.32 (1H, d, J = 9.0 Hz), 7.47-7.63 (2H, m), 8.68 (1H, d, J = 8.3 Hz), 11.26 (1H, brs).

### Example 62

### N-(1-(3-fluoro-4-(trifluoromethoxy)phenyl)-2-hydroxy-2-methylpropyl)-6-oxo-2-(pyrrolidin-1-yl)-1,6-dihydropyrimidine-4-carboxamide

### A) 2-amino-2-(3-fluoro-4-(trifluoromethoxy)phenyl)acetonitrile

3-Fluoro-4-(trifluoromethoxy)benzaldehyde (10 g) was dissolved in 2 M ammonia/methanol solution (96 mL), and titanium(IV) tetraisopropoxide (15.5 mL) was added under ice-cooling. The reaction mixture was stirred at the same temperature for 15 min, trimethylsilane carbonitrile (9.61 mL) was added, and the mixture was stirred at room temperature for 20 hr. The solvent was evaporated under reduced pressure, and ethyl acetate and saturated aqueous sodium hydrogen carbonate solution were added to the residue. Insoluble material was filtered off through celite, and the filtrate was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (9.4 g).
¹H NMR (300 MHz, CDCl₃) δ 2.01 (2H, brs), 4.93 (1H, s), 7.29-7.53 (3H, m).

### B) tert-butyl (cyano(3-fluoro-4-(trifluoromethoxy)phenyl)methyl)carbamate

To a solution of 2-amino-2-(3-fluoro-4-(trifluoromethoxy)phenyl)acetonitrile (9.4 g) in tetrahydrofuran (200 mL) were added di-tert-butyl dicarbonate (10.3 mL) and triethylamine (7.27 mL) at room temperature. The reaction mixture was stirred at 40°C for 20 hr, water was added, and the mixture was extracted with ethyl acetate. The extract was washed with saturated brine, and dried over anhydrous magnesium sulfate. The solvent was evaporated under reduced pressure. The residue was purified by silica gel column chromatography (ethyl acetate/hexane) to give the title compound (8.47 g).
MS: [M+H]⁺ 335.1.

### C) tert-butyl (2-amino-1-(3-fluoro-4-(trifluoromethoxy)phenyl)-2-oxoethyl)carbamate

To a mixture of tert-butyl (cyano(3-fluoro-4-(trifluoromethoxy)phenyl)methyl)carbamate (9.4 g), potassium carbonate (3.89 g) and dimethyl sulfoxide (200 mL) was added 35% aqueous hydrogen peroxide (4.66 mL) at room temperature. The reaction mixture was stirred at room temperature for 4 hr, water was added, and the mixture was extracted with ethyl acetate. The extract was washed with water, and saturated brine, and dried over anhydrous magnesium sulfate. The solvent was evaporated under reduced pressure. The residue was purified by silica gel column chromatography (ethyl acetate/hexane) to give the title compound (3.84 g). MS: found: 253.0.

### D) methyl 2-((tert-butoxycarbonyl)amino)-2-(3-fluoro-4-(trifluoromethoxy)phenyl)acetate

To a solution of tert-butyl (2-amino-1-(3-fluoro-4-(trifluoromethoxy)phenyl)-2-oxoethyl)carbamate (3.84 g) in methanol (100 mL) was added 8 M aqueous sodium hydroxide solution (2.8 mL) at room temperature. The reaction mixture was heated under reflux overnight, and methanol was evaporated under reduced pressure. The residue was neutralized with 1 M hydrochloric acid, and extracted with ethyl acetate. The extract was washed with saturated brine, and dried over anhydrous magnesium sulfate. The solvent was evaporated under reduced pressure. To a mixture of the residue (3.85 g), potassium carbonate (1.81 g) and N,N-dimethylformamide (50 mL) was added methyl iodide (0.750 mL) at room temperature. The reaction mixture was stirred at room temperature for 2 hr, water was added, and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (1.90 g).
MS: found: 268.0.

### E) tert-butyl (1-(3-fluoro-4-(trifluoromethoxy)phenyl)-2-hydroxy-2-methylpropyl)carbamate

To a solution of methyl 2-((tert-butoxycarbonyl)amino)-2-(3-fluoro-4-(trifluoromethoxy)phenyl)acetate (1.90 g) in tetrahydrofuran (60 mL) was slowly added 3 M methylmagnesium bromide/diethyl ether solution (5.17 mL) at 0°C. The reaction mixture was stirred at room temperature for 3 hr, 1 M hydrochloric acid was added at 0°C, and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (1.53 g).
¹H NMR (300 MHz, CDCl₃) δ 1.06 (3H, s), 1.29-1.50 (13H, m), 4.38-4.52 (1H, m), 5.47-5.60 (1H, m), 7.07-7.30 (3H, m).

### F) 1-amino-1-(3-fluoro-4-(trifluoromethoxy)phenyl)-2-methylpropan-2-ol hydrochloride

To tert-butyl (1-(3-fluoro-4-(trifluoromethoxy)phenyl)-2-hydroxy-2-methylpropyl)carbamate (3.01 g) was added 4 M hydrogen chloride/ethyl acetate solution (30 mL). The reaction mixture was stirred at room temperature for 1 hr and the solvent was evaporated under reduced pressure. The residue was washed with diisopropyl ether to give the title compound (2.22 g).
MS: found: 268.1.

### G) N-(1-(3-fluoro-4-(trifluoromethoxy)phenyl)-2-hydroxy-2-methylpropyl)-6-oxo-2-(pyrrolidin-1-yl)-1,6-dihydropyrimidine-4-carboxamide

By a method similar to that of Example 1, step C and Example 15, step B, the title compound was obtained.

### Example 63

### 2-(dimethylamino)-N-(2-methoxy-1-(4-(trifluoromethoxy)phenyl)ethyl)-5-methyl-6-oxo-1,6-dihydropyrimidine-4-carboxamide

### A) ethyl 2-(dimethylamino)-5-methyl-6-oxo-1,6-dihydropyrimidine-4-carboxylate

A mixture of 1,1-dimethylguanidinium sulfate (1.0 g), diethyl 3-methyloxaloacetate (1.36 mL) and Eaton reagent (7.0 g) was stirred at 80°C for 5 hr. Ice water was added to the reaction mixture, 8 M aqueous sodium hydroxide solution was added, and the mixture was extracted with ethyl acetate. The extract was dried over anhydrous sodium sulfate, and the solvent was evaporated under reduced pressure. The residue was purified by silica gel column chromatography (ethyl acetate/hexane) to give the title compound (0.16 g).
MS: [M+H]⁺ 226.1.

### B) 2-(dimethylamino)-5-methyl-6-oxo-1,6-dihydropyrimidine-4-carboxylic acid

By a method similar to that of Example 4, step C and Example 1, step C, the title compound was obtained.

### Example 64

### N-(1-(3-fluoro-4-(trifluoromethyl)phenyl)-2-methoxyethyl)-6-oxo-2-(pyrrolidin-1-yl)-1,6-dihydropyrimidine-4-carboxamide

### A) 1-(3-fluoro-4-(trifluoromethyl)phenyl)-2-methoxyethanamine hydrochloride

By a method similar to that of Example 1, step A and step B, the title compound was obtained.
MS: found: 238.1.

### B) N-(1-(3-fluoro-4-(trifluoromethyl)phenyl)-2-methoxyethyl)-6-methoxy-2-(pyrrolidin-1-yl)pyrimidine-4-carboxamide

By a method similar to that of Example 1, step C, the title compound was obtained.
MS: [M+H]⁺ 443.1.

### C) N-(1-(3-fluoro-4-(trifluoromethyl)phenyl)-2-methoxyethyl)-6-oxo-2-(pyrrolidin-1-yl)-1,6-dihydropyrimidine-4-carboxamide

By a method similar to that of Example 15, step B, the title compound was obtained.

### Example 65

### N-(3-methoxy-1-(4-(trifluoromethoxy)phenyl)propyl)-6-oxo-2-(pyrrolidin-1-yl)-1,6-dihydropyrimidine-4-carboxamide

### A) 1-(4-(trifluoromethoxy)phenyl)prop-2-en-1-ol

To a solution of 4-(trifluoromethoxy)benzaldehyde (12.0 g) in tetrahydrofuran (200 mL) was slowly added 1 M vinylmagnesium bromide/tetrahydrofuran solution (69.5 mL) under a nitrogen atmosphere at -78°C. The reaction mixture was heated slowly to room temperature, and stirred under a nitrogen atmosphere at room temperature for 16 hr. To the reaction mixture was added saturated aqueous ammonium chloride solution, and the mixture was extracted with ethyl acetate. The extract was washed with saturated brine, and dried over anhydrous sodium sulfate. The solvent was evaporated under reduced pressure. The residue was purified by silica gel column chromatography (petroleum ether/ethyl acetate) to give the title compound (11.8 g).
¹H NMR (400 MHz, DMSO-d₆) δ 5.02-5.13 (2H, m), 5.26 (1H, dt, J = 17.2, 1.6 Hz), 5.64 (1H, d, J = 4.4 Hz), 5.87-5.99 (1H, m), 7.31 (2H, d, J = 8.0 Hz), 7.44 (2H, dd, J = 6.8, 1.6 Hz).

### B) 1-(4-(trifluoromethoxy)phenyl)prop-2-en-1-one

To a solution of 1-(4-(trifluoromethoxy)phenyl)prop-2-en-1-ol (9.80 g) in dichloromethane (150 mL) was added manganese dioxide (IV) (39.1 g). The reaction mixture was stirred at room temperature for 2 days. Insoluble material was filtered off, and the filtrate was concentrated under reduced pressure. The residue was purified by silica gel column chromatography (petroleum ether/ethyl acetate) to give the title compound (2.75 g).
¹H NMR (400 MHz, CDCl₃) δ 5.97 (1H, dd, J = 10.8, 1.6 Hz), 6.46 (1H, dd, J = 17.2, 1.6 Hz), 7.13 (1H, dd, J = 17.2, 10.8 Hz), 7.31 (2H, dd, J = 8.8, 0.8 Hz), 8.00 (2H, dd, J = 6.8, 2.0 Hz).

### C) 3-methoxy-1-(4-(trifluoromethoxy)phenyl)propan-1-one

A mixture of 1-(4-(trifluoromethoxy)phenyl)prop-2-en-1-one (2.75 g), methanol (407 mg), bis(acetonitrile)dichloropalladium(II) (328 mg) and dichloromethane (30 mL) was stirred under a nitrogen atmosphere at room temperature for 16 hr, and dichloromethane was added. Insoluble material was filtrated off. The filtrate was distilled under reduced pressure, and the residue was purified by silica gel column chromatography (petroleum ether/ethyl acetate) to give the title compound (2.80 g).
¹H NMR (400 MHz, CDCl₃) δ 3.22 (2H, t, J = 6.4 Hz), 3.38 (3H, s), 3.82 (2H, t, J = 6.4 Hz), 7.29 (2H, d, J = 8.4 Hz), 8.02 (2H, dd, J = 6.8, 2.0 Hz).

### D) 3-methoxy-1-(4-(trifluoromethoxy)phenyl)propan-1-amine hydrochloride

By a method similar to that of Example 1, step B, the title compound was obtained.
MS: [M+H]⁺ 250.0.

### E) 6-methoxy-N-(3-methoxy-1-(4-(trifluoromethoxy)phenyl)propyl)-2-(pyrrolidin-1-yl)pyrimidine-4-carboxamide

By a method similar to that of Example 1, step C, the title compound was obtained.
MS: [M+H]⁺ 455.1.

### F) N-(3-methoxy-1-(4-(trifluoromethoxy)phenyl)propyl)-6-oxo-2-(pyrrolidin-1-yl)-1,6-dihydropyrimidine-4-carboxamide

By a method similar to that of Example 15, step B, the title compound was obtained.

### Example 66

### 2-(6,6-difluoro-3-azabicyclo[3.1.0]hex-3-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 67

### 2-((3S)-3-isopropoxypyrrolidin-1-yl)-N-((1S)-2-methoxy-1-(4-(trifluoromethoxy)phenyl)ethyl)-6-oxo-1,6-dihydropyrimidine-4-carboxamide

### A) tert-butyl (S)-3-isopropoxypyrrolidine-1-carboxylate

A mixture of tert-butyl (S)-3-hydroxypyrrolidine-1-carboxylate (1.0 g), silver oxide (I) (1.49 g) and 2-iodopropane (2.67 mL) was stirred under a nitrogen atmosphere at 40°C overnight. The reaction mixture was filtered through celite, and the solvent of the filtrate was evaporated under reduced pressure. The residue was purified by silica gel column chromatography (ethyl acetate/hexane) to give the title compound (223 mg).
¹H NMR (300 MHz, CDCl₃) δ 1.11-1.22 (6H, m), 1.46 (9H, s), 1.79-1.98 (2H, m), 3.17-3.53 (4H, m), 3.63 (1H, quin, J = 6.1 Hz), 4.00-4.19 (1H, m).

### B) (S)-3-isopropoxypyrrolidine hydrochloride

To a mixture of tert-butyl (S)-3-isopropoxypyrrolidine-1-carboxylate (220 mg) and ethyl acetate (1 mL) was added 4 M hydrogen chloride/ethyl acetate solution (3 mL). The mixture was stirred at room temperature overnight, and the solvent was evaporated under reduced pressure to give the title compound (220 mg).
¹H NMR (300 MHz, DMSO-d₆) δ 1.09 (6H, d, J = 6.4 Hz), 1.87-1.96 (2H, m), 3.02-3.25 (4H, m), 3.64 (1H, dt, J = 12.3, 6.1 Hz), 4.27 (1H, d, J = 2.6 Hz), 8.89-9.60 (2H, m).

### C) 2-((3S)-3-isopropoxypyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 68

### N-((1S)-2-methoxy-1-(4-(trifluoromethoxy)phenyl)ethyl)-2-(3-oxa-8-azabicyclo[3.2.1]oct-8-yl)-6-oxo-1,6-dihydropyrimidine-4-carboxamide

By a method similar to that of Example 39, the title compound was obtained.

### Example 69

### 2-((3R)-3-fluoropyrrolidin-1-yl)-N-((1S)-2-methoxy-1-(4-(trifluoromethoxy)phenyl)ethyl)-6-oxo-1,6-dihydropyrimidine-4-carboxamide

By a method similar to that of Example 39, the title compound was obtained.

### Example 70

### N-(1-(4-cyclopropylphenyl)-2-methoxyethyl)-6-oxo-2-(pyrrolidin-1-yl)-1,6-dihydropyrimidine-4-carboxamide

### A) 1-(4-bromophenyl)-2-methoxyethanone

By a method similar to that of Example 1, step A, the title compound was obtained.
¹H NMR (300 MHz, CDCl₃) δ 3.50 (3H, s), 4.65 (2H, s), 7.55-7.68 (2H, m), 7.75-7.87 (2H, m).

### B) 1-(4-cyclopropylphenyl)-2-methoxyethanone

A mixture of 1-(4-bromophenyl)-2-methoxyethanone (2.22 g), cyclopropylboronic acid (1.082 g), (1,1-bis(diphenylphosphino)ferrocene)dichloropalladium(II) (0.355 g), tripotassium phosphate (4.11 g) in 1,2-dimethoxyethane (30 mL) and water (10 mL) was stirred under a nitrogen atmosphere at 85°C for 16 hr. Water was added to the reaction mixture, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (1.58 g). MS: [M+H]⁺ 191.1.

### C) N-(1-(4-cyclopropylphenyl)-2-methoxyethyl)-6-methoxy-2-(pyrrolidin-1-yl)pyrimidine-4-carboxamide

A reaction similar to that of Example 1, step B and step C was performed to give the title compound.
MS: [M+H]⁺ 397.2.

### D) N-(1-(4-cyclopropylphenyl)-2-methoxyethyl)-6-oxo-2-(pyrrolidin-1-yl)-1,6-dihydropyrimidine-4-carboxamide

By a method similar to that of Example 15, step B, the title compound was obtained.

### Example 71

### 2-cyclopropyl-N-(2-methoxy-1-(4-(trifluoromethoxy)phenyl)ethyl)-6-oxo-1,6-dihydropyrimidine-4-carboxamide

### A) 2-cyclopropyl-6-oxo-1,6-dihydropyrimidine-4-carboxylic acid

By a method similar to that of Example 34, step A, the title compound was obtained.
¹H NMR (300 MHz, DMSO-d₆) δ 1.00-1.08 (4H, m), 1.88-2.03 (1H, m), 4.76 (1H, brs), 6.57 (1H, s), 13.23 (1H, brs).

### B) 2-cyclopropyl-N-(2-methoxy-1-(4-(trifluoromethoxy)phenyl)ethyl)-6-oxo-1,6-dihydropyrimidine-4-carboxamide

By a method similar to that of Example 1, step C, the title compound was obtained.

### Example 72

### N-((1S)-2-methoxy-1-(4-(trifluoromethoxy)phenyl)ethyl)-6-oxo-2-(3-(trifluoromethyl)pyrrolidin-1-yl)-1,6-dihydropyrimidine-4-carboxamide

By a method similar to that of Example 39, the title compound was obtained.

### Example 73

### N-((1S)-1-(3-fluoro-4-(trifluoromethoxy)phenyl)-2-methoxyethyl)-2-(6-oxa-3-azabicyclo[3.1.1]hept-3-yl)-6-oxo-1,6-dihydropyrimidine-4-carboxamide

### A) N,2-dimethoxy-N-methylacetamide

To a mixture of N,O-dimethylhydroxylamine hydrochloride (24.7 g), potassium carbonate (63.7 g) and acetonitrile (330 mL) was added 2-methoxyacetylchloride (25.0 g) at 10°C or below. The reaction mixture was stirred at room temperature overnight. Insoluble material was filtered off, and the filtrate was concentrated under reduced pressure. The residue was distilled (boiling point: 60°C/0.8 KPa) to give the title compound (25.8 g).
¹H NMR (300 MHz, CDCl₃) δ 3.20 (3H, s), 3.47 (3H, d, J = 0.8 Hz), 3.69 (3H, s), 4.22 (2H, s).

### B) 1-(3-fluoro-4-(trifluoromethoxy)phenyl)-2-methoxyethanone

To a mixture of 4-bromo-2-fluoro-1-(trifluoromethoxy)benzene (25.0 g), N,2-dimethoxy-N-methylacetamide (15.4 g) and tetrahydrofuran (400 mL) was slowly added 1.6 M n-butyllithium/hexane solution (72.4 mL) under a nitrogen atmosphere at -78°C. The reaction mixture was stirred under a nitrogen atmosphere at -78°C for 20 min, neutralized with 0.1 M hydrochloric acid, and extracted with ethyl acetate. The extract was washed with water and saturated brine, and dried over anhydrous sodium sulfate. The solvent was evaporated under reduced pressure. The residue was purified by silica gel column chromatography (hexane/ethyl acetate) to give the title compound (15.7 g).
¹H NMR (300 MHz, CDCl₃) δ 3.50 (3H, s), 4.63 (2H, s), 7.42 (1H, ddd, J = 8.6, 7.3, 1.5 Hz), 7.75-7.81 (1H, m), 7.83 (1H, dd, J = 10.2, 1.9 Hz).

### C) 1-(3-fluoro-4-(trifluoromethoxy)phenyl)-N-hydroxy-2-methoxyethanimine

To a solution of 1-(3-fluoro-4-(trifluoromethoxy)phenyl)-2-methoxyethanone (7.56 g) in ethanol (250 mL) were added hydroxylamine hydrochloride (4.17 g) and triethylamine (8.36 mL) at room temperature. The reaction mixture was stirred at room temperature for 16 hr and the solvent was evaporated under reduced pressure. Water was added to the residue and the mixture was extracted with ethyl acetate. The extract was washed with water and saturated brine, and dried over anhydrous magnesium sulfate. The solvent was evaporated under reduced pressure. The residue was purified by silica gel column chromatography (hexane/ethyl acetate) to give the title compound (7.88 g).
MS: [M+H]⁺ 268.0.

### D) tert-butyl (1-(3-fluoro-4-(trifluoromethoxy)phenyl)-2-methoxyethyl)carbamate

To a solution of 1-(3-fluoro-4-(trifluoromethoxy)phenyl)-N-hydroxy-2-methoxyethanimine (7.88 g) in ethanol (200 mL) was added 10% palladium-carbon (containing water (50%), 1.00 g). The reaction mixture was stirred under a hydrogen atmosphere at room temperature overnight. Insoluble material was filtered off, and the filtrate was concentrated under reduced pressure. To a solution of the residue in tetrahydrofuran (200 mL) were added di-tert-butyl dicarbonate (7.53 mL) and triethylamine (6.17 mL), and the mixture was stirred at room temperature for 16 hr. Water was added to the reaction mixture, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (8.25 g). ¹H NMR (300 MHz, CDCl₃) δ 1.42 (9H, brs), 3.35 (3H, s), 3.49-3.67 (2H, m), 4.78 (1H, brs), 5.34 (1H, brs), 7.08-7.29 (3H, m).

### E) tert-butyl ((1S)-1-(3-fluoro-4-(trifluoromethoxy)phenyl)-2-methoxyethyl)carbamate

A racemate (12.48 g) of tert-butyl (1-(3-fluoro-4-(trifluoromethoxy)phenyl)-2-methoxyethyl)carbamate was fractionated by HPLC (column: CHIRALPAK AD, 50 mmID×500 mmL, manufactured by Daicel Corporation, mobile phase: hexane/ethanol = 950/50) to give the title compound (5.73 g) having a shorter retention time.
MS: found: 254.0.

### F) (1S)-1-(3-fluoro-4-(trifluoromethoxy)phenyl)-2-methoxyethanamine hydrochloride

A mixture of tert-butyl ((1S)-1-(3-fluoro-4-(trifluoromethoxy)phenyl)-2-methoxyethyl)carbamate (5.73 g) and 4 M hydrogen chloride/ethyl acetate solution (100 mL) was stirred at room temperature for 3 hr. The solvent was evaporated under reduced pressure, and the residue was washed with diisopropyl ether to give the title compound (3.22 g). MS: found: 254.0.

### G) 2-(6-oxa-3-azabicyclo[3.1.1]hept-3-yl)-N-((S)-1-(3-fluoro-4-(trifluoromethoxy)phenyl)-2-methoxyethyl)-6-((4-methoxybenzyl)oxy)pyrimidine-4-carboxamide

By a method similar to that of Example 28, step D, the title compound was obtained.
MS: [M+H]⁺ 593.2.

### H) N-((1S)-1-(3-fluoro-4-(trifluoromethoxy)phenyl)-2-methoxyethyl)-2-(6-oxa-3-azabicyclo[3.1.1]hept-3-yl)-6-oxo-1,6-dihydropyrimidine-4-carboxamide

By a method similar to that of Example 24, step C, the title compound was obtained.

### Example 74

### N-((1S)-1-(3-fluoro-4-(trifluoromethoxy)phenyl)-2-methoxyethyl)-2-((3S)-3-methoxypyrrolidin-1-yl)pyrimidine-4-carboxamide

By a method similar to that of Example 9, step A and B, the title compound was obtained.

### Example 75

### 2-(dimethylamino)-N-(2-methoxy-1-(4-(1H-pyrazol-1-yl)phenyl)ethyl)-6-oxo-1,6-dihydropyrimidine-4-carboxamide

### A) 2-(4-bromophenyl)-2-(methoxymethyl)-1,3-dioxolane

A mixture of 1-(4-bromophenyl)-2-methoxyethanone (3.00 g), p-toluenesulfonic acid monohydrate (0.249 g), ethylene glycol (1.63 g) and toluene (50 mL) was stirred using a Dean-Stark apparatus at 140°C overnight. The reaction mixture was filtered through a silica gel pad (NH) and the solvent was evaporated under reduced pressure to give the title compound (3.97 g).
¹H NMR (300 MHz, CDCl₃) δ 3.39 (3H, s), 3.57 (2H, s), 3.76-3.92 (2H, m), 4.02-4.19 (2H, m), 7.35-7.43 (2H, m), 7.43-7.52 (2H, m).

### B) 1-(4-(2-(methoxymethyl)-1,3-dioxolan-2-yl)phenyl)-1H-pyrazole

A mixture of 2-(4-bromophenyl)-2-(methoxymethyl)-1,3-dioxolane (3.5 g), 1H-pyrazole (0.960 g), quinolin-8-ol (0.372 g), copper iodide (I) (0.244 g), potassium carbonate (3.54 g) and dimethyl sulfoxide (50 mL) was stirred under a nitrogen atmosphere at 140°C overnight, water was added, and the mixture was extracted with ethyl acetate. The extract was washed with saturated aqueous ammonium chloride solution and saturated brine, dried over anhydrous magnesium sulfate and filtered through an NH silica gel pad. The filtrate was concentrated under reduced pressure, and the residue was purified by silica gel column chromatography (hexane/ethyl acetate) to give the title compound (2.53 g).
MS: [M+H]⁺ 261.1.

### C) 2-methoxy-1-(4-(1H-pyrazol-1-yl)phenyl)ethanone

A mixture of 1-(4-(2-(methoxymethyl)-1,3-dioxolan-2-yl)phenyl)-1H-pyrazole (2.6 g), 1 M hydrochloric acid (15 mL) and tetrahydrofuran (30 mL) was stirred at room temperature overnight, saturated brine was added, and the mixture was extracted with ethyl acetate. The extract was washed with saturated brine, and dried over anhydrous magnesium sulfate. The solvent was evaporated under reduced pressure to give the title compound (2.15 g).
¹H NMR (300 MHz, CDCl₃) δ 3.52 (3H, s), 4.71 (2H, s), 6.48-6.56 (1H, m), 7.78 (1H, d, J = 1.9 Hz), 7.79-7.87 (2H, m), 8.02 (1H, d, J = 2.6 Hz), 8.03-8.10 (2H, m).

### D) 2-methoxy-1-(4-(1H-pyrazol-1-yl)phenyl)ethanamine

To a solution of 2-methoxy-1-(4-(1H-pyrazol-1-yl)phenyl)ethanone (4.77 g) in ethanol (111 mL) were added hydroxylamine hydrochloride (2.033 g) and triethylamine (6.20 mL) at room temperature. The reaction mixture was stirred at room temperature for 72 hr and the solvent was evaporated under reduced pressure. Water was added to the residue and the mixture was extracted with ethyl acetate. The extract was washed with water and saturated brine, and dried over anhydrous sodium sulfate. The solvent was evaporated under reduced pressure. To a solution of the residue in methanol (316 mL) was added 10% palladium-carbon (containing water (50%), 500 mg). The reaction mixture was stirred under a hydrogen atmosphere at room temperature overnight. The catalyst was filtered off, and the filtrate was distilled under reduced pressure to give the title compound (4.39 g).
¹H NMR (300 MHz, CDCl₃) δ 1.75 (2H, brs), 3.31-3.43 (4H, m), 3.51 (1H, dd, J = 9.3, 4.0 Hz), 4.24 (1H, dd, J = 8.7, 4.0 Hz),6.42-6.50 (1H, m), 7.43-7.52 (2H, m), 7.61-7.69 (2H, m), 7.71 (1H, d, J = 1.5 Hz), 7.91 (1H, d, J = 2.5 Hz).

### E) 2-(dimethylamino)-N-(2-methoxy-1-(4-(1H-pyrazol-1-yl)phenyl)ethyl)-6-oxo-1,6-dihydropyrimidine-4-carboxamide

By a method similar to that of Example 1, step C, the title compound was obtained.

### Example 76

### 2-(ethyl(methyl)amino)-N-(1S)-1-(3-fluoro-4-(trifluoromethoxy)phenyl)-2-methoxyethyl)-6-oxo-1,6-dihydropyrimidine-4-carboxamide

### A) methyl 2-(ethyl(methyl)amino)-6-methoxypyrimidine-4-carboxylate

By a method similar to that of Example 9, step B, the title compound was obtained.
MS: [M+H]⁺ 226.1.

### B) 2-(ethyl(methyl)amino)-6-methoxypyrimidine-4-carboxylic acid

By a method similar to that of Example 4, step C, the title compound was obtained.
MS: [M+H]⁺ 212.1.

### C) (S)-2-(ethyl(methyl)amino)-N-(1-(3-fluoro-4-(trifluoromethoxy)phenyl)-2-methoxyethyl)-6-methoxypyrimidine-4-carboxamide

By a method similar to that of Example 1, step C, the title compound was obtained.
MS: [M+H]⁺ 447.2.

### D) 2-(ethyl(methyl)amino)-N-((1S)-1-(3-fluoro-4-(trifluoromethoxy)phenyl)-2-methoxyethyl)-6-oxo-1,6-dihydropyrimidine-4-carboxamide

By a method similar to that of Example 15, step B, the title compound was obtained.

### Example 77

### 5-chloro-N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide

### A) (S)-N-(1-(3-fluoro-4-(trifluoromethoxy)phenyl)-2-methoxyethyl)-6-methoxy-2-(pyrrolidin-1-yl)pyrimidine-4-carboxamide

By a method similar to that of Example 1, step C, the title compound was obtained.
MS: [M+H]⁺ 459.2.

### B) (S)-5-chloro-N-(1-(3-fluoro-4-(trifluoromethoxy)phenyl)-2-methoxyethyl)-6-methoxy-2-(pyrrolidin-1-yl)pyrimidine-4-carboxamide

To a mixture of (S)-N-(1-(3-fluoro-4-(trifluoromethoxy)phenyl)-2-methoxyethyl)-6-methoxy-2-(pyrrolidin-1-yl)pyrimidine-4-carboxamide (50 mg) and acetonitrile (1 mL) was added N-chlorosuccinimide (14.6 mg) under ice-cooling. Under a nitrogen atmosphere, the mixture was stirred under ice-cooling for 15 min, at room temperature for 3 hr, and at 80°C overnight. The solvent in the reaction mixture was evaporated under reduced pressure to give the title compound (64 mg).
MS: [M+H]⁺ 493.1.

### C) 5-chloro-N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide

By a method similar to that of Example 15, step B, the title compound was obtained.

### Example 78

### N-((1S)-1-(3-fluoro-4-(trifluoromethoxy)phenyl)-2-methoxyethyl)-2-(1,2-oxazolidin-2-yl)-6-oxo-1,6-dihydropyrimidine-4-carboxamide

### A) (S)-N-(1-(3-fluoro-4-(trifluoromethoxy)phenyl)-2-methoxyethyl)-2-(isoxazolidin-2-yl)-6-((4-methoxybenzyl)oxy)pyrimidine-4-carboxamide

By a method similar to that of Example 28, step D, the title compound was obtained.
MS: [M+H]⁺ 567.2.

### B) N-((1S)-1-(3-fluoro-4-(trifluoromethoxy)phenyl)-2-methoxyethyl)-2-(1,2-oxazolidin-2-yl)-6-oxo-1,6-dihydropyrimidine-4-carboxamide

By a method similar to that of Example 24, step C, the title compound was obtained.

### Example 79

### N-((1S)-1-(3-fluoro-4-(trifluoromethoxy)phenyl)-2-methoxyethyl)-2-isopropyl-6-oxo-1,6-dihydropyrimidine-4-carboxamide

### A) 2-isopropyl-6-oxo-1,6-dihydropyrimidine-4-carboxylic acid

By a method similar to that of Example 34, step A, the title compound was obtained.
MS: [M+H]⁺ 183.1.

### B) N-((1S)-1-(3-fluoro-4-(trifluoromethoxy)phenyl)-2-methoxyethyl)-2-isopropyl-6-oxo-1,6-dihydropyrimidine-4-carboxamide

By a method similar to that of Example 1, step C, the title compound was obtained.

### Example 80

### 2-tert-butyl-N-((1S)-1-(3-fluoro-4-(trifluoromethoxy)phenyl)-2-methoxyethyl)-6-oxo-1,6-dihydropyrimidine-4-carboxamide

### A) 2-(tert-butyl)-6-oxo-1,6-dihydropyrimidine-4-carboxylic acid

By a method similar to that of Example 34, step A, the title compound was obtained.
MS: [M+H]⁺ 197.1.

### B) 2-tert-butyl-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 81

### 2-cyclopropyl-N-((1S)-1-(3-fluoro-4-(trifluoromethoxy)phenyl)-2-methoxyethyl)-6-oxo-1,6-dihydropyrimidine-4-carboxamide

By a method similar to that of Example 34, step A and Example 1, step C, the title compound was obtained.

### Example 82

### N-((1S)-1-(3-fluoro-4-(trifluoromethoxy)phenyl)-2-methoxyethyl)-2-(isobutyl(methyl)amino)-6-oxo-1,6-dihydropyrimidine-4-carboxamide

### A) 2-chloro-N-((1S)-1-(3-fluoro-4-(trifluoromethoxy)phenyl)-2-methoxyethyl)-6-methoxypyrimidine-4-carboxamide

By a method similar to that of Example 9, step A, the title compound was obtained.
MS: [M+H]⁺ 424.1.

### B) N-((1S)-1-(3-fluoro-4-(trifluoromethoxy)phenyl)-2-methoxyethyl)-2-(isobutyl(methyl)amino)-6-oxo-1,6-dihydropyrimidine-4-carboxamide

By a method similar to that of Example 39, the title compound was obtained.

### Example 83

### 2-(cyclopent-1-en-1-yl)-N-((1S)-1-(3-fluoro-4-(trifluoromethoxy)phenyl)-2-methoxyethyl)-6-oxo-1,6-dihydropyrimidine-4-carboxamide

### A) 2-(cyclopent-1-en-1-yl)-N-((1S)-1-(3-fluoro-4-(trifluoromethoxy)phenyl)-2-methoxyethyl)-6-methoxypyrimidine-4-carboxamide

By a method similar to that of Example 58, step A, the title compound was obtained.
MS: [M+H]⁺ 456.1.

### B) 2-(cyclopent-1-en-1-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 15, step B, the title compound was obtained.

### Example 84

### 2-(cyclohex-1-en-1-yl)-N-((1S)-1-(3-fluoro-4-(trifluoromethoxy)phenyl)-2-methoxyethyl)-6-oxo-1,6-dihydropyrimidine-4-carboxamide

### A) 2-(cyclohex-1-en-1-yl)-N-((1S)-1-(3-fluoro-4-(trifluoromethoxy)phenyl)-2-methoxyethyl)-6-methoxypyrimidine-4-carboxamide

By a method similar to that of Example 58, step A, the title compound was obtained.
MS: [M+H]⁺ 470.1.

### B) 2-(cyclohex-1-en-1-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 15, step B, the title compound was obtained.

### Example 85

### 2-((cyclopropylmethyl)(methyl)amino)-N-((1S)-1-(3-fluoro-4-(trifluoromethoxy)phenyl)-2-methoxyethyl)-6-oxo-1,6-dihydropyrimidine-4-carboxamide

By a method similar to that of Example 39, the title compound was obtained.

### Example 86

### N-((1S)-1-(3-fluoro-4-(trifluoromethoxy)phenyl)-2-methoxyethyl)-6-oxo-2-(tetrahydro-2H-pyran-4-yl)-1,6-dihydropyrimidine-4-carboxamide

### A) 2-(3,6-dihydro-2H-pyran-4-yl)-N-((1S)-1-(3-fluoro-4-(trifluoromethoxy)phenyl)-2-methoxyethyl)-6-methoxypyrimidine-4-carboxamide

By a method similar to that of Example 58, step A, the title compound was obtained.
MS: [M+H]⁺ 472.2.

### B) N-((1S)-1-(3-fluoro-4-(trifluoromethoxy)phenyl)-2-methoxyethyl)-6-oxo-2-(tetrahydro-2H-pyran-4-yl)-1,6-dihydropyrimidine-4-carboxamide

An operation similar to that of Example 58, step B, Example 15, step B was performed to give the title compound.

### Example 87

### N-((1S)-1-(3-fluoro-4-(trifluoromethoxy)phenyl)-2-methoxyethyl)-6-oxo-2-pheny1-1,6-dihydropyrimidine-4-carboxamide

### A) 6-oxo-2-phenyl-1,6-dihydropyrimidine-4-carboxylic acid

To diethyl oxaloacetate sodium salt (2.10 g) was added 2 M aqueous sodium hydroxide solution (25 mL) at room temperature, and the mixture was stirred at room temperature for 30 min. Benzamidine hydrochloride (1.566 g) was added, and the mixture was stirred at room temperature overnight, and further stirred at 60°C for 5 hr. The reaction mixture was acidified with 2 M hydrochloric acid at 0°C, and the precipitate was collected by filtration to give a pale-brown solid (1.3 g). A mixture of the obtained solid (0.65 g) and ethyl acetate (100 mL) was heated under reflux for 10 hr, and the solid was collected by filtration to give the title compound (0.30 g).
MS: [M+H]⁺ 217.1.

### B) N-((1S)-1-(3-fluoro-4-(trifluoromethoxy)phenyl)-2-methoxyethyl)-6-oxo-2-pheny1-1,6-dihydropyrimidine-4-carboxamide

By a method similar to that of Example 1, step C, the title compound was obtained.

### Example 88

### N-((1S)-1-(3-fluoro-4-(trifluoromethoxy)phenyl)-2-methoxyethyl)-6-oxo-2-(pyridin-2-yl)-1,6-dihydropyrimidine-4-carboxamide

By a method similar to that of Example 1, step C, the title compound was obtained.

### Example 89

### N-((1S)-1-(3-fluoro-4-(trifluoromethoxy)phenyl)-2-methoxyethyl)-6-oxo-2-(pyridin-4-yl)-1,6-dihydropyrimidine-4-carboxamide

By a method similar to that of Example 87, step A and Example 1, step C, the title compound was obtained.

### Example 90

### N-((1S)-1-(3-fluoro-4-(trifluoromethoxy)phenyl)-2-methoxyethyl)-6-oxo-2-(pyridin-3-yl)-1,6-dihydropyrimidine-4-carboxamide

By a method similar to that of Example 87, step A and Example 1, step C, the title compound was obtained.

### Example 91

### 2-cyclohexyl-N-((1S)-1-(3-fluoro-4-(trifluoromethoxy)phenyl)-2-methoxyethyl)-6-oxo-1,6-dihydropyrimidine-4-carboxamide

By a method similar to that of Example 58, step B, Example 15, step B, the title compound was obtained.

### Example 92

### N-((1S)-1-(3-fluoro-4-(trifluoromethoxy)phenyl)-2-methoxyethyl)-2-(4-methyl-1H-imidazol-1-yl)-6-oxo-1,6-dihydropyrimidine-4-carboxamide

By a method similar to that of Example 42 and Example 15, step B, the title compound was obtained.

### Example 93

### N-((1S)-1-(3-fluoro-4-(trifluoromethoxy)phenyl)-2-methoxyethyl)-6-oxo-2-(tetrahydrofuran-2-yl)-1,6-dihydropyrimidine-4-carboxamide

### A) 2-(4,5-dihydrofuran-2-yl)-N-((1S)-1-(3-fluoro-4-(trifluoromethoxy)phenyl)-2-methoxyethyl)-6-methoxypyrimidine-4-carboxamide

By a method similar to that of Example 58, step A, the title compound was obtained.
MS: [M+H]⁺ 458.1.

### B) N-((1S)-1-(3-fluoro-4-(trifluoromethoxy)phenyl)-2-methoxyethyl)-6-methoxy-2-(tetrahydrofuran-2-yl)pyrimidine-4-carboxamide

By a method similar to that of Example 58, step B, the title compound was obtained.
MS: [M+H]⁺ 460.1.

### C) N-((1S)-1-(3-fluoro-4-(trifluoromethoxy)phenyl)-2-methoxyethyl)-6-oxo-2-(tetrahydrofuran-2-yl)-1,6-dihydropyrimidine-4-carboxamide

By a method similar to that of Example 15, step B, the title compound was obtained.

### Example 94

### N-((1S)-1-(3-fluoro-4-(trifluoromethoxy)phenyl)-2-methoxyethyl)-2-(isopropyl(methyl)amino)-6-oxo-1,6-dihydropyrimidine-4-carboxamide

By a method similar to that of Example 39, the title compound was obtained.

### Example 95

### 2-tert-butyl-N-(1-(3-fluoro-4-(trifluoromethoxy)phenyl)-2-hydroxy-2-methylpropyl)-6-oxo-1,6-dihydropyrimidine-4-carboxamide

### A) ethyl 2-((diphenylmethylene)amino)-2-(3-fluoro-4-(trifluoromethoxy)phenyl)acetate

A mixture of ethyl 2-((diphenylmethylene)amino)acetate (24.36 g), 4-bromo-2-fluoro-1-(trifluoromethoxy)benzene (22.48 g) and tripotassium phosphate (55.3 g) in toluene (289 mL) was purged with argon, and bis(tri-tert-butylphosphine)palladium(0) (2.218 g) was added at room temperature. The reaction mixture was stirred at 100°C for 24 hr. To the reaction mixture were added water and ethyl acetate, insoluble material was filtrated off, and the solvent was evaporated under reduced pressure. The residue was purified by silica gel column chromatography (hexane/ethyl acetate) to give the title compound (27.2 g).
MS: [M+H]⁺ 446.1.

### B) tert-butyl (1-(3-fluoro-4-(trifluoromethoxy)phenyl)-2-hydroxy-2-methylpropyl)carbamate

To a solution of ethyl 2-((diphenylmethylene)amino)-2-(3-fluoro-4-(trifluoromethoxy)phenyl)acetate (27.2 g) in tetrahydrofuran (305 mL) were slowly added a solution (183 mL) of 1 M methylmagnesium bromide in tetrahydrofuran at 0°C. The reaction mixture was stirred under an argon atmosphere at 0°C for 1 hr. To the reaction mixture was added 2 M hydrochloric acid (245 mL) at 0°C, and the mixture was stirred at room temperature for 2 hr. Tetrahydrofuran in the reaction mixture was removed under reduced pressure and the mixture was extracted with diethyl ether. The aqueous layer was neutralized with 2 M aqueous sodium hydroxide solution, and extracted with ethyl acetate. The organic layer was dried over anhydrous magnesium sulfate, and the solvent was concentrated under reduced pressure to give a crude product (7.60 g). The obtained crude product was used for the next reaction without purification. A solution of the crude product (7.60 g) and di-tert-butyl dicarbonate (7.26 mL), triethylamine (5.95 mL) in tetrahydrofuran (142 mL) solution was stirred at room temperature overnight. The reaction mixture was concentrated under reduced pressure, and the residue was purified by silica gel column chromatography (hexane/ethyl acetate) to give the title compound (9.70 g).
MS: [M-H]- 366.0.

### C) tert-butyl ((1S)-1-(3-fluoro-4-(trifluoromethoxy)phenyl)-2-hydroxy-2-methylpropyl)carbamate

A racemate (9.70 g) of tert-butyl (1-(3-fluoro-4-(trifluoromethoxy)phenyl)-2-hydroxy-2-methylpropyl)carbamate was fractionated by HPLC (column: CHIRALPAK AD, 50 mmID×500 mmL, manufactured by Daicel Corporation, mobile phase: hexane/ethanol = 950/50) to give the title compound (4.65 g) having a shorter retention time.
MS: [M-H]- 366.0.

### D) (S)-1-amino-1-(3-fluoro-4-(trifluoromethoxy)phenyl)-2-methylpropan-2-ol hydrochloride

To a mixture of tert-butyl ((1S)-1-(3-fluoro-4-(trifluoromethoxy)phenyl)-2-hydroxy-2-methylpropyl)carbamate (2.86 g) and ethyl acetate (40 mL) was added 4 M hydrogen chloride/ethyl acetate solution (40 mL). The reaction mixture was stirred at room temperature for 4 hr, and the solvent was concentrated under reduced pressure. The residue was washed with diisopropyl ether to give the title compound (2.39 g). MS: found: 268.1.

### E) 2-tert-butyl-N-((1S)-1-(3-fluoro-4-(trifluoromethoxy)phenyl)-2-hydroxy-2-methylpropyl)-6-oxo-1,6-dihydropyrimidine-4-carboxamide

By a method similar to that of Example 1, step C, the title compound was obtained.

### Example 96

### 2-cyclopropyl-N-((1S)-1-(3-fluoro-4-(trifluoromethoxy)phenyl)-2-hydroxy-2-methylpropyl)-6-oxo-1,6-dihydropyrimidine-4-carboxamide

By a method similar to that of Example 34, step A and Example 1, step C, the title compound was obtained.

### Example 97

### N-((1S)-1-(3-fluoro-4-(trifluoromethoxy)phenyl)-2-methoxyethyl)-6-oxo-2-(prop-1-en-2-yl)-1,6-dihydropyrimidine-4-carboxamide

### A) N-((1S)-1-(3-fluoro-4-(trifluoromethoxy)phenyl)-2-methoxyethyl)-6-methoxy-2-(prop-1-en-2-yl)pyrimidine-4-carboxamide

By a method similar to that of Example 58, step A, the title compound was obtained.
MS: [M+H]⁺ 430.1.

### B) N-((1S)-1-(3-fluoro-4-(trifluoromethoxy)phenyl)-2-methoxyethyl)-6-oxo-2-(prop-1-en-2-yl)-1,6-dihydropyrimidine-4-carboxamide

By a method similar to that of Example 15, step B, the title compound was obtained.

### Example 98

### N-((1S)-1-(3-fluoro-4-(trifluoromethoxy)phenyl)-2-methoxyethyl)-2-(1-methoxy-2-methylpropan-2-yl)-6-oxo-1,6-dihydropyrimidine-4-carboxamide

### A) methyl 3-methoxy-2,2-dimethylpropanoate

To a mixture of sodium hydride (60% in oil, 0.666 g) and tetrahydrofuran (20 mL) was added dropwise methyl 3-hydroxy-2,2-dimethylpropanoate (1.93 mL) under ice-cooling. Under a nitrogen atmosphere, the mixture was stirred under ice-cooling for 1 hr, methyl iodide (1.42 mL) was added, and the mixture was stirred at room temperature overnight. The reaction was quenched with a saturated aqueous ammonium chloride solution at room temperature, and the mixture was diluted with water, and extracted with diethyl ether. The extract was washed with saturated brine, and dried over anhydrous magnesium sulfate. The solvent was evaporated under reduced pressure to give the title compound (2.18 g).
¹H NMR (300 MHz, CDCl₃) δ 1.19 (6H, s), 3.33 (3H, s), 3.38 (2H, s), 3.69 (3H, s).

### B) 3-methoxy-2,2-dimethylpropanimidamide hydrochloride

To a mixture of ammonium chloride (1.10 g) and toluene (50 mL) was added dropwise trimethylaluminum (about 1.4 M hexane solution, 14.7 mL) under ice-cooling, and the mixture was stirred under a nitrogen atmosphere under ice-cooling for 30 min. A mixture of methyl 3-methoxy-2,2-dimethylpropanoate (1.0 g) and toluene (10 mL) was added to the reaction mixture under ice-cooling and stirred at 80°C overnight. After cooling to 0°C, methanol was added to the reaction mixture, and the mixture was stirred at room temperature for 1 hr. The reaction mixture was filtered, and the solvent was evaporated under reduced pressure to give the title compound (1.35 g).
¹H NMR (300 MHz, DMSO-d₆) δ 1.21 (6H, s), 3.26 (3H, s), 3.43 (2H, s), 8.69 (2H, brs), 9.14 (2H, brs).

### C) 2-(1-methoxy-2-methylpropan-2-yl)-6-oxo-1,6-dihydropyrimidine-4-carboxylic acid

By a method similar to that of Example 34, step A, the title compound was obtained.
MS: [M+H]⁺ 227.1.

### D) N-((1S)-1-(3-fluoro-4-(trifluoromethoxy)phenyl)-2-methoxyethyl)-2-(1-methoxy-2-methylpropan-2-yl)-6-oxo-1,6-dihydropyrimidine-4-carboxamide

By a method similar to that of Example 1, step C, the title compound was obtained.

### Example 99

### N-((1S)-1-(3-fluoro-4-(trifluoromethoxy)phenyl)-2-methoxyethyl)-2-(2-methoxy-1,3-thiazol-5-yl)-6-oxo-1,6-dihydropyrimidine-4-carboxamide

### A) N-((1S)-1-(3-fluoro-4-(trifluoromethoxy)phenyl)-2-methoxyethyl)-6-methoxy-2-(2-methoxy-1,3-thiazol-5-yl)pyrimidine-4-carboxamide

A mixture of 2-chloro-N-((1S)-1-(3-fluoro-4-(trifluoromethoxy)phenyl)-2-methoxyethyl)-6-methoxypyrimidine-4-carboxamide (115.7 mg), (2-methoxy-1,3-thiazol-5-yl)boronic acid (87 mg), chloro(2-dicyclohexylphosphino-2',6'-dimethoxy-1,1'-biphenyl) (2-(2-aminoethylphenyl))palladium(II) methyl tert-butyl ether adduct (20.77 mg), dicyclohexyl(2',6'-dimethoxybiphenyl-2-yl)phosphine (11.21 mg), 2 M aqueous cesium carbonate solution (0.273 mL) and 2-methyl-2-butanol (10.9 mL) was heated under microwave irradiation at 130°C for 40 min. After cooling to room temperature, the reaction mixture was poured into water, and the mixture was extracted with ethyl acetate. The extract was washed with water and saturated brine, dried over anhydrous sodium sulfate, and the solvent was evaporated under reduced pressure. The residue was fractionated by HPLC (C18, mobile phase: water/acetonitrile (0.1% TFA-containing system)), a saturated aqueous sodium hydrogen carbonate solution was added to the obtained fraction, and the mixture was extracted with ethyl acetate, dried over anhydrous magnesium sulfate, and concentrated under reduced pressure to give the title compound (22 mg).
MS: [M+H]⁺ 503.1.

### B) N-((1S)-1-(3-fluoro-4-(trifluoromethoxy)phenyl)-2-methoxyethyl)-2-(2-methoxy-1,3-thiazol-5-yl)-6-oxo-1,6-dihydropyrimidine-4-carboxamide

By a method similar to that of Example 15, step B, the title compound was obtained.

### Example 100

### N-(1-(3-fluoro-4-(trifluoromethoxy)phenyl)-2-methoxyethyl)-6-oxo-4-(pyrrolidin-1-yl)-1,6-dihydropyrimidine-2-carboxamide

### A) 2-chloro-4-methoxy-6-(pyrrolidin-1-yl)pyrimidine

To a solution of 2,4-dichloro-6-(pyrrolidin-1-yl)pyrimidine (1.0 g) in tetrahydrofuran (20 mL) was added sodium methoxide/methanol solution (28%, 0.929 g) at 0°C. The reaction mixture was stirred at room temperature for 3 days, water was added and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (150 mg).
¹H NMR (300 MHz, CDCl₃) δ 1.92-2.06 (4H, m), 3.11-3.72 (4H, m), 3.91 (3H, s), 5.46 (1H, s).

### B) methyl 4-methoxy-6-(pyrrolidin-1-yl)pyrimidine-2-carboxylate

A mixture of 2-chloro-4-methoxy-6-(pyrrolidin-1-yl)pyrimidine (150 mg), palladium(II) acetate (15.8 mg), 1,1'-bis(diphenylphosphino)ferrocene (58.4 mg), sodium acetate (86 mg) and methanol (8.0 mL) was stirred under a carbon monoxide atmosphere in an autoclave at 120°C for 3 hr. The reaction mixture was cooled to room temperature, and insoluble material was filtered off. Under reduced pressure, the solvent was evaporated, and the residue was purified by silica gel column chromatography (ethyl acetate/hexane) to give the title compound (137.5 mg).
MS: [M+H]⁺ 238.1.

### C) N-(1-(3-fluoro-4-(trifluoromethoxy)phenyl)-2-methoxyethyl)-4-methoxy-6-(pyrrolidin-1-yl)pyrimidine-2-carboxamide

By a method similar to that of Example 14, step B, the title compound was obtained.
MS: [M+H]⁺ 459.2.

### D) N-(1-(3-fluoro-4-(trifluoromethoxy)phenyl)-2-methoxyethyl)-6-oxo-4-(pyrrolidin-1-yl)-1,6-dihydropyrimidine-2-carboxamide

By a method similar to that of Example 15, step B, the title compound was obtained.

### Example 101

### 2-cyclobutyl-N-((1S)-1-(3-fluoro-4-(trifluoromethoxy)phenyl)-2-methoxyethyl)-6-oxo-1,6-dihydropyrimidine-4-carboxamide

By a method similar to that of Example 87, step A and Example 1, step C, the title compound was obtained.

### Example 102

### N-((1S)-1-(3-fluoro-4-(trifluoromethoxy)phenyl)-2-hydroxy-2-methylpropyl)-6-oxo-2-(pyridin-2-yl)-1,6-dihydropyrimidine-4-carboxamide

By a method similar to that of Example 1, step C, the title compound was obtained.

### Example 103

### N-((1S)-1-(3-fluoro-4-(trifluoromethoxy)phenyl)-2-methoxyethyl)-2-(1-methylcyclopropyl)-6-oxo-1,6-dihydropyrimidine-4-carboxamide

### A) 1-methylcyclopropanecarboximidamide hydrochloride

To a mixture of ammonium chloride (1.71 g) and toluene (40 mL) was added a toluene solution (15%, 14.2 mL) of trimethylaluminum at 0°C, and the mixture was stirred at room temperature for 1 hr. To the reaction mixture was added methyl 1-methylcyclopropane-1-carboxylate (0.75 mL), and the mixture was stirred at 80°C overnight, further stirred at 100°C for 10 hr. To the reaction mixture was added a tetrahydrofuran suspension of silica gel at 0°C, and the mixture was stirred at room temperature for 0.5 hr, filtered through celite, and insoluble material was washed with methanol. The obtained filtrate was concentrated under reduced pressure to give the title compound (0.50 g).
¹H NMR (300 MHz, DMSO-d₆) δ 0.85-0.91 (2H, m), 1.16-1.22 (2H, m), 1.31 (3H, s), 7.18-7.36 (2H, m), 8.58 (1H, brs), 8.82 (1H, brs).

### B) 2-(1-methylcyclopropyl)-6-oxo-1,6-dihydropyrimidine-4-carboxylic acid

To diethyl oxaloacetate sodium salt (1.69 g) was added 2 M aqueous sodium hydroxide solution (20 mL) at room temperature, and the mixture was stirred at room temperature for 10 min. To the reaction mixture was added 1-methylcyclopropanecarboximidamide hydrochloride (0.90 g) and the mixture was stirred at room temperature for 1 hr, and further stirred at 70°C for 40 min. To the reaction mixture was added 2 M hydrochloric acid (20 mL) at 0°C, and the mixture was stirred at room temperature overnight. The precipitate was collected by filtration to give the title compound (0.14 g).
MS: [M+H]⁺ 195.1.

### C)N-((1S)-1-(3-fluoro-4-(trifluoromethoxy)phenyl)-2-methoxyethyl)-2-(1-methylcyclopropyl)-6-oxo-1,6-dihydropyrimidine-4-carboxamide

To a mixture of 2-(1-methylcyclopropyl)-6-oxo-1,6-dihydropyrimidine-4-carboxylic acid (64.4 mg) and (1S)-1-(3-fluoro-4-(trifluoromethoxy)phenyl)-2-methoxyethanamine hydrochloride (80 mg), 1-(3-dimethylaminopropyl)-3-ethylcarbodiimide hydrochloride (79 mg), 1-hydroxybenzotriazole (56 mg) and N,N-dimethylformamide (1.0 mL) was added triethylamine (0.077 mL) at room temperature. The reaction mixture was stirred at room temperature overnight, poured into water, and extracted with ethyl acetate. The extract was washed with water and saturated aqueous sodium hydrogen carbonate solution, and dried over anhydrous sodium sulfate. The solvent was evaporated under reduced pressure. The residue was purified by silica gel column chromatography (ethyl acetate/hexane) to give the title compound (80 mg).
¹H NMR (300 MHz, CDCl₃) δ 0.99-1.08 (2H, m), 1.39-1.49 (2H, m), 1.55 (3H, s), 3.42 (3H, s), 3.73 (2H, d, J = 4.2 Hz), 5.15-5.24 (1H, m), 7.04 (1H, s), 7.12-7.32 (3H, m), 8.51 (1H, d, J = 7.9 Hz), 10.51 (1H, brs).

### Example 104

### N-((1S)-1-(3-fluoro-4-(trifluoromethoxy)phenyl)-2-methoxyethyl)-6-oxo-2-(1,3-thiazol-4-yl)-1,6-dihydropyrimidine-4-carboxamide

By a method similar to that of Example 103, step A, Example 87, step A and Example 1, step C, the title compound was obtained.

### Example 105

### N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide

### A) 5-methoxypyridine-2-carboximidamide hydrochloride

A mixture of 5-fluoropyridine-2-carbonitrile (1.3 g) and methanol solution (28%, 1.6 mL) of sodium methoxide was stirred at 0°C for 10 hr. To the reaction mixture were added ethyl acetate and water, and the mixture was extracted with ethyl acetate. The extract was washed with saturated brine, and dried over anhydrous sodium sulfate. The solvent was evaporated under reduced pressure. To a solution (50 mL) of the residue (6.8 g) in methanol was added sodium methoxide (0.44 g), and the mixture was stirred at room temperature for 10 hr. Ammonium chloride (2.85 g) was added, and the mixture was stirred at room temperature overnight. The solvent was evaporated under reduced pressure, and the residue was washed with diethyl ether to give the title compound (7.27 g).
¹H NMR (300 MHz, DMSO-d₆) δ 3.93 (3H, s), 7.72 (1H, dd, J = 8.9, 3.0 Hz), 8.39 (1H, d, J = 8.7 Hz), 8.49 (1H, d, J = 2.8 Hz), 8.58 (4H, brs).

### B) 2-(5-methoxypyridin-2-yl)-6-oxo-1,6-dihydropyrimidine-4-carboxylic acid

By a method similar to that of Example 87, step A, the title compound was obtained.
MS: [M+H]⁺ 248.1.

### C) N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide

By a method similar to that of Example 1, step C, the title compound was obtained.

### Example 106

### N-((1S)-1-(3-fluoro-4-(trifluoromethoxy)phenyl)-2-methoxyethyl)-6-oxo-2-(1,3-thiazol-2-yl)-1,6-dihydropyrimidine-4-carboxamide

### A) (S)-N-(1-(3-fluoro-4-(trifluoromethoxy)phenyl)-2-methoxyethyl)-6-methoxy-2-(thiazol-2-yl)pyrimidine-4-carboxamide

A mixture of (S)-2-chloro-N-(1-(3-fluoro-4-(trifluoromethoxy)phenyl)-2-methoxyethyl)-6-methoxypyrimidine-4-carboxamide (100 mg), 2-(tributylstannyl)thiazole (0.089 mL), tetrakis(triphenylphosphine)palladium(0) (13.6 mg) and toluene (2 ml) was stirred under a nitrogen atmosphere at 110°C for 2 hr. 2-(tributylstannyl)thiazole (0.022 mL) was added to the reaction mixture, and the mixture was stirred under a nitrogen atmosphere at 110°C overnight. The solvent in the reaction mixture was evaporated under reduced pressure. The residue was purified by silica gel column chromatography (NH, ethyl acetate/hexane) to give the title compound (97 mg).
MS: [M+H]⁺ 473.1.

### B) N-((1S)-1-(3-fluoro-4-(trifluoromethoxy)phenyl)-2-methoxyethyl)-6-oxo-2-(1,3-thiazol-2-yl)-1,6-dihydropyrimidine-4-carboxamide

By a method similar to that of Example 15, step B, the title compound was obtained.

### Example 107

### N-((1S)-1-(3-fluoro-4-(trifluoromethoxy)phenyl)-2-methoxyethyl)-6-oxo-2-(2-thienyl)-1,6-dihydropyrimidine-4-carboxamide

### A) thiophene-2-carboximidamide hydrochloride

To a solution of sodium methoxide (0.88 g) in methanol (50 mL) was added 2-cyanothiophene (4.27 mL), and the mixture was stirred at room temperature for 10 hr. Ammonium chloride (2.70 g) was added, and the mixture was stirred at room temperature overnight. Unreacted ammonium chloride was filtered off. The solvent was evaporated under reduced pressure, and the residue was washed with diethyl ether to give the title compound (5.91 g).

### MS: [M+H]⁺ 127.1.

### B) N-((1S)-1-(3-fluoro-4-(trifluoromethoxy)phenyl)-2-methoxyethyl)-6-oxo-2-(2-thienyl)-1,6-dihydropyrimidine-4-carboxamide

By a method similar to that of Example 87, step A and Example 1, step C, the title compound was obtained.

### Example 108

### N-((1S)-1-(3-fluoro-4-(trifluoromethoxy)phenyl)-2-methoxyethyl)-2-(4-methoxyphenyl)-6-oxo-1,6-dihydropyrimidine-4-carboxamide

By a method similar to that of Example 107, step A, Example 87, step A and Example 1, step C, the title compound was obtained.

### Example 109

### N-((1S)-1-(3-fluoro-4-(trifluoromethoxy)phenyl)-2-methoxyethyl)-2-(3-methoxyphenyl)-6-oxo-1,6-dihydropyrimidine-4-carboxamide

By a method similar to that of Example 107, step A, Example 87, step A and Example 1, step C, the title compound was obtained.

### Example 110

### N-((1S)-1-(3-fluoro-4-(trifluoromethoxy)phenyl)-2-methoxyethyl)-2-(4-methylphenyl)-6-oxo-1,6-dihydropyrimidine-4-carboxamide

By a method similar to that of Example 107, step A, Example 87, step A and Example 1, step C, the title compound was obtained.

### Example 111

### N-((1S)-1-(3-fluoro-4-(trifluoromethoxy)phenyl)-2-methoxyethyl)-6-oxo-2-(prop-1-yn-1-yl)-1,6-dihydropyrimidine-4-carboxamide

### A) but-2-yn imidamide hydrochloride

By a method similar to that of Example 98, step B, the title compound was obtained.
¹H NMR (300 MHz, DMSO-d₆) δ 2.17 (3H, s), 8.85-10.02 (4H, m).

### B) 6-oxo-2-(prop-1-yn-1-yl)-1,6-dihydropyrimidine-4-carboxylic acid

By a method similar to that of Example 34, step A, the title compound was obtained.
MS: [M+H]⁺ 179.0.

### C) N-((1S)-1-(3-fluoro-4-(trifluoromethoxy)phenyl)-2-methoxyethyl)-6-oxo-2-(prop-1-yn-1-yl)-1,6-dihydropyrimidine-4-carboxamide

By a method similar to that of Example 1, step C, the title compound was obtained.

### Example 112

### N-((1S)-1-(3-fluoro-4-(trifluoromethoxy)phenyl)-2-methoxyethyl)-2-(5-methyl-1,3-thiazol-2-yl)-6-oxo-1,6-dihydropyrimidine-4-carboxamide

By a method similar to that of Example 106, step A and Example 15, step B, the title compound was obtained.

### Example 113

### N-((1S)-1-(3-fluoro-4-(trifluoromethoxy)phenyl)-2-methoxyethyl)-2-(2-methoxypropan-2-yl)-6-oxo-1,6-dihydropyrimidine-4-carboxamide

### A) 2-methoxy-2-methylpropanimidamide hydrochloride

By a method similar to that of Example 98, step A and B, the title compound was obtained.
¹H NMR (300 MHz, DMSO-d₆) δ 1.45 (6H, s), 3.19 (3H, s), 8.67 (4H, brs).

### B) 2-(2-methoxypropan-2-yl)-6-oxo-1,6-dihydropyrimidine-4-carboxylic acid

By a method similar to that of Example 34, step A, the title compound was obtained.
MS: [M+H]⁺ 213.1.

### C) N-((1S)-1-(3-fluoro-4-(trifluoromethoxy)phenyl)-2-methoxyethyl)-2-(2-methoxypropan-2-yl)-6-oxo-1,6-dihydropyrimidine-4-carboxamide

By a method similar to that of Example 1, step C, the title compound was obtained.

### Example 114

### 2-(cyclopent-1-en-1-yl)-N-((1S)-1-(3-fluoro-4-(trifluoromethoxy)phenyl)-2-hydroxy-2-methylpropyl)-6-oxo-1,6-dihydropyrimidine-4-carboxamide

### A) 2-(cyclopent-1-en-1-yl)-N-((1S)-1-(3-fluoro-4-(trifluoromethoxy)phenyl)-2-hydroxy-2-methylpropyl)-6-((4-methoxybenzyl)oxy)pyrimidine-4-carboxamide

A mixture of methyl 2,6-dichloropyrimidine-4-carboxylate (3.55 g), 4-methoxybenzylalcohol (4.28 mL), 1,8-diazabicyclo[5.4.0]undec-7-ene (2.58 mL) and acetonitrile (70 mL) was stirred under a nitrogen atmosphere at room temperature overnight. Water was added to the reaction mixture, and the mixture was extracted with ethyl acetate. The extract was washed with water and saturated brine, and dried over anhydrous sodium sulfate. The solvent was evaporated under reduced pressure, and the residue was purified by silica gel column chromatography (ethyl acetate/hexane) to give a pale-yellow oil (3.03 g). A mixture of the obtained oil (106.3 mg), 2-(cyclopent-1-en-1-yl)-4,4,5,5-tetramethyl-1,3,2-dioxaborolane (54.7 mg), bis(di-tert-butyl(4-dimethylaminophenyl)phosphine)dichloropalladium(II) (18.1 mg), tripotassium phosphate (115 mg) and N,N-dimethylformamide (12.8 mL) was heated under microwave irradiation at 130°C for 1.5 hr. After cooling to room temperature, the reaction mixture was poured into water, and the mixture was extracted with ethyl acetate. The extract was washed with water and saturated brine, dried over anhydrous sodium sulfate, and the solvent was evaporated under reduced pressure. The residue was purified by silica gel column chromatography (NH, ethyl acetate/hexane) to give a pale-yellow oil (10.7 mg). Using the obtained oil (10.7 mg), a reaction similar to that of Example 14, step B was performed to give the title compound (6.4 mg).
MS: [M+H]⁺ 576.1.

### B) 2-(cyclopent-1-en-1-yl)-N-((1S)-1-(3-fluoro-4-(trifluoromethoxy)phenyl)-2-hydroxy-2-methylpropyl)-6-oxo-1,6-dihydropyrimidine-4-carboxamide

By a method similar to that of Example 24, step C, the title compound was obtained.

### Example 115

### 2-(cyclopent-1-en-1-yl)-N-((1S)-2-methoxy-1-(4-(trifluoromethoxy)phenyl)ethyl)-6-oxo-1,6-dihydropyrimidine-4-carboxamide

### A) 2-(cyclopent-1-en-1-yl)-6-methoxy-N-((1S)-2-methoxy-1-(4-(trifluoromethoxy)phenyl)ethyl)pyrimidine-4-carboxamide

By a method similar to that of Example 58, step A, the title compound was obtained.
MS: [M+H]⁺ 438.2

### B) 2-(cyclopent-1-en-1-yl)-N-((1S)-2-methoxy-1-(4-(trifluoromethoxy)phenyl)ethyl)-6-oxo-1,6-dihydropyrimidine-4-carboxamide

By a method similar to that of Example 15, step B, the title compound was obtained.

### Example 116

### N-(1-(3-fluoro-4-(1H-pyrrol-1-yl)phenyl)-2-methoxyethyl)-6-oxo-2-(pyridin-2-yl)-1,6-dihydropyrimidine-4-carboxamide

### A) 1-(4-bromo-2-fluorophenyl)-1H-pyrrole

A mixture of 4-bromo-2-fluoroaniline (12.4 g), 2,5-dimethoxytetrahydrofuran (8.7 g) and acetic acid (40 mL) was stirred at 110°C for 3 hr. The reaction mixture was cooled to room temperature, and the solvent was evaporated under reduced pressure. Water was added to the residue and the mixture was extracted with ethyl acetate. The extract was washed with saturated brine, dried over anhydrous sodium sulfate, and the solvent was evaporated under reduced pressure. The residue was purified by silica gel column chromatography (ethyl acetate/petroleum ether) to give the title compound (10 g).
¹H NMR (400 MHz, CDCl₃) δ 6.35 (2H, t, J = 2.2 Hz), 6.98-7.00 (2H, m), 7.24 (1H, t, J = 4.2 Hz), 7.32-7.40 (2H, m).

### B) 1-(3-fluoro-4-(1H-pyrrol-1-yl)phenyl)-2-methoxyethanone

By a method similar to that of Example 73, step B, the title compound was obtained.
¹H NMR (400 MHz, CDCl₃) δ 3.52 (3H, s), 4.67 (2H, s), 6.40 (2H, t, J = 2.0 Hz), 7.14 (2H, t, J = 2.0 Hz), 7.49 (1H, t, J = 8.0 Hz), 7.79-7.86 (2H, m).

### C) 1-(3-fluoro-4-(1H-pyrrol-1-yl)phenyl)-2-methoxyethanol

To a solution of 1-(3-fluoro-4-(1H-pyrrol-1-yl)phenyl)-2-methoxyethanone (3.6 g) in methanol (30 mL) was added sodium borohydride (0.90 g) at room temperature. The reaction mixture was stirred at room temperature for 1 hr, and the solvent was evaporated under reduced pressure. Ethyl acetate was added to the residue, and the mixture was washed with water and saturated brine, and dried over anhydrous sodium sulfate. The solvent was evaporated under reduced pressure. The residue was purified by silica gel column chromatography (ethyl acetate/petroleum ether) to give the title compound (2.0 g).
¹H NMR (400 MHz, CDCl₃) δ 2.70-3.10 (1H, brs), 3.40-3.44 (4H, m), 3.55-3.58 (1H, m), 4.87-4.91 (1H, m), 6.35 (2H, t, J = 2.2 Hz), 7.01-7.02 (2H, m), 7.19 (1H, d, J = 8.0 Hz), 7.25-7.29 (1H, m), 7.35 (1H, t, J = 8.0 Hz).

### D) 1-(3-fluoro-4-(1H-pyrrol-1-yl)phenyl)-2-methoxyethanamine

To a mixture of 1-(3-fluoro-4-(1H-pyrrol-1-yl)phenyl)-2-methoxyethanol (2.3 g), 1H-isoindole-1,3(2H)-dione (1.9 g), triphenylphosphine (4.0 g) and tetrahydrofuran (100 mL) was added diisopropyl azodicarboxylate (3.0 g) at 0°C. The reaction mixture was stirred at room temperature for 3 hr and the solvent was evaporated under reduced pressure. Water was added to the residue and the mixture was extracted with ethyl acetate. The extract was washed with saturated brine, dried over anhydrous sodium sulfate, and the solvent was evaporated under reduced pressure. The residue was purified by silica gel column chromatography (ethyl acetate/petroleum ether) to give a colorless oil. A solution (20 mL) of the oil (1.8 g) and dimethylamine in methanol was stirred at 70°C for 1 hr. The solvent was evaporated under reduced pressure, and the residue was acidified with water and 2 M hydrochloric acid, and washed with ethyl acetate. The aqueous layer was alkalified with aqueous sodium hydroxide solution, and the mixture was extracted with ethyl acetate. The extract was washed with saturated brine, dried over anhydrous sodium sulfate, and the solvent was evaporated under reduced pressure. The residue was purified by silica gel column chromatography (methanol/ethyl acetate) to give the title compound (0.5 g).
MS: [M+H]⁺ 235.1.

### E) N-(1-(3-fluoro-4-(1H-pyrrol-1-yl)phenyl)-2-methoxyethyl)-6-oxo-2-(pyridin-2-yl)-1,6-dihydropyrimidine-4-carboxamide

By a method similar to that of Example 1, step C, the title compound was obtained.

### Example 117

### N-((1S)-1-(3-fluoro-4-(trifluoromethoxy)phenyl)-2-methoxyethyl)-2-(4-methoxypyridin-2-yl)-6-oxo-1,6-dihydropyrimidine-4-carboxamide

### A) 4-methoxypicolinonitrile

To a solution of 4-methoxypyridine-N-oxide (4.6 g) in acetonitrile (100 mL) were added, at room temperature, trimethylsilyl cyanide (5.88 mL) and N,N-dimethylcarbamoyl chloride (4.74 g), and the mixture was stirred under a nitrogen stream at 70°C for 5 hr. To the reaction mixture was added a saturated aqueous sodium hydrogen carbonate solution, and the mixture was extracted with ethyl acetate. The extract was dried over anhydrous sodium sulfate, and the solvent was evaporated under reduced pressure. The residue was purified by silica gel column chromatography (ethyl acetate/hexane) and recrystallized (ethyl acetate/hexane) to give the title compound (1.68 g).
MS: [M+H]⁺ 135.1.

### B) N-((1S)-1-(3-fluoro-4-(trifluoromethoxy)phenyl)-2-methoxyethyl)-2-(4-methoxypyridin-2-yl)-6-oxo-1,6-dihydropyrimidine-4-carboxamide

By a method similar to that of Example 107, step A, Example 87, step A and Example 1, step C, the title compound was obtained.

### Example 118

### N-((1S)-1-(3-fluoro-4-(trifluoromethoxy)phenyl)-2-methoxyethyl)-6-oxo-2-(5-(trifluoromethyl)pyridin-2-yl)-1,6-dihydropyrimidine-4-carboxamide

By a method similar to that of Example 107, step A, Example 87, step A and Example 1, step C, the title compound was obtained.

### Example 119

### N-((1S)-1-(3-fluoro-4-(trifluoromethoxy)phenyl)-2-methoxyethyl)-2-(3-methylphenyl)-6-oxo-1,6-dihydropyrimidine-4-carboxamide

By a method similar to that of Example 107, step A, Example 87, step A and Example 1, step C, the title compound was obtained.

### Example 120

### N-((1S)-1-(3-fluoro-4-(trifluoromethoxy)phenyl)-2-methoxyethyl)-6-oxo-2-(3-thienyl)-1,6-dihydropyrimidine-4-carboxamide

By a method similar to that of Example 107, step A, Example 87, step A and Example 1, step C, the title compound was obtained.

### Example 121

### N-((1S)-1-(3-fluoro-4-(trifluoromethoxy)phenyl)-2-methoxyethyl)-2-(2-furyl)-6-oxo-1,6-dihydropyrimidine-4-carboxamide

By a method similar to that of Example 107, step A, Example 87, step A and Example 1, step C, the title compound was obtained.

### Example 122

### N-((1S)-1-(3-fluoro-4-(trifluoromethoxy)phenyl)-2-methoxyethyl)-2-(3-furyl)-6-oxo-1,6-dihydropyrimidine-4-carboxamide

By a method similar to that of Example 107, step A, Example 87, step A and Example 1, step C, the title compound was obtained.

### Example 123

### N-((1S)-1-(3-fluoro-4-(trifluoromethoxy)phenyl)-2-methoxyethyl)-2-(6-methylpyridin-2-yl)-6-oxo-1,6-dihydropyrimidine-4-carboxamide

### A) 6-methylpicolinimidamide hydrochloride

By a method similar to that of Example 107, step A, the title compound was obtained.
MS: [M+H]⁺ 136.1.

### B) 2-(6-methylpyridin-2-yl)-6-oxo-1,6-dihydropyrimidine-4-carboxylic acid

To a mixture of diethyl oxaloacetate sodium salt (1.47 g), water (6.0 mL) and ethanol (1.0 mL) was added 8 M aqueous sodium hydroxide solution (0.874 mL) at room temperature, and the mixture was stirred at room temperature for 30 min. A mixture of 6-methylpicolinimidamide hydrochloride (1.0 g) and water (3 mL), and 8 M aqueous sodium hydroxide solution (0.874 mL) were added to the reaction mixture, and the mixture was stirred at 70°C for 3 hr. The reaction mixture was acidified with 6 M hydrochloric acid, and the precipitate was collected by filtration. The obtained solid was washed with methanol to give the title compound (810 mg).
MS: [M+H]⁺ 232.1.

### C) N-((1S)-1-(3-fluoro-4-(trifluoromethoxy)phenyl)-2-methoxyethyl)-2-(6-methylpyridin-2-yl)-6-oxo-1,6-dihydropyrimidine-4-carboxamide

By a method similar to that of Example 1, step C, the title compound was obtained.

### Example 124

### N-((1S)-1-(3-fluoro-4-(trifluoromethoxy)phenyl)-2-methoxyethyl)-2-(6-methoxypyridin-2-yl)-6-oxo-1,6-dihydropyrimidine-4-carboxamide

By a method similar to that of Example 123, step B and Example 1, step C, the title compound was obtained.
MS: [M+H]⁺ 483.1.

### Example 125

### N-((1S)-1-(3-fluoro-4-(trifluoromethoxy)phenyl)-2-methoxyethyl)-2-(1-methoxycyclopropyl)-6-oxo-1,6-dihydropyrimidine-4-carboxamide

### A) 1-methoxycyclopropanecarboximidamide hydrochloride

By a method similar to that of Example 98, step A and B, the title compound was obtained.
¹H NMR (300 MHz, DMSO-d₆) δ 1.23-1.50 (4H, m), 3.26 (3H, s), 8.19-9.21 (4H, m).

### B) N-((1S)-1-(3-fluoro-4-(trifluoromethoxy)phenyl)-2-methoxyethyl)-2-(1-methoxycyclopropyl)-6-oxo-1,6-dihydropyrimidine-4-carboxamide

By a method similar to that of Example 34, step A and Example 1, step C, the title compound was obtained.

### Example 126

### N-((1S)-1-(3-fluoro-4-(trifluoromethoxy)phenyl)-2-methoxyethyl)-2-(3-methoxy-4-methylphenyl)-6-oxo-1,6-dihydropyrimidine-4-carboxamide

By a method similar to that of Example 107, step A, Example 34, step A and Example 1, step C, the title compound was obtained.

### Example 127

### N-((1S)-1-(3-fluoro-4-(trifluoromethoxy)phenyl)-2-methoxyethyl)-2-(3-methoxycyclopent-1-en-1-yl)-6-oxo-1,6-dihydropyrimidine-4-carboxamide

### A) 3-iodocyclopent-2-en-1-one

To a solution of triphenylphosphine (6.42 g) in acetonitrile (200 mL) was added iodine (6.21 g) at room temperature. Under an argon atmosphere, the mixture was stirred at room temperature for 4 hr, and cyclopentane-1,3-dione (2.0 g) and triethylamine (3.41 mL) were added to the reaction mixture. Under an argon atmosphere, the reaction mixture was stirred at 90°C for 4 hr. The solvent was evaporated under reduced pressure, and the residue was purified by silica gel column chromatography (ethyl acetate/hexane) to give the title compound (3.49 g).
¹H NMR (300 MHz, CDCl₃) δ 2.47-2.52 (2H, m), 3.04-3.10 (2H, m), 6.69 (1H, t, J = 1.8 Hz).

### B) 3-iodocyclopent-2-en-1-ol

To a solution of 3-iodocyclopent-2-en-1-one (1.0 g) in ethanol (15 mL) was added sodium borohydride (0.182 g) at 0°C. The reaction mixture was stirred at 0°C for 0.5 hr, and at room temperature for 2 hr. To the reaction mixture was added water at room temperature, and the mixture was extracted with ethyl acetate. The extract was washed with water and saturated brine, and dried over anhydrous sodium sulfate. The solvent was evaporated under reduced pressure to give the title compound (723 mg).
¹H NMR (300 MHz, CDCl₃) δ 1.52 (1H, s), 1.71-1.83 (1H, m), 2.31-2.43 (1H, m), 2.53-2.65 (1H, m), 2.77-2.90 (1H, m), 4.68-4.77 (1H, m), 6.24 (1H, q, J = 2.1 Hz).

### C) 1-iodo-3-methoxycyclopentene

To a solution (14 mL) of 3-iodocyclopent-2-en-1-ol (720 mg) in tetrahydrofuran was added sodium hydride (60% in oil, 274 mg) at 0°C, and the mixture was stirred under an argon atmosphere at 0°C for 30 min. To the reaction mixture was added methyl iodide (0.643 mL) at 0°C, and the mixture was stirred at room temperature for 30 min. To the reaction mixture was added water at 0°C, and the mixture was extracted with ethyl acetate. The extract was washed with water and saturated brine, and dried over anhydrous sodium sulfate. The solvent was evaporated under reduced pressure, and the residue was purified by silica gel column chromatography (ethyl acetate/hexane) to give the title compound (628 mg).
¹H NMR (300 MHz, CDCl₃) δ 1.79-1.91 (1H, m), 2.17-2.30 (1H, m), 2.51-2.63 (1H, m), 2.74-2.87 (1H, m), 3.31 (3H, s), 4.27-4.34 (1H, m), 6.28-6.32 (1H, m).

### D) 2-(3-methoxycyclopent-1-en-1-yl)-4,4,5,5-tetramethyl-1,3,2-dioxaborolane

To a solution of 1-iodo-3-methoxycyclopentene (1.10 g) in diethyl ether (49 mL) was dropwise added 1.6 M n-butyllithium/hexane solution (6.14 mL) at -78°C. The mixture was stirred at -78°C for 1 hr, and 2-isopropoxy-4,4,5,5-tetramethyl-1,3,2-dioxaborolane (2.0 mL) was added at -78°C. The reaction mixture was warmed to room temperature, and stirred at room temperature for 1 hr. The reaction mixture was neutralized with 1 M hydrochloric acid at 0°C and extracted with diethyl ether. The extract was washed with water and saturated brine, and dried over anhydrous sodium sulfate. The solvent was evaporated under reduced pressure, and the residue was purified by silica gel column chromatography (ethyl acetate/hexane) to give the title compound (264 mg).
¹H NMR (300 MHz, CDCl₃) δ 1.27 (12H, s), 1.70-1.82 (1H, m), 2.08-2.20 (1H, m), 2.30-2.43 (1H, m), 2.53-2.67 (1H, m), 3.34 (3H, s), 4.46-4.53 (1H, m), 6.55-6.59 (1H, m).

### E) N-((1S)-1-(3-fluoro-4-(trifluoromethoxy)phenyl)-2-methoxyethyl)-6-((4-methoxybenzyl)oxy)-2-(3-methoxycyclopent-1-en-1-yl)pyrimidine-4-carboxamide

A reaction similar to that of Example 114, step A was performed to give the title compound.
MS: [M+H]⁺ 592.2.

### F) N-((1S)-1-(3-fluoro-4-(trifluoromethoxy)phenyl)-2-methoxyethyl)-2-(3-methoxycyclopent-1-en-1-yl)-6-oxo-1,6-dihydropyrimidine-4-carboxamide

By a method similar to that of Example 24, step C, the title compound was obtained.

### Example 128

### 2-(cyclopent-1-en-1-yl)-N-(2-methoxy-1-(4-(1H-pyrazol-1-yl)phenyl)ethyl)-6-oxo-1,6-dihydropyrimidine-4-carboxamide

### A) 2-(cyclopent-1-en-1-yl)-6-((4-methoxybenzyl)oxy)-N-(2-methoxy-1-(4-(1H-pyrazol-1-yl)phenyl)ethyl)pyrimidine-4-carboxamide

By a method similar to that of Example 114, step A, the title compound was obtained.
MS: [M+H]⁺ 526.2.

### B) 2-(cyclopent-1-en-1-yl)-N-(2-methoxy-1-(4-(1H-pyrazol-1-yl)phenyl)ethyl)-6-oxo-1,6-dihydropyrimidine-4-carboxamide

By a method similar to that of Example 24, step C, the title compound was obtained.

### Example 129

### 2-(cyclopent-1-en-1-yl)-N-(1-(3-fluoro-4-(1H-pyrazol-1-yl)phenyl)-2-methoxyethyl)-6-oxo-1,6-dihydropyrimidine-4-carboxamide

### A) 1-(3-fluoro-4-(1H-pyrazol-1-yl)phenyl)-2-methoxyethanone

To a mixture of magnesium (1.2 g) and tetrahydrofuran (2.0 mL) was added dropwise a solution of 4-bromo-1,2-difluorobenzene (8.0 g) in tetrahydrofuran (30 mL) under a nitrogen atmosphere at room temperature. The reaction mixture was stirred at room temperature for 1 hr, cooled to -10°C, and a solution of N,2-dimethoxy-N-methylacetamide (7.0 g) in tetrahydrofuran (30 mL) was added dropwise. After stirring at room temperature for 3 hr, saturated aqueous ammonium chloride solution was added to the reaction mixture, and the mixture was extracted with ethyl acetate. The extract was washed with saturated brine, dried over anhydrous sodium sulfate, and the solvent was evaporated under reduced pressure. The residue was purified by silica gel column chromatography (ethyl acetate/petroleum ether) to give a yellow oil (2.5 g). To a solution of the obtained oil (2.3 g), 1H-pyrazole (1.0 g) in dimethyl sulfoxide (30 mL) was added potassium carbonate (3.1 g) at room temperature. After stirring at 90°C for 3 hr, the mixture was cooled to room temperature, and poured into water, and extracted with ethyl acetate. The extract was washed with saturated brine, dried over anhydrous sodium sulfate, and the solvent was evaporated under reduced pressure. The residue was purified by silica gel column chromatography (ethyl acetate/petroleum ether) to give the title compound (1.3 g).
¹H NMR (400 MHz, CDCl₃) δ 3.52 (3H, s), 4.69 (2H, s), 6.54 (1H, t, J = 2.2 Hz), 7.79-7.89 (3H, m), 8.12-8.17 (2H, m).

### B) 1-(3-fluoro-4-(1H-pyrazol-1-yl)phenyl)-2-methoxyethanamine

By a method similar to that of Example 75, step D, the title compound was obtained.

### MS: [M+H]⁺ 236.1.

### C) 2-(cyclopent-1-en-1-yl)-N-(1-(3-fluoro-4-(1H-pyrazol-1-yl)phenyl)-2-methoxyethyl)-6-((4-methoxybenzyl)oxy)pyrimidine-4-carboxamide

By a method similar to that of Example 114, step A, the title compound was obtained.
MS: [M+H]⁺ 544.3.

### D) 2-(cyclopent-1-en-1-yl)-N-(1-(3-fluoro-4-(1H-pyrazol-1-yl)phenyl)-2-methoxyethyl)-6-oxo-1,6-dihydropyrimidine-4-carboxamide

By a method similar to that of Example 24, step C, the title compound was obtained.

### Example 130

### N-((1S)-1-(3-fluoro-4-(trifluoromethoxy)phenyl)-2-methoxyethyl)-2-(3-methylpyridin-2-yl)-6-oxo-1,6-dihydropyrimidine-4-carboxamide

By a method similar to that of Example 107, step A, Example 87, step A and Example 1, step C, the title compound was obtained.

### Example 131

### 2-(3,4-dimethoxyphenyl)-N-((1S)-1-(3-fluoro-4-(trifluoromethoxy)phenyl)-2-methoxyethyl)-6-oxo-1,6-dihydropyrimidine-4-carboxamide

By a method similar to that of Example 107, step A, Example 87, step A and Example 1, step C, the title compound was obtained.

### Example 132

### N-((1S)-1-(3-fluoro-4-(trifluoromethoxy)phenyl)-2-methoxyethyl)-2-(4-methylpyridin-2-yl)-6-oxo-1,6-dihydropyrimidine-4-carboxamide

By a method similar to that of Example 123, step B and Example 1, step C, the title compound was obtained.

### Example 133

### N-((1S)-1-(3-fluoro-4-(trifluoromethoxy)phenyl)-2-methoxyethyl)-2-(5-methylpyridin-2-yl)-6-oxo-1,6-dihydropyrimidine-4-carboxamide

By a method similar to that of Example 123, step B and Example 1, step C, the title compound was obtained.

### Example 134

### N-((1S)-1-(3-fluoro-4-(trifluoromethoxy)phenyl)-2-methoxyethyl)-2-(2-methylcyclopropyl)-6-oxo-1,6-dihydropyrimidine-4-carboxamide

### A) methyl 2-methylcyclopropanecarboxylate

To a mixture of 2-methylcyclopropanecarboxylic acid (1.0 g), diethyl ether (18 mL) and methanol (2.0 mL) was added trimethylsilyldiazomethane (0.6 M hexane solution, 16.7 mL) at room temperature. After stirring at room temperature for 2 hr, acetic acid was added to the reaction mixture, and the solvent was evaporated under reduced pressure. The residue was dissolved in diethyl ether, and the mixture was washed with saturated aqueous sodium hydrogen carbonate solution and saturated brine, and dried over anhydrous magnesium sulfate. The solvent was evaporated under reduced pressure to give the title compound (0.94 g).
¹H NMR (300 MHz, CDCl₃) δ 0.67 (1H, s), 1.09-1.14 (3H, m), 1.14-1.19 (1H, m), 1.29-1.45 (2H, m), 3.64-3.78 (3H, m).

### B) 2-(2-methylcyclopropyl)-6-oxo-1,6-dihydropyrimidine-4-carboxylic acid

By a method similar to that of Example 98, step B and Example 123, step B, the title compound was obtained. MS: [M+H]⁺ 194.9.

### C) N-((1S)-1-(3-fluoro-4-(trifluoromethoxy)phenyl)-2-methoxyethyl)-2-(2-methylcyclopropyl)-6-oxo-1,6-dihydropyrimidine-4-carboxamide

By a method similar to that of Example 1, step C, the title compound was obtained.

### Example 135

### 2-(4-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide

### A) 4-cyclopropylpicolinonitrile

To a mixture of 4-chloropicolinonitrile (200 mg), cyclopropylboronic acid (248 mg), potassium carbonate (698 mg), toluene (3.0 mL) and water (0.5 mL) was added bis(di-tert-butyl(4-dimethylaminophenyl)phosphine)dichloropalladium(II) (102 mg), and the mixture was stirred under microwave irradiation at 130°C for 30 min. The reaction mixture was diluted with saturated brine, and extracted with ethyl acetate. The extract was dried over anhydrous magnesium sulfate and the solvent was evaporated under reduced pressure. The residue was purified by column chromatography (ethyl acetate/hexane) to give the title compound (119 mg).
MS: [M+H]⁺ 145.2.

### B) 4-cyclopropylpicolinimidamide hydrochloride

By a method similar to that of Example 107, step A, the title compound was obtained.
MS: [M+H]⁺ 162.1.

### C) 2-(4-cyclopropylpyridin-2-yl)-6-oxo-1,6-dihydropyrimidine-4-carboxylic acid

By a method similar to that of Example 123, step B, the title compound was obtained.
MS: [M+H]⁺ 258.1.

### D) 2-(4-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 136

### 2-(5-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-cyclopropylpicolinonitrile

To a mixture of 5-chloropicolinonitrile (1.0 g), cyclopropylboronic acid (1.24 g), tripotassium phosphate (5.36 g), toluene (10 mL) and water (2.0 mL) was added bis(di-tert-butyl(4-dimethylaminophenyl)phosphine)dichloropalladium(II) (511 mg), and the mixture was heated under reflux under a nitrogen atmosphere for 3 hr. The reaction mixture was diluted with water, and extracted with ethyl acetate. The extract was dried over anhydrous magnesium sulfate, and the solvent was evaporated under reduced pressure. The residue was suspended in ethyl acetate, and insoluble material was filtered off. The solvent in the filtrate was evaporated under reduced pressure. The residue was purified by column chromatography (ethyl acetate/hexane) to give the title compound (100 mg).
MS: [M+H]⁺ 145.1.

### B) 5-cyclopropylpicolinimidamide hydrochloride

By a method similar to that of Example 107, step A, the title compound was obtained.
MS: [M+H]⁺ 162.1.

### C) 2-(5-cyclopropylpyridin-2-yl)-6-oxo-1,6-dihydropyrimidine-4-carboxylic acid

By a method similar to that of Example 123, step B, the title compound was obtained.
MS: [M+H]⁺ 258.1.

### D) 2-(5-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 137

### 2-(5-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 103, step A, Example 87, step A and Example 1, step C, the title compound was obtained.

### Example 138

### 2-(5-ethoxypyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 105, step A, Example 87, step A and Example 1, step C, the title compound was obtained.

### Example 139

### N-((1S)-1-(3-fluoro-4-(trifluoromethoxy)phenyl)-2-methoxyethyl)-2-(5-isopropoxypyridin-2-yl)-6-oxo-1,6-dihydropyrimidine-4-carboxamide

By a method similar to that of Example 103, step A, Example 87, step A and Example 1, step C, the title compound was obtained.

### Example 140

### 2-(2-fluorophenyl)-N-((1S)-1-(3-fluoro-4-(trifluoromethoxy)phenyl)-2-methoxyethyl)-6-oxo-1,6-dihydropyrimidine-4-carboxamide

By a method similar to that of Example 58, step A and Example 15, step B, the title compound was obtained.

### Example 141

### N-((1S)-1-(3-fluoro-4-(trifluoromethoxy)phenyl)-2-methoxyethyl)-2-(2-methoxyphenyl)-6-oxo-1,6-dihydropyrimidine-4-carboxamide

By a method similar to that of Example 58, step A and Example 15, step B, the title compound was obtained.

### Example 142

### 2-(5-chloropyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-chloropicolinimidamide hydrochloride

By a method similar to that of Example 107, step A, the title compound was obtained.
MS: [M+H]⁺ 156.1.

### B) 2-(5-chloropyridin-2-yl)-6-oxo-1,6-dihydropyrimidine-4-carboxylic acid

By a method similar to that of Example 123, step B, the title compound was obtained.
MS: [M+H]⁺ 252.0.

### C) 2-(5-chloropyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 143

### 2-(5-(difluoromethoxy)pyridin-2-yl)-N-((1S)-1-(3-fluoro-4-(trifluoromethoxy)phenyl)-2-methoxyethyl)-6-oxo-1,6-dihydropyrimidine-4-carboxamide

### A) methyl 5-(difluoromethoxy)picolinate

To a mixture of potassium carbonate (1.35 g) and N,N-dimethylformamide (2.0 mL) was added a mixture of methyl 5-hydroxypicolinate (1.0 g), sodium chlorodifluoroacetate (1.99 g) and N,N-dimethylformamide (4.0 mL) at 90°C, and the mixture was stirred at 90°C for 2 hr. After cooling to room temperature, water was added. The precipitate was filtered off, and the filtrate was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (860 mg).
MS: [M+H]⁺ 204.1.

### B) 5-(difluoromethoxy)picolinimidamide hydrochloride

By a method similar to that of Example 98, step B, the title compound was obtained.
MS: [M+H]⁺ 188.1.

### C) 2-(5-(difluoromethoxy)pyridin-2-yl)-6-oxo-1,6-dihydropyrimidine-4-carboxylic acid

By a method similar to that of Example 123, step B, the title compound was obtained.
MS: [M+H]⁺ 284.0.

### D) 2-(5-(difluoromethoxy)pyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 144

### N-((1S)-1-(3-fluoro-4-(trifluoromethoxy)phenyl)-2-methoxyethyl)-2-(5-methoxypyrazin-2-yl)-6-oxo-1,6-dihydropyrimidine-4-carboxamide

### A) 5-methoxypyrazine-2-carboximidamide hydrochloride

To a mixture of 5-chloropyrazine-2-carbonitrile (300 mg) and methanol (5 mL) was added a solution (28%, 456 mg) of sodium methoxide in methanol at room temperature, and the mixture was stirred overnight. To the reaction mixture was added ammonium chloride (138 mg), and the mixture was stirred at room temperature for 3 hr. The reaction mixture was filtered, and the solvent was evaporated under reduced pressure. The residue was washed with ethyl acetate to give the title compound (350 mg).
MS: [M+H]⁺ 153.1.

### B) 2-(5-methoxypyrazin-2-yl)-6-oxo-1,6-dihydropyrimidine-4-carboxylic acid

By a method similar to that of Example 123, step B, the title compound was obtained.
MS: [M+H]⁺ 249.1.

### C) N-((1S)-1-(3-fluoro-4-(trifluoromethoxy)phenyl)-2-methoxyethyl)-2-(5-methoxypyrazin-2-yl)-6-oxo-1,6-dihydropyrimidine-4-carboxamide

By a method similar to that of Example 1, step C, the title compound was obtained.

### Example 145

### 2-(3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

### A) 3-fluoropicolinimidamide hydrochloride

To a mixture of ammonium chloride (1.34 g) and toluene (15 mL) was added a solution (1.5 M, 15.3 mL) of diisobutylaluminum hydride in toluene at 0°C, and the mixture was stirred at 80°C for 20 min. To the reaction mixture was added 3-fluoropyridine-2-carbonitrile (0.6 g), and the mixture was stirred at 80°C overnight. To the reaction mixture was added methanol at 0°C, and the mixture was stirred at room temperature for 3 hr, filtered, and insoluble material was washed with methanol. The obtained filtrate was concentrated under reduced pressure, and the residue was washed with ethyl acetate to give the title compound (1.15 g).
MS: [M+H]⁺ 140.1.

### B) 2-(3-fluoropyridin-2-yl)-6-oxo-1,6-dihydropyrimidine-4-carboxylic acid

By a method similar to that of Example 87, step A, the title compound was obtained.

### MS: [M+H]⁺ 236.1.

### C) 2-(3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 146

### N-((1S)-1-(3-fluoro-4-(trifluoromethoxy)phenyl)-2-methoxyethyl)-2-(3-methoxypyridin-2-yl)-6-oxo-1,6-dihydropyrimidine-4-carboxamide

### A) 3-methoxypicolinimidamide hydrochloride

By a method similar to that of Example 145, step A, the title compound was obtained.
MS: [M+H]⁺ 152.1.

### B) 2-(3-methoxypyridin-2-yl)-6-oxo-1,6-dihydropyrimidine-4-carboxylic acid

By a method similar to that of Example 87, step A, the title compound was obtained.
MS: [M+H]⁺ 248.1.

### C) N-((1S)-1-(3-fluoro-4-(trifluoromethoxy)phenyl)-2-methoxyethyl)-2-(3-methoxypyridin-2-yl)-6-oxo-1,6-dihydropyrimidine-4-carboxamide

By a method similar to that of Example 1, step C, the title compound was obtained.

### Example 147

### N-((1S)-1-(3-fluoro-4-(trifluoromethoxy)phenyl)-2-methoxyethyl)-6-((3S)-3-methoxypyrrolidin-1-yl)pyrazine-2-carboxamide

### A) (S)-6-chloro-N-(1-(3-fluoro-4-(trifluoromethoxy)phenyl)-2-methoxyethyl)pyrazine-2-carboxamide

By a method similar to that of Example 9, step A, the title compound was obtained.
MS: [M-H]- 391.9.

### B) N-((1S)-1-(3-fluoro-4-(trifluoromethoxy)phenyl)-2-methoxyethyl)-6-((3S)-3-methoxypyrrolidin-1-yl)pyrazine-2-carboxamide

A mixture of (S)-6-chloro-N-(1-(3-fluoro-4-(trifluoromethoxy)phenyl)-2-methoxyethyl)pyrazine-2-carboxamide (100 mg), (S)-3-methoxypyrrolidine hydrochloride (52.4 mg), potassium carbonate (105 mg) and N,N-dimethylacetamide (1.0 mL) was stirred at 100°C overnight. The reaction mixture was diluted with water, and the precipitate was collected by filtration. The obtained solid was washed with ethyl acetate/hexane to give the title compound (50 mg).
¹H NMR (300 MHz, CDCl₃) δ 2.03-2.20 (1H, m), 2.22-2.34 (1H, m), 3.35-3.44 (6H, m), 3.55-3.82 (6H, m), 4.16 (1H, tt, J = 4.4, 2.4 Hz), 5.21-5.37 (1H, m), 7.15-7.32 (3H, m), 8.07 (1H, s), 8.49 (1H, d, J = 8.1 Hz), 8.59 (1H, s).

### Example 148

### 6-chloro-N-((1S)-1-(3-fluoro-4-(trifluoromethoxy)phenyl)-2-hydroxy-2-methylpropyl)pyrazine-2-carboxamide

By a method similar to that of Example 9, step A, the title compound was obtained.

### Example 149

### N-((1S)-1-(3-fluoro-4-(trifluoromethoxy)phenyl)-2-methoxyethyl)-6-(2-oxopyrrolidin-1-yl)pyrazine-2-carboxamide

By a method similar to that of Example 23, the title compound was obtained.

### Example 150

### N-((1S)-1-(3-fluoro-4-(trifluoromethoxy)phenyl)-2-methoxyethyl)-2-(5-methyl-3-thienyl)-6-oxo-1,6-dihydropyrimidine-4-carboxamide

### A) 5-methylthiophene-3-carboximidamide hydrochloride

By a method similar to that of Example 107, step A, the title compound was obtained.
MS: [M+H]⁺ 141.1.

### B) 2-(5-methylthiophen-3-yl)-6-oxo-1,6-dihydropyrimidine-4-carboxylic acid

By a method similar to that of Example 123, step B, the title compound was obtained.
MS: [M+H]⁺ 237.1.

### C) N-((1S)-1-(3-fluoro-4-(trifluoromethoxy)phenyl)-2-methoxyethyl)-2-(5-methyl-3-thienyl)-6-oxo-1,6-dihydropyrimidine-4-carboxamide

By a method similar to that of Example 1, step C, the title compound was obtained.

### Example 151

### N-((1S)-1-(3-fluoro-4-(trifluoromethoxy)phenyl)-2-methoxyethyl)-2-(5-methyl-2-thienyl)-6-oxo-1,6-dihydropyrimidine-4-carboxamide

### A) 5-methylthiophene-2-carboximidamide hydrochloride

By a method similar to that of Example 107, step A, the title compound was obtained.
MS: [M+H]⁺ 141.1.

### B) 2-(5-methylthiophen-2-yl)-6-oxo-1,6-dihydropyrimidine-4-carboxylic acid

By a method similar to that of Example 123, step B, the title compound was obtained.
MS: [M+H]⁺ 237.1.

### C) N-((1S)-1-(3-fluoro-4-(trifluoromethoxy)phenyl)-2-methoxyethyl)-2-(5-methyl-2-thienyl)-6-oxo-1,6-dihydropyrimidine-4-carboxamide

By a method similar to that of Example 1, step C, the title compound was obtained.

### Example 152

### 2-(3,5-dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide

### A) 3,5-dimethoxypicolinonitrile

To 2-cyano-3,5-difluoropyridine (1.0 g) was added a solution (28%, 4.13 g) of sodium methoxide in methanol at 0°C, methanol (3.0 mL) was added, and the mixture was stirred at room temperature overnight. Water was added to the reaction mixture and the mixture was extracted with ethyl acetate. The extract was washed with saturated brine, and dried over anhydrous sodium sulfate. The solvent was evaporated under reduced pressure to give the title compound (1.1 g).
MS: [M+H]⁺ 165.1.

### B) 3,5-dimethoxypicolinimidamide hydrochloride

To a mixture of ammonium chloride (1.08 g) and toluene (15 mL) was added a solution (13.4 mL) of 1.5 M diisobutylaluminum hydride in toluene at 0°C, and the mixture was stirred at 80°C for 20 min. To the reaction mixture was added 3,5-dimethoxypicolinonitrile (1.1 g), and the mixture was stirred at 80°C overnight, and at 120°C for 5 hr. To the reaction mixture was added methanol at 0°C, and the mixture was stirred at room temperature for 3 hr, filtered, and insoluble material was washed with methanol. The obtained filtrate was concentrated under reduced pressure, and the residue was washed with ethyl acetate to give the title compound (1.39 g).
MS: [M+H]⁺ 182.1.

### C) 2-(3,5-dimethoxypyridin-2-yl)-6-oxo-1,6-dihydropyrimidine-4-carboxylic acid

To diethyl oxaloacetate sodium salt (1.61 g) was added 2 M aqueous sodium hydroxide solution (19.2 mL) at room temperature, and the mixture was stirred at room temperature for 10 min. 3,5-Dimethoxypicolinimidamide hydrochloride (1.39 g) was added, and the mixture was stirred at room temperature overnight, and further at 70°C for 1 hr. To the reaction mixture was added 2 M hydrochloric acid (20 mL) at 0°C, and the mixture was stirred at room temperature overnight. The precipitate was collected by filtration to give the title compound (0.45 g).
MS: [M+H]⁺ 278.1.

### D) 2-(3,5-dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide

To a mixture of 2-(3,5-dimethoxypyridin-2-yl)-6-oxo-1,6-dihydropyrimidine-4-carboxylic acid (100 mg) and (1S)-1-(3-fluoro-4-(trifluoromethoxy)phenyl)-2-methoxyethanamine hydrochloride (80 mg), 1-(3-dimethylaminopropyl)-3-ethylcarbodiimide hydrochloride (90 mg), 1-hydroxybenzotriazole (63.4 mg) and N,N-dimethylformamide (1.0 mL) was added triethylamine (0.096 mL) at room temperature. The reaction mixture was stirred at room temperature for 3 hr, water was added to the reaction mixture, and the precipitated solid was collected by filtration. The solid was purified by silica gel column chromatography (ethyl acetate/hexane) to give the title compound (120 mg).
¹H NMR (300 MHz, CDCl₃) δ 3.43 (3H, s), 3. 80 (2H, qd, J = 9.9, 4.0 Hz), 4.00 (3H, s), 4.01 (3H, s), 5.25-5.34 (1H, m), 6.94 (1H, d, J = 2.3 Hz), 7.16 (1H, s), 7.19-7.34 (3H, m), 8.01 (1H, d, J = 2.3 Hz), 8.97 (1H, d, J = 7.9 Hz), 11.04 (1H, brs).

### Example 153

### N-((1S)-2-methoxy-1-(4-(trifluoromethoxy)phenyl)ethyl)-2-(1-methylcyclopropyl)-6-oxo-1,6-dihydropyrimidine-4-carboxamide

By a method similar to that of Example 1, step C, the title compound was obtained.

### Example 154

### 6-(5-fluoropyridin-2-yl)-N-((1S)-1-(3-fluoro-4-(trifluoromethoxy)phenyl)-2-methoxyethyl)pyrazine-2-carboxamide

A mixture of (S)-6-chloro-N-(1-(3-fluoro-4-(trifluoromethoxy)phenyl)-2-methoxyethyl)pyrazine-2-carboxamide (150 mg), lithium 2-(5-fluoropyridine)cyclic-triol borate (176 mg), palladium(II) acetate (8.6 mg), triphenylphosphine (40.0 mg), copper iodide (I) (36.3 mg) and N,N-dimethylacetamide (1 mL) was stirred under a nitrogen atmosphere at 80°C for 2 hr. The reaction mixture was washed with water and saturated brine, and dried over anhydrous magnesium sulfate. The solvent was evaporated under reduced pressure. The residue was purified by silica gel column chromatography (ethyl acetate/hexane) and recrystallized from ethyl acetate/hexane to give the title compound (62 mg).
¹H NMR (300 MHz, CDCl₃) δ 3.47 (3H, s), 3. 83 (2H, d, J = 4.5 Hz), 5.30-5.44 (1H, m), 7.21-7.36 (3H, m), 7.57-7.69 (1H, m), 8.38 (1H, dd, J = 8.7, 4.5 Hz), 8.57 (1H, d, J = 7.9 Hz), 8.60-8.63 (1H, m), 9.40 (1H, s), 9.79 (1H, s).

### Example 155

### N-((1S)-1-(3-fluoro-4-(trifluoromethoxy)phenyl)-2-methoxyethyl)-6-(5-methoxypyridin-2-yl)pyrazine-2-carboxamide

A mixture of 6-(5-fluoropyridin-2-yl)-N-((1S)-1-(3-fluoro-4-(trifluoromethoxy)phenyl)-2-methoxyethyl)pyrazine-2-carboxamide (50 mg), a solution (28%, 42.5 mg) of sodium methoxide in methanol and methanol (1 mL) was stirred under a nitrogen atmosphere at room temperature overnight, and the mixture was stirred at 50°C for 6 hr. The reaction mixture was diluted with water, and extracted with ethyl acetate. The extract was washed with saturated brine, and dried over anhydrous magnesium sulfate. The solvent was evaporated under reduced pressure. The residue was purified by silica gel column chromatography (ethyl acetate/hexane) and recrystallized from ethyl acetate/hexane to give the title compound (34.3 mg).
¹H NMR (300 MHz, CDCl₃) δ 3.47 (3H, s), 3.83 (2H, d, J = 4.1 Hz), 3.97 (3H, s), 5.25-5.46 (1H, m), 7.19-7.35 (3H, m), 7.39 (1H, dd, J = 8.7, 3.0 Hz), 8.30 (1H, d, J = 8.7 Hz), 8.44 (1H, d, J = 2.6 Hz), 8.60 (1H, d, J = 8.3 Hz), 9.33 (1H, s), 9.77 (1H, s).

### Example 156

### 6-(dimethylamino)-N-((1S)-1-(3-fluoro-4-(trifluoromethoxy)phenyl)-2-hydroxy-2-methylpropyl)pyrazine-2-carboxamide

By a method similar to that of Example 147, step B, the title compound was obtained.

### Example 157

### 6-(azetidin-1-yl)-N-((1S)-1-(3-fluoro-4-(trifluoromethoxy)phenyl)-2-hydroxy-2-methylpropyl)pyrazine-2-carboxamide

By a method similar to that of Example 147, step B, the title compound was obtained.

### Example 158

### N-((1S)-1-(3-fluoro-4-(trifluoromethoxy)phenyl)-2-hydroxy-2-methylpropyl)-6-(1-methyl-1H-pyrazol-4-yl)pyrazine-2-carboxamide

A mixture of (S)-6-chloro-N-(1-(3-fluoro-4-(trifluoromethoxy)phenyl)-2-hydroxy-2-methylpropyl)pyrazine-2-carboxamide (50 mg), 1-methyl-4-(4,4,5,5-tetramethyl-1,3,2-dioxaborolan-2-yl)-1H-pyrazole (51.0 mg), bis(di-tert-butyl(4-dimethylaminophenyl)phosphine)dichloropalladium(II) (8.7 mg), potassium carbonate (50.8 mg), 1,2-dimethoxyethane (0.9 mL) and water (0.3 mL) was stirred under a nitrogen atmosphere at 100°C for 3 hr. The reaction mixture was diluted with water, and extracted with ethyl acetate. The extract was washed with saturated brine, and dried over anhydrous magnesium sulfate. The solvent was evaporated under reduced pressure. The residue was purified by silica gel column chromatography (ethyl acetate/hexane) and recrystallized from ethyl acetate/hexane to give the title compound (11.0 mg).
¹H NMR (300 MHz, CDCl₃) δ 1.18 (3H, s), 1.45 (3H, s), 1.60 (1H, s), 4.04 (3H, s), 4.96 (1H, d, J = 8.7 Hz), 7.20-7.38 (3H, m), 8.01 (1H, s), 8.09 (1H, s), 8.84 (1H, d, J = 9.0 Hz), 8.91 (1H, s), 9.11 (1H, s).

### Example 159

### 2-(5-fluoro-2-furyl)-N-((1S)-1-(3-fluoro-4-(trifluoromethoxy)phenyl)-2-methoxyethyl)-6-oxo-1,6-dihydropyrimidine-4-carboxamide

### A) 5-fluorofuran-2-carboximidamide hydrochloride

By a method similar to that of Example 98, step B, the title compound was obtained.
MS: [M+H]⁺ 129.1.

### B) 2-(5-fluoro-2-furyl)-6-oxo-1,6-dihydropyrimidine-4-carboxylic acid

By a method similar to that of Example 123, step B, the title compound was obtained.
MS: [M+H]⁺ 225.1.

### C) 2-(5-fluoro-2-furyl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 160

### N-((1S)-1-(3-fluoro-4-(trifluoromethoxy)phenyl)-2-methoxyethyl)-6-oxo-2-(1-phenylcyclopropyl)-1,6-dihydropyrimidine-4-carboxamide

By a method similar to that of Example 145, step A, Example 87, step A and Example 1, step C, the title compound was obtained.

### Example 161

### N-((1S)-1-(3-fluoro-4-(trifluoromethoxy)phenyl)-2-methoxyethyl)-6-oxo-2-(1-(trifluoromethyl)cyclopropyl)-1,6-dihydropyrimidine-4-carboxamide

By a method similar to that of Example 98, step B, Example 87, step A and Example 1, step C, the title compound was obtained.

### Example 162

### 2-(bicyclo[3.1.0]hex-1-yl)-N-((1S)-1-(3-fluoro-4-(trifluoromethoxy)phenyl)-2-methoxyethyl)-6-oxo-1,6-dihydropyrimidine-4-carboxamide

To a mixture of sodium hydride (60% in oil, 0.761 g) and DMSO (30 mL) was added trimethylsulfoxonium iodide (4.19 g) under ice-cooling, and the mixture was stirred at room temperature for 30 min. To the mixture was added a mixture of methyl cyclopent-1-en carboxylate (2 g) and DMSO (30 mL), and the mixture was stirred at room temperature for 1 hr. To the reaction mixture was added a saturated aqueous ammonium chloride solution, and the mixture was extracted with diethyl ether/hexane. The extract was washed with saturated brine, and dried over anhydrous magnesium sulfate. The solvent was evaporated under reduced pressure to give an oil (0.69 g). To a mixture of ammonium chloride (1.32 g) and toluene (20 mL) was added a solution (about 1.4 M, 17.6 mL) of trimethylaluminum in hexane at room temperature, and the mixture was stirred under a nitrogen atmosphere at room temperature for 30 min. To the reaction mixture was added a mixture of the oil (0.69 g) and toluene (10 mL), and the mixture was stirred under a nitrogen atmosphere at 70°C overnight. To the reaction mixture was added methanol under ice-cooling, and the mixture was stirred under ice-cooling for 1 hr, and filtered through celite. The solvent was evaporated under reduced pressure. The residue was suspended in ethyl acetate/methanol, and insoluble material was removed by filtration. The solvent in the filtrate was evaporated under reduced pressure to give a solid (480 mg). To a mixture of diethyl oxaloacetate sodium salt (722 mg), water (1.5 mL) and ethanol (0.3 mL) was added 8 M aqueous sodium hydroxide solution (0.43 mL) at room temperature, and the mixture was stirred at room temperature for 30 min. A mixture of the obtained solid (460 mg) and water (0.5 mL) and 8 M aqueous sodium hydroxide solution (0.286 mL) was added. The mixture was stirred at room temperature overnight. The reaction mixture was acidified with 6 M hydrochloric acid, and the precipitate was collected by filtration to give a solid (253 mg). To a mixture of the obtained solid (130 mg), (S)-1-(3-fluoro-4-(trifluoromethoxy)phenyl)-2-methoxyethanamine hydrochloride (171 mg), triethylamine (0.247 mL) and N,N-dimethylformamide (3.0 mL) were added 1-(3-dimethylaminopropyl)-3-ethylcarbodiimide hydrochloride (136 mg) and 1-hydroxybenzotriazole (96 mg), and the mixture was stirred at room temperature overnight. The reaction mixture was diluted with water, and the precipitate was collected by filtration. The obtained solid was purified by silica gel column chromatography (ethyl acetate/hexane) and recrystallized from ethyl acetate/hexane to give the title compound (72.4 mg).
¹H NMR (300 MHz, CDCl₃) δ 1.20 (1H, t, J = 5.3 Hz), 1.31-1.52 (2H, m), 1.69-1.99 (3H, m), 2.05-2.30 (3H, m), 3.42 (3H, s), 3.73 (2H, d, J = 4.5 Hz), 5.12-5.30 (1H, m), 7.03 (1H, d, J = 0.8 Hz), 7.11-7.32 (3H, m), 8.55 (1H, d, J = 7.9 Hz), 10.88 (1H, brs).

### Example 163

### N-((1S)-1-(3-fluoro-4-(trifluoromethoxy)phenyl)-2-hydroxy-2-methylpropyl)-6-((3S)-3-methoxypyrrolidin-1-yl)pyrazine-2-carboxamide

By a method similar to that of Example 147, step B, the title compound was obtained.

### Example 164

### N-((1S)-1-(3-fluoro-4-(trifluoromethoxy)phenyl)-2-methoxyethyl)-2-(1-methylcyclobutyl)-6-oxo-1,6-dihydropyrimidine-4-carboxamide

### A) 1-methylcyclobutanecarboximidamide hydrochloride

By a method similar to that of Example 98, step B, the title compound was obtained.
MS: [M+H]⁺ 113.1.

### B) 2-(1-methylcyclobutyl)-6-oxo-1,6-dihydropyrimidine-4-carboxylic acid

By a method similar to that of Example 123, step B, the title compound was obtained.
MS: [M+H]⁺ 209.1.

### C) N-((1S)-1-(3-fluoro-4-(trifluoromethoxy)phenyl)-2-methoxyethyl)-2-(1-methylcyclobutyl)-6-oxo-1,6-dihydropyrimidine-4-carboxamide

By a method similar to that of Example 1, step C, the title compound was obtained.

### Example 165

### N-((1S)-2-methoxy-1-(4-(trifluoromethoxy)phenyl)ethyl)-2-(1-methylcyclobutyl)-6-oxo-1,6-dihydropyrimidine-4-carboxamide

By a method similar to that of Example 1, step C, the title compound was obtained.

### Example 166

### 2-(cyclopropylethynyl)-N-((1S)-1-(3-fluoro-4-(trifluoromethoxy)phenyl)-2-methoxyethyl)-6-oxo-1,6-dihydropyrimidine-4-carboxamide

### A) 3-cyclopropylpropiolic imidamide hydrochloride

By a method similar to that of Example 98, step B, the title compound was obtained.
MS: [M+H]⁺ 109.1.

### B) 2-(cyclopropylethynyl)-6-oxo-1,6-dihydropyrimidine-4-carboxylic acid

By a method similar to that of Example 123, step B, the title compound was obtained.
MS: [M+H]⁺ 204.9.

### C) 2-(cyclopropylethynyl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 167

### 6-cyclopropyl-N-((1S)-1-(3-fluoro-4-(trifluoromethoxy)phenyl)-2-hydroxy-2-methylpropyl)pyrazine-2-carboxamide

By a method similar to that of Example 135, step A, the title compound was obtained.

### Example 168

### N-((1S)-1-(3-fluoro-4-(trifluoromethoxy)phenyl)-2-methoxyethyl)-6-oxo-2-(1-phenylcyclobutyl)-1,6-dihydropyrimidine-4-carboxamide

### A) 1-phenylcyclobutanecarbonitrile

Sodium hydride (60% in oil, 2.56 g) was added to N,N-dimethylformamide (15 mL) at 0°C, a solution of phenylacetonitrile (2.95 mL) and 1,3-dibromopropane (2.60 mL) in N,N-dimethylformamide (15 mL) was added dropwise at 0°C, and the mixture was stirred at room temperature for 2 hr. Water was added to the reaction mixture, and the mixture was extracted with diethyl ether. The extract was washed with water, and dried over anhydrous sodium sulfate. The solvent was evaporated under reduced pressure to give the title compound (3.95 g).
¹H NMR (300 MHz, CDCl₃) δ 2.08 (1H, dtt, J = 11.5, 9.0, 4.4 Hz), 2.35-2.53 (1H, m), 2.56-2.70 (2H, m), 2.77-2.89 (2H, m), 7.28-7.35 (1H, m), 7.35-7.45 (4H, m).

### B) N-((1S)-1-(3-fluoro-4-(trifluoromethoxy)phenyl)-2-methoxyethyl)-6-oxo-2-(1-phenylcyclobutyl)-1,6-dihydropyrimidine-4-carboxamide

By a method similar to that of Example 145, step A, Example 87, step A and Example 1, step C, the title compound was obtained.

### Example 169

### N-((1S)-1-(3-fluoro-4-(trifluoromethoxy)phenyl)-2-methoxyethyl)-6-oxo-2-(2-(pyridin-2-yl)propan-2-yl)-1,6-dihydropyrimidine-4-carboxamide

### A) 2-methyl-2-(pyridin-2-yl)propanenitrile

To a solution of 2-fluoropyridine (2.58 mL) in toluene (50 mL) were added isobutyronitrile (8.29 g) and potassium hexamethyldisilazide (11% toluene solution, 90 mL) at room temperature, and the mixture was stirred at 80°C overnight. Water was added to the reaction mixture, and the mixture was extracted with diethyl ether. The extract was washed with saturated brine, and dried over anhydrous sodium sulfate. The solvent was evaporated under reduced pressure to give the title compound (3.60 g).
¹H NMR (300 MHz, CDCl₃) δ 1 .77 (6H, s), 7.20-7.28 (1H, m), 7.56-7.62 (1H, m), 7.69-7.76 (1H, m), 8.59-8.63 (1H, m).

### B) N-((1S)-1-(3-fluoro-4-(trifluoromethoxy)phenyl)-2-methoxyethyl)-6-oxo-2-(2-(pyridin-2-yl)propan-2-yl)-1,6-dihydropyrimidine-4-carboxamide

By a method similar to that of Example 145, step A, Example 87, step A and Example 1, step C, the title compound was obtained.

### Example 170

### N-((1S)-1-(3-fluoro-4-(trifluoromethoxy)phenyl)-2-methoxyethyl)-6-oxo-2-(2-phenylpropan-2-yl)-1,6-dihydropyrimidine-4-carboxamide

By a method similar to that of Example 145, step A, Example 87, step A and Example 1, step C, the title compound was obtained.

### Example 171

### N-((1S)-1-(3-fluoro-4-(trifluoromethoxy)phenyl)-2-methoxyethyl)-2-(4-methoxycyclopent-1-en-1-yl)-6-oxo-1,6-dihydropyrimidine-4-carboxamide

### A) tert-butyl((3,4-dibromocyclopentyl)oxy)diphenylsilane

To a solution of tert-butyl(cyclopent-3-en-1-yloxy)diphenylsilane (5.514 g) in carbon tetrachloride (152 mL) was added dropwise bromine (1.34 mL) at room temperature. The reaction mixture was stirred at room temperature for 4 hr and the solvent was evaporated under reduced pressure. The residue was purified by silica gel column chromatography (ethyl acetate/hexane) to give the title compound (6.32 g).
¹H NMR (300 MHz, CDCl₃) δ 1.07 (9H, s), 2.12-2.31 (2H, m), 2.56-2.82 (2H, m), 4.25-4.33 (1H, m), 4.47-4.61 (2H, m), 7.33-7.49 (6H, m), 7.64 (4H, ddd, J = 7.9, 2.8, 1.7 Hz).

### B) ((3-bromocyclopent-3-en-1-yl)oxy)(tert-butyl)diphenylsilane

To a solution of tert-butyl((3,4-dibromocyclopentyl)oxy)diphenylsilane (6.32 g) in tetrahydrofuran (47.5 mL) was added a suspension of potassium tert-butoxide (5.19 g) in tetrahydrofuran (47.5 mL) at 0°C. The reaction mixture was stirred under a nitrogen atmosphere at 0°C for 2 hr. Water was added to the reaction mixture at 0°C, and the mixture was extracted with diethyl ether. The extract was washed with saturated brine, dried over anhydrous sodium sulfate, and the solvent was evaporated under reduced pressure. The residue was purified by silica gel column chromatography (ethyl acetate/hexane) to give the title compound (1.86 g).
¹H NMR (300 MHz, CDCl₃) δ 1.05 (9H, s), 2.31-2.50 (2H, m), 2.64 (2H, dq, J = 5.7, 1.9 Hz), 4.48-4.61 (1H, m), 5.73 (1H, quin, J = 2.3 Hz), 7.32-7.47 (6H, m), 7.61-7.69 (4H, m).

### C) tert-butyl(diphenyl)((3-(4,4,5,5-tetramethyl-1,3,2-dioxaborolan-2-yl)cyclopent-3-en-1-yl)oxy)silane

A mixture of ((3-bromocyclopent-3-en-1-yl)oxy)(tert-butyl)diphenylsilane (1.31 g), 4,4,4',4',5,5,5',5'-octamethyl-2,2'-bi-1,3,2-dioxaborolane (0.989 g), potassium acetate (0.64 g), tris(dibenzylideneacetone)dipalladium (0) (40 mg), dicyclohexyl(2',4',6'-triisopropylbiphenyl-2-yl)phosphine (0.064 g) and 1,2-dimethoxyethane (46.6 mL) was stirred under an argon atmosphere at 90°C for 2 hr. The reaction mixture was cooled to room temperature, water was added, and the mixture was extracted with ethyl acetate. The extract was washed with water and saturated brine, dried over anhydrous sodium sulfate, and the solvent was evaporated under reduced pressure. The residue was purified by silica gel column chromatography (ethyl acetate/hexane) to give the title compound (1.372 g).
¹H NMR (300 MHz, CDCl₃) δ 1.04 (9H, s), 1.26 (12H, s), 2.47 (2H, dt, J = 4.6, 2.0 Hz), 2.55-2.67 (2H, m), 4.50-4.63 (1H, m), 6.37-6.45 (1H, m), 7.31-7.45 (6H, m), 7.62-7.70 (4H, m).

### D) methyl 6-((4-methoxybenzyl)oxy)-2-(4-methoxycyclopent-1-en-1-yl)pyrimidine-4-carboxylate

A mixture of methyl 2,6-dichloropyrimidine-4-carboxylate (3.55 g), 4-methoxybenzylalcohol (4.28 mL), 1,8-diazabicyclo[5.4.0]undec-7-ene (2.58 mL) and acetonitrile (70 mL) was stirred under a nitrogen atmosphere at room temperature overnight. Water was added to the reaction mixture, and the mixture was extracted with ethyl acetate. The extract was washed with water and saturated brine, and dried over anhydrous sodium sulfate. The solvent was evaporated under reduced pressure, and the residue was purified by silica gel column chromatography (ethyl acetate/hexane) to give a pale-yellow oil (3.03 g). A mixture of the obtained oil (369 mg), tert-butyl(diphenyl)((3-(4,4,5,5-tetramethyl-1,3,2-dioxaborolan-2-yl)cyclopent-3-en-1-yl)oxy)silane (1.34 g), bis(di-tert-butyl(4-dimethylaminophenyl)phosphine)dichloropalladium(II) (211 mg), tripotassium phosphate (1.334 g) and N,N-dimethylformamide (59.7 mL) was heated at 90°C for 1.5 hr. After cooling to room temperature, the reaction mixture was poured into water, and the mixture was extracted with ethyl acetate. The extract was washed with water and saturated brine, dried over anhydrous sodium sulfate, and the solvent was evaporated under reduced pressure. The residue was purified by silica gel column chromatography (ethyl acetate/hexane) to give a yellow oil (679.3 mg). To a solution of the obtained oil (599 mg) in tetrahydrofuran (18.5 mL) was added tetrabutylammonium fluoride (1.196 mL) at 0°C. After stirring at room temperature for 5 hr, water was added to the reaction mixture, and the mixture was extracted with ethyl acetate. The extract was washed with saturated brine, dried over anhydrous sodium sulfate, and the solvent was evaporated under reduced pressure. The residue was purified by silica gel column chromatography (ethyl acetate/hexane) to give a yellow oil (284.1 mg). To a solution of the obtained oil (238.5 mg) in N,N-dimethylformamide (11.7 mL) was added sodium hydride (60% in oil, 34.8 mg) at 0°C. The reaction mixture was stirred at 0°C for 30 min, and methyl iodide (0.0544 mL) was added. The reaction mixture was stirred at room temperature for 1 hr, methyl iodide (0.0544 mL) was added, and the mixture was further stirred for 1 hr. To the reaction mixture was added methyl iodide (0.0544 mL) again, and the mixture was stirred at room temperature for 1 hr, and cooled to 0°C. To the reaction mixture were added sodium hydride (60% in oil, 46.4 mg) and methyl iodide (0.0726 mL) at 0°C, and the mixture was stirred at room temperature overnight. To the reaction mixture was added methyl iodide (0.0544 mL) at room temperature, and the mixture was stirred at room temperature for 1 hr. Sodium hydride (60% in oil, 46.4 mg) and methyl iodide (0.091 mL) were added, and the mixture was stirred at room temperature for 1 hr. Water was added to the reaction mixture, and the mixture was extracted with ethyl acetate. The extract was washed with saturated brine, dried over anhydrous sodium sulfate, and the solvent was evaporated under reduced pressure. The residue was purified by silica gel column chromatography (ethyl acetate/hexane) to give the title compound (100.3 mg).
MS: [M+H]⁺ 371.3.

### E) N-((1S)-1-(3-fluoro-4-(trifluoromethoxy)phenyl)-2-methoxyethyl)-6-((4-methoxybenzyl)oxy)-2-(4-methoxycyclopent-1-en-1-yl)pyrimidine-4-carboxamide

By a method similar to that of Example 14, step B, the title compound was obtained.
MS: [M+H]⁺ 592.3.

### F) N-((1S)-1-(3-fluoro-4-(trifluoromethoxy)phenyl)-2-methoxyethyl)-2-(4-methoxycyclopent-1-en-1-yl)-6-oxo-1,6-dihydropyrimidine-4-carboxamide

By a method similar to that of Example 24, step C, the title compound was obtained.

### Example 172

### N-((1S)-1-(3-fluoro-4-(trifluoromethoxy)phenyl)-2-methoxyethyl)-2-(1-methylcyclopentyl)-6-oxo-1,6-dihydropyrimidine-4-carboxamide

### A) 1-methylcyclopentanecarboximidamide hydrochloride

By a method similar to that of Example 98, step B, the title compound was obtained.
MS: [M+H]⁺ 127.2.

### B) 2-(1-methylcyclopentyl)-6-oxo-1,6-dihydropyrimidine-4-carboxylic acid

By a method similar to that of Example 123, step B, the title compound was obtained.
MS: [M+H]⁺ 223.1.

### C) N-((1S)-1-(3-fluoro-4-(trifluoromethoxy)phenyl)-2-methoxyethyl)-2-(1-methylcyclopentyl)-6-oxo-1,6-dihydropyrimidine-4-carboxamide

By a method similar to that of Example 1, step C, the title compound was obtained.

### Example 173

### N-((1S)-2-methoxy-1-(4-(trifluoromethoxy)phenyl)ethyl)-2-(1-methylcyclopentyl)-6-oxo-1,6-dihydropyrimidine-4-carboxamide

By a method similar to that of Example 1, step C, the title compound was obtained.

### Example 174

### N-(2-hydroxy-2-methyl-1-(4-(trifluoromethoxy)phenyl)propyl)-6-(1-methyl-1H-pyrazol-3-yl)pyrazine-2-carboxamide

### A) tert-butyl 6-(1-methyl-1H-pyrazol-3-yl)pyrazine-2-carboxylate

A mixture of tert-butyl 6-chloropyrazine-2-carboxylate (100 mg), 1-methyl-3-(4,4,5,5-tetramethyl-1,3,2-dioxaborolan-2-yl)-1H-pyrazole (126 mg), bis(di-tert-butyl(4-dimethylaminophenyl)phosphine)dichloropalladium(II) (42.5 mg), tripotassium phosphate (198 mg) and N,N-dimethylformamide (3.0 mL) was stirred under an argon atmosphere at 90°C for 12 hr. Water was added to the reaction mixture at room temperature, and the mixture was extracted with ethyl acetate. The extract was washed with water and saturated brine, and dried over anhydrous sodium sulfate. The solvent was evaporated under reduced pressure. The residue was purified by silica gel column chromatography (ethyl acetate/hexane) to give the title compound (92.7 mg).
¹H NMR (300 MHz, CDCl₃) δ 1.66 (9H, s), 4.01 (3H, s), 7.07 (1H, d, J = 2.3 Hz), 7.44 (1H, d, J = 2.3 Hz), 9.07 (1H, s), 9.35 (1H, s).

### B) N-(2-hydroxy-2-methyl-1-(4-(trifluoromethoxy)phenyl)propyl)-6-(1-methyl-1H-pyrazol-3-yl)pyrazine-2-carboxamide

By a method similar to that of Example 14, step B, the title compound was obtained.

### Example 175

### N-(2-hydroxy-2-methyl-1-(4-(trifluoromethoxy)phenyl)propyl)-6-(1H-pyrazol-1-yl)pyrazine-2-carboxamide

### A) tert-butyl 6-(1H-pyrazol-1-yl)pyrazine-2-carboxylate

To a mixture of 1H-pyrazole (76 mg) and N,N-dimethylformamide (5.0 mL) was added sodium hydride (60% in oil, 44.7 mg) under ice-cooling, and the mixture was stirred under a nitrogen atmosphere for 30 min. A mixture of tert-butyl 6-chloropyrazine-2-carboxylate (200 mg) and N,N-dimethylformamide (2.0 mL) was added, and the mixture was stirred at room temperature overnight. To the reaction mixture was added a saturated aqueous ammonium chloride solution, and the mixture was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (147 mg).
¹H NMR (300 MHz, CDCl₃) δ 1.67 (9H, s), 6.53 (1H, dd, J = 2.6, 1.5 Hz), 7.81 (1H, d, J = 1.5 Hz), 8.64 (1H, d, J = 2.6 Hz), 9.08 (1H, s), 9.48 (1H, s).

### B) methyl 2-((tert-butoxycarbonyl)amino)-2-(4-(trifluoromethoxy)phenyl)acetate

To a mixture of 4-(trifluoromethoxy)benzaldehyde (19.0 g) and ammonium carbonate (25.9 g) in ethanol (114 mL) and water (45.6 mL) was slowly added an aqueous solution (71.1 mL) of potassium cyanide (8.14 g) at 50°C. The reaction mixture was stirred at 60°C for 3 hr, cooled to room temperature, and ethanol was evaporated under reduced pressure. Concentrated hydrochloric acid was added to the residue at 0°C to adjust the pH to 1, and the resulting solid was collected by filtration and washed with water. To an aqueous solution (100 mL) of potassium hydroxide (23.6 g) was added the solid obtained by the above-mentioned operation at room temperature, and the reaction mixture was stirred at 90°C for 3 days. The reaction mixture was cooled to room temperature, and neutralized with concentrated hydrochloric acid. The resulting solid was collected by filtration and washed with water to give 2-amino-2-(4-(trifluoromethoxy)phenyl)acetic acid as a crude product (13.3 g). To a solution of the obtained crude product (13.3 g) in tetrahydrofuran (113 mL) were added di-tert-butyl dicarbonate (19.7 mL) and 2 M aqueous sodium hydroxide solution (85 mL) at room temperature. The reaction mixture was stirred at room temperature overnight, poured into water, and washed with diethyl ether. 1 M Hydrochloric acid was added to the aqueous layer at 0°C to adjust the pH to 3, and the mixture was extracted with ethyl acetate. The extract was washed with saturated brine, and dried over anhydrous magnesium sulfate. The solvent was evaporated under reduced pressure to give 2-((tert-butoxycarbonyl)amino)-2-(4-(trifluoromethoxy)phenyl)acetic acid as a crude product (11.3 g). To a solution of the obtained crude product (11.3 g) in N,N-dimethylformamide (84 mL) were added methyl iodide (2.53 mL) and potassium carbonate (5.59 g) at room temperature. The reaction mixture was stirred at room temperature for 2 hr, poured into water, and extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (8.20 g).
MS: [M-H]- 348.1.

### C) tert-butyl (2-hydroxy-2-methyl-1-(4-(trifluoromethoxy)phenyl)propyl)carbamate

To a solution of methyl 2-((tert-butoxycarbonyl)amino)-2-(4-(trifluoromethoxy)phenyl)acetate (5.00 g) in tetrahydrofuran (71.6 mL) was slowly added 1 M methylmagnesium bromide/tetrahydrofuran solution (57.3 mL) at 0°C. The reaction mixture was stirred under an argon atmosphere at 0°C for 1 hr, saturated aqueous ammonium chloride solution was added at 0°C, and the mixture was extracted with ethyl acetate. The extract was washed with saturated brine, and dried over anhydrous magnesium sulfate. The solvent was evaporated under reduced pressure. The residue was purified by silica gel column chromatography (hexane/ethyl acetate) to give the title compound (3.99 g).
MS: [M-H]- 348.2.

### D) 1-amino-2-methyl-1-(4-(trifluoromethoxy)phenyl)propan-2-ol hydrochloride

To tert-butyl (2-hydroxy-2-methyl-1-(4-(trifluoromethoxy)phenyl)propyl)carbamate (2.50 g) was added 4 M hydrogen chloride/ethyl acetate solution (71.6 mL). The reaction mixture was stirred at room temperature for 1 hr, and the solvent was evaporated under reduced pressure. Diisopropyl ether was added to the residue, and the resulting solid was collected by filtration to give the title compound (2.01 g). MS: found: 250.1.

### E) N-(2-hydroxy-2-methyl-1-(4-(trifluoromethoxy)phenyl)propyl)-6-(1H-pyrazol-1-yl)pyrazine-2-carboxamide

To a mixture of tert-butyl 6-(1H-pyrazol-1-yl)pyrazine-2-carboxylate (140 mg) and ethyl acetate (1.0 mL) was added 4 M hydrogen chloride/ethyl acetate solution (2.0 mL) at room temperature, and the mixture was stirred at room temperature for 30 min. The reaction mixture was heated to 50°C, stirred for 1 hr and the solvent was evaporated under reduced pressure. Trifluoroacetic acid (2.0 mL) was added to the residue, and the mixture was stirred at room temperature overnight. The solvent was evaporated under reduced pressure to give a solid (162 mg). To a mixture of the obtained solid (160 mg), 1-amino-2-methyl-1-(4-(trifluoromethoxy)phenyl)propan-2-ol hydrochloride (100 mg) and N,N-dimethylformamide (2.0 mL) were added N,N-diisopropylethylamine (0.245 mL) and O-(7-azabenzotriazol-1-yl)-N,N,N',N'-tetramethyluronium hexafluorophosphate (200 mg) at room temperature, and the mixture was stirred at room temperature for 2 hr. The reaction mixture was diluted with water, and extracted with ethyl acetate. The extract was washed with saturated brine, and dried over anhydrous magnesium sulfate. The solvent was evaporated under reduced pressure. The residue was purified by silica gel column chromatography (ethyl acetate/hexane) and recrystallized from ethyl acetate/hexane to give the title compound (76 mg).
¹H NMR (300 MHz, CDCl₃) δ 1.16 (3H, s), 1.45 (3H, s), 1.59 (1H, s), 5.00 (1H, d, J = 8.7 Hz), 6.61 (1H, dd, J = 2.6, 1.9 Hz), 7.21 (2H, d, J = 7.9 Hz), 7.47 (2H, d, J = 8.7 Hz), 7.86 (1H, d, J = 1.1 Hz), 8.54 (1H, d, J = 2.3 Hz), 8.66 (1H, d, J = 9.0 Hz), 9.23 (1H, s), 9.53 (1H, s).

### Example 176

### 6-(1H-benzimidazol-1-yl)-N-(2-hydroxy-2-methyl-1-(4-(trifluoromethoxy)phenyl)propyl)pyrazine-2-carboxamide

### A) tert-butyl 6-(1H-benzo[d]imidazol-1-yl)pyrazine-2-carboxylate

By a method similar to that of Example 175, step A, the title compound was obtained.
MS: [M+H]⁺ 297.1.

### B) 6-(1H-benzimidazol-1-yl)-N-(2-hydroxy-2-methyl-1-(4-(trifluoromethoxy)phenyl)propyl)pyrazine-2-carboxamide

A mixture of tert-butyl 6-(1H-benzo[d]imidazol-1-yl)pyrazine-2-carboxylate (98 mg) and trifluoroacetic acid (2 mL) was stirred at room temperature for 30 min. The solvent was evaporated under reduced pressure to give a solid (137 mg). To a mixture of the obtained solid (60 mg), 1-amino-2-methyl-1-(4-(trifluoromethoxy)phenyl)propan-2-ol hydrochloride (36.6 mg) and N,N-dimethylformamide (2 mL) were added N,N-diisopropylethylamine (0.112 mL) and O-(7-azabenzotriazol-1-yl)-N,N,N',N'-tetramethyluronium hexafluorophosphate (73.1 mg) at room temperature, and the mixture was stirred at room temperature overnight. The reaction mixture was diluted with water, and extracted with ethyl acetate. The extract was washed with saturated brine, and dried over anhydrous magnesium sulfate. The solvent was evaporated under reduced pressure. The residue was purified by silica gel column chromatography (ethyl acetate/hexane) and recrystallized from ethyl acetate/hexane to give the title compound (46 mg).
¹H NMR (300 MHz, CDCl₃) δ 1.16 (3H, s), 1.47 (3H, s), 1.72 (1H, s), 5.03 (1H, d, J = 9.0 Hz), 7.21 (2H, d, J = 7.9 Hz), 7.44-7.53 (4H, m), 7.90-7.98 (1H, m), 8.07-8.14 (1H, m), 8.63 (1H, s), 8.75 (1H, d, J = 9.0 Hz), 9.18 (1H, s), 9.34 (1H, s).

### Example 177

### N-(2-hydroxy-2-methyl-1-(4-(trifluoromethoxy)phenyl)propyl)-6-(4-methyl-1H-imidazol-1-yl)pyrazine-2-carboxamide

### A) tert-butyl 6-(4-methyl-1H-imidazol-1-yl)pyrazine-2-carboxylate

A mixture of tris(dibenzylideneacetone)dipalladium (85 mg), 2-di-tert-butylphosphino-3,4,5,6-tetramethyl-2',4',6'-triisopropyl-1,1'-biphenyl (90 mg), toluene (0.7 mL) and tert-amyl alcohol (0.1 mL) was stirred under a nitrogen atmosphere at 120°C for 5 min. To the reaction mixture was added a mixture of tert-butyl 6-chloropyrazine-2-carboxylate (200 mg), 4-methyl-1H-imidazole (115 mg) and potassium phosphate (396 mg), and the mixture was stirred at 120°C overnight. The reaction mixture was diluted with water, and extracted with ethyl acetate. The extract was washed with saturated brine, and dried over anhydrous magnesium sulfate. The solvent was evaporated under reduced pressure. The residue was purified by silica gel column chromatography (NH, ethyl acetate/hexane) to give the title compound (116 mg).
MS: [M+H]⁺ 261.1.

### B) N-(2-hydroxy-2-methyl-1-(4-(trifluoromethoxy)phenyl)propyl)-6-(4-methyl-1H-imidazol-1-yl)pyrazine-2-carboxamide

By a method similar to that of Example 176, step B, the title compound was obtained.

### Example 178

### 2-(3-fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

### A) methyl 3-fluoro-5-methoxypicolinimidate

To a mixture of 2-cyano-3,5-difluoropyridine (2.0 g) and methanol (30 mL) was added sodium methoxide (1.8 g) at 0°C, and the mixture was stirred under a nitrogen atmosphere, and at room temperature overnight. Water was added to the reaction mixture, and the mixture was extracted with ethyl acetate. The extract was washed with saturated brine, and dried over anhydrous sodium sulfate. The solvent was evaporated under reduced pressure. The residue was purified by silica gel column chromatography (ethyl acetate/hexane) to give the title compound (0.09 g).
MS: [M+H]⁺ 185.1.

### B) 3-fluoro-5-methoxypicolinimidamide hydrochloride

To a solution of methyl 3-fluoro-5-methoxypicolinimidate (0.09 g) in methanol (10 mL) was added ammonium chloride (0.039 g) at room temperature, and the mixture was stirred at room temperature overnight. The solvent in the reaction mixture was evaporated under reduced pressure to give the title compound (0.09 g).
MS: [M+H]⁺ 170.1.

### C) 2-(3-fluoro-5-methoxypyridin-2-yl)-6-oxo-1,6-dihydropyrimidine-4-carboxylic acid

To a mixture of diethyl oxaloacetate sodium salt (123 mg), water (0.5 mL) and ethanol (0.1 mL) was added 8 M aqueous sodium hydroxide solution (0.073 mL) at room temperature, and the mixture was stirred at room temperature for 30 min. A mixture of 3-fluoro-5-methoxypicolinimidamide hydrochloride (0.090 g) and water (0.2 mL) and 8 M aqueous sodium hydroxide solution (0.049 mL) were added to the reaction mixture, and the mixture was stirred at room temperature overnight. The reaction mixture was acidified with 6 M hydrochloric acid, and the precipitate was collected by filtration to give the title compound (71 mg).
MS: [M+H]⁺ 266.1

### D) 2-(3-fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

To a solution of 2-(3-fluoro-5-methoxypyridin-2-yl)-6-oxo-1,6-dihydropyrimidine-4-carboxylic acid (70 mg) and (1S)-1-(3-fluoro-4-(trifluoromethoxy)phenyl)-2-methoxyethanamine hydrochloride (76 mg) in N,N-dimethylformamide (5.0 mL) were added 1-(3-dimethylaminopropyl)-3-ethylcarbodiimide hydrochloride (76 mg), 1-hydroxybenzotriazole (53.5 mg) and triethylamine (0.184 mL) at room temperature. The reaction mixture was stirred at room temperature overnight, poured into water, and the mixture was extracted with ethyl acetate. The extract was washed with water and brine, and dried over anhydrous magnesium sulfate. The solvent was evaporated under reduced pressure. The residue was fractionated by HPLC (C18, mobile phase: water/acetonitrile (0.05% TFA-containing system)), to the obtained fraction was added a saturated aqueous sodium hydrogen carbonate solution, and the mixture was extracted with ethyl acetate, dried over anhydrous sodium sulfate, and concentrated under reduced pressure to give the title compound (62.7 mg).
¹H NMR (300 MHz, CDCl₃) δ 3.44 (3H, s), 3.76 (2H, d, J = 4.5 Hz), 4.00 (3H, s), 5.21-5.28 (1H, m), 7.15 (1H, dd, J = 12.2, 2.4 Hz), 7.18-7.23 (2H, m), 7.25-7.32 (2H, m), 8.22-8.26 (1H, m), 8.84 (1H, d, J = 7.8 Hz), 10.71-11.07 (1H, m).

### Example 179

### N-((1S)-1-(3-fluoro-4-(trifluoromethoxy)phenyl)-2-methoxyethyl)-2-(5-methoxy-3-methylpyridin-2-yl)-6-oxo-1,6-dihydropyrimidine-4-carboxamide

### A) 5-bromo-3-methylpicolinonitrile

A mixture of 2,5-dibromo-3-methyl-pyridine (2.58 mL), copper(I) cyanide (2.14 g) and N,N-dimethylformamide (20 mL) was stirred under a nitrogen stream at 170°C for 2 hr. Water was added to the reaction mixture, and the mixture was extracted with ethyl acetate. Insoluble material was removed by filtration, and the filtrate was washed with water, and dried over anhydrous sodium sulfate. The solvent was evaporated under reduced pressure. The residue was purified by silica gel column chromatography (ethyl acetate/hexane) to give the title compound (1.75 g).
MS: [M+H]⁺ 197.0.

### B) 5-methoxy-3-methylpicolinonitrile

To a mixture of sodium methoxide (0.88 g) and methanol (20 mL) was added 5-bromo-3-methylpicolinonitrile (1.6 g), and the mixture was stirred under a nitrogen stream at 60°C overnight. To the reaction mixture was added a solution (28%, 4.7 g) of sodium methoxide in methanol and the mixture was heated under reflux under a nitrogen stream at 90°C for 3 hr. Water was added to the reaction mixture, and the mixture was extracted with ethyl acetate. The extract was washed with water, and dried over anhydrous sodium sulfate. The solvent was evaporated under reduced pressure. The residue was purified by silica gel column chromatography (ethyl acetate/hexane) to give the title compound (0.83 g).
MS: [M+H]⁺ 149.1.

### C) N-((1S)-1-(3-fluoro-4-(trifluoromethoxy)phenyl)-2-methoxyethyl)-2-(5-methoxy-3-methylpyridin-2-yl)-6-oxo-1,6-dihydropyrimidine-4-carboxamide

By a method similar to that of Example 145, step A, Example 123, step B and Example 1, step C, the title compound was obtained.

### Example 180

### 2-(5-fluoro-3-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-fluoro-3-methoxypicolinonitrile

To a mixture of 2-cyano-3,5-difluoropyridine (2.0 g) and methanol (30 mL) was added sodium methoxide (1.8 g) at 0°C and the mixture was stirred under a nitrogen stream at room temperature overnight. Water was added to the reaction mixture, and the mixture was extracted with ethyl acetate. The extract was washed with saturated brine, and dried over anhydrous sodium sulfate. The solvent was evaporated under reduced pressure. The residue was purified by silica gel column chromatography (ethyl acetate/hexane) to give a mixture (1:1, 1.08 g) of the title compound and 3-fluoro-5-methoxypicolinonitrile.
MS: [M+H]⁺ 153.1.

### B) 5-fluoro-3-methoxypicolinimidamide hydrochloride

By a method similar to that of Example 145, step A, the title compound was obtained as a mixture with 3-fluoro-5-methoxypicolinimidamide hydrochloride.
MS: [M+H]⁺ 170.1.

### C) 2-(5-fluoro-3-methoxypyridin-2-yl)-6-oxo-1,6-dihydropyrimidine-4-carboxylic acid

By a method similar to that of Example 87, step A, the title compound was obtained as a mixture with 2-(3-fluoro-5-methoxypyridin-2-yl)-6-oxo-1,6-dihydropyrimidine-4-carboxylic acid.
MS: [M+H]⁺ 266.1.

### D) 2-(5-fluoro-3-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 181

### N-(2-hydroxy-2-methyl-1-(4-(trifluoromethoxy)phenyl)propyl)-6-(pyridin-3-yloxy)pyrazine-2-carboxamide

### A) tert-butyl 6-(pyridin-3-yloxy)pyrazine-2-carboxylate

To a solution of tert-butyl 6-chloropyrazine-2-carboxylate (200 mg) in N,N-dimethylformamide (13.3 mL) were added 3-hydroxypyridine (89 mg) and cesium carbonate (607 mg) at room temperature. The reaction mixture was heated under microwave irradiation at 100°C for 10 min. The reaction mixture was cooled to room temperature, poured into water, and the mixture was extracted with ethyl acetate. The extract was washed with water and saturated brine, dried over anhydrous sodium sulfate, and the solvent was evaporated under reduced pressure. The residue was purified by silica gel column chromatography (NH, ethyl acetate/hexane) to give a pale-yellow oil (216 mg).
MS: [M+H]⁺ 274.1.

### B) N-(2-hydroxy-2-methyl-1-(4-(trifluoromethoxy)phenyl)propyl)-6-(pyridin-3-yloxy)pyrazine-2-carboxamide

By a method similar to that of Example 176, step B, the title compound was obtained.

### Example 182

### N-((1S)-1-(3-fluoro-4-(trifluoromethoxy)phenyl)-2-methoxyethyl)-2-(3-methyl-2-furyl)-6-oxo-1,6-dihydropyrimidine-4-carboxamide

### A) 3-methylfuran-2-carboximidamide hydrochloride

By a method similar to that of Example 98, step B, the title compound was obtained.
MS: [M+H]⁺ 125.1.

### B) 2-(3-methyl-2-furyl)-6-oxo-1,6-dihydropyrimidine-4-carboxylic acid

By a method similar to that of Example 123, step B, the title compound was obtained.
MS: [M+H]⁺ 221.1.

### C) N-((1S)-1-(3-fluoro-4-(trifluoromethoxy)phenyl)-2-methoxyethyl)-2-(3-methyl-2-furyl)-6-oxo-1,6-dihydropyrimidine-4-carboxamide

By a method similar to that of Example 1, step C, the title compound was obtained.

### Example 183

### 2-(3-ethoxypyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 152, step A, Example 145, step A, Example 87, step A and Example 1, step C, the title compound was obtained.

### Example 184

### 2-(3-chloro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

### A) methyl 3-chloro-5-methoxypicolinate

To a mixture of methyl 5-bromo-3-chloropicolinate (3.0 g) and N,N-dimethylformamide (5 ml) was added a solution (28%, 2.43 g) of sodium methoxide in methanol at room temperature, and the mixture was stirred under a nitrogen atmosphere at room temperature for 1 hr. To the reaction mixture was added saturated aqueous ammonium chloride solution, and the mixture was diluted with water, and extracted with ethyl acetate. The extract was washed with saturated brine, and dried over anhydrous magnesium sulfate. The solvent was evaporated under reduced pressure. The residue was purified by silica gel column chromatography (ethyl acetate/hexane) to give the title compound (947 mg).
MS: [M+H]⁺ 202.0.

### B) 3-chloro-5-methoxypicolinimidamide hydrochloride

By a method similar to that of Example 98, step B, the title compound was obtained.
MS: [M+H]⁺ 186.1.

### C) 2-(3-chloro-5-methoxypyridin-2-yl)-6-oxo-1,6-dihydropyrimidine-4-carboxylic acid

By a method similar to that of Example 123, step B, the title compound was obtained.
MS: [M+H]⁺ 281.8.

### D) 2-(3-chloro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 185

### 2-(5-cyclopropyl-3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-cyclopropyl-3-fluoropyridine-2-carboximidamide hydrochloride

By a method similar to that of Example 145, step A, the title compound was obtained.
MS: [M+H]⁺ 180.1.

### B) 2-(5-cyclopropyl-3-fluoropyridin-2-yl)-6-oxo-1,6-dihydropyrimidine-4-carboxylic acid

By a method similar to that of Example 123, step B, the title compound was obtained.
MS: [M+H]⁺ 276.1.

### C) 2-(5-cyclopropyl-3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 186

### 2-(5-(difluoromethoxy)-3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-(difluoromethoxy)-3-fluoropyridine-2-carbonitrile

Under a chlorodifluoromethane atmosphere, to a mixture of 3-fluoro-5-hydroxypyridine-2-carbonitrile (204 mg), benzyltriethylammonium chloride (102 mg) and tetrahydrofuran (9.8 mL) was added 8 M aqueous sodium hydroxide solution (0.183 mL) at 0°C. The reaction mixture was stirred at 0°C for 1.5 hr. The reaction mixture was further stirred at room temperature for 3 hr, cooled to 0°C again, and stirred at said temperature overnight. The reaction mixture was warmed to room temperature again, and stirred at room temperature for 5 hr. Water was added to the reaction mixture at 0°C, and the mixture was extracted with ethyl acetate. The extract was washed with water and saturated brine, and dried over anhydrous sodium sulfate. The solvent was evaporated under reduced pressure. The residue was purified by silica gel column chromatography (hexane/ethyl acetate) to give the title compound (63.9 mg).
MS: [M+H]⁺ 189.1.

### B) 5-(difluoromethoxy)-3-fluoropyridine-2-carboximidamide

By a method similar to that of Example 145, step A, the title compound was obtained.
MS: [M+H]⁺ 206.1.

### C) 2-(5-(difluoromethoxy)-3-fluoropyridin-2-yl)-6-oxo-1,6-dihydropyrimidine-4-carboxylic acid

By a method similar to that of Example 123, step B, the title compound was obtained.
MS: [M+H]⁺ 302.1.

### D) 2-(5-(difluoromethoxy)-3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

### Example 187

### 2-(3,5-diethoxypyridin-2-yl)-N-((lS)-1-(3-fluoro-4-(trifluoromethoxy)phenyl)-2-methoxyethyl)-6-oxo-1,6-dihydropyrimidine-4-carboxamide

By a method similar to that of Example 152, step A, Example 145, step A, Example 87, step C and Example 1, step C, the title compound was obtained.

### Example 188

### 5-fluoro-N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide

### A) 5-fluoro-6-hydroxy-2-(5-methoxypyridin-2-yl)pyrimidine-4(3H)-one

To a mixture of 5-methoxypyridine-2-carboximidamide hydrochloride (1.0 g) and methanol (15 mL) were added sodium methoxide (2.06 g) and dimethyl 2-fluoromalonate (0.80 g) at room temperature, and the mixture was heated under reflux at 80°C for 10 hr. Water was added to the reaction mixture at 0°C, and the mixture was acidified with 1 M hydrochloric acid. The precipitate was collected by filtration to give the title compound (0.73 g).
MS: [M+H]⁺ 237.8.

### B) 4,6-dichloro-5-fluoro-2-(5-methoxypyridin-2-yl)pyrimidine

A mixture of 5-fluoro-6-hydroxy-2-(5-methoxypyridin-2-yl)pyrimidine-4(3H)-one (0.64 g) and phosphorus oxychloride (3.07 mL) was stirred at 120°C for 3 hr. Water was added to the reaction mixture at 0°C, and the mixture was neutralized with 8 M aqueous sodium hydroxide solution. The precipitate was collected by filtration to give the title compound (0.40 g). MS: [M+H]⁺ 274.0.

### C) 4-chloro-5-fluoro-6-methoxy-2-(5-methoxypyridin-2-yl)pyrimidine

To a solution of 4,6-dichloro-5-fluoro-2-(5-methoxypyridin-2-yl)pyrimidine (0.4 g) in tetrahydrofuran (15 mL) was added sodium methoxide (0.31 g) at 0°C, and the mixture was stirred at 0°C for 40 min. The reaction mixture was neutralized with 1 M hydrochloric acid at 0°C, and extracted with ethyl acetate. The extract was washed with water and saturated brine, and dried over anhydrous sodium sulfate. The solvent was evaporated under reduced pressure to give the title compound (0.40 g).
MS: [M+H]⁺ 270.1.

### D) methyl 5-fluoro-6-methoxy-2-(5-methoxypyridin-2-yl)pyrimidine-4-carboxylate

By a method similar to that of Example 100, step B, the title compound was obtained.
MS: [M+H]⁺ 294.1.

### E) 5-fluoro-2-(5-methoxypyridin-2-yl)-6-oxo-1,6-dihydropyrimidine-4-carboxylic acid

A mixture of methyl 5-fluoro-6-methoxy-2-(5-methoxypyridin-2-yl)pyrimidine-4-carboxylate (100 mg) and 6 M hydrochloric acid (2.84 mL) was stirred at 100°C overnight. The reaction mixture was concentrated under reduced pressure to give the title compound (90 mg).
MS: [M+H]⁺ 266.1.

### F) 5-fluoro-N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide

By a method similar to that of Example 1, step C, the title compound was obtained.

### Example 189

### 2-(5-cyclopropyl-3-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

### A) 5-cyclopropyl-3-methoxypicolinonitrile

By a method similar to that of Example 135, step A, the title compound was obtained.
MS: [M+H]⁺ 175.1.

### B) 5-cyclopropyl-3-methoxypicolinimidamide hydrochloride

By a method similar to that of Example 145, step A, the title compound was obtained.
MS: [M+H]⁺ 192.1.

### C) 2-(5-cyclopropyl-3-methoxypyridin-2-yl)-6-oxo-1,6-dihydropyrimidine-4-carboxylic acid

To a mixture of diethyl oxaloacetate sodium salt (1.01 g), water (2.5 mL) and ethanol (0.5 mL) was added 8 M aqueous sodium hydroxide solution (0.603 mL) at room temperature, and the mixture was stirred at room temperature for 30 min. 5-Cyclopropyl-3-methoxypicolinimidamide hydrochloride (915 mg), 8 M aqueous sodium hydroxide solution (0.402 mL) and water (0.5 mL) were added to the reaction mixture, and the mixture was stirred at 60°C for 1 hr. To a mixture of diethyl oxaloacetate sodium salt (1.01 g), water (2.5 mL) and ethanol (0.5 mL) was added 8 M aqueous sodium hydroxide solution (0.603 mL) at room temperature, and the mixture was stirred at room temperature for 30 min. This mixture was added to the reaction mixture, and the mixture was stirred at 65°C overnight. The reaction mixture was acidified with 6 M hydrochloric acid under ice-cooling, and the solvent was evaporated under reduced pressure. The residue was washed with 2-propanol, suspended in hot methanol, and insoluble material was removed by filtration. The solvent in the filtrate was evaporated under reduced pressure to give a solid (689 mg). The obtained solid (510 mg) was purified by silica gel column chromatography (Diol, methanol/ethyl acetate) and recrystallized from methanol to give the title compound (26.8 mg).
MS: [M+H]⁺ 288.1.

### D) 2-(5-cyclopropyl-3-methoxypyridin-2-yl)-N-((1S)-1-(3-fluoro-4-(trifluoromethoxy)phenyl)-2-methoxyethyl)-6-oxo-1,6-dihydropyrimidine-4-carboxamide

By a method similar to that of Example 1, step C, the title compound was obtained.

Example compounds produced according to the above-mentioned methods, or a method analogous thereto are shown in the following Tables. In the Tables, MS shows measured values.

**[Table 1-1]**

| **Ex. No.** | **IUPAC name** | **Structure** | **salt** | **MS** |
|---|---|---|---|---|
| 1 | N-(2-methoxy-1-(4-(trifluoromethoxy)phenyl)ethyl)-2-phenyl-1,3-thiazole-4-carboxamide | 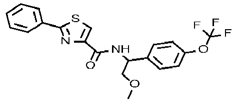 | | 423.1 |
| 2 | 2-anilino-N-(2-methoxy-1-(4-(trifluoromethoxy)phenyl)ethyl)-pyrimidine-4-carboxamide | 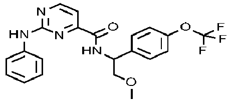 | | 433.0 |
| 3 | N-(2-methoxy-1-(4-(trifluoromethoxy)phenyl)ethyl)-2-((1-methyl-1H-pyrazol-4-yl)amino)pyrimidine-4-carboxamide | 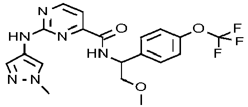 | | 437.1 |
| 4 | 2-anilino-6-methoxy-N-(2-methoxy-1-(4-(trifluoromethoxy)phenyl)ethyl)-pyrimidine-4-carboxamide | 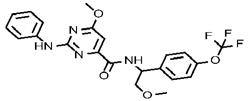 | | 463.1 |
| 5 | N-(2-methoxy-1-(4-(trifluoromethoxy)phenyl)ethyl)-pyrimidine-4-carboxamide | 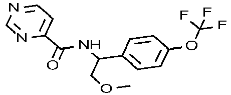 | | 339.9 |
| 6 | 2-anilino-N-(2-methoxy-1-(4-(trifluoromethoxy)phenyl)ethyl)-6-oxo-1,6-dihydropyrimidine-4-carboxamide | 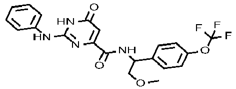 | | 447.0 |
| 7 | 2-anilino-N-(2-methoxy-1-(4-(trifluoromethoxy)phenyl)ethyl)-6-methylpyrimidine-4-carboxamide | 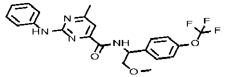 | | 447.1 |
| 8 | 5-amino-N-(2-methoxy-1-(4-(trifluoromethoxy)phenyl)ethyl)-pyrimidine-4-carboxamide | 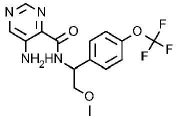 | | 355.1 |
| 9 | 2-(benzylamino)-N-(2-methoxy-1-(4-(trifluoromethoxy)-phenyl)ethyl)pyrimidine-4-carboxamide | 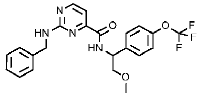 | | 447.1 |
| 10 | N-(2-methoxy-1-(4-(trifluoromethoxy)phenyl)ethyl)-5-(methylamino)pyrimidine-4-carboxamide | 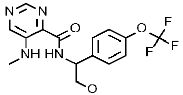 | | 371.1 |

**[Table 1-2]**

| | | | | |
|---|---|---|---|---|
| 11 | 2-anilino-6-chloro-N-(2-methoxy-1-(4-(trifluoromethoxy)-phenyl)ethyl)pyrimidine-4-carboxamide | 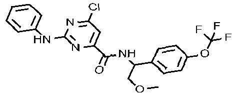 | | 467.1 |
| 12 | 2-anilino-6-cyano-N-(2-methoxy-1-(4-(trifluoromethoxy)-phenyl)ethyl)pyrimidine-4-carboxamide | 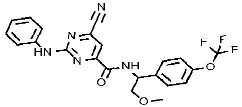 | | 456.0 |
| 13 | N-(2-methoxy-1-(4-(trifluoromethoxy)phenyl)ethyl)-2-(1H-pyrazol-5-yl)pyrimidine-4-carboxamide | 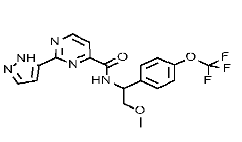 | | 408.1 |
| 14 | 2-anilino-5-fluoro-N-(2-methoxy-1-(4-(trifluoromethoxy)-phenyl)ethyl)pyrimidine-4-carboxamide | 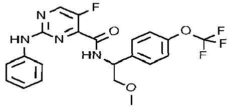 | | 451.1 |
| 15 | 2-(benzyl(methyl)amino)-N-(2-methoxy-1-(4-(trifluoro-methoxy)phenyl)ethyl)-6-oxo-1,6-dihydropyrimidine-4-carboxamide | 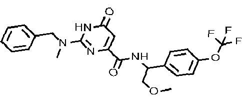 | | 477.2 |
| 16 | 2-(cyclopropylamino)-N-(2-methoxy-1-(4-(trifluoro-methoxy)phenyl)ethyl)-6-oxo-1,6-dihydropyrimidine-4-carboxamide | 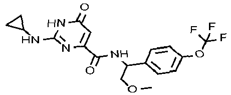 | | 413.1 |
| 17 | 2-anilino-N-(2-methoxy-1-(4-(trifluoromethoxy)phenyl)ethyl)-5-(methylamino)pyrimidine-4-carboxamide | 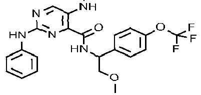 | | 462.1 |
| 18 | N-(2-methoxy-1-(4-(trifluoromethoxy)phenyl)ethyl)-2-(methylamino)-6-oxo-1,6-dihydropyrimidine-4-carboxamide | 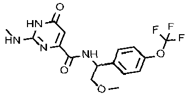 | | 387.1 |
| 19 | 2-(dimethylamino)-N-(2-methoxy-1-(4-(trifluoromethoxy)-phenyl)ethyl)-6-oxo-1,6-dihydropyrimidine-4-carboxamide | 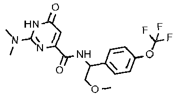 | | 401.1 |
| 20 | 2-(dimethylamino)-N-((1S)-2-methoxy-1-(4-(trifluoro-methoxy)phenyl)ethyl)-6-oxo-1,6-dihydropyrimidine-4-carboxamide | 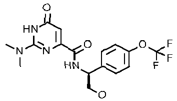 | | 401.1 |

**[Table 1-3]**

| | | | | |
|---|---|---|---|---|
| 21 | 2-(dimethylamino)-N-((1R)-2-methoxy-1-(4-(trifluoro-methoxy)phenyl)ethyl)-6-oxo-1,6-dihydropyrimidine-4-carboxamide | 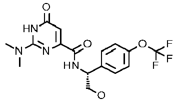 | | 401.1 |
| 22 | 2-((3,4-dimethoxyphenyl)amino)-6-hydroxy-N-(2-methoxy-1-(4-(trifluoromethoxy)phenyl)-ethyl)pyrimidine-4-carboxamide | 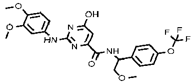 | | 509.2 |
| 23 | 2-((cyclopropylcarbonyl)amino)-N-(2-methoxy-1-(4-(trifluoromethoxy)phenyl)-ethyl)pyrimidine-4-carboxamide | 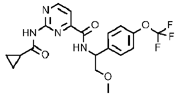 | | 425.1 |
| 24 | N-(2-methoxy-1-(4-(trifluoromethoxy)phenyl)ethyl)-6-oxo-1,6-dihydropyrimidine-4-carboxamide | 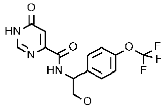 | | 356.0 |
| 25 | N-(2-methoxy-1-(4-(trifluoromethoxy)phenyl)ethyl)-2-methyl-6-oxo-1,6-dihydropyrimidine-4-carboxamide | 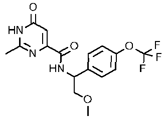 | | 372.1 |
| 26 | 2-benzyl-N-(2-methoxy-1-(4-(trifluoromethoxy)phenyl)ethyl)-6-oxo-1,6-dihydropyrimidine-4-carboxamide | 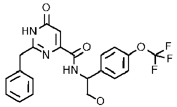 | | 448.1 |
| 27 | N-(2-methoxy-1-(4-(trifluoro-methoxy)phenyl)ethyl)-6-oxo-2-(pyrrolidin-1-yl)-1,6-dihydropyrimidine-4-carboxamide | 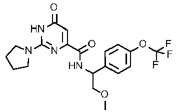 | | 427.2 |
| 28 | 2-(3-fluoroazetidin-1-yl)-N-((1S)-2-methoxy-1-(4-(trifluoromethoxy)phenyl)ethyl)-6-oxo-1,6-dihydropyrimidine-4-carboxamide | 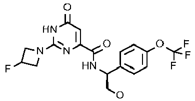 | | 431.0 |
| 29 | N-(2-methoxy-1-(4-(trifluoromethoxy)phenyl)ethyl)-2-(1-methyl-1H-pyrazol-3-yl)-6-oxo-1,6-dihydropyrimidine-4-carboxamide | 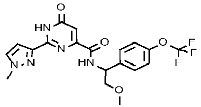 | | 438.1 |
| 30 | 2-((2-methoxyethyl)(methyl)amino)-N-(2-methoxy-1-(4-(trifluoromethoxy)phenyl)ethyl)-6-oxo-1,6-dihydropyrimidine-4-carboxamide | 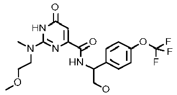 | | 445.1 |

**[Table 1-4]**

| | | | | |
|---|---|---|---|---|
| 31 | 2-(dimethylamino)-N-(1-(3-fluoro-4-(trifluoromethoxy)phenyl)-2-methoxyethyl)-6-oxo-1,6-dihydropyrimidine-4-carboxamide | 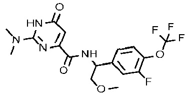 | | 419.1 |
| 32 | 2-(dimethylamino)-N-((1R)-1-(3-fluoro-4-(trifluoromethoxy)phenyl)-2-methoxyethyl)-6-oxo-1,6-dihydropyrimidine-4-carboxamide | 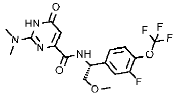 | | 419.0 |
| 33 | 2-(dimethylamino)-N-((1S)-1-(3-fluoro-4-(trifluoromethoxy)phenyl)-2-methoxyethyl)-6-oxo-1,6-dihydropyrimidine-4-carboxamide | 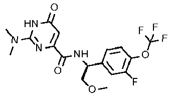 | | 419.0 |
| 34 | 2-methoxy-N-(2-methoxy-1-(4-(trifluoromethoxy)phenyl)ethyl)-6-oxo-1,6-dihydropyrimidine-4-carboxamide | 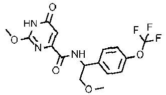 | | 388.0 |
| 35 | N-(2-methoxy-1-(4-(trifluoromethoxy)phenyl)ethyl)-2-(methylsulfanyl)-6-oxo-1,6-dihydropyrimidine-4-carboxamide | 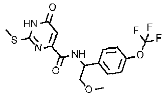 | | 404.1 |
| 36 | 2-(cyclopropyl(methyl)amino)-N-((1S)-2-methoxy-1-(4-(trifluoromethoxy)phenyl)ethyl)-6-oxo-1,6-dihydropyrimidine-4-carboxamide | 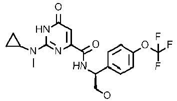 | | 427.1 |
| 37 | 2-(diethylamino)-N-((1S)-2-methoxy-1-(4-(trifluoromethoxy)phenyl)ethyl)-6-oxo-1,6-dihydropyrimidine-4-carboxamide | 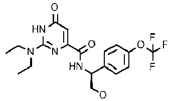 | | 429.1 |
| 38 | 2-(ethyl(methyl)amino)-N-((1S)-2-methoxy-1-(4-(trifluoromethoxy)phenyl)ethyl)-6-oxo-1,6-dihydropyrimidine-4-carboxamide | 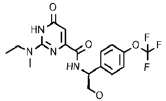 | | 415.1 |
| 39 | N-((1S)-2-methoxy-1-(4-(trifluoromethoxy)phenyl)ethyl)-2-(3-methylpyrrolidin-1-yl)-6-oxo-1,6-dihydropyrimidine-4-carboxamide | 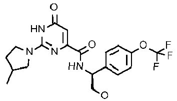 | | 441.1 |
| 40 | 2-((3R)-3-methoxypyrrolidin-1-yl)-N-((1S)-2-methoxy-1-(4-(trifluoromethoxy)phenyl)ethyl)-6-oxo-1,6-dihydropyrimidine-4-carboxamide | 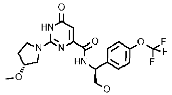 | | 457.1 |

**[Table 1-5]**

| | | | | |
|---|---|---|---|---|
| 41 | 6-chloro-N-(2-methoxy-1-(4-(trifluoromethoxy)phenyl)ethyl)-pyrazine-2-carboxamide | 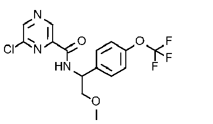 | | 373.9 |
| 42 | N-(2-methoxy-1-(4-(trifluoromethoxy)phenyl)ethyl)-6-(pyrrolidin-1-yl)pyrazine-2-carboxamide | 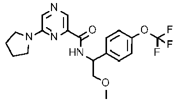 | | 411.1 |
| 43 | N-((1S)-2-methoxy-1-(4-(trifluoromethoxy)phenyl)ethyl)-2-(2-methylpyrrolidin-1-yl)-6-oxo-1,6-dihydropyrimidine-4-carboxamide | 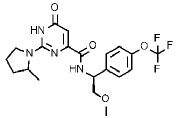 | | 441.1 |
| 44 | 2-(3-azabicyclo[3.1.0]hex-3-yl)-N-((1S)-2-methoxy-1-(4-(trifluoromethoxy)phenyl)ethyl)-6-oxo-1,6-dihydropyrimidine-4-carboxamide | 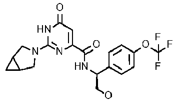 | | 439.1 |
| 45 | 2-((3S)-3-methoxypyrrolidin-1-yl)-N-((1S)-2-methoxy-1-(4-(trifluoromethoxy)phenyl)ethyl)-6-oxo-1,6-dihydropyrimidine-4-carboxamide | 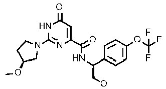 | | 457.1 |
| 46 | 2-(3,3-difluoropyrrolidin-1-yl)-N-((1S)-2-methoxy-1-(4-(trifluoromethoxy)phenyl)ethyl)-6-oxo-1,6-dihydropyrimidine-4-carboxamide | 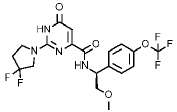 | | 463.1 |
| 47 | N-(2-methoxy-1-(4-(trifluoromethoxy)phenyl)ethyl)-6-oxo-2-(piperidin-1-yl)-1,6-dihydropyrimidine-4-carboxamide | 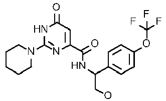 | | 441.1 |
| 48 | N-(2-methoxy-1-(4-(trifluoromethoxy)phenyl)ethyl)-2-(morpholin-4-yl)-6-oxo-1,6-dihydropyrimidine-4-carboxamide | 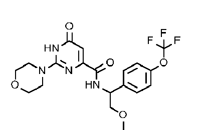 | | 443.1 |
| 49 | 2-(azepan-1-yl)-N-(2-methoxy-1-(4-(trifluoromethoxy)phenyl)-ethyl)-6-oxo-1,6-dihydropyrimidine-4-carboxamide | 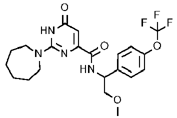 | | 455.1 |
| 50 | 2-((2R)-2-(methoxymethyl)pyrrolidin-1-yl)-N-((1S)-2-methoxy-1-(4-(trifluoromethoxy)phenyl)ethyl)-6-oxo-1,6-dihydropyrimidine-4-carboxamide | 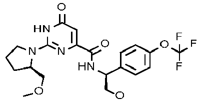 | | 471.2 |

**[Table 1-6]**

| | | | | |
|---|---|---|---|---|
| 51 | N-(2-methoxy-1-(4-(trifluoromethoxy)phenyl)ethyl)-6-oxo-2-(pyridin-2-yl)-1,6-dihydropyrimidine-4-carboxamide | 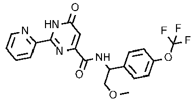 | | 435.0 |
| 52 | N-((1S)-2-methoxy-1-(4-(trifluoromethoxy)phenyl)ethyl)-6-oxo-2-(pyrrolidin-1-yl)-1,6-dihydropyrimidine-4-carboxamide | 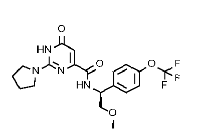 | | 427.1 |
| 53 | N-(2-methoxy-1-(4-(trifluoromethoxy)phenyl)ethyl)-6-oxo-2-(1H-pyrazol-1-yl)-1,6-dihydropyrimidine-4-carboxamide | 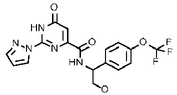 | | 424.0 |
| 54 | 2-(azetidin-1-yl)-N-(2-methoxy-1-(4-(trifluoromethoxy)phenyl)ethyl)-6-oxo-1,6-dihydropyrimidine-4-carboxamide | 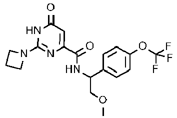 | | 413.1 |
| 55 | 2-(3-methoxyazetidin-1-yl)-N-((1S)-2-methoxy-1-(4-(trifluoromethoxy)phenyl)ethyl)-6-oxo-1,6-dihydropyrimidine-4-carboxamide | 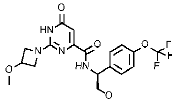 | | 443.1 |
| 56 | 2-((2S)-2-(methoxymethyl)pyrrolidin-1-yl)-N-((1S)-2-methoxy-l-(4-(trifluoromethoxy)phenyl)ethyl)-6-oxo-1,6-dihydropyrimidine-4-carboxamide | 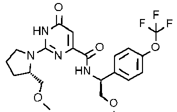 | | 471.1 |
| 57 | N-(2-methoxy-1-(4-(trifluoromethoxy)phenyl)ethyl)-2-(1-methyl-1H-pyrazol-4-yl)-6-oxo-1,6-dihydropyrimidine-4-carboxamide | 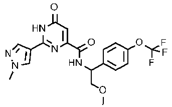 | | 438.1 |
| 58 | 2-cyclopentyl-N-((1S)-2-methoxy-1-(4-(trifluoromethoxy)-phenyl)ethyl)-6-oxo-1,6-dihydropyrimidine-4-carboxamide | 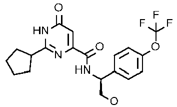 | | 426.1 |
| 59 | N-(1-(3-fluoro-4-(trifluoromethoxy)phenyl)-2-methoxyethyl)-6-oxo-2-(pyrrolidin-1-yl)-1,6-dihydropyrimidine-4-carboxamide | 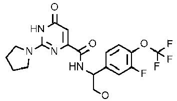 | | 445.1 |
| 60 | N-((1R)-1-(3-fluoro-4-(trifluoromethoxy)phenyl)-2-methoxyethyl)-6-oxo-2-(pyrrolidin-1-yl)-1,6-dihydropyrimidine-4-carboxamide | 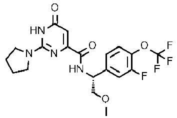 | | 445.1 |

**[Table 1-7]**

| | | | | |
|---|---|---|---|---|
| 61 | N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide | 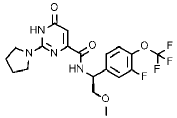 | | 445.1 |
| 62 | N-(1-(3-fluoro-4-(trifluoromethoxy)phenyl)-2-hydroxy-2-methylpropyl)-6-oxo-2-(pyrrolidin-1-yl)-1,6-dihydropyrimidine-4-carboxamide | 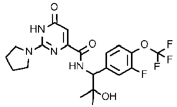 | | 459.1 |
| 63 | 2-(dimethylamino)-N-(2-methoxy-1-(4-(trifluoromethoxy)-phenyl)ethyl)-5-methyl-6-oxo-1,6-dihydropyrimidine-4-carboxamide | 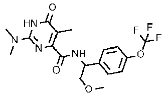 | | 415.1 |
| 64 | N-(1-(3-fluoro-4-(trifluoromethyl)phenyl)-2-methoxyethyl)-6-oxo-2-(pyrrolidin-1-yl)-1,6-dihydropyrimidine-4-carboxamide | 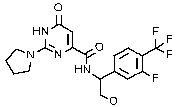 | | 429.1 |
| 65 | N-(3-methoxy-1-(4-(trifluoromethoxy)phenyl)propyl)-6-oxo-2-(pyrrolidin-1-yl)-1,6-dihydropyrimidine-4-carboxamide | 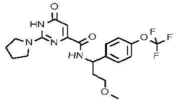 | | 441.1 |
| 66 | 2-(6,6-difluoro-3-azabicyclo[3.1.0]hex-3-yl)-N-((1S)-2-methoxy-1-(4-(trifluoromethoxy)phenyl)ethyl)-6-oxo-1,6-dihydropyrimidine-4-carboxamide | 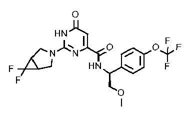 | | 475.1 |
| 67 | 2-((3S)-3-isopropoxypyrrolidin-1-yl)-N-((1S)-2-methoxy-1-(4-(trifluoromethoxy)phenyl)ethyl)-6-oxo-1,6-dihydropyrimidine-4-carboxamide | 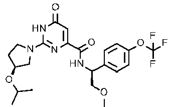 | | 485.1 |
| 68 | N-((1S)-2-methoxy-1-(4-(trifluoromethoxy)phenyl)ethyl)-2-(3-oxa-8-azabicyclo[3.2.1]oct-8-yl)-6-oxo-1,6-dihydropyrimidine-4-carboxamide | 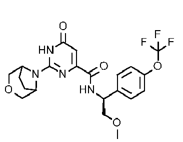 | | 469.1 |
| 69 | 2-((3R)-3-fluoropyrrolidin-1-yl)-N-((1S)-2-methoxy-1-(4-(trifluoromethoxy)phenyl)ethyl)-6-oxo-1,6-dihydropyrimidine-4-carboxamide | 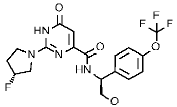 | | 445.1 |
| 70 | N-(1-(4-cyclopropylphenyl)-2-methoxyethyl)-6-oxo-2-(pyrrolidin-1-yl)-1,6-dihydropyrimidine-4-carboxamide | 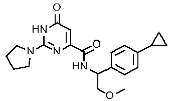 | | 383.1 |

**[Table 1-8]**

| | | | | |
|---|---|---|---|---|
| 71 | 2-cyclopropyl-N-(2-methoxy-1-(4-(trifluoromethoxy)phenyl)ethyl)-6-oxo-1,6-dihydropyrimidine-4-carboxamide | 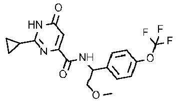 | | 398.1 |
| 72 | N-((1S)-2-methoxy-1-(4-(trifluoromethoxy)phenyl)ethyl)-6-oxo-2-(3-(trifluoromethyl)pyrrolidin-1-yl)-1,6-dihydropyrimidine-4-carboxamide | 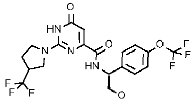 | | 494.9 |
| 73 | N-((1S)-1-(3-fluoro-4-(trifluoromethoxy)phenyl)-2-methoxyethyl)-2-(6-oxa-3-azabicyclo[3.1.1]hept-3-yl)-6-oxo-1,6-dihydropyrimidine-4-carboxamide | 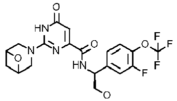 | | 473.1 |
| 74 | N-((1S)-1-(3-fluoro-4-(trifluoromethoxy)phenyl)-2-methoxyethyl)-2-((3S)-3-methoxypyrrolidin-1-yl)pyrimidine-4-carboxamide | 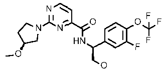 | | 459.1 |
| 75 | 2-(dimethylamino)-N-(2-methoxy-1-(4-(1H-pyrazol-1-yl)phenyl)ethyl)-6-oxo-1,6-dihydropyrimidine-4-carboxamide | 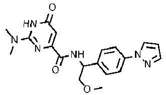 | | 383.0 |
| 76 | 2-(ethyl(methyl)amino)-N-((1S)-1-(3-fluoro-4-(trifluoromethoxy)phenyl)-2-methoxyethyl)-6-oxo-1,6-dihydropyrimidine-4-carboxamide | 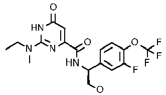 | | 433.0 |
| 77 | 5-chloro-N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide | 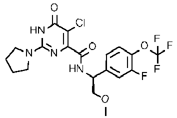 | | 479.0 |
| 78 | N- ((1S)-1-(3-fluoro-4-(trifluoromethoxy)phenyl)-2-methoxyethyl)-2-(1,2-oxazolidin-2-yl)-6-oxo-1,6-dihydropyrimidine-4-carboxamide | 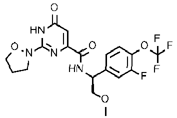 | | 447.0 |
| 79 | N-((1S)-1-(3-fluoro-4-(trifluoromethoxy)phenyl)-2-methoxyethyl)-2-isopropyl-6-oxo-1,6-dihydropyrimidine-4-carboxamide | 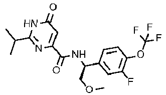 | | 418.1 |
| 80 | 2-tert-butyl-N-((1S)-1-(3-fluoro-4-(trifluoromethoxy)phenyl)-2-methoxyethyl)-6-oxo-1,6-dihydropyrimidine-4-carboxamide | 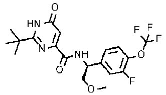 | | 432.1 |

**[Table 1-9]**

| | | | | |
|---|---|---|---|---|
| 81 | 2-cyclopropyl-N-((1S)-1-(3-fluoro-4-(trifluoromethoxy)phenyl)-2-methoxyethyl)-6-oxo-1,6-dihydropyrimidine-4-carboxamide | 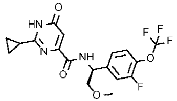 | | 416.0 |
| 82 | N-((1S)-1-(3-fluoro-4-(trifluoromethoxy)phenyl)-2-methoxyethyl)-2-(isobutyl(methyl)amino)-6-oxo-1,6-dihydropyrimidine-4-carboxamide | 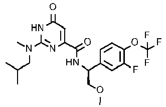 | | 461.1 |
| 83 | 2-(cyclopent-1-en-1-yl)-N-((1S)-1-(3-fluoro-4-(trifluoro-methoxy)phenyl)-2-methoxyethyl)-6-oxo-1,6-dihydropyrimidine-4-carboxamide | 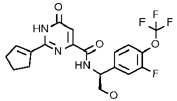 | | 442.1 |
| 84 | 2-(cyclohex-1-en-1-yl)-N-((1S)-1-(3-fluoro-4-(trifluoromethoxy)phenyl)-2-methoxyethyl)-6-oxo-1,6-dihydropyrimidine-4-carboxamide | 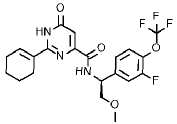 | | 456.1 |
| 85 | 2-((cyclopropylmethyl)-(methyl)amino)-N-((1S)-1-(3-fluoro-4-(trifluoromethoxy)-phenyl)-2-methoxyethyl)-6-oxo-1,6-dihydropyrimidine-4-carboxamide | 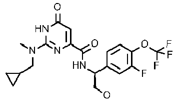 | | 459.1 |
| 86 | N-((1S)-1-(3-fluoro-4-(trifluoromethoxy)phenyl)-2-methoxyethyl)-6-oxo-2-(tetrahydro-2H-pyran-4-yl)-1,6-dihydropyrimidine-4-carboxamide | 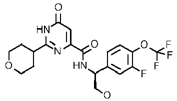 | | 460.2 |
| 87 | N-((1S)-1-(3-fluoro-4-(trifluoromethoxy)phenyl)-2-methoxyethyl)-6-oxo-2-phenyl-1,6-dihydropyrimidine-4-carboxamide | 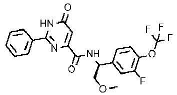 | | 452.0 |
| 88 | N-((1S)-1-(3-fluoro-4-(trifluoromethoxy)phenyl)-2-methoxyethyl)-6-oxo-2-(pyridin-2-yl)-1,6-dihydropyrimidine-4-carboxamide | 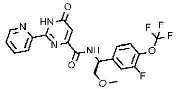 | | 450.9 |
| 89 | N-((1S)-1-(3-fluoro-4-(trifluoromethoxy)phenyl)-2-methoxyethyl)-6-oxo-2-(pyridin-4-yl)-1,6-dihydropyrimidine-4-carboxamide | 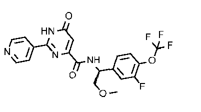 | | 450.9 |
| 90 | N-((1S)-1-(3-fluoro-4-(trifluoromethoxy)phenyl)-2-methoxyethyl)-6-oxo-2-(pyridin-3-yl)-1,6-dihydropyrimidine-4-carboxamide | 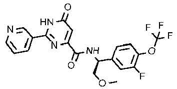 | | 450.9 |

**[Table 1-10]**

| | | | | |
|---|---|---|---|---|
| 91 | 2-cyclohexyl-N-((1S)-1-(3-fluoro-4-(trifluoromethoxy)phenyl)-2-methoxyethyl)-6-oxo-1,6-dihydropyrimidine-4-carboxamide | 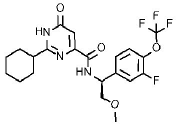 | | 458.1 |
| 92 | N-((1S)-1-(3-fluoro-4-(trifluoromethoxy)phenyl)-2-methoxyethyl)-2-(4-methyl-1H-imidazol-1-yl)-6-oxo-1,6-dihydropyrimidine-4-carboxamide | 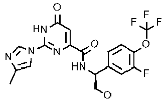 | | 456.1 |
| 93 | N-((1S)-1-(3-fluoro-4-(trifluoromethoxy)phenyl)-2-methoxyethyl)-6-oxo-2-(tetrahydrofuran-2-yl)-1,6-dihydropyrimidine-4-carboxamide | 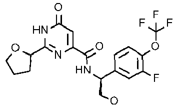 | | 446.0 |
| 94 | N-((1S)-1-(3-fluoro-4-(trifluoromethoxy)phenyl)-2-methoxyethyl)-2-(isopropyl(methyl)amino)-6-oxo-1,6-dihydropyrimidine-4-carboxamide | 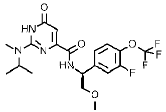 | | 447.0 |
| 95 | 2-tert-butyl-N-((1S)-1-(3-fluoro-4-(trifluoro-methoxy)phenyl)-2-hydroxy-2-methylpropyl)-6-oxo-1,6-dihydropyrimidine-4-carboxamide | 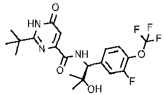 | | 446.0 |
| 96 | 2-cyclopropyl-N-((1S)-1-(3-fluoro-4-(trifluoro-methoxy)phenyl)-2-hydroxy-2-methylpropyl)-6-oxo-1,6-dihydropyrimidine-4-carboxamide | 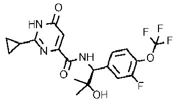 | | 430.0 |
| 97 | N-((1S)-1-(3-fluoro-4-(trifluoromethoxy)phenyl)-2-methoxyethyl)-6-oxo-2-(prop-1-en-2-yl)-1,6-dihydropyrimidine-4-carboxamide | 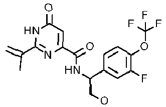 | | 416.0 |
| 98 | N-((1S)-1-(3-fluoro-4-(trifluoromethoxy)phenyl)-2-methoxyethyl)-2-(1-methoxy-2-methylpropan-2-yl)-6-oxo-1,6-dihydropyrimidine-4-carboxamide | 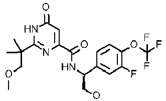 | | 462.1 |
| 99 | N-((1S)-1-(3-fluoro-4-(trifluoromethoxy)phenyl)-2-methoxyethyl)-2-(2-methoxy-1,3-thiazol-5-yl)-6-oxo-1,6-dihydropyrimidine-4-carboxamide | 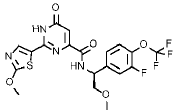 | | 486.9 |
| 100 | N-(1-(3-fluoro-4-(trifluoromethoxy)phenyl)-2-methoxyethyl)-6-oxo-4-(pyrrolidin-1-yl)-1,6-dihydropyrimidine-2-carboxamide | 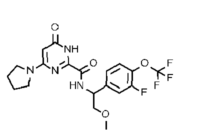 | | 445.1 |

**[Table 1-11]**

| | | | | |
|---|---|---|---|---|
| 101 | 2-cyclobutyl-N-((1S)-1-(3-fluoro-4-(trifluoromethoxy)phenyl)-2-methoxyethyl)-6-oxo-1,6-dihydropyrimidine-4-carboxamide | 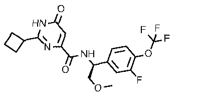 | | 430.1 |
| 102 | N-((1S)-1-(3-fluoro-4-(trifluoromethoxy)phenyl)-2-hydroxy-2-methylpropyl)-6-oxo-2-(pyridin-2-yl)-1,6-dihydropyrimidine-4-carboxamide | 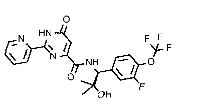 | | 467.1 |
| 103 | N-((1S)-1-(3-fluoro-4-(trifluoromethoxy)phenyl)-2-methoxyethyl)-2-(1-methylcyclopropyl)-6-oxo-1,6-dihydropyrimidine-4-carboxamide | 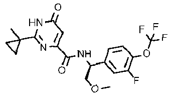 | | 430.1 |
| 104 | N-((1S)-1-(3-fluoro-4-(trifluoromethoxy)phenyl)-2-methoxyethyl)-6-oxo-2-(1,3-thiazol-4-yl)-1,6-dihydropyrimidine-4-carboxamide | 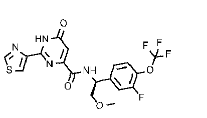 | | 459.1 |
| 105 | N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide | 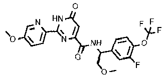 | | 483.0 |
| 106 | N-((1S)-1-(3-fluoro-4-(trifluoromethoxy)phenyl)-2-methoxyethyl)-6-oxo-2-(1,3-thiazol-2-yl)-1,6-dihydropyrimidine-4-carboxamide | 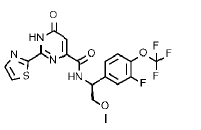 | | 459.1 |
| 107 | N-((1S)-1-(3-fluoro-4-(trifluoromethoxy)phenyl)-2-methoxyethyl)-6-oxo-2-(2-thienyl)-1,6-dihydropyrimidine-4-carboxamide | 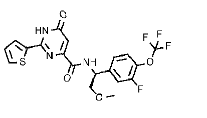 | | 455.9 |
| 108 | N-((1S)-1-(3-fluoro-4-(trifluoromethoxy)phenyl)-2-methoxyethyl)-2-(4-methoxyphenyl)-6-oxo-1,6-dihydropyrimidine-4-carboxamide | 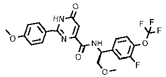 | | 482.1 |
| 109 | N-((1S)-1-(3-fluoro-4-(trifluoromethoxy)phenyl)-2-methoxyethyl)-2-(3-methoxyphenyl)-6-oxo-1,6-dihydropyrimidine-4-carboxamide | 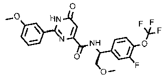 | | 480.0 |
| 110 | N-((1S)-1-(3-fluoro-4-(trifluoromethoxy)phenyl)-2-methoxyethyl)-2-(4-methylphenyl)-6-oxo-1,6-dihydropyrimidine-4-carboxamide | 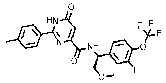 | | 466.1 |

**[Table 1-12]**

| | | | | |
|---|---|---|---|---|
| 111 | N-((1S)-1-(3-fluoro-4-(trifluoromethoxy)phenyl)-2-methoxyethyl)-6-oxo-2-(prop-1-yn-1-yl)-1,6-dihydropyrimidine-4-carboxamide | 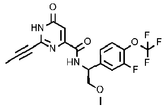 | | 414.1 |
| 112 | N-((1S)-1-(3-fluoro-4-(trifluoromethoxy)phenyl)-2-methoxyethyl)-2-(5-methyl-1,3-thiazol-2-yl)-6-oxo-1,6-dihydropyrimidine-4-carboxamide | 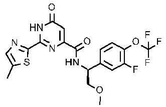 | | 473.1 |
| 113 | N-((1S)-1-(3-fluoro-4-(trifluoromethoxy)phenyl)-2-methoxyethyl)-2-(2-methoxypropan-2-yl)-6-oxo-1,6-dihydropyrimidine-4-carboxamide | 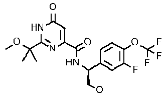 | | 448.1 |
| 114 | 2-(cyclopent-1-en-1-yl)-N-((1S)-1-(3-fluoro-4-(trifluoromethoxy)phenyl)-2-hydroxy-2-methylpropyl)-6-oxo-1,6-dihydropyrimidine-4-carboxamide | 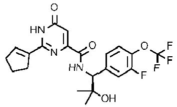 | | 456.1 |
| 115 | 2-(cyclopent-1-en-1-yl)-N-((1S)-2-methoxy-1-(4-(trifluoromethoxy)phenyl)ethyl)-6-oxo-1,6-dihydropyrimidine-4-carboxamide | 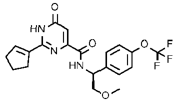 | | 424.1 |
| 116 | N-(1-(3-fluoro-4-(1H-pyrrol-1-yl)phenyl)-2-methoxyethyl)-6-oxo-2-(pyridin-2-yl)-1,6-dihydropyrimidine-4-carboxamide | 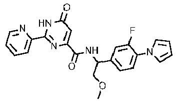 | | 434.1 |
| 117 | N-((1S)-1-(3-fluoro-4-(trifluoromethoxy)phenyl)-2-methoxyethyl)-2-(4-methoxypyridin-2-yl)-6-oxo-1,6-dihydropyrimidine-4-carboxamide | 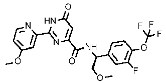 | | 483.1 |
| 118 | N-((1S)-1-(3-fluoro-4-(trifluoromethoxy)phenyl)-2-methoxyethyl)-6-oxo-2-(5-(trifluoromethyl)pyridin-2-yl)-1,6-dihydropyrimidine-4-carboxamide | 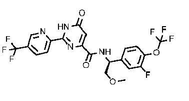 | | 521.0 |
| 119 | N-((1S)-1-(3-fluoro-4-(trifluoromethoxy)phenyl)-2-methoxyethyl)-2-(3-methylphenyl)-6-oxo-1,6-dihydropyrimidine-4-carboxamide | 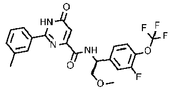 | | 466.1 |
| 120 | N-((1S)-1-(3-fluoro-4-(trifluoromethoxy)phenyl)-2-methoxyethyl)-6-oxo-2-(3-thienyl)-1,6-dihydropyrimidine-4-carboxamide | 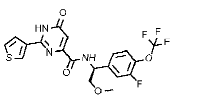 | | 458.0 |

**[Table 1-13]**

| | | | | |
|---|---|---|---|---|
| 121 | N-((1S)-1-(3-fluoro-4-(trifluoromethoxy)phenyl)-2-methoxyethyl)-2-(2-furyl)-6-oxo-1,6-dihydropyrimidine-4-carboxamide | 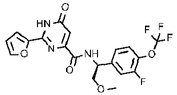 | | 442.0 |
| 122 | N-((1S)-1-(3-fluoro-4-(trifluoromethoxy)phenyl)-2-methoxyethyl)-2-(3-furyl)-6-oxo-1,6-dihydropyrimidine-4-carboxamide | 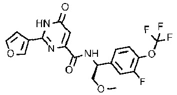 | | 442.0 |
| 123 | N-((1S)-1-(3-fluoro-4-(trifluoromethoxy)phenyl)-2-methoxyethyl)-2-(6-methylpyridin-2-yl)-6-oxo-1,6-dihydropyrimidine-4-carboxamide | 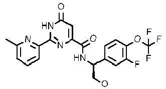 | | 467.0 |
| 124 | N-((1S)-1-(3-fluoro-4-(trifluoromethoxy)phenyl)-2-methoxyethyl)-2-(6-methoxypyridin-2-yl)-6-oxo-1,6-dihydropyrimidine-4-carboxamide | 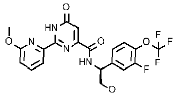 | | 483.1 |
| 125 | N-((1S)-1-(3-fluoro-4-(trifluoromethoxy)phenyl)-2-methoxyethyl)-2-(1-methoxycyclopropyl)-6-oxo-1,6-dihydropyrimidine-4-carboxamide | 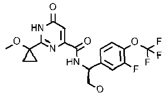 | | 446.0 |
| 126 | N-((1S)-1-(3-fluoro-4-(trifluoromethoxy)phenyl)-2-methoxyethyl)-2-(3-methoxy-4-methylphenyl)-6-oxo-1,6-dihydropyrimidine-4-carboxamide | 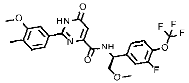 | | 496.0 |
| 127 | N-((1S)-1-(3-fluoro-4-(trifluoromethoxy)phenyl)-2-methoxyethyl)-2-(3-methoxycyclopent-1-en-1-yl)-6-oxo-1,6-dihydropyrimidine-4-carboxamide | 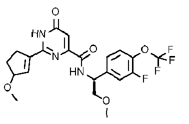 | | 472.1 |
| 128 | 2-(cyclopent-1-en-1-yl)-N-(2-methoxy-1-(4-(1H-pyrazol-1-yl)phenyl)ethyl)-6-oxo-1,6-dihydropyrimidine-4-carboxamide | 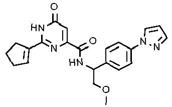 | | 406.2 |
| 129 | 2-(cyclopent-1-en-1-yl)-N-(1-(3-fluoro-4-(1H-pyrazol-1-yl)phenyl)-2-methoxyethyl)-6-oxo-1,6-dihydropyrimidine-4-carboxamide | 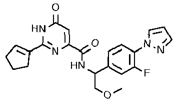 | | 424.1 |
| 130 | N-((1S)-1-(3-fluoro-4-(trifluoromethoxy)phenyl)-2-methoxyethyl)-2-(3-methylpyridin-2-yl)-6-oxo-1,6-dihydropyrimidine-4-carboxamide | 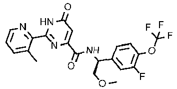 | | 467.1 |

**[Table 1-14]**

| | | | | |
|---|---|---|---|---|
| 131 | 2-(3,4-dimethoxyphenyl)-N-((1S)-1-(3-fluoro-4-(trifluoromethoxy)phenyl)-2-methoxyethyl)-6-oxo-1,6-dihydropyrimidine-4-carboxamide | 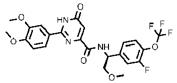 | | 512.0 |
| 132 | N-((1S)-1-(3-fluoro-4-(trifluoromethoxy)phenyl)-2-methoxyethyl)-2-(4-methylpyridin-2-yl)-6-oxo-1,6-dihydropyrimidine-4-carboxamide | 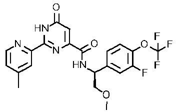 | | 467.1 |
| 133 | N-((1S)-1-(3-fluoro-4-(trifluoromethoxy)phenyl)-2-methoxyethyl)-2-(5-methylpyridin-2-yl)-6-oxo-1,6-dihydropyrimidine-4-carboxamide | 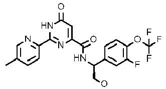 | | 467.1 |
| 134 | N-((1S)-1-(3-fluoro-4-(trifluoromethoxy)phenyl)-2-methoxyethyl)-2-(2-methylcyclopropyl)-6-oxo-1,6-dihydropyrimidine-4-carboxamide | 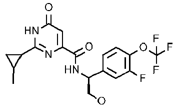 | | 430.1 |
| 135 | 2-(4-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide | 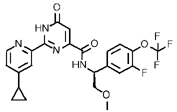 | | 493.1 |
| 136 | 2-(5-cyclopropylpyridin-2-yl)-N-((1S)-1-(3-fluoro-4-(trifluoromethoxy)phenyl)-2-methoxyethyl)-6-oxo-1,6-dihydropyrimidine-4-carboxamide | 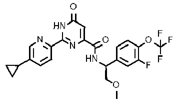 | | 493.2 |
| 137 | 2-(5-fluoropyridin-2-yl)-N-((1S)-1-(3-fluoro-4-(trifluoromethoxy)phenyl)-2-methoxyethyl)-6-oxo-1,6-dihydropyrimidine-4-carboxamide | 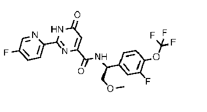 | | 469.0 |
| 138 | 2-(5-ethoxypyridin-2-yl)-N-((1S)-1-(3-fluoro-4-(trifluoromethoxy)phenyl)-2-methoxyethyl)-6-oxo-1,6-dihydropyrimidine-4-carboxamide | 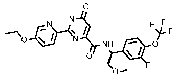 | | 497.1 |
| 139 | N-((1S)-1-(3-fluoro-4-(trifluoromethoxy)phenyl)-2-methoxyethyl)-2-(5-isopropoxypyridin-2-yl)-6-oxo-1,6-dihydropyrimidine-4-carboxamide | 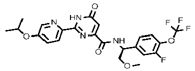 | | 511.1 |
| 140 | 2-(2-fluorophenyl)-N-((1S)-1-(3-fluoro-4-(trifluoromethoxy)phenyl)-2-methoxyethyl)-6-oxo-1,6-dihydropyrimidine-4-carboxamide | 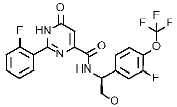 | | 470.0 |

**[Table 1-15]**

| | | | | |
|---|---|---|---|---|
| 141 | N-((1S)-1-(3-fluoro-4-(trifluoromethoxy)phenyl)-2-methoxyethyl)-2-(2-methoxyphenyl)-6-oxo-1,6-dihydropyrimidine-4-carboxamide | 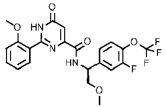 | | 482.0 |
| 142 | 2-(5-chloropyridin-2-yl)-N-((1S)-1-(3-fluoro-4-(trifluoromethoxy)phenyl)-2-methoxyethyl)-6-oxo-1,6-dihydropyrimidine-4-carboxamide | 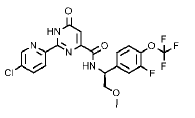 | | 487.0 |
| 143 | 2-(5-(difluoromethoxy)pyridin-2-yl)-N-((1S)-1-(3-fluoro-4-(trifluoromethoxy)phenyl)-2-methoxyethyl)-6-oxo-1,6-dihydropyrimidine-4-carboxamide | 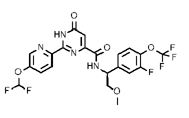 | | 519.1 |
| 144 | N-((1S)-1-(3-fluoro-4-(trifluoromethoxy)phenyl)-2-methoxyethyl)-2-(5-methoxypyrazin-2-yl)-6-oxo-1,6-dihydropyrimidine-4-carboxamide | 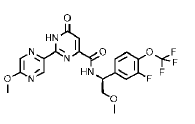 | | 484.1 |
| 145 | 2-(3-fluoropyridin-2-yl)-N-((1S)-1-(3-fluoro-4-(trifluoromethoxy)phenyl)-2-methoxyethyl)-6-oxo-1,6-dihydropyrimidine-4-carboxamide | 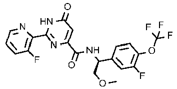 | | 471.1 |
| 146 | N-((1S)-1-(3-fluoro-4-(trifluoromethoxy)phenyl)-2-methoxyethyl)-2-(3-methoxypyridin-2-yl)-6-oxo-1,6-dihydropyrimidine-4-carboxamide | 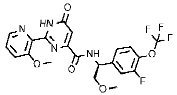 | | 483.1 |
| 147 | N-((1S)-1-(3-fluoro-4-(trifluoromethoxy)phenyl)-2-methoxyethyl)-6-((3S)-3-methoxypyrrolidin-1-yl)pyrazine-2-carboxamide | 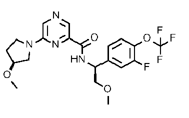 | | 459.1 |
| 148 | 6-chloro-N-((1S)-1-(3-fluoro-4-(trifluoromethoxy)phenyl)-2-hydroxy-2-methylpropyl)pyrazine-2-carboxamide | 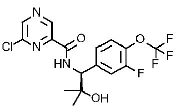 | | 405.9 |
| 149 | N-((1S)-1-(3-fluoro-4-(trifluoromethoxy)phenyl)-2-methoxyethyl)-6-(2-oxopyrrolidin-1-yl)pyrazine-2-carboxamide | 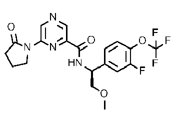 | | 443.1 |
| 150 | N-((1S)-1-(3-fluoro-4-(trifluoromethoxy)phenyl)-2-methoxyethyl)-2-(5-methyl-3-thienyl)-6-oxo-1,6-dihydropyrimidine-4-carboxamide | 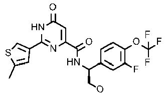 | | 472.0 |

**[Table 1-16]**

| | | | | |
|---|---|---|---|---|
| 151 | N-((1S)-1-(3-fluoro-4-(trifluoromethoxy)phenyl)-2-methoxyethyl)-2-(5-methyl-2-thienyl)-6-oxo-1,6-dihydropyrimidine-4-carboxamide | 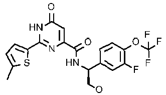 | | 472.0 |
| 152 | 2-(3,5-dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide | 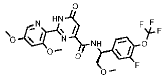 | | 513.1 |
| 153 | N-((1S)-2-methoxy-1-(4-(trifluoromethoxy)phenyl)ethyl)-2-(1-methylcyclopropyl)-6-oxo-1,6-dihydropyrimidine-4-carboxamide | 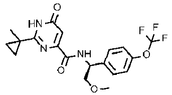 | | 412.1 |
| 154 | 6-(5-fluoropyridin-2-yl)-N-((1S)-1-(3-fluoro-4-(trifluoromethoxy)phenyl)-2-methoxyethyl)pyrazine-2-carboxamide | 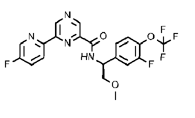 | | 455.1 |
| 155 | N-((1S)-1-(3-fluoro-4-(trifluoromethoxy)phenyl)-2-methoxyethyl)-6-(5-methoxypyridin-2-yl)pyrazine-2-carboxamide | 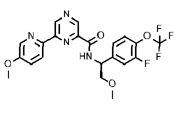 | | 467.1 |
| 156 | 6-(dimethylamino)-N-((1S)-1-(3-fluoro-4-(trifluoromethoxy)phenyl)-2-hydroxy-2-methylpropyl)pyrazine-2-carboxamide | 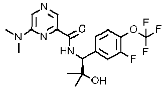 | | 417.1 |
| 157 | 6-(azetidin-1-yl)-N-((1S)-1-(3-fluoro-4-(trifluoromethoxy)phenyl)-2-hydroxy-2-methylpropyl)pyrazine-2-carboxamide | 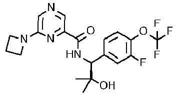 | | 429.1 |
| 158 | N-((1S)-1-(3-fluoro-4-(trifluoromethoxy)phenyl)-2-hydroxy-2-methylpropyl)-6-(1-methyl-1H-pyrazol-4-yl)pyrazine-2-carboxamide | 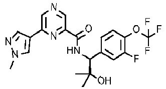 | | 454.1 |
| 159 | 2-(5-fluoro-2-furyl)-N-((1S)-1-(3-fluoro-4-(trifluoromethoxy)phenyl)-2-methoxyethyl)-6-oxo-1,6-dihydropyrimidine-4-carboxamide | 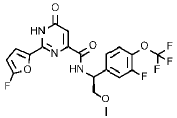 | | 460.1 |
| 160 | N-((1S)-1-(3-fluoro-4-(trifluoromethoxy)phenyl)-2-methoxyethyl)-6-oxo-2-(1-phenylcyclopropyl)-1,6-dihydropyrimidine-4-carboxamide | 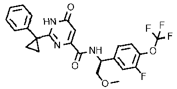 | | 492.1 |

**[Table 1-17]**

| | | | | |
|---|---|---|---|---|
| 161 | N-((1S)-1-(3-fluoro-4-(trifluoromethoxy)phenyl)-2-methoxyethyl)-6-oxo-2-(1-(trifluoromethyl)cyclopropyl)-1,6-dihydropyrimidine-4-carboxamide | 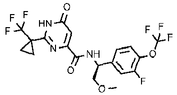 | | 484.0 |
| 162 | 2-(bicyclo[3.1.0]hex-1-yl)-N-((1S)-1-(3-fluoro-4-(trifluoromethoxy)phenyl)-2-methoxyethyl)-6-oxo-1,6-dihydropyrimidine-4-carboxamide | 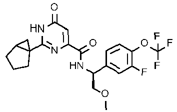 | | 456.1 |
| 163 | N-((1S)-1-(3-fluoro-4-(trifluoromethoxy)phenyl)-2-hydroxy-2-methylpropyl)-6-((3S)-3-methoxypyrrolidin-1-yl)pyrazine-2-carboxamide | 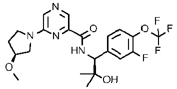 | | 473.1 |
| 164 | N-((1S)-1-(3-fluoro-4-(trifluoromethoxy)phenyl)-2-methoxyethyl)-2-(1-methylcyclobutyl)-6-oxo-1,6-dihydropyrimidine-4-carboxamide | 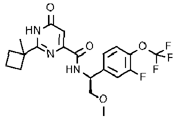 | | 444.2 |
| 165 | N-((1S)-2-methoxy-1-(4-(trifluoromethoxy)phenyl)ethyl)-2-(1-methylcyclobutyl)-6-oxo-1,6-dihydropyrimidine-4-carboxamide | 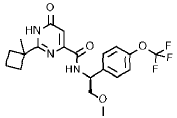 | | 426.1 |
| 166 | 2-(cyclopropylethynyl)-N-((1S)-1-(3-fluoro-4-(trifluoromethoxy)phenyl)-2-methoxyethyl)-6-oxo-1,6-dihydropyrimidine-4-carboxamide | 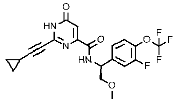 | | 440.0 |
| 167 | 6-cyclopropyl-N-((1S)-1-(3-fluoro-4-(trifluoromethoxy)phenyl)-2-hydroxy-2-methylpropyl)pyrazine-2-carboxamide | 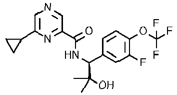 | | 414.1 |
| 168 | N-((1S)-1-(3-fluoro-4-(trifluoromethoxy)phenyl)-2-methoxyethyl)-6-oxo-2-(1-phenylcyclobutyl)-1,6-dihydropyrimidine-4-carboxamide | 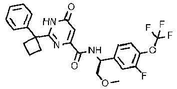 | | 506.1 |
| 169 | N-((1S)-1-(3-fluoro-4-(trifluoromethoxy)phenyl)-2-methoxyethyl)-6-oxo-2-(2-(pyridin-2-yl)propan-2-yl)-1,6-dihydropyrimidine-4-carboxamide | 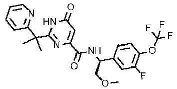 | | 495.1 |
| 170 | N-((1S)-1-(3-fluoro-4-(trifluoromethoxy)phenyl)-2-methoxyethyl)-6-oxo-2-(2-phenylpropan-2-yl)-1,6-dihydropyrimidine-4-carboxamide | 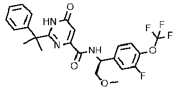 | | 494.1 |

**[Table 1-18]**

| | | | | |
|---|---|---|---|---|
| 171 | N-((1S)-1-(3-fluoro-4-(trifluoromethoxy)phenyl)-2-methoxyethyl)-2-(4-methoxycyclopent-1-en-1-yl)-6-oxo-1,6-dihydropyrimidine-4-carboxamide | 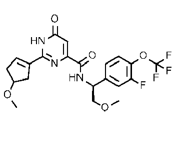 | | 472.1 |
| 172 | N-((1S)-1-(3-fluoro-4-(trifluoromethoxy)phenyl)-2-methoxyethyl)-2-(1-methylcyclopentyl)-6-oxo-1,6-dihydropyrimidine-4-carboxamide | 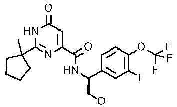 | | 458.1 |
| 173 | N-((1S)-2-methoxy-1-(4-(trifluoromethoxy)phenyl)ethyl)-2-(1-methylcyclopentyl)-6-oxo-1,6-dihydropyrimidine-4-carboxamide | 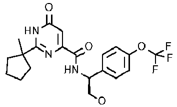 | | 440.1 |
| 174 | N-(2-hydroxy-2-methyl-1-(4-(trifluoromethoxy)phenyl)propyl)-6-(1-methyl-1H-pyrazol-3-yl)pyrazine-2-carboxamide | 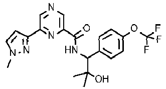 | | 436.1 |
| 175 | N-(2-hydroxy-2-methyl-1-(4-(trifluoromethoxy)phenyl)propyl)-6-(1H-pyrazol-1-yl)pyrazine-2-carboxamide | 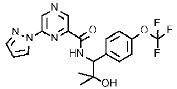 | | 422.0 |
| 176 | 6-(1H-benzimidazol-1-yl)-N-(2-hydroxy-2-methyl-1-(4-(trifluoromethoxy)phenyl)propyl)-pyrazine-2-carboxamide | 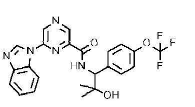 | | 472.1 |
| 177 | N-(2-hydroxy-2-methyl-1-(4-(trifluoromethoxy)phenyl)propyl)-6-(4-methyl-1H-imidazol-1-yl)pyrazine-2-carboxamide | 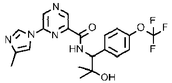 | | 436.1 |
| 178 | 2-(3-fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide | 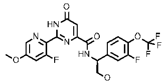 | | 501.1 |
| 179 | N-((1S)-1-(3-fluoro-4-(trifluoromethoxy)phenyl)-2-methoxyethyl)-2-(5-methoxy-3-methylpyridin-2-yl)-6-oxo-1,6-dihydropyrimidine-4-carboxamide | 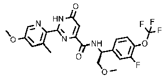 | | 497.1 |
| 180 | 2-(5-fluoro-3-methoxypyridin-2-yl)-N-((1S)-1-(3-fluoro-4-(trifluoromethoxy)phenyl)-2-methoxyethyl)-6-oxo-1,6-dihydropyrimidine-4-carboxamide | 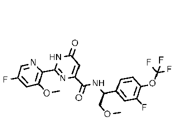 | | 501.1 |

**[Table 1-19]**

| | | | | |
|---|---|---|---|---|
| 181 | N-(2-hydroxy-2-methyl-1-(4-(trifluoromethoxy)phenyl)-propyl)-6-(pyridin-3-yloxy)pyrazine-2-carboxamide | 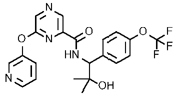 | | 449.0 |
| 182 | N-((1S)-1-(3-fluoro-4-(trifluoromethoxy)phenyl)-2-methoxyethyl)-2-(3-methyl-2-furyl)-6-oxo-1,6-dihydropyrimidine-4-carboxamide | 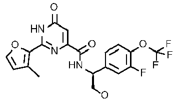 | | 453.9 |
| 183 | 2-(3-ethoxypyridin-2-yl)-N-((1S)-1-(3-fluoro-4-(trifluoromethoxy)phenyl)-2-methoxyethyl)-6-oxo-1,6-dihydropyrimidine-4-carboxamide | 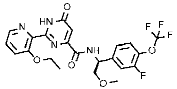 | | 497.1 |
| 184 | 2-(3-chloro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide | 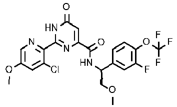 | | 517.0 |
| 185 | 2-(5-cyclopropyl-3-fluoropyridin-2-yl)-N-((1S)-1-(3-fluoro-4-(trifluoro-methoxy)phenyl)-2-methoxyethyl)-6-oxo-1,6-dihydropyrimidine-4-carboxamide | 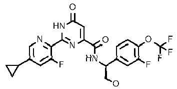 | | 511.1 |
| 186 | 2-(5-(difluoromethoxy)-3-fluoropyridin-2-yl)-N-((1S)-1-(3-fluoro-4-(trifluoro-methoxy)phenyl)-2-methoxyethyl)-6-oxo-1,6-dihydropyrimidine-4-carboxamide | 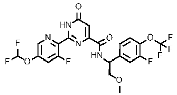 | | 537.1 |
| 187 | 2-(3,5-diethoxypyridin-2-yl)-N-((1S)-1-(3-fluoro-4-(trifluoromethoxy)phenyl)-2-methoxyethyl)-6-oxo-1,6-dihydropyrimidine-4-carboxamide | 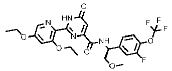 | | 541.2 |
| 188 | 5-fluoro-N-((1S)-1-(3-fluoro-4-(trifluoromethoxy)phenyl)-2-methoxyethyl)-2-(5-methoxypyridin-2-yl)-6-oxo-1,6-dihydropyrimidine-4-carboxamide | 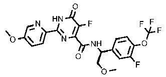 | | 498.9 |

**[Table 1-20]**

| | | | | |
|---|---|---|---|---|
| 189 | 2-(5-cyclopropyl-3-methoxypyridin-2-yl)-N-((1S)-1-(3-fluoro-4-(trifluoro-methoxy)phenyl)-2-methoxyethyl)-6-oxo-1,6-dihydropyrimidine-4-carboxamide | 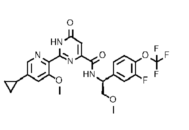 | | 523.1 |

### Experimental Example 1

### PDE enzyme inhibitory assay

Human PDE2A3 full-length gene was transduced into Sf9, and human PDE2A3 enzyme was purified by His-tag affinity column and gel filtration. The enzyme was stored at -70°C until use. PDE activity was measured by using a SPA (Scintillation Proximity Assay) (GE Healthcare). To evaluate the inhibitory activity of the compound, 10 µl of serial diluted compounds were reacted with 20 µl of PDE enzyme in assay buffer (50 mM HEPES-NaOH, 8.3 mM MgCl₂, 1.7 mM EGTA, 0.1 % BSA (pH 7.4)) for 30 min at room temperature. Final concentration of DMSO in the reaction solution was 1 %. Compounds were tested in duplicate in 96-well half-area plates (Corning) or 384-well OptiPlate (PerkinElmer). To start the reaction, 10 µl of substrate [³H] cGMP (final concentration 77 nM, PerkinElmer) was added to a total volume of 40 µl. After reaction for 60 min at room temperature, 20 µl of 20 mg/mL yttrium SPA beads containing zinc sulfate was added to terminate the reaction. After being settled for further 1 hour, the assay plates were counted in a scintillation counter (PerkinElmer) to allow calculation of inhibition rate. Inhibition rate was calculated on the basis of 0% control wells with enzyme and DMSO, and 100% control wells without enzyme. The results are shown in Table 2.

**Table 2**

| Example No. | inhibitory rate (%) (1 µM) | Example No. | inhibitory rate (%) (1 µM) |
|---|---|---|---|
| 20 | 98 | 111 | 94 |
| 33 | 99 | 114 | 104 |
| 45 | 97 | 115 | 100 |
| 52 | 102 | 116 | 99 |
| 55 | 98 | 120 | 103 |
| 61 | 98 | 121 | 95 |
| 62 | 99 | 122 | 102 |
| 67 | 100 | 129 | 98 |
| 73 | 99 | 152 | 96 |
| 76 | 101 | 153 | 100 |
| 80 | 94 | 158 | 97 |
| 81 | 93 | 163 | 94 |
| 83 | 98 | 171 | 103 |
| 87 | 98 | 178 | 99 |
| 88 | 92 | 179 | 94 |
| 95 | 104 | 184 | 95 |
| 96 | 94 | 185 | 94 |
| 102 | 102 | 186 | 92 |
| 103 | 108 | 187 | 105 |
| 105 | 104 | 189 | 99 |
| 107 | 97 | | |

### Formulation Example 1

(1) compound of Example 1 10.0 g
(2) lactose 70.0 g
(3) cornstarch 50.0 g
(4) soluble starch 7.0 g
(5) magnesium stearate 3.0 g

The compound of Example 1 (10.0 g) and magnesium stearate (3.0 g) are granulated in aqueous solution (70 mL) of soluble starch (7.0 g as soluble starch) and then dried, the resulting mixture is mixed with lactose (70.0 g) and cornstarch (50.0 g) (lactose, cornstarch, soluble starch and magnesium stearate are all products in compliance with Japanese Pharmacopoeia 16^{th} Edition). The mixture compressed to give tablets.

### Industrial Applicability

According to the present invention, a compound having a PDE2A selective inhibitory action, and useful as a prophylactic or therapeutic drug for schizophrenia, Alzheimer's disease and the like can be provided.

This application is based on a patent application No. 2013-220194 filed in Japan (filing date: October 23, 2013), the contents of which are incorporated in full herein.

## Claims

1. A compound represented by the formula (I): 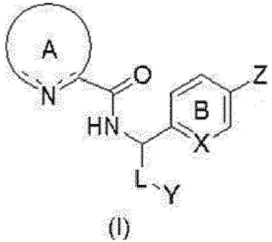 wherein
ring A is an optionally further substituted 5- or 6-membered nitrogen-containing heterocycle, 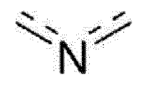 as a ring A-constituting atom is =N- or -N=,
ring B is a benzene ring or a pyridine ring, each of which is optionally further substituted,
X is a carbon atom or a nitrogen atom,
L is a bond or an optionally substituted C₁₋₂ alkylene group,
Y is
(1) the formula -CH₂-O-R¹ wherein R¹ is a substituent, or
(2) the formula: 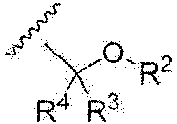 wherein R² and R³ are each independently a hydrogen atom or a substituent, R⁴ is a substituent, or R³ and R⁴ are joined to optionally form, together with the adjacent carbon atom, an optionally further substituted ring, provided when L is a bond, then R³ and R⁴ are not 3-pyridyl groups at the same time, and
Z is a substituent,
(provided that 3-amino-N-[1-(4-chlorophenyl)-3-methoxypropyl]pyrazine-2-carboxamide is excluded), or a salt thereof.

2. The compound according to claim 1, wherein Y is
(1) the formula -CH₂-O-R¹ wherein R¹ is an optionally substituted C₁₋₆ alkyl group; or
(2) the formula: 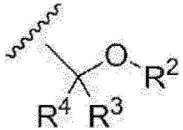 wherein R² and R³ are each independently a hydrogen atom or an optionally substituted C₁₋₆ alkyl group, and R⁴ is an optionally substituted C₁₋₆ alkyl group,
or a salt thereof.

3. The compound according to claim 1, wherein ring A is an optionally further substituted 5- or 6-membered nitrogen-containing heterocycle; 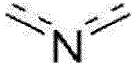 as a ring A-constituting atom is =N- or -N=;
ring B is a benzene ring or a pyridine ring, each of which is optionally further substituted;
X is a carbon atom or a nitrogen atom;
L is a bond or an optionally substituted C₁₋₂ alkylene group;
Y is
(1) the formula -CH₂-O-R¹ wherein R¹ is an optionally substituted C₁₋₆ alkyl group; or
(2) the formula: 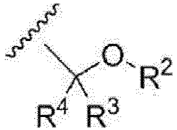 wherein R² and R³ are each independently a hydrogen atom or an optionally substituted C₁₋₆ alkyl group, and R⁴ is an optionally substituted C₁₋₆ alkyl group; and
Z is
(1) an optionally substituted C₁₋₆ alkoxy group,
(2) an optionally substituted C₁₋₆ alkyl group,
(3) an optionally substituted C₃₋₁₀ cycloalkyl group, or
(4) an optionally substituted heterocyclic group,
or a salt thereof.

4. The compound according to claim 1, wherein ring A is a 5- or 6-membered nitrogen-containing heterocycle optionally substituted by 1 to 3 substituents selected from
(1) a C₁₋₆ alkyl group optionally substituted by 1 to 3 substituents selected from
(i) a C₆₋₁₄ aryl group,
(ii) a C₁₋₆ alkoxy group, and
(iii) a heterocyclic group,
(2) a halogen atom,
(3) a cyano group,
(4) a C₂₋₆ alkenyl group,
(5) a C₂₋₆ alkynyl group optionally substituted by 1 to 3 C₃₋₁₀ cycloalkyl groups,
(6) a C₃₋₁₀ cycloalkyl group optionally substituted by 1 to 3 substituents selected from
(i) an optionally halogenated C₁₋₆ alkyl group,
(ii) a C₆₋₁₄ aryl group, and
(iii) a C₁₋₆ alkoxy group,
(7) a C₃₋₁₀ cycloalkenyl group optionally substituted by 1 to 3 C₁₋₆ alkoxy groups,
(8) a C₆₋₁₄ aryl group optionally substituted by 1 to 3 substituents selected from
(i) a C₁₋₆ alkyl group,
(ii) a halogen atom, and
(iii) a C₁₋₆ alkoxy group,
(9) a C₁₋₆ alkoxy group,
(10) a C₁₋₆ alkylthio group,
(11) an amino group optionally mono- or di-substituted by a substituent selected from
(i) a C₁₋₆ alkyl group optionally substituted by 1 to 3 substituents selected from a C₁₋₆ alkoxy group, a C₃₋₁₀ cycloalkyl group and a C₆₋₁₄ aryl group,
(ii) a C₃₋₁₀ cycloalkyl group,
(iii) a C₆₋₁₄ aryl group optionally substituted by 1 to 3 C₁₋₆ alkoxy groups,
(iv) a heterocyclic group optionally substituted by 1 to 3 C₁₋₆ alkyl groups, and
(v) a C₃₋₁₀ cycloalkyl-carbonyl group,
(12) a non-aromatic heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 substituents selected from a halogen atom and a C₁₋₆ alkoxy group, and
(iv) an oxo group,
(13) an aromatic heterocyclic group optionally substituted by 1 to 3 substituents selected from
(i) an optionally halogenated C₁₋₆ alkoxy group,
(ii) a halogen atom,
(iii) an optionally halogenated C₁₋₆ alkyl group, and
(iv) a C₃₋₁₀ cycloalkyl group,
(14) a heterocyclyloxy group, and
(15) an oxo group; 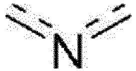 as a ring A-constituting atom is =N- or -N=;
ring B is a benzene ring optionally substituted by a halogen atom;
X is a carbon atom;
L is a bond or methylene;
Y is
(1) the formula -CH₂-O-R¹ wherein R¹ is a C₁₋₆ alkyl group; or
(2) the formula: 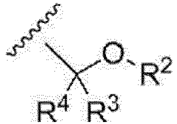
wherein R² is a hydrogen atom;
R³ is a C₁₋₆ alkyl group; and
R⁴ is a C₁₋₆ alkyl group; and
Z is
(1) an optionally halogenated C₁₋₆ alkoxy group,
(2) an optionally halogenated C₁₋₆ alkyl group,
(3) a C₃₋₁₀ cycloalkyl group, or
(4) a heterocyclic group,
or a salt thereof.

5. The compound according to claim 1, wherein ring A is a 5- or 6-membered nitrogen-containing heterocycle optionally substituted by 1 to 3 substituents selected from
(1) a C₁₋₆ alkyl group optionally substituted by 1 to 3 substituents selected from
(i) a C₆₋₁₄ aryl group,
(ii) a C₁₋₆ alkoxy group, and
(iii) a heterocyclic group,
(2) a cyano group,
(3) a C₃₋₁₀ cycloalkyl group optionally substituted by 1 to 3 substituents selected from
(i) an optionally halogenated C₁₋₆ alkyl group,
(ii) a C₆₋₁₄ aryl group, and
(iii) a C₁₋₆ alkoxy group,
(4) a C₃₋₁₀ cycloalkenyl group optionally substituted by 1 to 3 C₁₋₆ alkoxy groups,
(5) a C₆₋₁₄ aryl group optionally substituted by 1 to 3 substituents selected from
(i) a C₁₋₆ alkyl group,
(ii) a halogen atom, and
(iii) a C₁₋₆ alkoxy group,
(6) an amino group optionally mono- or di-substituted by a substituent selected from
(i) a C₁₋₆ alkyl group optionally substituted by 1 to 3 substituents selected from a C₁₋₆ alkoxy group, a C₃₋₁₀ cycloalkyl group and a C₆₋₁₄ aryl group,
(ii) a C₃₋₁₀ cycloalkyl group,
(iii) a C₆₋₁₄ aryl group optionally substituted by 1 to 3 C₁₋₆ alkoxy groups,
(iv) a heterocyclic group optionally substituted by 1 to 3 C₁₋₆ alkyl groups, and
(v) a C₃₋₁₀ cycloalkyl-carbonyl group,
(7) a non-aromatic heterocyclic group optionally substituted by 1 to 3 substituents selected from
(i) a C₁₋₆ alkoxy group,
(ii) a halogen atom,
(iii) a C₁₋₆ alkyl group optionally substituted by 1 to 3 substituents selected from a halogen atom and a C₁₋₆ alkoxy group, and
(iv) an oxo group,
(8) an aromatic heterocyclic group optionally substituted by 1 to 3 substituents selected from
(i) an optionally halogenated C₁₋₆ alkoxy group,
(ii) a halogen atom,
(iii) an optionally halogenated C₁₋₆ alkyl group, and
(iv) a C₃₋₁₀ cycloalkyl group, and
(9) an oxo group; 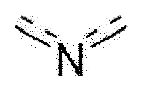 as a ring A-constituting atom is =N- or -N=;
ring B is a benzene ring optionally substituted by a halogen atom;
X is a carbon atom;
L is a bond;
Y is
(1) the formula -CH₂-O-R¹ wherein R¹ is a C₁₋₆ alkyl group; or
(2) the formula: 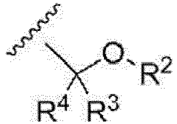
wherein R² is a hydrogen atom;
R³ is a C₁₋₆ alkyl group; and
R⁴ is a C₁₋₆ alkyl group; and
Z is
(1) an optionally halogenated C₁₋₆ alkoxy group, or
(2) a heterocyclic group,
or a salt thereof.

6. The compound according to claim 1, wherein ring A is a 5- or 6-membered nitrogen-containing heterocycle optionally substituted by 1 to 3 substituents selected from
(1) a C₁₋₆ alkyl group,
(2) a cyano group,
(3) a C₃₋₁₀ cycloalkyl group optionally substituted by 1 to 3, optionally halogenated C₁₋₆ alkyl group,
(4) a C₃₋₁₀ cycloalkenyl group optionally substituted by 1 to 3 C₁₋₆ alkoxy groups,
(5) a C₆₋₁₄ aryl group,
(6) an amino group optionally mono- or di-substituted by a C₁₋₆ alkyl group,
(7) a non-aromatic heterocyclic group optionally substituted by 1 to 3 C₁₋₆ alkoxy groups,
(8) an aromatic heterocyclic group optionally substituted by 1 to 3 substituents selected from
(i) an optionally halogenated C₁₋₆ alkoxy group,
(ii) a halogen atom, and
(iii) an optionally halogenated C₁₋₆ alkyl group, and
(9) an oxo group; 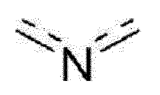 as a ring A-constituting atom is =N- or -N=;
ring B is a benzene ring optionally substituted by a halogen atom;
X is a carbon atom;
L is a bond;
Y is
(1) the formula -CH₂-O-R¹ wherein R¹ is a C₁₋₆ alkyl group; or
(2) the formula: 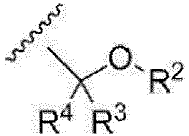
wherein R² is a hydrogen atom;
R³ is a C₁₋₆ alkyl group; and
R⁴ is a C₁₋₆ alkyl group; and
Z is
(1) an optionally halogenated C₁₋₆ alkoxy group, or
(2) a heterocyclic group,
or a salt thereof.

7. The compound according to claim 1, wherein ring A is a 5- or 6-membered nitrogen-containing heterocycle optionally substituted by 1 to 3 substituents selected from
(1) a C₃₋₁₀ cycloalkyl group optionally substituted by 1 to 3 C₁₋₆ alkyl groups,
(2) a non-aromatic heterocyclic group,
(3) an aromatic heterocyclic group optionally substituted by 1 to 3 substituents selected from
(i) a C₁₋₆ alkoxy group, and
(ii) a halogen atom, and
(4) an oxo group; 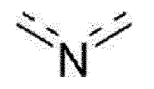 as a ring A-constituting atom is =N- or -N=;
ring B is a benzene ring optionally substituted by a halogen atom;
X is a carbon atom;
L is a bond;
Y is a group represented by the formula -CH₂-O-R¹ wherein R¹ is a C₁₋₆ alkyl group; and
Z is a halogenated C₁₋₆ alkoxy group,
or a salt thereof.

8. The compound according to claim 1, wherein ring A is a dihydropyrimidine ring optionally substituted by 1 to 3 substituents selected from
(1) a pyrrolidinyl group,
(2) a pyridyl group optionally substituted by 1 to 3 substituents selected from
(i) a C₁₋₆ alkoxy group, and
(ii) a halogen atom, and
(3) an oxo group; 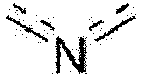 as a ring A-constituting atom is =N- or -N=;
ring B is a benzene ring optionally substituted by a halogen atom;
X is a carbon atom;
L is a bond;
Y is the formula -CH₂-O-R¹ wherein R¹ is a C₁₋₆ alkyl group; and Z is a halogenated C₁₋₆ alkoxy group,
or a salt thereof.

9. N-((1S)-1-(3-fluoro-4-(trifluoromethoxy)phenyl)-2-methoxyethyl)-6-oxo-2-(pyrrolidin-1-yl)-1,6-dihydropyrimidine-4-carboxamide, or a salt thereof.

10. 2-(3,5-Dimethoxypyridin-2-yl)-N-((1S)-1-(3-fluoro-4-(trifluoromethoxy)phenyl)-2-methoxyethyl)-6-oxo-1,6-dihydropyrimidine-4-carboxamide, or a salt thereof.

11. 2-(3-Fluoro-5-methoxypyridin-2-yl)-N-((1S)-1-(3-fluoro-4-(trifluoromethoxy)phenyl)-2-methoxyethyl)-6-oxo-1,6-dihydropyrimidine-4-carboxamide, or a salt thereof.

12. A medicament comprising the compound according to any one of claims 1 to 11, or a salt thereof.

13. The medicament according to claim 12, which is a phosphodiesterase 2A inhibitor.

14. The medicament according to claim 12, which is a prophylactic or therapeutic drug for schizophrenia.

15. The compound according to any one of claims 1 to 11 for use in the prophylaxis or treatment of schizophrenia, or a salt thereof.

16. A method of inhibiting phosphodiesterase 2A in a mammal, comprising administering an effective amount of the compound according to any one of claims 1 to 11 or a salt thereof to the mammal.

17. A method for the prophylaxis or treatment of schizophrenia in a mammal, comprising administering an effective amount of the compound according to any one of claims 1 to 11 or a salt thereof to the mammal.

18. Use of the compound according to any one of claims 1 to 11 or a salt thereof in the production of a prophylactic or therapeutic drug for schizophrenia.
